(19) Europäisches Patentamt
European Patent Office
Office européen des brevets

(11) EP 4 263 556 B1

(12) **EUROPEAN PATENT SPECIFICATION**

(45) Date of publication and mention of the grant of the patent:
**12.02.2025 Bulletin 2025/07**

(21) Application number: **21830696.7**

(22) Date of filing: **16.12.2021**

(51) International Patent Classification (IPC):
***C07D 498/22*** *(2006.01)* ***A61P 35/00*** *(2006.01)*
***A61K 31/395*** *(2006.01)*

(52) Cooperative Patent Classification (CPC):
**A61P 35/00; C07D 498/22**

(86) International application number:
**PCT/EP2021/086195**

(87) International publication number:
**WO 2022/129333 (23.06.2022 Gazette 2022/25)**

(54) **BRANCHED MACROCYCLIC 4-(PYRAZOL-5-YL)-INDOLE DERIVATIVES AS INHIBITORS OF MCL-1**

VERZWEIGTE MAKROZYKLISCHE 4-(PYRAZOL-5-YL)-INDOLDERIVATE ALS INHIBITOREN VON MCL-1

DÉRIVÉS MACROCYCLIQUES RAMIFIÉS DE 4-(PYRAZOL-5-YL)-INDOLE EN TANT QU'INHIBITEURS DE MCL-1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.12.2020 EP 20214811**

(43) Date of publication of application:
**25.10.2023 Bulletin 2023/43**

(73) Proprietor: **JANSSEN Pharmaceutica NV**
**2340 Beerse (BE)**

(72) Inventors:
- **ROMBOUTS, Frederik, Jan, Rita**
  **2340 Beerse (BE)**
- **VELTER, Adriana-Ingrid**
  **2340 Beerse (BE)**
- **BUIJNSTERS, Petrus, Jacobus, Johannes, Antonius**
  **2340 Beerse (BE)**
- **FERRER CABRERA, Sofia**
  **2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2020/063792**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 263 556 B1

## Description

FIELD OF THE INVENTION

The present invention relates to pharmaceutical agents useful for therapy and/or prophylaxis in a subject, pharmaceutical composition comprising such compounds, and their use as MCL-1 inhibitors, useful for treating or preventing diseases such as cancer.

BACKGROUND OF THE INVENTION

Cellular apoptosis or programmed cell death is critical to the development and homeostasis of many organs including the hematopoietic system. Apoptosis can be initiated via the extrinsic pathway, which is mediated by death receptors, or by the intrinsic pathway using the B cell lymphoma (BCL-2) family of proteins. Myeloid cell leukemia-1 (MCL-1) is a member of the BCL-2 family of cell survival regulators and is a critical mediator of the intrinsic apoptosis pathway. MCL-1 is one of five principal anti-apoptotic BCL-2 proteins (MCL-1, BCL-2, BCL-XL, BCL-w, and BFL1/A1) responsible for maintaining cell survival. MCL-1 continuously and directly represses the activity of the pro-apoptotic BCL-2 family proteins Bak and Bax and indirectly blocks apoptosis by sequestering BH3 only apoptotic sensitizer proteins such as Bim and Noxa. The activation of Bak/Bax following various types of cellular stress leads to aggregation on the mitochondrial outer membrane and this aggregation facilitates pore formation, loss of mitochondrial outer membrane potential, and subsequent release of cytochrome C into the cytosol. Cytosolic cytochrome C binds Apaf-1 and initiates recruitment of procaspase 9 to form apoptosome structures (Cheng et al. eLife 2016; 5: e17755). The assembly of apoptosomes activates the executioner cysteine proteases 3/7 and these effector caspases then cleave a variety of cytoplasmic and nuclear proteins to induce cell death (Julian et al. Cell Death and Differentiation 2017; 24, 1380-1389).

Avoiding apoptosis is an established hallmark of cancer development and facilitates the survival of tumor cells that would otherwise be eliminated due to oncogenic stresses, growth factor deprivation, or DNA damage (Hanahan and Weinberg. Cell 2011;1-44). Thus, unsurprisingly, MCL-1 is highly upregulated in many solid and hematologic cancers relative to normal non-transformed tissue counterparts. The overexpression of MCL-1 has been implicated in the pathogenesis of several cancers where it correlated with poor outcome, relapse, and aggressive disease. Additionally, overexpression of MCL-1 has been implicated in the pathogenesis of the following cancers: prostate, lung, pancreatic, breast, ovarian, cervical, melanoma, B-cell chronic lymphocytic leukemia (CLL), acute myeloid leukemia (AML), and acute lymphoblastic leukemia (ALL). The human MCL-1 genetic locus (1q21) is frequently amplified in tumors and quantitatively increases total MCL-1 protein levels (Beroukhim et al. Nature 2010;463 (7283) 899-905). MCL-1 also mediates resistance to conventional cancer therapeutics and is transcriptionally upregulated in response to inhibition of BCL-2 function (Yecies et al. Blood 2010;115 (16)3304-3313).

A small molecule BH3 inhibitor of BCL-2 has demonstrated clinical efficacy in patients with chronic lymphocytic leukemia and is FDA approved for patients with CLL or AML (Roberts et al. NEJM 2016;374:311-322). The clinical success of BCL-2 antagonism led to the development of several MCL-1 BH3 mimetics that show efficacy in preclinical models of both hematologic malignancies and solid tumors (Kotschy et al. Nature 2016;538 477-486, Merino et al. Sci. Transl. Med;2017 (9)).

MCL-1 regulates several cellular processes in addition to its canonical role in mediating cell survival including mitochondrial integrity and non-homologous end joining following DNA damage (Chen et al. JCI 2018;128(1):500-516). The genetic loss of MCL-1 shows a range of phenotypes depending on the developmental timing and tissue deletion. MCL-1 knockout models reveal there are multiple roles for MCL-1 and loss of function impacts a wide range of phenotypes. Global MCL-1-deficient mice display embryonic lethality and studies using conditional genetic deletion have reported mitochondrial dysfunction, impaired activation of autophagy, reductions in B and T lymphocytes, increased B and T cell apoptosis, and the development of heart failure/ cardiomyopathy (Wang et al. Genes and Dev 2013;27 1351-1364, Steimer et al. Blood 2009;(113) 2805-2815).

WO2018178226 discloses MCL-1 inhibitors and methods of use thereof.

WO2017182625 discloses macrocyclic MCL-1 inhibitors for treating cancer.

WO2018178227 discloses the synthesis of MCL-1 inhibitors.

WO2020063792 and WO2020221272 disclose indole macrocyclic derivatives.

WO2020103864 discloses macrocyclic indoles as MCL-1 inhibitors.

There remains a need for MCL-1 inhibitors, useful for the treatment or prevention of cancers such as prostate, lung, pancreatic, breast, ovarian, cervical, melanoma, B-cell chronic lymphocytic leukemia (CLL), acute myeloid leukemia (AML), and acute lymphoblastic leukemia (ALL).

The compounds of the present invention might have improved potency, improved lipophilicity (ChromLogD), improved CYP inhibition and improved cardiotoxic properties (CTMC) compared to prior art compounds.

SUMMARY OF THE INVENTION

The present invention concerns novel compounds of Formula (I):

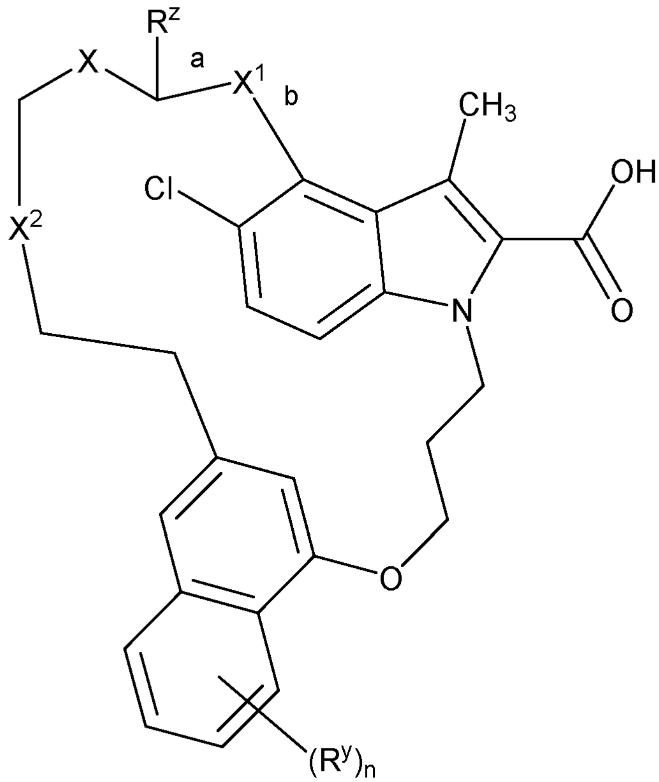

(I)

,

and the tautomers and the stereoisomeric forms thereof, wherein
$X^1$ represents (a-1) or (a-2):

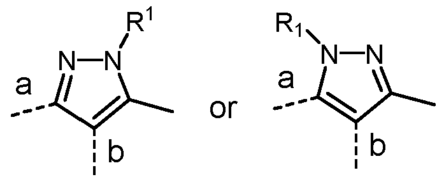

(a-1) (a-2) ,

wherein 'a' and 'b' indicate how variable $X^1$ is attached to the remainder of the molecule;
$R^1$ represents hydrogen; methyl; or $C_{2-6}$alkyl optionally substituted with one substituent selected from the group consisting of $Het^1$, $-OR^3$, and $-NR^{4a}R^{4b}$;
X represents -O- or $-CH_2-$;
in case X represents $-CH_2-$:
$R^z$ represents $-O-C_{1-4}$alkyl optionally substituted with one substituent selected from the group consisting of $-OR^5$, $-NR^{6a}R^{6b}$, $Ar^1$, and $Het^2$; or
$R^z$ is taken together with $R^1$ of (a-2), so that $R^z$ together with the atoms to which it is attached and (a-2) forms a bicyclic ring of formula (b-1) or (b-2):

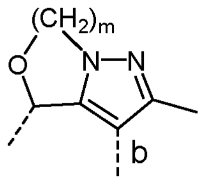

(b-1)

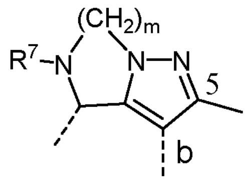

(b-2)

10 ;

in case X represents -O- :

$R^z$ represents $C_{2-4}$alkenyl or $C_{1-4}$alkyl, wherein said $C_{2-4}$alkenyl or $C_{1-4}$alkyl is optionally substituted with one substituent selected from the group consisting of $-OR^5$, - $NR^{6a}R^{6b}$, $Ar^1$, and $Het^2$;

$Het^1$ represents morpholinyl, N-methylpiperazinyl, or tetrahydropyranyl;

$R^3$ represents hydrogen, $C_{1-4}$alkyl, or $-C_{2-4}$alkyl-O-$C_{1-4}$alkyl;

$R^{4a}$ and $R^{4b}$ are each independently selected from the group consisting of hydrogen and

$C_{1-4}$alkyl;

$X^2$ represents

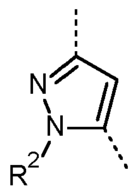

which can be attached to the remainder of the molecule in both directions;

$R^2$ represents hydrogen; methyl; or $C_{2-6}$alkyl optionally substituted with one substituent selected from the group consisting of $Het^1$, $-OR^3$, and $-NR^{4a}R^{4b}$;

$Het^2$ represents morpholinyl or N-methylpiperazinyl;

$Ar^1$ represents phenyl optionally substituted with one -O-$C_{1-4}$alkyl;

$R^y$ represents hydrogen or halo;

$R^5$ represents hydrogen, $C_{1-4}$alkyl, or $-C_{2-4}$alkyl-O-$C_{1-4}$alkyl;

$R^{6a}$ and $R^{6b}$ are each independently selected from the group consisting of hydrogen and $C_{1-4}$alkyl;

$R^7$ represents hydrogen, $C_{1-4}$alkyl, or $-C_{2-4}$alkyl-O-$C_{1-4}$alkyl;

n is 1 or 2;

m is 2;

and the pharmaceutically acceptable salts and the solvates thereof.

The present invention also relates to a pharmaceutical composition comprising a therapeutically effective

amount of a compound of Formula (I), a pharmaceutically acceptable salt, or a solvate thereof, and a pharmaceutically acceptable carrier or excipient.

Additionally, the invention relates to a compound of Formula (I), a pharmaceutically acceptable salt, or a solvate thereof, for use as a medicament, and to a compound of Formula (I), a pharmaceutically acceptable salt, or a solvate thereof, for use in the treatment or in the prevention of cancer.

In a particular embodiment, the invention relates to a compound of Formula (I), a pharmaceutically acceptable salt, or a solvate thereof, for use in the treatment or in the prevention of cancer.

Also disclosed herein is the use of a compound of Formula (I), a pharmaceutically acceptable salt, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable salt, or a solvate thereof.

Also disclosed herein is a product comprising a compound of Formula (I), a pharmaceutically acceptable salt, or a solvate thereof, and an additional pharmaceutical agent, as a combined preparation for simultaneous, separate or sequential use in the treatment or prevention of cancer.

DETAILED DESCRIPTION OF THE INVENTION

The term 'halo' or 'halogen' as used herein represents fluoro, chloro, bromo and iodo.

The prefix '$C_{x-y}$' (where x and y are integers) as used herein refers to the number of carbon atoms in a given group.

The term '$C_{1-4}$alkyl' as used herein as a group or part of a group represents a straight or branched chain fully saturated hydrocarbon radical having from 1 to 4 carbon atoms, such as methyl, ethyl, *n*-propyl, isopropyl, *n*-butyl, *s*-butyl, *t*-butyl and the like.

The term '$C_{2-4}$alkyl' as used herein as a group or part of a group represents a straight or branched chain fully saturated hydrocarbon radical having from 2 to 4 carbon atoms, such as ethyl, *n*-propyl, isopropyl, *n*-butyl, *s*-butyl, *t*-butyl and the like.

The term '$C_{2-6}$alkyl' as used herein as a group or part of a group represents a straight or branched chain fully saturated hydrocarbon radical having from 2 to 6 carbon atoms, such as ethyl, *n*-propyl, isopropyl, *n*-butyl, *s*-butyl, *t*-butyl, *n*-pentyl, *n*-hexyl and the like.

In general, whenever the term 'substituted' is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture.

Combinations of substituents and/or variables are permissible only if such combinations result in chemically stable compounds. 'Stable compound' is meant to indicate a compound that is sufficiently robust to survive isolation to a useful degree of purity from a reaction mixture.

The skilled person will understand that the term 'optionally substituted' means that the atom or radical indicated in the expression using 'optionally substituted' may or may not be substituted (this means substituted or unsubstituted respectively).

When two or more substituents are present on a moiety they may, where possible and unless otherwise indicated or clear from the context, replace hydrogens on the same atom or they may replace hydrogen atoms on different atoms in the moiety.

$Het^1$ may be attached to the remainder of the molecule of Formula (I) through any available ring carbon or nitrogen atom as appropriate, if not otherwise specified.

$Het^2$ may be attached to the remainder of the molecule of Formula (I) through any available ring carbon or nitrogen atom as appropriate, if not otherwise specified.

It will be clear for the skilled person that

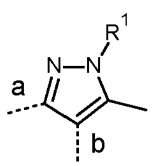

is an alternative representation for

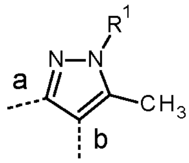

It will be clear for the skilled person that

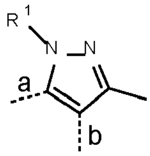

is an alternative representation for

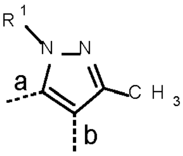

.

It will be clear that a compound of Formula (I) includes compounds of Formula (I-x) and (I-y) (both directions of $X^2$ being

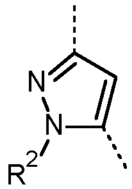

)

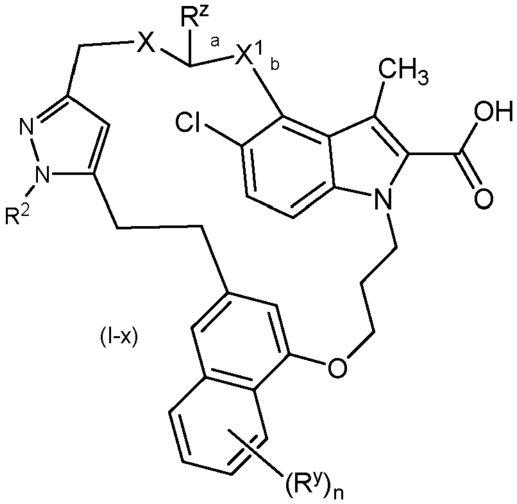

(I-x)

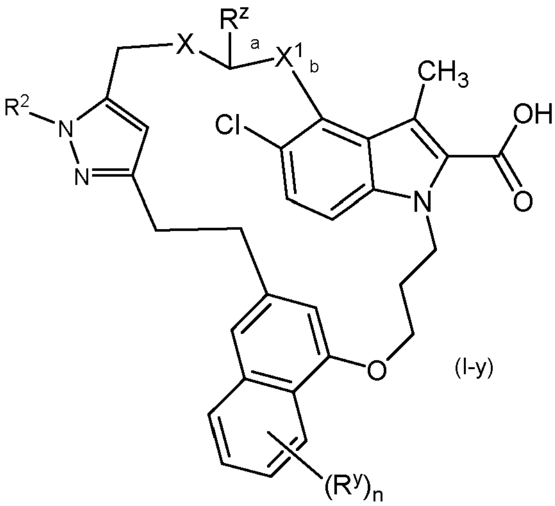

(I-y)

It will be clear that a compound of Formula (I) wherein X represents $-CH_2-$; and $R^z$ is taken together with $R^1$ of (a-2), so that $R^z$ together with the atoms to which it is attached and (a-2) forms a bicyclic ring of formula (b-1), corresponds with a compound of Formula (I-z):

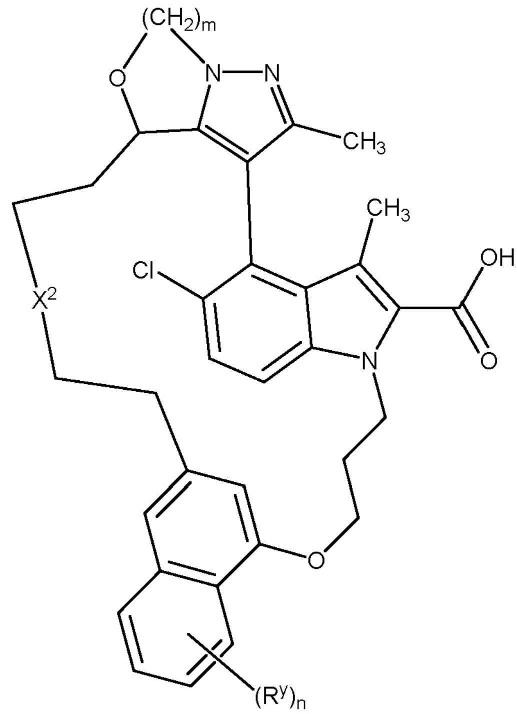

(I-z)

.

When any variable occurs more than one time in any constituent, each definition is independent.

When any variable occurs more than one time in any formula (e.g. Formula (I)), each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, or subject (e.g., human) that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R, S*) around one or more atoms, contemplates each possible stereoisomer, or mixture of two or more stereoisomers.

Hereinbefore and hereinafter, the term "compound(s) of Formula (I)" is meant to include the tautomers thereof and the stereoisomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present invention.

In particular, the compounds disclosed herein possess axial chirality, by virtue of restricted rotation around a biaryl bond and as such may exist as mixtures of atropisomers. When a compound is a pure atropisomer, the stereochemistry at each chiral center may be specified by either $R_a$ or $S_a$. Such designations may also be used for

mixtures that are enriched in one atropisomer. Further description of atropisomerism and axial chirality and rules for assignment of configuration can be found in Eliel, E.L. & Wilen, S. H. 'Stereochemistry of Organic Compounds' John Wiley and Sons, Inc. 1994.

Diastereomers (or diastereoisomers) are stereoisomers that are not enantiomers, i.e. they are not related as mirror images. If a compound contains a double bond, the substituents may be in the *E* or the *Z* configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, atropisomers, diastereomers, racemates, *E* isomers, *Z* isomers, cis isomers, trans isomers and mixtures thereof, whenever chemically possible.

The meaning of all those terms, i.e. enantiomers, atropisomers, diastereomers, racemates, *E* isomers, *Z* isomers, cis isomers, trans isomers and mixtures thereof are known to the skilled person.

The absolute configuration is specified according to the Cahn-Ingold-Prelog system. The configuration at an asymmetric atom is specified by either *R* or *S*. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light. Optically active ($R_a$)- and ($S_a$)-atropisomers may be prepared using chiral synthons, chiral reagents or chiral catalysts, or resolved using conventional techniques well known in the art, such as chiral HPLC.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (*R*), this means that the compound is substantially free of the (*S*) isomer; when a compound of Formula (I) is for instance specified as *E*, this means that the compound is substantially free of the *Z* isomer; when a compound of Formula (I) is for instance specified as cis, this means that the compound is substantially free of the trans isomer; when a compound of Formula (I) is for instance specified as $R_a$, this means that the compound is substantially free of the $S_a$ atropisomer.

Pharmaceutically acceptable salts, in particular pharmaceutically acceptable additions salts, include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I), and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, and solvates thereof, involves liquid chromatography using a chiral stationary phase. Said pure stereochemically isomeric forms may also be derived from the corresponding pure stereochemically isomeric

forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$, $^{13}N$, $^{15}O$, $^{17}O$, $^{18}O$, $^{32}P$, $^{33}P$, $^{35}S$, $^{18}F$, $^{36}Cl$, $^{122}I$, $^{123}I$, $^{125}I$, $^{131}I$, $^{75}Br$, $^{76}Br$, $^{77}Br$ and $^{82}Br$. Preferably, the isotope is selected from the group of $^{2}H$, $^{3}H$, $^{11}C$ and $^{18}F$. More preferably, the isotope is $^{2}H$. In particular, deuterated compounds are intended to be included within the scope of the present invention.

Certain isotopically-labeled compounds of the present invention (e.g., those labeled with $^{3}H$ and $^{14}C$) may be useful for example in substrate tissue distribution assays. Tritiated ($^{3}H$) and carbon-14 ($^{14}C$) isotopes are useful for their ease of preparation and detectability. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

The present invention relates in particular to compounds of Formula (I) as defined herein, and the tautomers and the stereoisomeric forms thereof, wherein

$X^1$ represents (a-1) or (a-2):

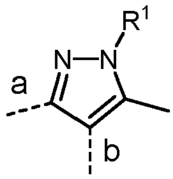

or

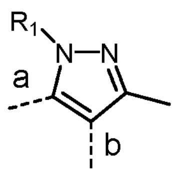

(a-1) (a-2) ,

wherein 'a' and 'b' indicate how variable $X^1$ is attached to the remainder of the molecule;

$R^1$ represents hydrogen; methyl; or $C_{2-6}$alkyl optionally substituted with one substituent selected from the group consisting of $Het^1$, $-OR^3$, and $-NR^{4a}R^{4b}$;

X represents -O- or $-CH_2-$;

in case X represents $-CH_2-$:

$R^z$ represents $-O-C_{1-4}$alkyl optionally substituted with one substituent selected from the group consisting of $-OR^5$, $-NR^{6a}R^{6b}$, $Ar^1$, and $Het^2$; or

$R^z$ is taken together with $R^1$ of (a-2), so that $R^z$ together with the atoms to which it is attached and (a-2) forms a bicyclic ring of formula (b-1):

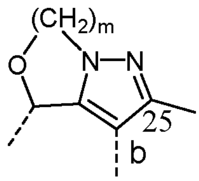

(b-1)

;

in case X represents -O- :

$R^z$ represents $C_{2-4}$alkenyl or $C_{1-4}$alkyl, wherein said $C_{2-4}$alkenyl or $C_{1-4}$alkyl is optionally substituted with one substituent selected from the group consisting of -$OR^5$, - $NR^{6a}R^{6b}$, $Ar^1$, and $Het^2$;

$Het^1$ represents morpholinyl, N-methylpiperazinyl, or tetrahydropyranyl;

$R^3$ represents hydrogen, $C_{1-4}$alkyl, or -$C_{2-4}$alkyl-O-$C_{1-4}$alkyl;

$R^{4a}$ and $R^{4b}$ are each independently selected from the group consisting of hydrogen and $C_{1-4}$alkyl;

$X^2$ represents

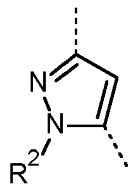

which can be attached to the remainder of the molecule in both directions;

$R^2$ represents hydrogen; methyl; or $C_{2-6}$alkyl optionally substituted with one substituent selected from the group consisting of $Het^1$, -$OR^3$, and -$NR^{4a}R^{4b}$;

$Het^2$ represents morpholinyl or N-methylpiperazinyl;

$Ar^1$ represents phenyl optionally substituted with one -O-$C_{1-4}$alkyl;

$R^y$ represents hydrogen or halo;

$R^5$ represents hydrogen, $C_{1-4}$alkyl, or -$C_{2-4}$alkyl-O-$C_{1-4}$alkyl;

$R^{6a}$ and $R^{6b}$ are each independently selected from the group consisting of hydrogen and $C_{1-4}$alkyl;

$R^7$ represents hydrogen, $C_{1-4}$alkyl, or -$C_{2-4}$alkyl-O-$C_{1-4}$alkyl;

n is 1 or 2;

m is 2;

and the pharmaceutically acceptable salts and the solvates thereof.

The present invention relates in particular to compounds of Formula (I) as defined herein, and the tautomers and the stereoisomeric forms thereof, wherein
$X^1$ represents (a-1) or (a-2):

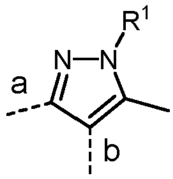 or 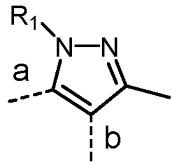

(a-1) (a-2) ,

wherein 'a' and 'b' indicate how variable $X^1$ is attached to the remainder of the molecule;

$R^1$ represents hydrogen; methyl; or $C_{2-6}$alkyl optionally substituted with one substituent selected from the group consisting of $Het^1$, $-OR^3$, and $-NR^{4a}R^{4b}$;

X represents -O- or $-CH_2-$;

in case X represents $-CH_2-$:

$R^z$ represents $-O-C_{1-4}$alkyl optionally substituted with one substituent selected from the group consisting of $-OR^5$, $-NR^{6a}R^{6b}$, $Ar^1$, and $Het^2$; or

$R^z$ is taken together with $R^1$ of (a-2), so that $R^z$ together with the atoms to which it is attached and (a-2) forms a bicyclic ring of formula (b-1):

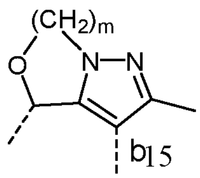

(b-1)

;

in case X represents -O- :

$R^z$ represents $C_{2-4}$alkenyl or $C_{1-4}$alkyl, wherein said $C_{2-4}$alkenyl or $C_{1-4}$alkyl is optionally substituted with one substituent selected from the group consisting of $-OR^5$, $-NR^{6a}R^{6b}$ , and $Het^2$;

$Het^1$ represents morpholinyl, N-methylpiperazinyl, or tetrahydropyranyl;

$R^3$ represents hydrogen, $C_{1-4}$alkyl, or $-C_{2-4}alkyl-O-C_{1-4}$alkyl;

$R^{4a}$ and $R^{4b}$ are each independently selected from the group consisting of hydrogen and $C_{1-4}$alkyl;

$X^2$ represents

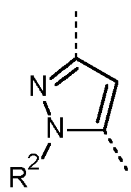

which can be attached to the remainder of the molecule in both directions;

$R^2$ represents hydrogen; methyl; or $C_{2-6}$alkyl optionally substituted with one substituent selected from the group consisting of $Het^1$, $-OR^3$, and $-NR^{4a}R^{4b}$;

$Het^2$ represents morpholinyl or N-methylpiperazinyl;

$Ar^1$ represents phenyl optionally substituted with one $-O-C_{1-4}$alkyl;

$R^y$ represents hydrogen or halo;

$R^5$ represents hydrogen, $C_{1-4}$alkyl, or $-C_{2-4}alkyl-O-C_{1-4}$alkyl;

$R^{6a}$ and $R^{6b}$ are each independently selected from the group consisting of hydrogen and $C_{1-4}$alkyl;

n is 1 or 2;

m is 2;
and the pharmaceutically acceptable salts and the solvates thereof.

The present invention relates in particular to compounds of Formula (I) as defined herein, and the tautomers and the stereoisomeric forms thereof, wherein
$X^1$ represents (a-1) or (a-2):

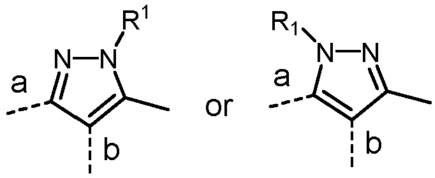

(a-1) (a-2) ,

wherein 'a' and 'b' indicate how variable $X^1$ is attached to the remainder of the molecule;

$R^1$ represents hydrogen; methyl; or $C_{2-6}$alkyl optionally substituted with one substituent selected from the group consisting of $Het^1$, $-OR^3$, and $-NR^{4a}R^{4b}$;

X represents $-CH_2-$;

$R^z$ is taken together with $R^1$ of (a-2), so that $R^z$ together with the atoms to which it is attached and (a-2) forms a bicyclic ring of formula (b-1):

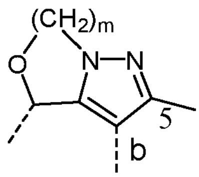

(b-1)

;

$Het^1$ represents morpholinyl, N-methylpiperazinyl, or tetrahydropyranyl;

$R^3$ represents hydrogen, $C_{1-4}$alkyl, or $-C_{2-4}alkyl-O-C_{1-4}$alkyl;

$R^{4a}$ and $R^{4b}$ are each independently selected from the group consisting of hydrogen and $C_{1-4}$alkyl;

$X^2$ represents

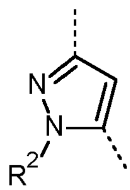

which can be attached to the remainder of the molecule in both directions;

$R^2$ represents hydrogen; methyl; or $C_{2-6}$alkyl optionally substituted with one substituent selected from the group

consisting of $Het^1$, $-OR^3$, and $-NR^{4a}R^{4b}$;

$Het^2$ represents morpholinyl or N-methylpiperazinyl;

$Ar^1$ represents phenyl optionally substituted with one $-O-C_{1-4}alkyl$;

$R^y$ represents hydrogen or halo;

$R^5$ represents hydrogen, $C_{1-4}alkyl$, or $-C_{2-4}alkyl-O-C_{1-4}alkyl$;

$R^{6a}$ and $R^{6b}$ are each independently selected from the group consisting of hydrogen and $C_{1-4}alkyl$;

n is 1 or 2;

m is 2;

and the pharmaceutically acceptable salts and the solvates thereof.

The present invention relates in particular to compounds of Formula (I) as defined herein, and the tautomers and the stereoisomeric forms thereof, wherein
$X^1$ represents (a-1) or (a-2):

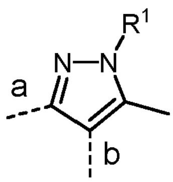
or
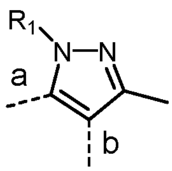

(a-1) (a-2) ,

wherein 'a' and 'b' indicate how variable $X^1$ is attached to the remainder of the molecule;
$R^1$ represents hydrogen; methyl; or $C_{2-6}alkyl$ optionally substituted with one substituent selected from the group consisting of $Het^1$, $-OR^3$, and $-NR^{4a}R^{4b}$;
X represents -O- or $-CH_2-$;
in case X represents $-CH_2-$:
$R^z$ represents $-O-C_{1-4}alkyl$ optionally substituted with one substituent selected from the group consisting of $-OR^5$, $-NR^{6a}R^{6b}$, $Ar^1$, and $Het^2$;
in case X represents -O- :

$R^z$ represents $C_{2-4}alkenyl$ or $C_{1-4}alkyl$, wherein said $C_{2-4}alkenyl$ or $C_{1-4}alkyl$ is optionally substituted with one substituent selected from the group consisting of $-OR^5$, $-NR^{6a}R^{6b}$, and $Het^2$;
$Het^1$ represents morpholinyl, N-methylpiperazinyl, or tetrahydropyranyl;
$R^3$ represents hydrogen, $C_{1-4}alkyl$, or $-C_{2-4}alkyl-O-C_{1-4}alkyl$;
$R^{4a}$ and $R^{4b}$ are each independently selected from the group consisting of hydrogen and $C_{1-4}alkyl$;
$X^2$ represents

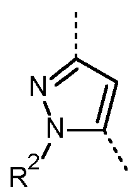

which can be attached to the remainder of the molecule in both directions;
$R^2$ represents hydrogen; methyl; or $C_{2-6}alkyl$ optionally substituted with one substituent selected from the group

consisting of $Het^1$, $-OR^3$, and $-NR^{4a}R^{4b}$;
$Het^2$ represents morpholinyl or N-methylpiperazinyl;
$Ar^1$ represents phenyl optionally substituted with one $-O-C_{1-4}alkyl$;
$R^y$ represents hydrogen or halo;
$R^5$ represents hydrogen, $C_{1-4}alkyl$, or $-C_{2-4}alkyl-O-C_{1-4}alkyl$;
$R^{6a}$ and $R^{6b}$ are each independently selected from the group consisting of hydrogen and $C_{1-4}alkyl$;
n is 1 or 2;
m is 2;
and the pharmaceutically acceptable salts and the solvates thereof.

The present invention relates in particular to compounds of Formula (I) as defined herein, and the tautomers and the stereoisomeric forms thereof, wherein
$X^1$ represents (a-1) or (a-2):

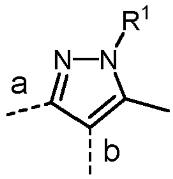 or 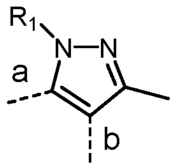

(a-1) (a-2) ,

wherein 'a' and 'b' indicate how variable $X^1$ is attached to the remainder of the molecule;
$R^1$ represents hydrogen; methyl; or $C_{2-6}alkyl$ optionally substituted with one $-OR^3$;
X represents -O- or $-CH_2-$;
in case X represents $-CH_2-$:
$R^z$ represents $-O-C_{1-4}alkyl$ optionally substituted with one $Ar^1$ substituent; or
$R^z$ is taken together with $R^1$ of (a-2), so that $R^z$ together with the atoms to which it is attached and (a-2) forms a bicyclic ring of formula (b-1):

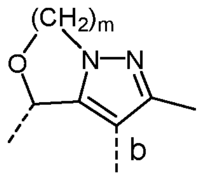

5

(b-1)

;

in case X represents -O- :

$R^z$ represents $C_{2-4}alkenyl$ or $C_{1-4}alkyl$, wherein said $C_{1-4}alkyl$ is optionally substituted with one substituent selected from the group consisting of $-OR^5$, $-NR^{6a}R^{6b}$, and $Het^2$;

$R^3$ represents $C_{1-4}alkyl$, or $-C_{2-4}alkyl-O-C_{1-4}alkyl$;

$X^2$ represents

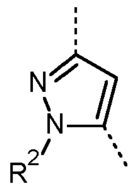

which can be attached to the remainder of the molecule in both directions;

$R^2$ represents $C_{2-6}$alkyl optionally substituted with one $-OR^3$;

$Het^2$ represents morpholinyl or N-methylpiperazinyl;

$Ar^1$ represents phenyl optionally substituted with one $-O-C_{1-4}$alkyl;

$R^y$ represents halo;

$R^5$ represents hydrogen, $C_{1-4}$alkyl, or $-C_{2-4}$alkyl-O-$C_{1-4}$alkyl;

$R^{6a}$ and $R^{6b}$ are each independently selected from the group consisting of hydrogen and $C_{1-4}$alkyl;

n is 1;

m is 2;

and the pharmaceutically acceptable salts and the solvates thereof.

In an embodiment, the present invention relates to those compounds of Formula (I) and the pharmaceutically acceptable salts, and the solvates thereof, or any subgroup thereof as mentioned in any of the other embodiments, wherein $R^y$ represents fluoro.

In an embodiment, the present invention relates to those compounds of Formula (I) and the pharmaceutically acceptable salts, and the solvates thereof, or any subgroup thereof as mentioned in any of the other embodiments, wherein

n represents 1; and
$R^y$ represents fluoro.

In an embodiment, the present invention relates to those compounds of Formula (I) and the pharmaceutically acceptable salts, and the solvates thereof, or any subgroup thereof as mentioned in any of the other embodiments, wherein $R^1$ represents methyl.

In an embodiment, the present invention relates to those compounds of Formula (I) and the pharmaceutically acceptable salts, and the solvates thereof, or any subgroup thereof as mentioned in any of the other embodiments, wherein n represents 1.

In an embodiment, the present invention relates to those compounds of Formula (I) and the pharmaceutically acceptable salts, and the solvates thereof, or any subgroup thereof as mentioned in any of the other embodiments, wherein n represents 2.

In an embodiment, the present invention relates to those compounds of Formula (I) and the pharmaceutically acceptable salts, and the solvates thereof, or any subgroup thereof as mentioned in any of the other embodiments, wherein $Het^1$ is attached to the remainder of the molecule of Formula (I) through a nitrogen atom.

In an embodiment, the present invention relates to those compounds of Formula (I) and the pharmaceutically acceptable salts, and the solvates thereof, or any subgroup thereof as mentioned in any of the other embodiments, wherein $Het^2$ is attached to the remainder of the molecule of Formula (I) through a nitrogen atom.

In an embodiment, the present invention relates to those compounds of Formula (I) and the pharmaceutically acceptable salts, and the solvates thereof, or any subgroup thereof as mentioned in any of the other embodiments, wherein X represents -O-.

In an embodiment, the present invention relates to those compounds of Formula (I) and the pharmaceutically acceptable salts, and the solvates thereof, or any subgroup thereof as mentioned in any of the other embodiments, wherein X represents $-CH_2-$.

In an embodiment, the present invention relates to those compounds of Formula (I) and the pharmaceutically acceptable salts, and the solvates thereof, or any subgroup thereof as mentioned in any of the other embodiments, wherein X represents $-CH_2-$; and $R^z$ is taken together with $R^1$ of (a-2), so that $R^z$ together with the atoms to which it is attached and (a-2) forms a bicyclic ring of formula (b-1).

In an embodiment, the present invention relates to those compounds of Formula (I) and the pharmaceutically acceptable salts, and the solvates thereof, or any subgroup thereof as mentioned in any of the other embodiments, wherein

in case X represents $-CH_2-$:
$R^z$ represents $-O-C_{1-4}$alkyl optionally substituted with one substituent selected from the group consisting of $-OR^5$, $-NR^{6a}R^{6b}$, $Ar^1$, and $Het^2$;
in case X represents -O- :
$R^z$ represents $C_{2-4}$alkenyl or $C_{1-4}$alkyl, wherein said $C_{2-4}$alkenyl or $C_{1-4}$alkyl is optionally substituted with one substituent selected from the group consisting of $-OR^5$, - $NR^{6a}R^{6b}$, $Ar^1$, and $Het^2$.

In an embodiment, the present invention relates to those compounds of Formula (I) and the pharmaceutically acceptable salts, and the solvates thereof, or any subgroup thereof as mentioned in any of the other embodiments, wherein

X represents $-CH_2-$; and
$R^z$ represents $-O-C_{1-4}$alkyl optionally substituted with one substituent selected from the group consisting of $-OR^5$, $-NR^{6a}R^{6b}$, $Ar^1$, and $Het^2$.

In an embodiment, the present invention relates to those compounds of Formula (I) and the pharmaceutically acceptable salts, and the solvates thereof, or any subgroup thereof as mentioned in any of the other embodiments, wherein

X represents -O- ; and
$R^z$ represents $C_{2-4}$alkenyl or $C_{1-4}$alkyl, wherein said $C_{2-4}$alkenyl or $C_{1-4}$alkyl is optionally substituted with one substituent selected from the group consisting of $-OR^5$, - $NR^{6a}R^{6b}$, $Ar^1$, and $Het^2$.

In an embodiment, the present invention relates to those compounds of Formula (I) and the pharmaceutically acceptable salts, and the solvates thereof, or any subgroup thereof as mentioned in any of the other embodiments, wherein

in case X represents $-CH_2-$:
$R^z$ is taken together with $R^1$ of (a-2), so that $R^z$ together with the atoms to which it is attached and (a-2) forms a bicyclic ring of formula (b-1):

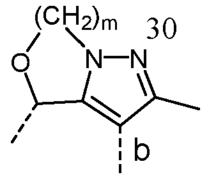

(b-1)

In an embodiment, the present invention relates to those compounds of Formula (I) and the pharmaceutically acceptable salts, and the solvates thereof, or any subgroup thereof as mentioned in any of the other embodiments, wherein

in case X represents $-CH_2-$:
$R^z$ is taken together with $R^1$ of (a-2), so that $R^z$ together with the atoms to which it is attached and (a-2) forms a bicyclic ring of formula (b-1) or (b-2):

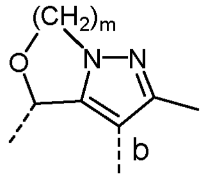
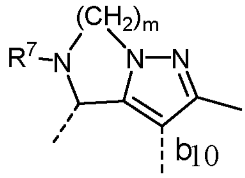

(b-1) (b-2)

In an embodiment, the present invention relates to those compounds of Formula (I) and the pharmaceutically acceptable salts, and the solvates thereof, or any subgroup thereof as mentioned in any of the other embodiments, wherein

X represents $-CH_2-$; and
$R^z$ is taken together with $R^1$ of (a-2), so that $R^z$ together with the atoms to which it is attached and (a-2) forms a bicyclic ring of formula (b-1) or (b-2):

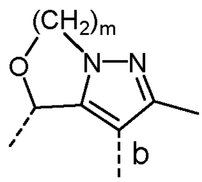
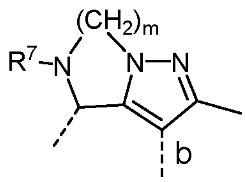

(b-1) (b-2)

In an embodiment, the present invention relates to those compounds of Formula (I) and the pharmaceutically acceptable salts, and the solvates thereof, or any subgroup thereof as mentioned in any of the other embodiments, wherein n is 1 and wherein $R^y$ is in position 3 as indicated below:

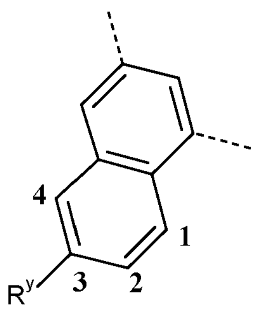

In an embodiment, the present invention relates to those compounds of Formula (I) and the pharmaceutically acceptable salts, and the solvates thereof, or any subgroup thereof as mentioned in any of the other embodiments, wherein n is 1 and wherein $R^y$ is in position 3 as indicated below; and wherein $R^y$ represents fluoro:

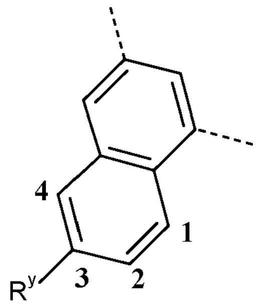

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-x):

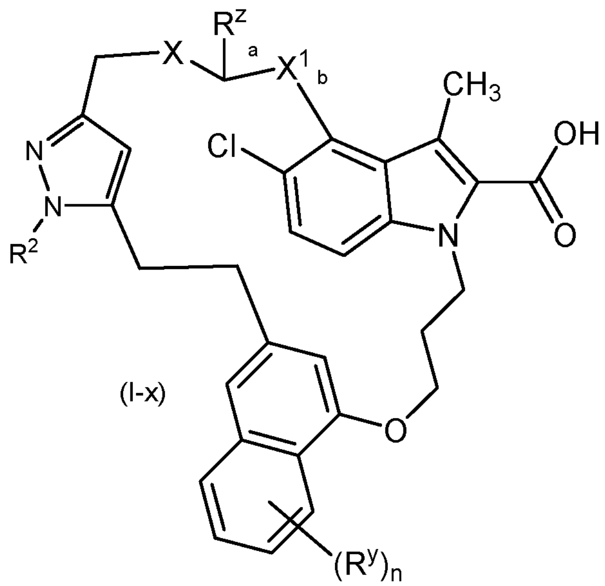

(I-x)

It will be clear that all variables in the structure of Formula (I-x), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-y):

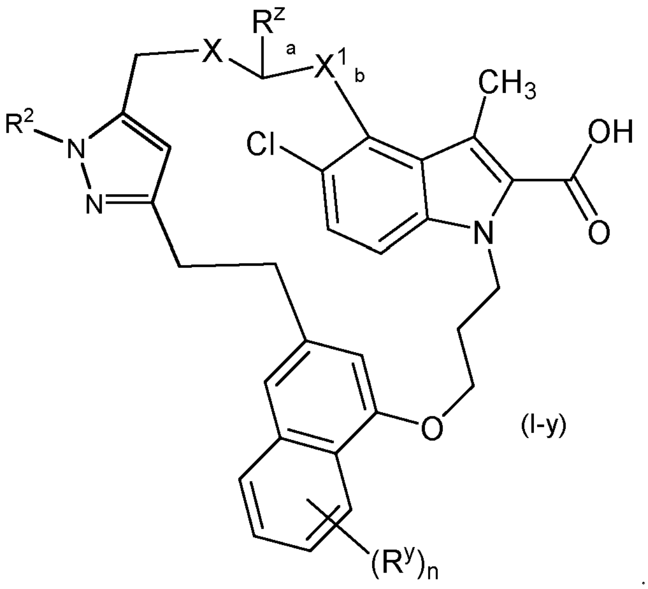

(I-y)

It will be clear that all variables in the structure of Formula (I-y), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-z):

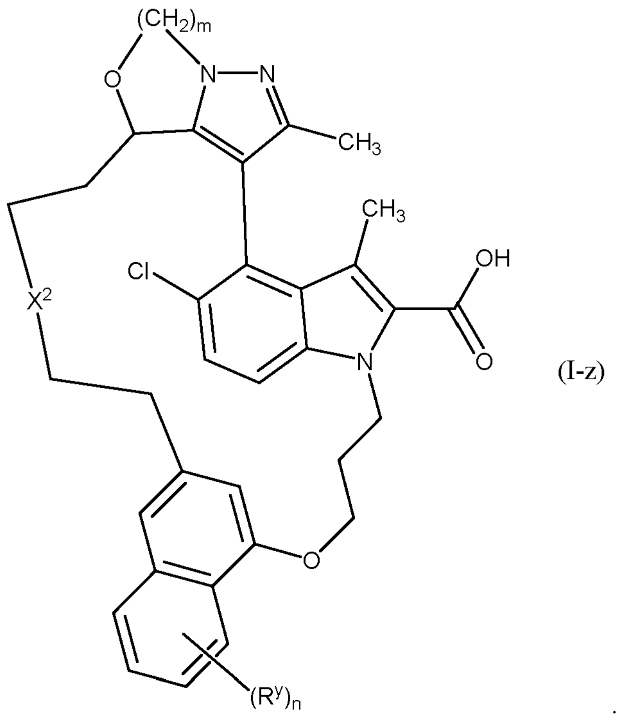

(I-z)

It will be clear that all variables in the structure of Formula (I-z), are defined as defined for the compounds of Formula (I) or any subgroup thereof as mentioned in any of the other embodiments.

In an embodiment, the present invention relates to a subgroup of Formula (I) as defined in the general reaction schemes.

In an embodiment the compound of Formula (I) is selected from the group consisting of any of the exemplified compounds,

tautomers and stereoisomeric forms thereof,

any pharmaceutically acceptable salts, and the solvates thereof.

All possible combinations of the above indicated embodiments are considered to be embraced within the scope of the invention.

METHODS FOR THE PREPARATION OF COMPOUNDS

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or prepared by standard synthetic processes commonly used by those skilled in the art of organic chemistry. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under $N_2$-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.

All variables are as defined for the compound of Formula (I) unless otherwise indicated or if it is clear from the context. The meaning of the chemical abbreviations used in the schemes are as defined below or as defined in the Table with abbreviations in the Examples.

Compounds of Formula (I) can be prepared according to Scheme 1,

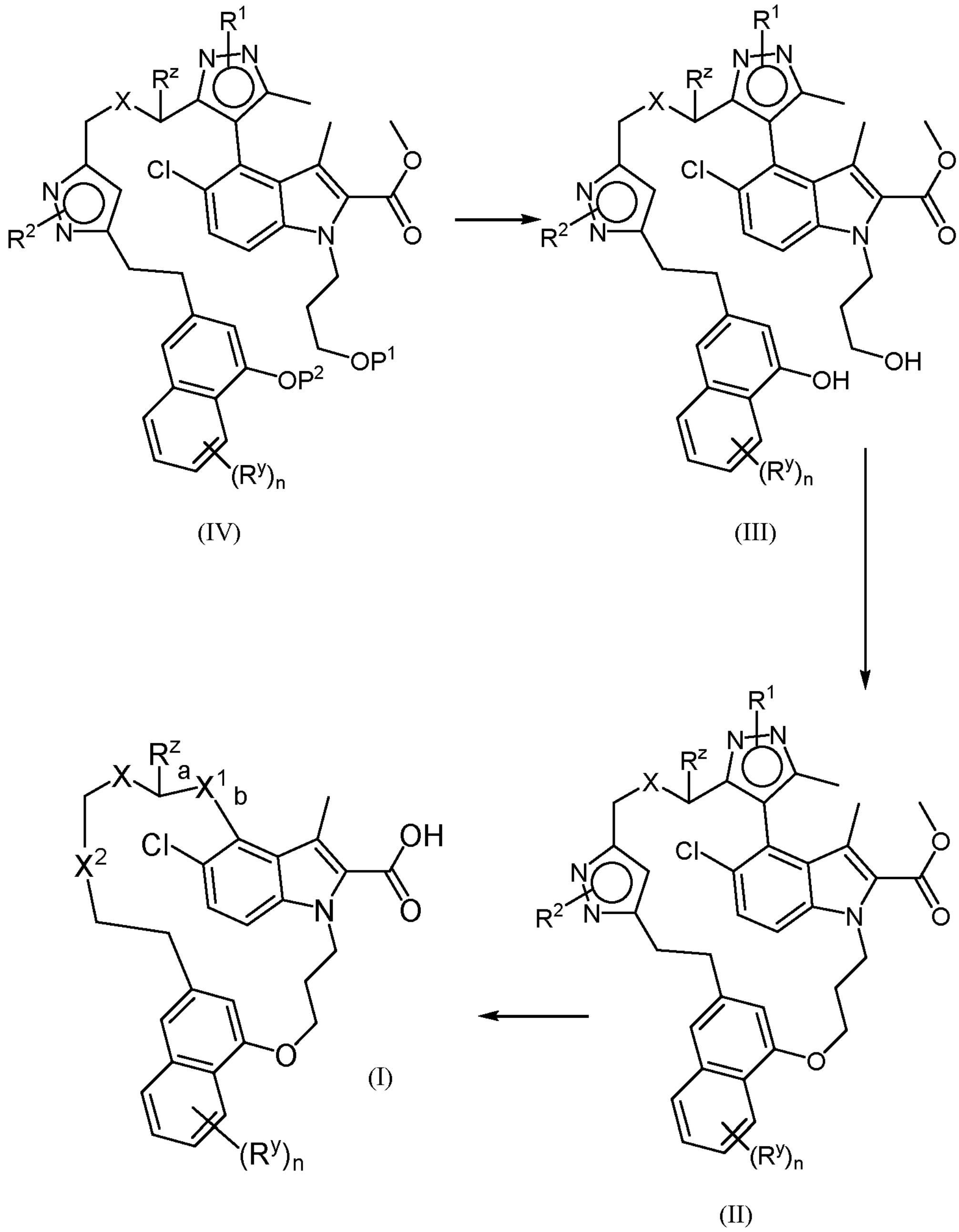

Scheme 1

- by reacting an intermediate of Formula (II) where X, $R^z$, $R^1$, $R^2$, and $(R^y)_n$ are defined as in Formula (I), with a suitable base such as, for example, LiOH or NaOH, in a suitable solvent such as water or a mixture of water and a suitable organic solvent such as dioxane or THF, or a mixture of MeOH and THF, at a suitable temperature such as room temperature or 50-60 °C.
- Intermediates of Formula (II) can be prepared by reacting an intermediate of Formula (III) where X, $R^z$, $R^1$, and $(R^y)_n$ are defined as in Formula (I), and $R^2$ is a suitable protecting group such as, for example, paramethoxybenzyl (PMB), dimethoxylbenzyl (DMB), or tetrahydropyranyl (THP), or $R^2$ is a suitable alkyl substituent such as, for example, methyl, with a suitable reagent, such as, for example, diethyl azodicarboxylate (DEAD) or di-tert-butyl azodicarboxylate (DTBAD), in the presence of a suitable phosphine such as, for example, $PPh_3$, in a suitable solvent such as, for example, THF, toluene, or a mixture thereof, at a suitable temperature such as, for example, room temperature or 70 °C.
- Intermediates of Formula (III) can be prepared by reacting an intermediate of Formula (IV) where X, $R^z$, $R^1$, $R^2$, and $(R^y)_n$ are as defined in Formula (III), and $P^1$ as well as $P^2$ are suitable protecting groups, such as, for example, tert-butyldimethylsilyl (TBDMS) or tert-butyldiphenylsilyl (TBDPS), with a suitable deprotecting reagent such as, for example, tetrabutylammonium fluoride (TBAF), in a suitable solvent such as, for example, THF, at a suitable temperature such as, for example, room temperature or 60 °C; or by sequential reaction with suitable deprotecting reagents such as, for example, $K_2CO_3$, in a suitable solvent such as, for example, MeOH, at a suitable temperature such as, for example, room temperature followed by reaction with suitable deprotecting reagents such as, for example, PPTS (pyridinium p-toluenesulfonate), in a suitable solvent such as, for example, MeOH, at a suitable temperature such as, for example room temperature or 50 °C.

- Alternatively, when $P^2$ in intermediates of Formula (IV) is a PMB group, an additional deprotection step might be necessary, using a suitable deprotection reagent such as, for example, TFA or 2,3-dichloro-5,6-dicyano-1,4-benzoquinone (DDQ), in a suitable solvent such as, for example, DCM, at a suitable temperature such as, for example, room temperature.

An intermediate of Formula (II) might have a protecting group in the $R^1$ position such as, for example, tetrahydropyranyl. In such a case, the intermediate of Formula (II) is reacted with a suitable deprotection reagent, such as, for example, pTsOH (p-toluenesulfonic acid) or HCl, in a suitable solvent such as, for example, iPrOH (2-propanol), at a suitable temperature such as, for example, room temperature. In a next step the obtained unprotected intermediate can be reacted with a suitable alkylating agent $R^1L$ (where L is as suitable leaving group) such as, for example, an alkyl halide, in the presence of a suitable base such as, for example, $Cs_2CO_3$, in a suitable solvent such as, for example, DMF (N,N-dimethylformamide), at a suitable temperature such as, for example, room temperature or 60 °C.

It will be clear for someone skilled in the art, that orthogonality of protective groups will have to be considered in this case, for instance when $R^1$ is a tetrahydropyranyl, $P^1$ and $P^2$ should be preferably TBDMS or TBDPS groups.

Alternatively, intermediates of Formula (II-a), wherein $R^{4a}$ and $R^{4b}$ are defined as in Formula (I), and p is defined as 0-2, can be prepared according to Scheme 2,

- by reacting an intermediate of Formula (II-b), wherein $L^2$ is a suitable leaving group such as, for example, mesylate, with a suitable amine such as, for example, morpholine or N-methylpiperazine, in a suitable solvent such as, for example, THF or DCM, at a suitable temperature such as, for example, room temperature.
- Intermediates of Formula (II-b) can be prepared by reacting an intermediate of Formula (II-c) with a suitable activating agent such as, for example, MsCl, in the presence of a suitable base such as, for example, triethylamine, in a suitable solvent such as, for example, THF or DCM, at a suitable temperature such as, for example, room temperature.

Alternatively, intermediates of Formula (II-a) can be prepared by reacting an intermediate of Formula (II-f) with a suitable amine such as, for example, methylamine, in the presence of a suitable reducing agent such as, for example, sodium cyanoborohydride, in a suitable solvent such as, for example, MeOH, at a suitable temperature such as, for example, room temperature.

Alternatively, intermediates of Formula (II-d), wherein $R^3$ is defined as in Formula (I) and p is defined as 0-2, can be prepared according to Scheme 2,

- by reacting an intermediate of Formula (II-c) with a suitable alkylating agent such as, for example, methyl iodide, in the presence of a suitable base such as, for example, NaH, in a suitable solvent such as, for example, THF, at a suitable temperature such as, for example, 0 °C or room temperature.

Alternatively, intermediates of Formula (II-e), where p is defined as 0-1, can be prepared according to Scheme 2,

- by reacting an intermediate of Formula (II-f) with a suitable alkyl nucleophile such as, for example, methylmagnesium bromide, in a suitable solvent such as, for example, THF, at a suitable temperature such as, for example, -78 °C.
- Intermediates of Formula (II-f) can be prepared by reacting an intermediates of Formula (II-c), wherein p is defined as 0-1, with a suitable oxidizing agent such as, for example, Dess-Martin periodinane, in a suitable solvent such as, for example, DCM, at a suitable temperature such as, for example, room temperature.

Intermediates of Formula (II-c) can be prepared according to Scheme 2, by reacting an intermediate of Formula (II-g) wherein $R^z$ is defined as $(CH_2)_pCH=CH_2$, and p is defined as 0-2, with a suitable hydroxylating reagent such as, for example, 9-borabicyclo[3.3.1]nonane dimer (9-BBN) followed by sodium perborate tetrahydrate, in a suitable solvent such as, for example, THF, at a suitable temperature such as, for example, 40 °C.

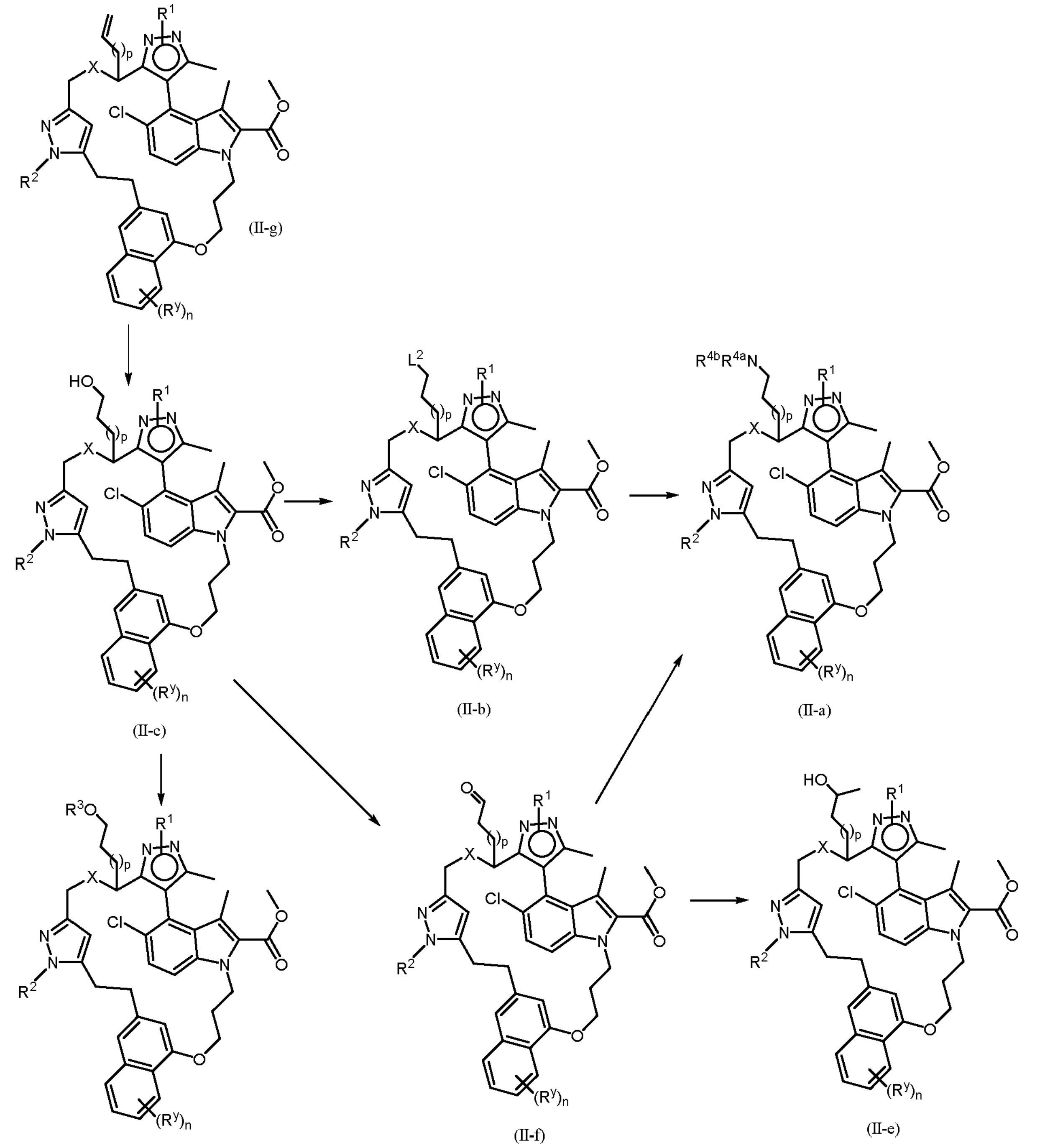

(II-g) (II-c) (II-b) (II-a) (II-d) (II-f) (II-e)

Scheme 2

Alternatively, both intermediates of Formula (II-h) and (II-i), wherein X, $R^z$, $R^1$ and $(R^y)_n$, and $R^2$ are defined as in Formula (I), can be prepared in two steps according to Scheme 3.

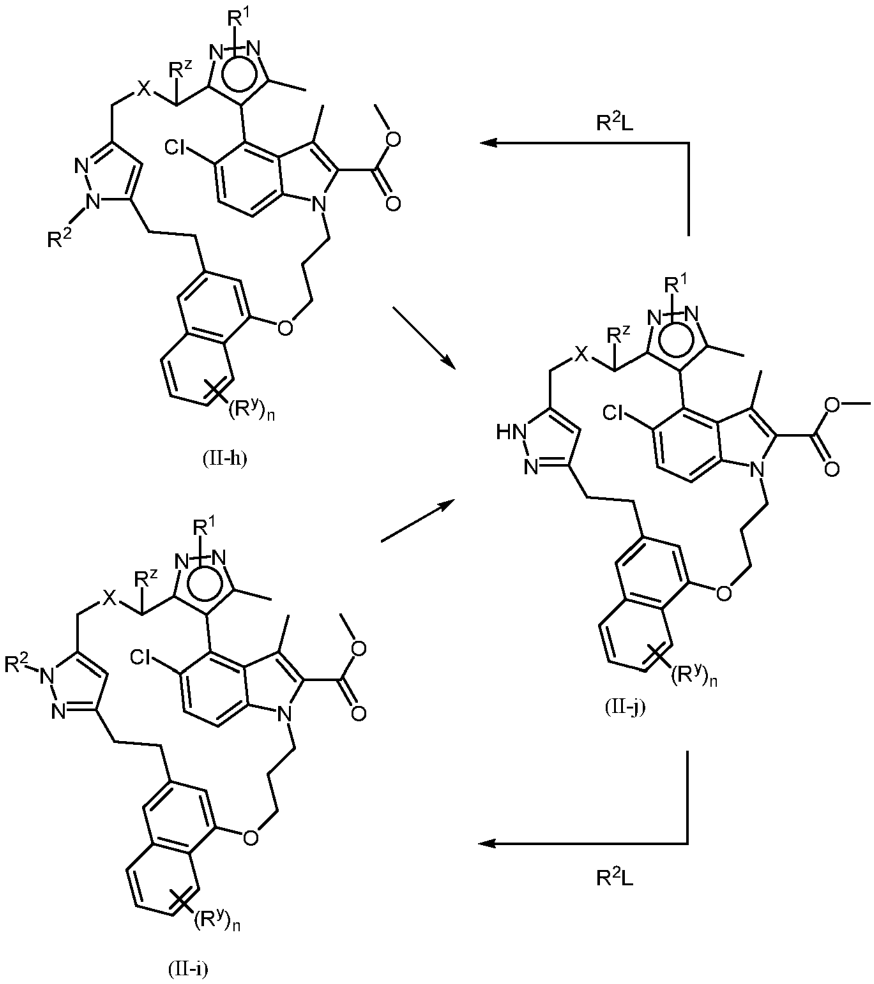


(II-h)

(II-j)

(II-i)

Scheme 3

- First, by reacting an intermediate of Formula (II-h) or (II-i), respectively, where $R^2$ is then defined as a suitable protecting group such as, for example, THP, with a suitable deprotection reagent such as, for example, HCl, in a suitable solvent such as, for example, dioxane or isopropanol, at a suitable temperature such as, for example, room temperature.
- Then, by reacting the obtained intermediate of Formula (II-j) with a suitable alkylating agent $R^2L$, such as, for example, an alkyl halide, in a suitable solvent, such as, for example, DMF, or acetonitrile, in the presence of a suitable base, such as, for example, triethylamine ($Et_3N$), N,N-diisopropylethylamine ($iPr_2EtN$), $Cs_2CO_3$, or 1,8-diazabicyclo[5.4.0] undec-7-ene (DBU), at a suitable temperature, such as, for example, room temperature or 60 °C, followed by a suitable separation of the isomers (II-h) and (II-i), such as, for example, a chromatographic separation.

Intermediates of Formula (IV), wherein $R^z$, $R^1$, $R^2$ and $(R^y)_n$ are defined as in Formula (I), $P^1$ and $P^2$ are suitable protective groups such as, for example, TBDMS or TBDPS, and X is defined as -O-, can be prepared according to Scheme 4,

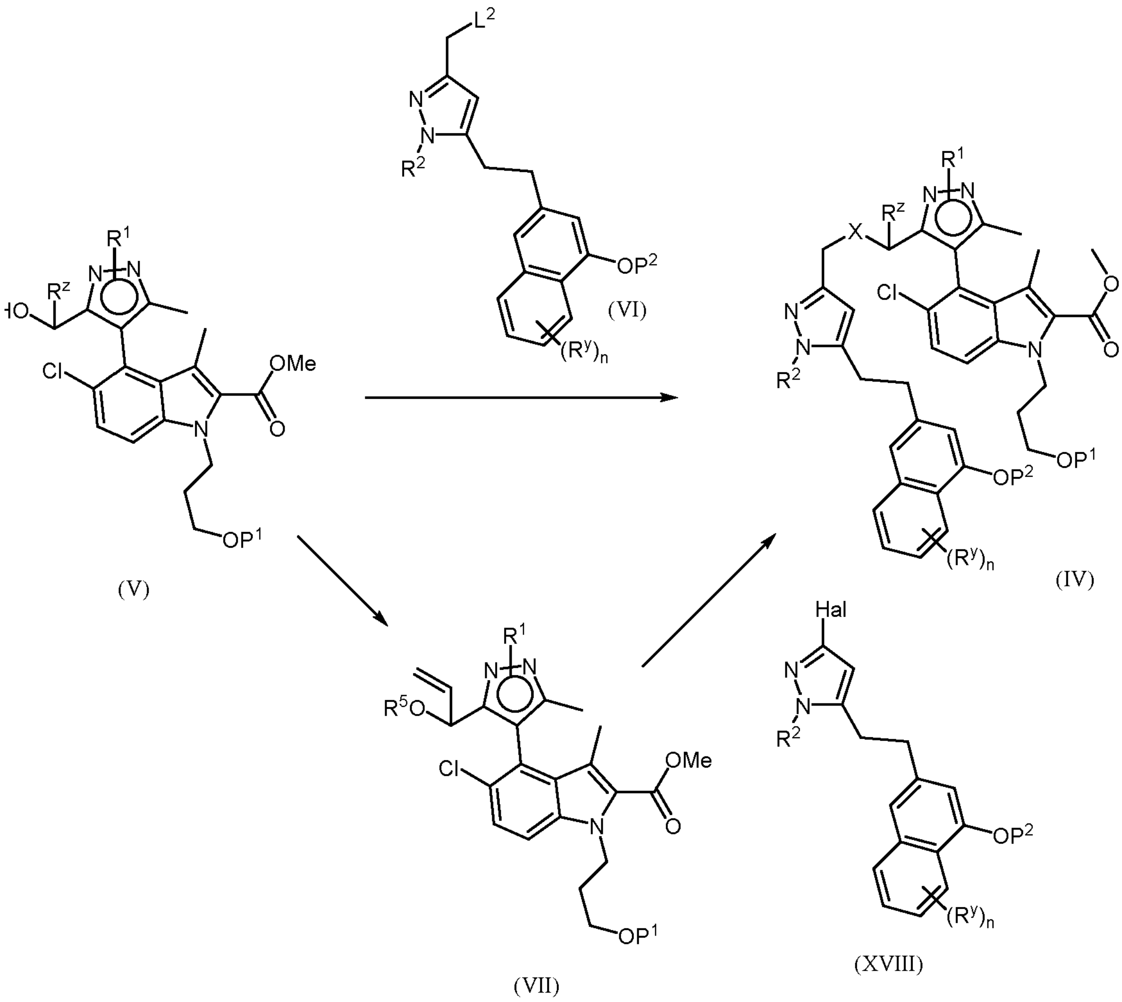

(VI)
(V)
(IV)
(VII)
(XVIII)

Scheme 4

- by reacting an intermediate of Formula (V) with an intermediate of Formula (VI), wherein $L^2$ is a suitable leaving group such as, for example, iodide, in the presence of a suitable base such as, for example, sodium hydride, in a suitable solvent such as, for example, THF, at a suitable temperature such as, for example, room temperature.

Intermediates of Formula (IV), wherein $R^1$, $R^2$ and $(R^y)_n$ are defined as in Formula (I), $P^1$ and $P^2$ are suitable protective groups such as, for example, TBDMS or TBDPS, and X is defined as $-CH_2-$ and $R^z$ is defined as $OR^5$, can be prepared according to Scheme 4,

- by reacting first an intermediate of Formula (VII) with a suitable hydroboration agent such as, for example, 9-BBN, in a suitable solvent such as, for example, THF, at a suitable temperature such as, for example, 75 °C. Then, by reacting the formed intermediate with an intermediate of Formula (VIII), wherein Hal is a suitable halide such as, for example, bromide , in the presence of a suitable catalyst such as, for example, [1,1'-bis(di-tert-butylphosphino)ferrocene] dichloropalladium(II), in the presence of a suitable base such as, for example, $K_3PO_4$, in a suitable solvent such as, for example, a mixture of THF and water, at a suitable temperature such as, for example, 80 °C.
- Intermediates of Formula (VII) can be prepared by reacting an intermediate of Formula (V), wherein $R^z$ is defined as vinyl, with a suitable alkylating agent such as, for example, p-methoxybenzyl chloride, in the presence of a suitable base such as, for example, sodium hydride, in the presence of a suitable iodide salt such as, for example, KI, in a suitable solvent such as, for example, DMF, at a suitable temperature such as, for example, 0 °C or room temperature.

Intermediates of Formula (V) wherein $R^z$ and $R^1$ are defined as in Formula (I), and $P^1$ is a suitable protecting group, such as, for example, TBDMS can be prepared according to Scheme 5,

- by reacting an intermediate of Formula (XLIX) with a suitable alkyl or alkenyl nucleophile such as, for example, an

alkylmagnesium halide or an alkenylmagnesium halide, in a suitable solvent such as, for example, THF, at a suitable temperature such as, for example, -78 °C.

- Intermediates of Formula (XLIX) can be prepared by reacting an intermediate of Formula (IX) with a suitable oxidizing agent such as, for example, Dess-Martin periodinane, in a suitable solvent such as, for example, DCM, at a suitable temperature such as, for example, 0 °C.
- Intermediates of Formula (IX) can be prepared by reacting an intermediate of Formula (X) with a suitable O-protected propyl halide or alkylsulfonate such as, for example, (3-bromopropoxy)(tert-butyl)dimethylsilane, in the presence of a suitable base such as, for example, $Cs_2CO_3$, in a suitable solvent such as, for example, DMF, at a suitable temperature such as, for example, room temperature.
- Intermediates of Formula (X) can be prepared by reacting an intermediate of Formula (XI), with a suitable deprotecting agent such as, for example, trifluoromethanesulfonic acid, TFA, or DDQ, in a suitable solvent such as, for example, $CH_2Cl_2$, at a suitable temperature such as, for example, room temperature.
- Intermediates of Formula (XI) can be prepared by reacting an intermediate of Formula (XII), with a suitable substituted pyrazole derivative such as, for example, 3-(((4-methoxybenzyl)oxy)methyl)-1,5-dimethyl-4-(4,4,5,5-tetramethyl-1,3,2-dioxaborolan-2-yl)-1H-pyrazole , in the presence of a suitable catalyst such as, for example, $Pd_2(dba)_3$, in the presence of a suitable phosphine ligand such as, for example, S-Phos, in the presence of a suitable base such as, for example, sodium bicarbonate, in a suitable solvent such as, for example, dioxane, water, or a mixture thereof, at a suitable temperature such as, for example, 100 °C.
- Intermediates of Formula (XII) can be prepared by reacting an intermediate of Formula (XIII), with a suitable acid, such as, for example, sulfuric acid, in a suitable solvent, such as, for example, acetic acid, at a suitable temperature, such as, for example, 70 °C.
- An intermediate of Formula (XIII) can be prepared by reacting (3-bromo-4-chlorophenyl)hydrazine with methyl 2-oxobutanoate, in the presence of a suitable acid, such as, for example, hydrochloric acid, in a suitable solvent, such as, for example, methanol, at a suitable temperature, such as, for example, 65 °C.

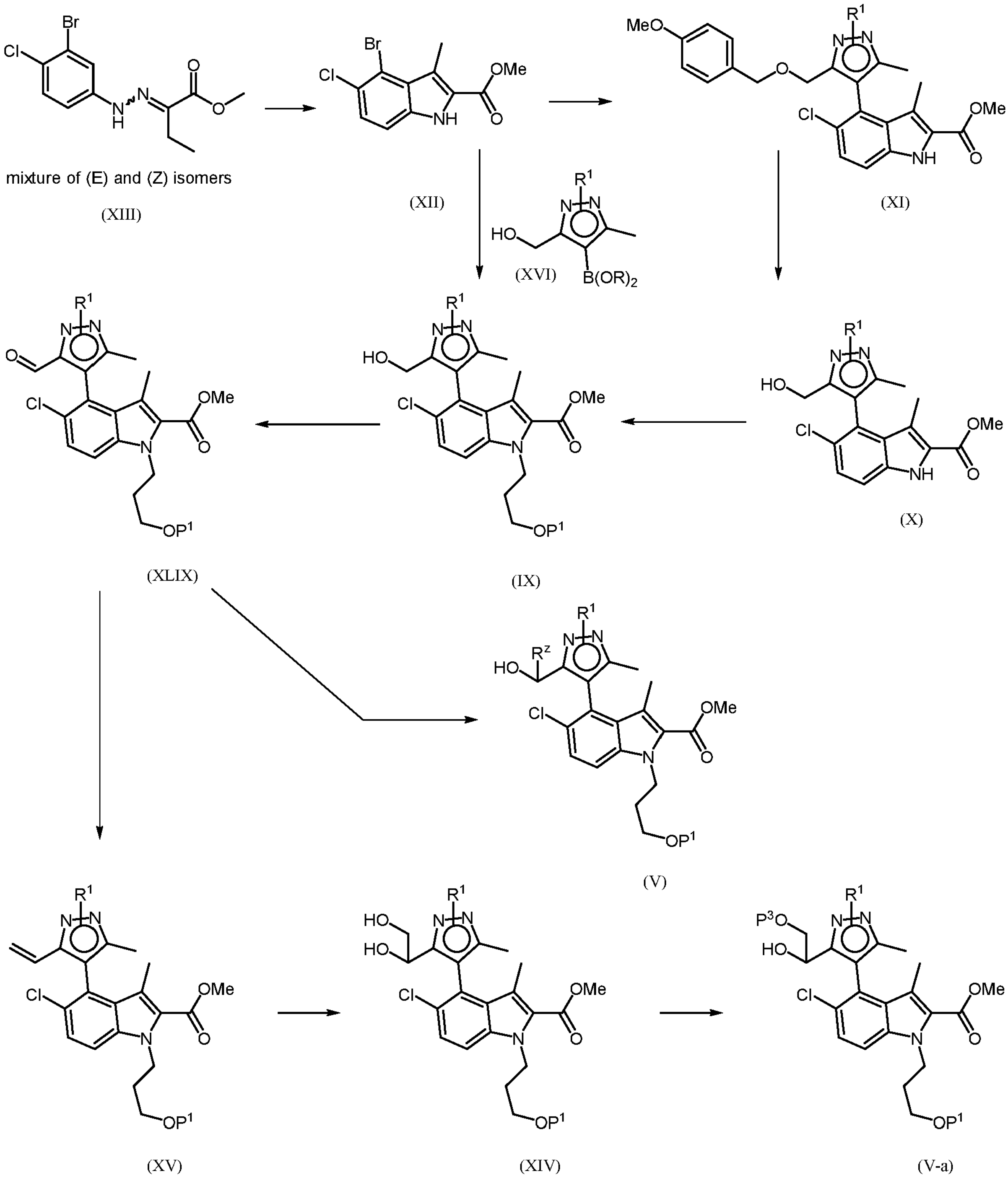


(XIII) (XII) (XI) (XVI) (X) (IX) (XLIX) (V) (XV) (XIV) (V-a)

Scheme 5

Alternatively, intermediates of Formula (V-a) wherein $R^z$ is defined as $CH_2OP^3$, with $P^3$ being a suitable protecting group such as, for example, TIPS (triisopropylsilyl), can be prepared according to Scheme 5,

- by reacting an intermediate of Formula (XIV) with a suitable protecting group precursor such as, for example, TIPSCl, in the presence of a suitable base such as, for example, imidazole, DMAP, or a mixture thereof, in a suitable solvent such as, for example, DCM, at a suitable temperature such as, for example, 0 °C.
- Intermediates of Formula (XIV) can be prepared by reacting an intermediate of Formula (XV) with a suitable dihydroxylating agent such as, for example, ADmix alpha, in the presence of a suitable additive such as, for example, methylsulfonamide, in a suitable solvent such as, for example, tBuOH, at a suitable temperature such as, for example, room temperature.

- Intermediates of Formula (XV) can be prepared by reacting an intermediate of Formula (XLIX) with a suitable phosphorus ylide, formed for example from a suitable phosphonium salt such as, for example, methyltriphenylphosphonium bromide, and a suitable base such as, for example, butyllithium, in a suitable solvent such as, for example, THF, at a suitable temperature such as, for example, -78 °C or room temperature.

Alternatively, intermediates of Formula (IX) can be prepared by reacting an intermediate of Formula (XII) with an intermediate of Formula (XVI), in the presence of a suitable catalyst such as, for example, bis(di-tert-butyl(4-dimethylaminophenyl)phosphine)dichloropalladium(II), in the presence of a suitable base such as, for example, potassium carbonate, in a suitable solvent such as, for example, dioxane, water, or a mixture thereof, at a suitable temperature such as, for example, 65 °C.

Intermediates of Formula (III), wherein $R^2$ and $(R^y)_n$ are defined as in Formula (I), and X is defined as $-CH_2-$, and $R^z$, taken together with $R^1$ forms a ring (b-1), with m as defined in Formula (I-z), can be prepared according to Scheme 6,

Scheme 6

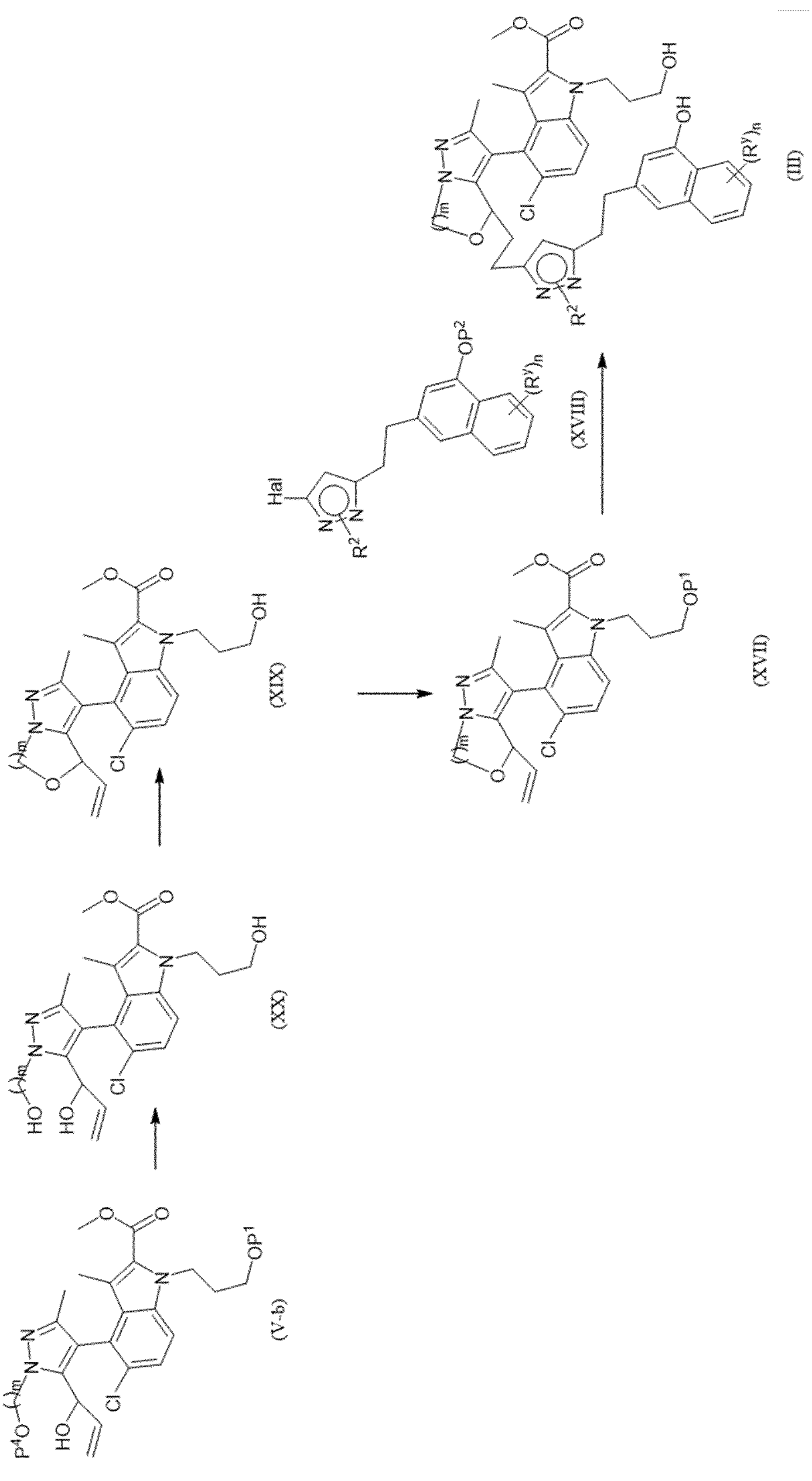

(V-b) (XX) (XIX) (XVII) (XVIII) (III)

- by reacting first an intermediate of Formula (XVII) with a suitable hydroboration agent such as, for example, 9-BBN, in

a suitable solvent such as, for example, THF, at a suitable temperature such as, for example, 75 °C. Then, by reacting the formed intermediate with an intermediate of Formula (XVIII), wherein Hal is a suitable halide such as, for example, bromide , in the presence of a suitable catalyst such as, for example, [1,1'-bis(di-tert-butylphosphino)ferrocene] dichloropalladium(II), in the presence of a suitable base such as, for example, $K_3PO_4$, in a suitable solvent such as, for example, a mixture of THF and water, at a suitable temperature such as, for example, 80 °C.

- Intermediates of Formula (XVII) can be prepared by reacting an intermediate of Formula (XIX) with a suitable protecting group precursor such as, for example, TBDMSCl, in the presence of a suitable base such as, for example, imidazole, or a mixture of imidazole and DMAP, in a suitable solvent such as, for example, DCM, at a suitable temperature such as, for example, room temperature.
- Intermediates of Formula (XIX) can be prepared by reacting an intermediate of Formula (XX) with a suitable catalyst such as, for example, pyridinium.p-toluenesulfonate, in a suitable solvent such as, for example, toluene, at a suitable temperature such as, for example, 115 °C.
- Intermediates of Formula (XX) can be prepared by reacting an intermediate of Formula (V-b), wherein $R^z$ is defined as vinyl and $R_1$ is defined as $-(CH_2)_mOP^4$, wherein $P^4$ is defined as a suitable protecting group such as, for example, THP, with a suitable deprotecting reagent such as, for example, PTSA, in a suitable solvent such as, for example, MeOH, at a suitable temperature such as, for example, room temperature.

Alternatively, Intermediates of Formula (II) wherein $R^2$ and $(R^y)_n$ are defined as in Formula (I), and X is defined as $-CH_2-$, and $R^z$, taken together with $R^1$ forms a ring (b-2), with m as defined in Formula (I), can be prepared according to Scheme 7,

- by reacting an intermediate of Formula (XXI) with a suitable alkylating agent such as, for example, methyl iodide, in the presence of a suitable base such as, for example, $Cs_2CO_3$, in a suitable solvent such as, for example, DMF, at a suitable temperature such as, for example, room temperature.
- Intermediates of Formula (XXI) can be prepared by reacting an intermediate of Formula (XXII) with a suitable acid such as, for example, HCl, in a suitable solvent such as, for example, iPrOH, at a suitable temperature such as, for example, room temperature.
- Intermediates of Formula (XXII) can be prepared by reacting an intermediate of Formula (XXIII) with a suitable reagent, such as, for example, diethyl azodicarboxylate (DEAD) or di-tert-butyl azodicarboxylate (DTBAD), in the presence of a suitable phosphine such as, for example, $PPh_3$, in a suitable solvent such as, for example, THF, toluene, or a mixture thereof, at a suitable temperature such as, for example, room temperature or 70 °C.
- Intermediates of Formula (XXIII) can be prepared by reacting an intermediate of Formula (XXIV) with a suitable deprotecting agent such as, for example, TBAF, in a suitable solvent such as, for example, THF, at a suitable temperature such as, for example, room temperature.
- Intermediates of Formula (XXIV) can be prepared by reacting first an intermediate of Formula (XXV) with a suitable hydroboration agent such as, for example, 9-BBN, in a suitable solvent such as, for example, THF, at a suitable temperature such as, for example, 75 °C. Then, by reacting the formed intermediate with an intermediate of Formula (XVIII), wherein Hal is a suitable halide such as, for example, bromide , in the presence of a suitable catalyst such as, for example, [1,1'-bis(di-tert-butylphosphino)ferrocene]dichloropalladium(II), in the presence of a suitable base such as, for example, $K_3PO_4$, in a suitable solvent such as, for example, a mixture of THF and water, at a suitable temperature such as, for example, 80 °C.
- Intermediates of Formula (XXV) can be prepared by reacting an intermediate of Formula (XXVI) with a suitable coupling reagent such as, for example, cyanomethylenetributylphosphorane, in a suitable solvent such as, for example, THF or toluene, or a mixture thereof, at a suitable temperature such as, for example, 80 °C.
- Intermediates of Formula (XXVI) can be prepared by reacting an intermediate of Formula (XXVII), where $P^4$ is defined as a suitable protecting group such as, for example, THP, with a suitable deprotecting reagent such as, for example, PTSA, in a suitable solvent such as, for example, MeOH, at a suitable temperature such as, for example, room temperature.
- Intermediates of Formula (XXVII) can be prepared by reacting an Intermediate of Formula (XXVIII) with a suitable vinyl nucleophile such as, for example, vinylmagnesium bromide, in the presence of a suitable catalyst such as, for example, dimethylzinc, in a suitable solvent such as, for example, THF, at a suitable temperature such as, for example, -78 °C.
- Intermediates of Formula (XXVIII) can be prepared by reacting an intermediate of Formula (XLIX-a) with a suitable sulfinamide such as, for example, tertbutylsulfinamide, in the presence of a suitable catalyst such as, for example, $Ti(OPr)_4$, in a suitable solvent such as, for example, THF, at a suitable temperature such as, for example, room temperature.

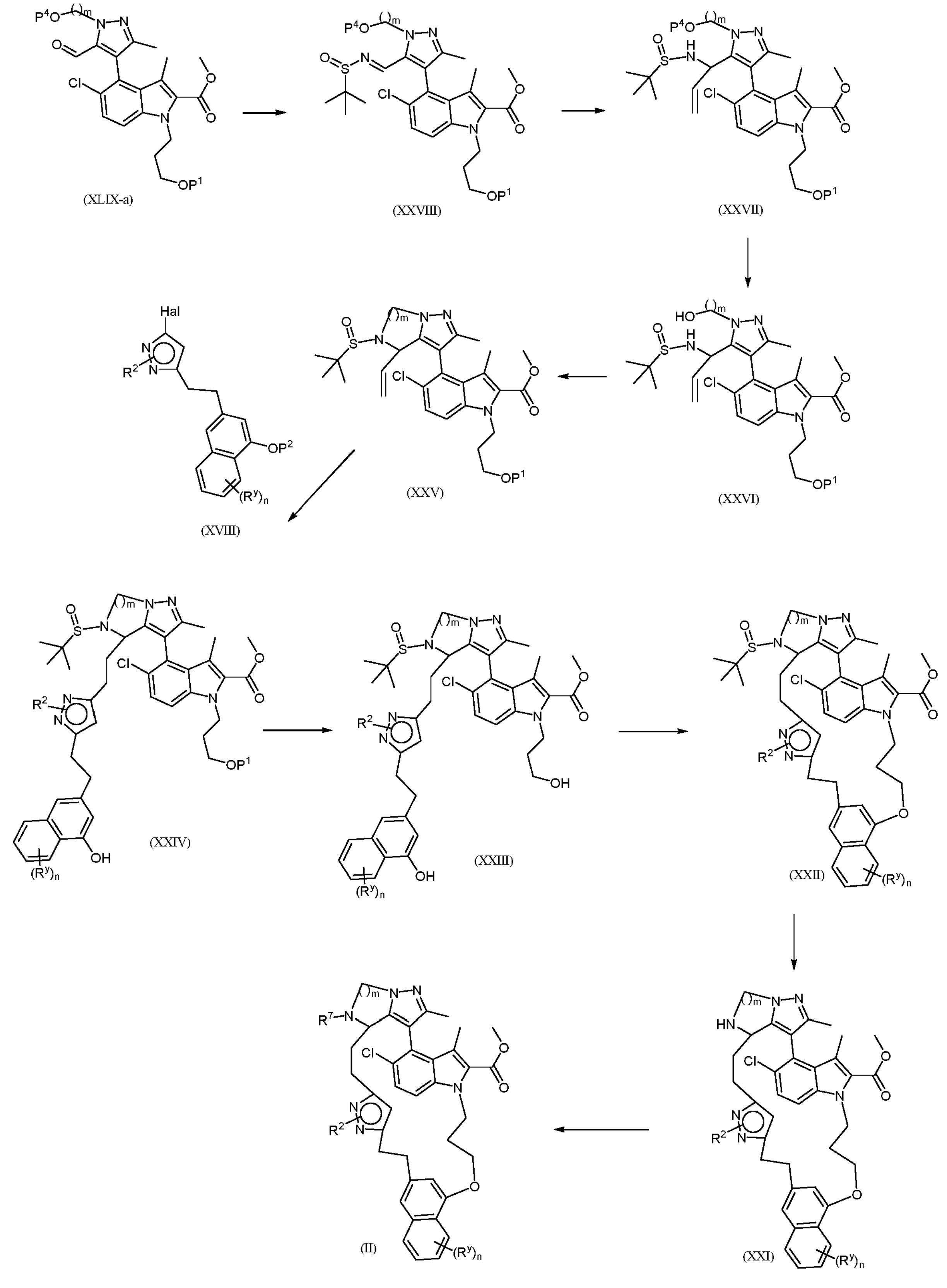

(XLIX-a) (XXVIII) (XXVII) (XXVI) (XXV) (XVIII) (XXIV) (XXIII) (XXII) (XXI) (II)

Scheme 7

Intermediates of Formula (VI) can be prepared according to Scheme 8,

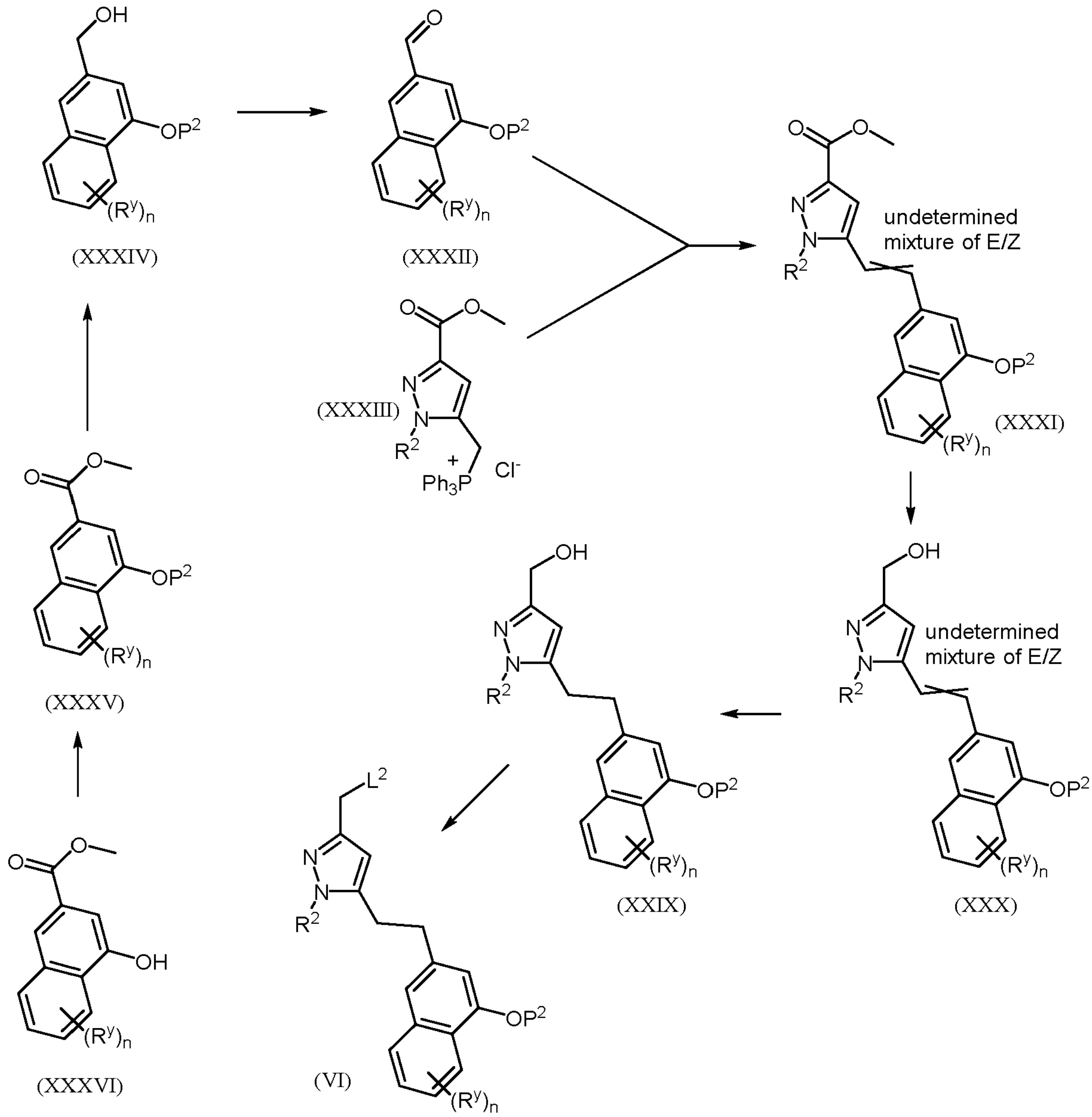


(XXXIV) (XXXII) (XXXIII) (XXXI) (XXXV) (XXIX) (XXX) (XXXVI) (VI)

Scheme 8

- by reacting first an intermediate of Formula (XXIX) with a suitable activating agent such as, for example, mesyl chloride, in the presence of a suitable base such as, for example, DIPEA, in a suitable solvent such as, for example, THF, at a suitable temperature such as, for example, 0 °C or room temperature; then by reacting the activated intermediate with a suitable leaving group precursor such as, for example, sodium iodide, in a suitable solvent such as, for example, THF, at a suitable temperature such as, for example, room temperature.
- Intermediates of Formula (XXIX) can prepared by reacting an intermediate of Formula (XXX) with a suitable hydrogenating reagent such as, for example, hydrogen gas, in the presence of a suitable catalyst such as, for example, Pd/C, in a suitable solvent such as, for example, MeOH, at a suitable temperature such as, for example, room temperature.
- Intermediates of Formula (XXX) can be prepared by reacting an intermediate of Formula (XXXI) with a suitable reducing agent such as, for example, $LiAlH_4$, in a suitable solvent such as, for example, THF, at a suitable temperature such as, for example, 0 °C.
- Intermediates of Formula (XXXI) can be prepared by reacting an intermediate of Formula (XXXII) with an intermediate of Formula (XXXIII), in the presence of a suitable base such as, for example, NaH, in a suitable solvent such as, for example, THF, at a suitable temperature such as, for example, -10 °C.
- Intermediates of Formula (XXXII) can be prepared by reacting an intermediate of Formula (XXXIV) with a suitable oxidizing agent such as, for example, $MnO_2$, in a suitable solvent such as, for example, acetonitrile, at a suitable

temperature such as, for example, 60 °C.

- Intermediates of Formula (XXXIV) can be prepared by reacting an intermediate of Formula (XXXV) with a suitable reducing agent such as, for example, $LiAlH_4$, in a suitable solvent such as, for example, THF, at a suitable temperature such as, for example, 0 °C.
- Intermediates of Formula (XXXV) can be prepared by reacting an intermediate of Formula (XXXVI) with a suitable protecting reagent such as, for example, tert-butyl(chloro)diphenylsilane (TBDPSCl) or 4-methoxybenzyl chloride (PMBCl), in the presence of a suitable base such as, for example, imidazole or NaH, in a suitable solvent such as, for example, DMF, at a suitable temperature such as, for example, room temperature.
- Intermediates of Formula (XXXIII) and Intermediates of Formula (XXXVI) are commercially available or can be prepared according to procedures described in literature.

Intermediates of Formula (XVI) wherein $R^1$ is defined as in Formula (I), and $B(OR)_2$ is a suitable boronate ester such as, for example, pinacol borate, can be prepared according to Scheme 9,

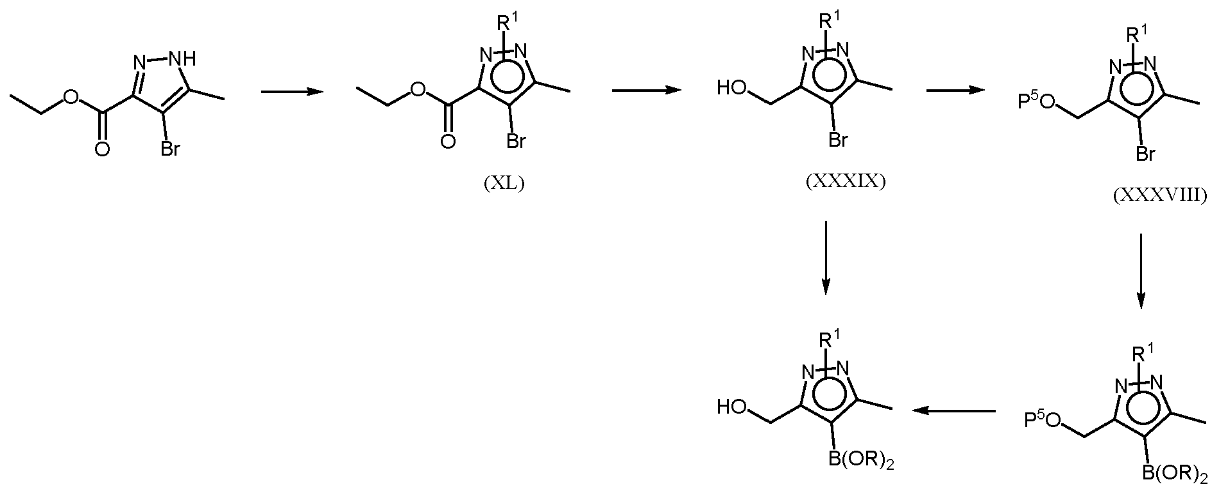

(XL) (XXXIX) (XXXVIII) (XVI) (XXXVII)

Scheme 9

- by reacting an intermediate of Formula (XXXVII) wherein $P^5$ is a suitable protecting group such as, for example, TBDMS, with a suitable deprotecting agent such as, for example, TBAF, in a suitable solvent such as, for example, methyl-THF, at a suitable temperature such as, for example, room temperature.
- Intermediates of Formula (XXXVII), where $P^5$ is a suitable protecting group such as, for example, TBDMS, can be prepared by reacting an intermediate of Formula (XXXVIII) with a suitable borylating agent such as, for example, 2-isopropoxy-4,4,5,5-tetramethyl-1,3,2-dioxaborolane, in the presence of a suitable base such as, for example, butyllithium, in a suitable solvent such as, for example, THF, at a suitable temperature such as, for example, -78 °C or room temperature.
- Intermediates of Formula (XXXVIII) can be prepared by reacting an intermediate of Formula (XXXIX) with a suitable protecting group precursor, such as, for example, TBDMSCl, in the presence of a suitable base such as, for example, imidazole, in a suitable solvent such as, for example, DCM, at a suitable temperature such as, for example, room temperature.
- Intermediates of Formula (XXXIX) can be prepared by reacting an intermediate of Formula (XL) with a suitable reducing agent such as, for example, sodium borohydride, in a suitable solvent such as, for example, a mixture of THF and MeOH, at a suitable temperature such as, for example, room temperature.
- Intermediates of Formula (XL) can be prepared by reacting 1H-pyrazole-3-carboxylic acid, 4-bromo-5-methyl-, ethyl ester, with a suitable alcohol, such as, for example, 2-(tetrahydro-2H-pyran-2-yloxy)ethanol, in the presence of a suitable coupling reagent such as, for example, cyanomethylenetributylphosphorane, in a suitable solvent such as, for example, THF, at a suitable temperature such as, for example, room temperature.
- Alternatively, intermediates of Formula (XVI) can be prepared by reacting an intermediate of Formula (XXXIX) with a suitable borylating agent such as, for example, pinacolborane, in the presence of a suitable base such as, for example, $Et_3N$, in the presence of a suitable catalyst such as, for example, bis(acetonitrile)dichloropalladium(II), in the presence of a suitable phosphine such as, for example, 2-dicyclohexylphosphino-2',6'-dimethoxybiphenyl, in a suitable solvent such as, for example, 1,4-dioxane, at a suitable temperature such as, for example, 80 °C.

Intermediates of Formula (XVIII), wherein $X^2$, $R^y$, and n are defined as in Formula (I), $P^2$ is a suitable protecting group such as, for example, TBDMS, and Hal is a suitable halide such as, for example, bromide, can be prepared according to Scheme 10,

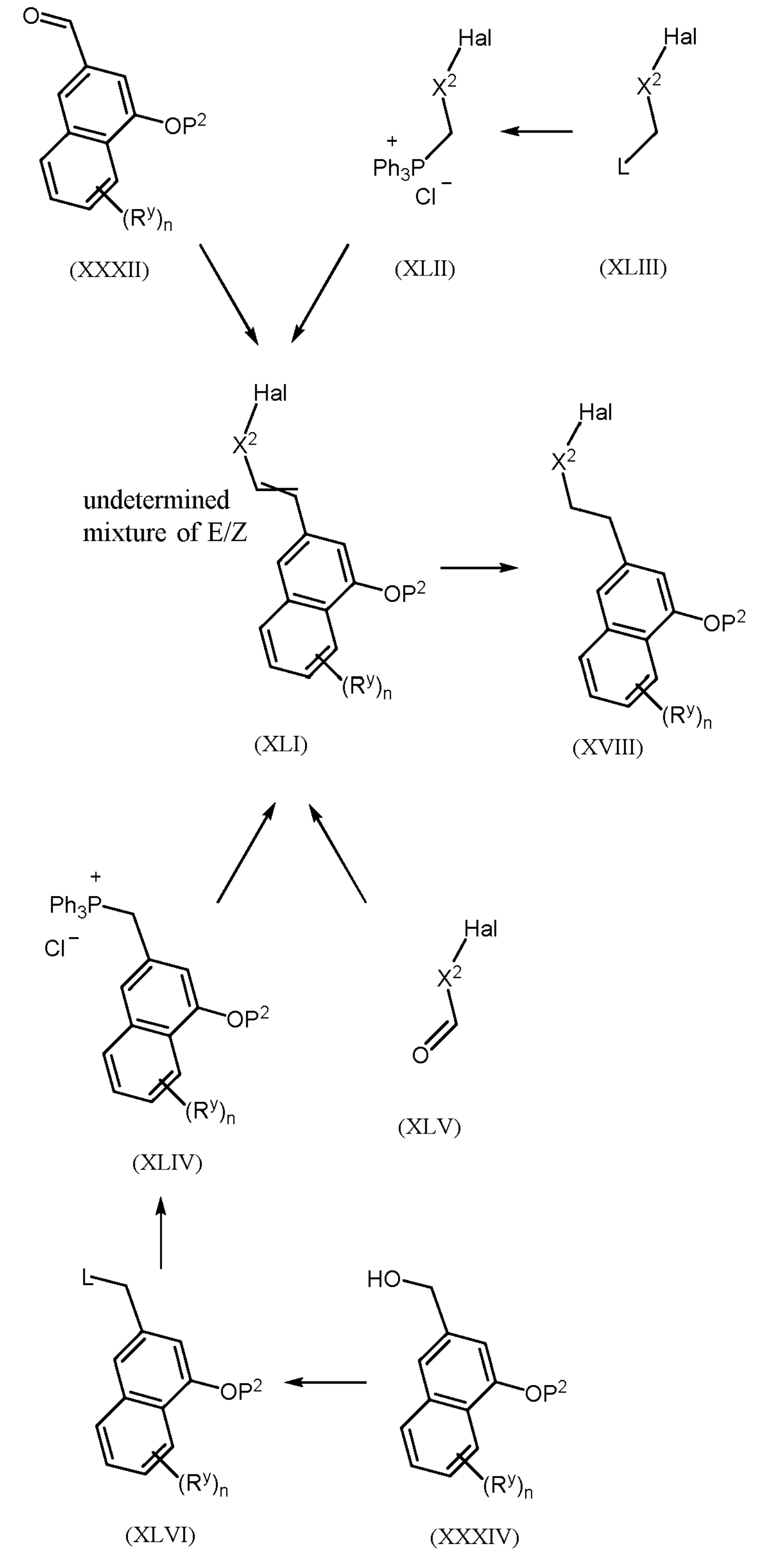


(XXXII) (XLII) (XLIII)

(XLI) (XVIII)

(XLIV) (XLV)

(XLVI) (XXXIV)

Scheme 10

- by reacting an intermediate of Formula (XLI) with a suitable hydrogenating agent such as, for example, hydrogen, in

the presence of a suitable catalyst such as, for example, $PtO_2$, in a suitable solvent such as, for example, ethyl acetate (EtOAc), at a suitable temperature such as, for example, room temperature.

- Intermediates of Formula (XLI) can be prepared by reacting an intermediate of Formula (XLII) with an intermediate of Formula (XXXII) in the presence of a suitable base such as, for example, NaH, in a suitable solvent such as, for example, THF, at a suitable temperature such as, for example, -30 °C or 0 °C.
- Intermediates of Formula (XLII) can be prepared by reacting a suitable haloheterocycle of Formula (XLIII) such as, for example, 3-bromo-5-(chloromethyl)-1-methyl-1H-pyrazole (CAS [2109428-60-4]), with a suitable phosphine such as, for example, $PPh_3$, in a suitable solvent such as, for example, acetonitrile (ACN), at a suitable temperature such as, for example, 85 °C.

Alternatively, intermediates of Formula (XLI) can be prepared according to Scheme 10,

- by reacting an intermediate of Formula (XLIV) with an intermediate of Formula (XLV) in the presence of a suitable base such as, for example, NaH, in a suitable solvent such as, for example, THF, at a suitable temperature such as, for example, -30 °C.
- Intermediates of Formula (XLIV) can be prepared by reacting an intermediate of Formula (XLVI) with a suitable phosphine such as, for example, triphenylphosphine, in a suitable solvent such as, for example, ACN, at a suitable temperature such as, for example, 85 °C.
- Intermediates of Formula (XLVI), wherein L is a suitable leaving group such as, for example, chloride, can be prepared by reacting first an intermediate of Formula (XXXIV) with a suitable activating reagent such as, for example, methanesulfonic anhydride, in a suitable solvent such as, for example, THF, at a suitable temperature such as, for example, room temperature; then by reacting the formed activated intermediate with a suitable chloride source such as, for example, LiCl, in a suitable solvent such as, for example, THF, at a suitable temperature such as, for example, room temperature.

Intermediates of Formula (XLIII), wherein $X^2$ is defined as in Formula (I), L is a suitable leaving group such as, for example, chloride, and Hal is a suitable halide such as, for example, bromide, can be prepared according to Scheme 11,

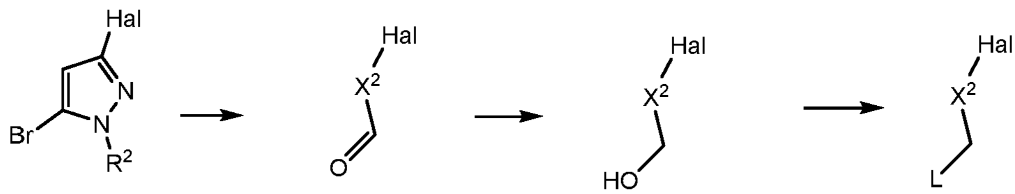

(XLVIII) (XLV) (XLVII) (XLIII)

Scheme 11

- by reacting an intermediate of Formula (XLVII) with a suitable activating agent such as, for example, thionyl chloride, in a suitable solvent such as, for example, DCM, at a suitable temperature such as, for example, room temperature.
- Intermediates of Formula (XLVII) can be prepared by reacting an intermediate of Formula (XLV) with a suitable reducing agent such as, for example, $NaBH_4$, in a suitable solvent such as, for example, methanol (MeOH), at a suitable temperature such as, for example, 15 °C.
- Intermediates of Formula (XLV) can be prepared by reacting a dihaloheterocycle such as, for example, an intermediate of Formula (XLVIII), with a suitable acylating agent such as, for example, DMF, in the presence of a suitable base such as, for example, BuLi, in a suitable solvent such as, for example, THF, at a suitable temperature such as, for example, -78 °C.
- Intermediates of Formula (XLVIII) are commercially available or can be prepared according to procedures described in literature.

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be

separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

PHARMACOLOGY OF COMPOUNDS

It has been found that the compounds of the present invention inhibit one of more MCL-1 activities, such as MCL-1 antiapoptotic activity.

An MCL-1 inhibitor is a compound that blocks one or more MCL-1 functions, such as the ability to bind and repress proapoptotic effectors Bak and Bax or BH3 only sensitizers such as Bim, Noxa or Puma.

The compounds of the present invention can inhibit the MCL-1 pro-survival functions. Therefore, the compounds of the present invention may be useful in treating and / or preventing, in particular treating, diseases that are susceptible to the effects of the immune system such as cancer.

In another embodiment of the present invention, the compounds of the present invention exhibit anti-tumoral properties, for example, through immune modulation.

In an embodiment, the present invention is directed to a compound of formula (I) for use in methods for treating and / or preventing a cancer, wherein the cancer is selected from those described herein, comprising administering to a subject in need thereof (preferably a human), a therapeutically effective amount of a compound of Formula (I), or pharmaceutically acceptable salt, or a solvate thereof.

In an embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of acute lymphoblastic leukemia (ALL), acute myeloid leukemia (AML), B cells acute lymphoblastic leukemia, B-cell chronic lymphocytic leukemia (CLL), bladder cancer, breast cancer, chronic lymphocytic leukemia, chronic myeloid leukemia, colon adenocarcinoma, diffuse large B cell lymphoma, esophageal cancer, follicular lymphoma, gastric cancer, head and neck cancer (including, but not limited to head and neck squamous cell carcinoma), hematopoietic cancer, hepatocellular carcinoma, Hodgkin lymphoma, liver cancer, lung cancer (including but not limited to lung adenocarcinoma), lymphoma, medulloblastoma, melanoma, monoclonal gammopathy of undetermined significance, multiple myeloma, myelodysplastic syndromes, myelofibrosis, myeloproliferative neoplasms, ovarian cancer, ovarian clear cell carcinoma, ovarian serous cystadenoma, pancreatic cancer, polycythemia vera, prostate cancer, rectum adenocarcinoma, renal cell carcinoma, smoldering multiple myeloma, T cell acute lymphoblastic leukemia, T cell lymphoma, and Waldenstroms macroglobulinemia.

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is preferably selected from the group consisting of acute lymphoblastic leukemia (ALL), acute myeloid leukemia (AML), B cells acute lymphoblastic leukemia, B-cell chronic lymphocytic leukemia (CLL), breast cancer, chronic lymphocytic leukemia, chronic myeloid leukemia, diffuse large B cell lymphoma, follicular lymphoma, hematopoietic cancer, Hodgkin lymphoma, lung cancer (including, but not limited to lung adenocarcinoma) lymphoma, monoclonal gammopathy of undetermined significance, multiple myeloma, myelodysplastic syndromes, myelofibrosis, myeloproliferative neoplasms, smoldering multiple myeloma, T cell acute lymphoblastic leukemia, T cell lymphoma and Waldenstroms macroglobulinemia.

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of adenocarcinoma, benign monoclonal gammopathy, biliary cancer (including, but not limited to, cholangiocarcinoma), bladder cancer, breast cancer (including, but not limited

to, adenocarcinoma of the breast, papillary carcinoma of the breast, mammary cancer, medullary carcinoma of the breast), brain cancer (including, but not limited to, meningioma), glioma (including, but not limited to, astrocytoma, oligodendroglioma; medulloblastoma), bronchus cancer, cervical cancer (including, but not limited to, cervical adenocarcinoma), chordoma, choriocarcinoma, colorectal cancer (including, but not limited to, colon cancer, rectal cancer, colorectal adenocarcinoma), epithelial carcinoma, endothelial sarcoma (including, but not limited to, Kaposi's sarcoma, multiple idiopathic hemorrhagic sarcoma), endometrial cancer (including, but not limited to, uterine cancer, uterine sarcoma), esophageal cancer (including, but not limited to, adenocarcinoma of the esophagus, Barrett' s adenocarinoma), Ewing sarcoma, gastric cancer (including, but not limited to, stomach adenocarcinoma), gastrointestinal stromal tumor (GIST), head and neck cancer (including, but not limited to, head and neck squamous cell carcinoma), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma. splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, kidney cancer (including, but not limited to, nephroblastoma a.k.a. Wilms' tumor, renal cell carcinoma), liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, *Lewis lung carcinoma,* lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES), ovarian cancer (including, but not limited to, cystadenocarcinoma, ovarian embryonal carcinoma, ovarian adenocarcinoma), pancreatic cancer (including, but not limited to, pancreatic andenocarcinoma, intraductal papillary mucinous neoplasm (IPMN), Islet cell tumors), prostate cancer (including, but not limited to, prostate adenocarcinoma), skin cancer (including, but not limited to, squamous cell carcinoma (SCC), keratoacanthoma (KA), melanoma, basal cell carcinoma (BCC)) and soft tissue sarcoma (e.g. malignant fibrous histiocytoma (MFH), liposarcoma, malignant peripheral nerve sheath tumor (MPNST), chondrosarcoma, fibrosarcoma, myxosarcoma).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of benign monoclonal gammopathy, breast cancer (including, but not limited to, adenocarcinoma of the breast, papillary carcinoma of the breast, mammary cancer, medullary carcinoma of the breast), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma. splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not

limited to, alpha chain disease, gamma chain disease, mu chain disease), immunocytic amyloidosis, liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, Lewis lung carcinoma, lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), and prostate cancer (including, but not limited to, prostate adenocarcinoma).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of prostate, lung, pancreatic, breast, ovarian, cervical, melanoma, B-cell chronic lymphocytic leukemia (CLL), acute myeloid leukemia (AML), and acute lymphoblastic leukemia (ALL).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is multiple myeloma.

The compounds according to the present invention or pharmaceutical compositions comprising said compounds, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1 and or CTLA-4 or engineered chimeric antigen receptor T cells (CART) targeting tumor associated antigens.

The compounds according to the present invention or pharmaceutical compositions comprising said compounds, may also be combined with radiotherapy or chemotherapeutic agents (including, but not limited to, anti-cancer agents) or any other pharmaceutical agent which is administered to a subject having cancer for the treatment of said subject's cancer or for the treatment or prevention of side effects associated with the treatment of said subject's cancer.

The compounds according to the present invention or pharmaceutical compositions comprising said compounds, may also be combined with other agents that stimulate or enhance the immune response, such as vaccines.

Therefore also disclosed herein are the methods for treating and / or preventing a cancer (wherein the cancer is selected from those described herein) comprising administering to a subject in need thereof (preferably a human), a therapeutically effective amount of co-therapy or combination therapy; wherein the co-therapy or combination therapy comprises a compound of Formula (I) of the present invention and one or more anti-cancer agent(s) selected from the group consisting of (a) immune modulatory agent (such as inhibitors of the PD1/PDL1 immune checkpoint axis, for example antibodies (or peptides) that bind to and/or inhibit the activity of PD-1 or the activity of PD-L1 and or CTLA-4); (b) engineered chimeric antigen receptor T cells (CART) targeting tumor associated antigens; (c) radiotherapy; (d) chemotherapy; and (e) agents that stimulate or enhance the immune response, such as vaccines.

The present invention is directed to compounds of Formula (I) and pharmaceutically acceptable salts, and solvates thereof, for use as a medicament.

The present invention is directed to compounds of Formula (I) and pharmaceutically acceptable salts, and solvates thereof, for use in the inhibition of MCL-1 activity.

As used herein, unless otherwise noted, the term "anti-cancer agents" shall encompass "anti-tumor cell growth agents" and "anti-neoplastic agents".

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for use in treating and / or preventing in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, MCL-1 mediated diseases or conditions, preferably cancer, more preferably a cancer as herein described (for example, multiple myeloma).

The present invention is directed to compounds of Formula (I) and pharmaceutically acceptable salts, and solvates thereof, for use in treating and / or preventing, in particular for use in treating, MCL-1 mediated diseases or conditions, preferably cancer, more preferably a cancer as herein described (for example, multiple myeloma).

The present invention relates to compounds of Formula (I) and pharmaceutically acceptable salts, and solvates

thereof, for the manufacture of a medicament.

The present invention relates to compounds of Formula (I) and pharmaceutically acceptable salts, and solvates thereof, for the manufacture of a medicament for the inhibition of MCL-1.

The present invention relates to compounds of Formula (I) and pharmaceutically acceptable salts, and solvates thereof, for the manufacture of a medicament for treating and / or preventing, in particular for treating, a cancer, preferably a cancer as herein described. More particularly, the cancer is a cancer which responds to inhibition of MCL-1 (for example, multiple myeloma).

The present invention is directed to compounds of Formula (I) and pharmaceutically acceptable salts, and solvates thereof, for the manufacture of a medicament for treating and / or preventing, in particular for treating, any one of the disease conditions mentioned hereinbefore.

The present invention is directed to compounds of Formula (I) and pharmaceutically acceptable salts, and solvates thereof, for the manufacture of a medicament for treating and / or preventing any one of the disease conditions mentioned hereinbefore.

The compounds of Formula (I) and pharmaceutically acceptable salts, and solvates thereof, can be administered to subjects, preferably humans, for treating and / or preventing of any one of the diseases mentioned hereinbefore.

In view of the utility of the compounds of Formula (I) and pharmaceutically acceptable salts, and solvates thereof, there is provided a compound of formula (I) for use in a method of treating subjects, preferably mammals such as humans, suffering from any of the diseases mentioned hereinbefore; or a method of slowing the progression of any of the diseases mentioned hereinbefore in subject, humans; or a method of preventing subjects, preferably mammals such as humans, from suffering from any one of the diseases mentioned hereinbefore.

Said methods comprise the administration, i.e. the systemic or topical administration, preferably oral or intravenous administration, more preferably oral administration, of an effective amount of a compound of Formula (I) or a pharmaceutically acceptable salt, or a solvate thereof, to subjects such as humans.

One skilled in the art will recognize that a therapeutically effective amount of the compounds of the present invention is the amount sufficient to have therapeutic activity and that this amount varies *inter alias,* depending on the type of disease, the concentration of the compound in the therapeutic formulation, and the condition of the patient. In an embodiment, a therapeutically effective daily amount may be from about 0.005 mg/kg to 100 mg/kg.

The amount of a compound according to the present invention, also referred to herein as the active ingredient, which is required to achieve a therapeutic effect may vary on case-by-case basis, for example with the specific compound, the route of administration, the age and condition of the recipient, and the particular disorder or disease being treated. The methods of the present invention may also include administering the active ingredient on a regimen of between one and four intakes per day. In these methods of the present invention, the compounds according to the invention are preferably formulated prior to administration.

The present invention also provides compositions for use in treating and / or preventing the disorders (preferably a cancer as described herein) referred to herein. Said compositions comprise a therapeutically effective amount of a compound of Formula (I), or a pharmaceutically acceptable salt, or a solvate thereof, and a pharmaceutically acceptable carrier or diluent.

While it is possible for the active ingredient (e.g. a compound of the present invention) to be administered alone, it is preferable to administer it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of the present invention may be prepared by any methods well known in the art of pharmacy, for example, using methods such as those described in, for example, Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own separate pharmaceutical dosage formulation.

Therefore, also disclosed herein is a product comprising, as a first active ingredient a compound according to the invention and as further, as an additional active ingredient one or more anti-cancer agent(s), as a combined preparation for simultaneous, separate or sequential use in the treatment of patients suffering from cancer.

The one or more other anti-cancer agents and the compound according to the present invention may be administered simultaneously (e.g. in separate or unitary compositions) or sequentially, in either order. In an embodiment, the two or more compounds are administered within a period and / or in an amount and / or a manner that is sufficient to ensure that an advantageous or synergistic effect is achieved. It will be appreciated that the preferred method and order of

administration and the respective dosage amounts and regimes for each component of the combination will depend on the particular other anti-cancer agent and the compound of the present invention being administered, their route of administration, the particular condition, in particular tumor, being treated and the particular host being treated.

The following examples further illustrate the present invention.

EXAMPLES

Several methods for preparing the Compounds of this invention are illustrated in the following examples. Unless otherwise noted, all starting materials were obtained from commercial suppliers and used without further purification, or alternatively can be synthesized by a skilled person by using well-known methods.

| **Abbreviation** | **Meaning** |
|---|---|
| **9-BBN** | 9-borabicyclo[3.3.1]nonane dimer |
| **ACN** | acetonitrile |
| **AcOH** | acetic acid |
| **Celite®** | diatomaceous earth |
| **Co** | Compound |
| **Co. No.** | Compound Number |
| **DCM** | dichloromethane |
| **DDQ** | 2,3-Dichloro-5,6-dicyano-1,4-benzoquinone |
| **Dess-Martin periodinane** | 3-oxo-1,3-dihydro-1$\lambda^5$,2-benziodoxole-1,1,1-triyl triacetate |
| **DIBAL** | diisobutylaluminium hydride |
| **Dicalite®** | diatomaceous earth |
| **DIPE** | diisopropyl ether |
| **DIPEA** | *N,N-diisopropylethylamine* |
| **DMF** | *N*,*N*-dimethylformamide |
| **DTBAD** | Di-tert-butyl Azodicarboxylate |
| **eq.** | equivalent(s) |
| **$Et_3N$ or TEA** | triethylamine |
| **EtOAc** | ethyl acetate |
| **EtOH** | ethanol |
| **h** | hour(s) |
| **HPLC** | high performance liquid chromatography |
| **$iPrNH_2$** | isopropylamine |
| **IPA or iPrOH** | isopropanol |
| **mCPBA** | meta-chloroperoxybenzoic acid |
| **KSAc** | potassium thioacetate |
| **Me** | methyl |
| **MeI** | methyl iodide |
| **MeOH** | methanol |
| **2-Me-THF or Me-THF** | 2-methyltetrahydrofuran |
| **MP** | melting point |
| **MsCl** | methanesulfonyl chloride |
| **nBuLi or BuLi** | n-butyllithium |

(continued)

| Abbreviation | Meaning |
|---|---|
| **$NaBH(OAc)_3$** | sodium triacetoxyborohydride |
| **Pd/C** | palladium on carbon |
| **$Pd(Amphos)_2Cl_2$** | dichlorobis[di-tert-butyl(p-dimethylaminophenyl)phosphino]palladium(II) |
| **$Pd_2(dba)_3$** | tris(dibenzylideneacetone)dipalladium(0) |
| **$Pd(dtbpf)Cl_2$** | 1,1'-Bis (di-t-butylphosphino)ferrocene palladium dichloride |
| **$PPh_3$** | triphenylphosphine |
| **pTsOH or PTSA** | p-toluenesulfonic acid |
| **$pTsOH.H_2O$** | p-toluenesulfonic acid monohydrate |
| **rac** | racemic |
| **RP** | reversed phase |
| **SFC** | supercritical fluid chromatography |
| **S-Phos** | 2-dicyclohexylphosphino-2',6'-dimethoxybiphenyl |
| **TBAF** | tetrabutylammonium fluoride |
| **TBDMSCl** | tert-butyldimethylsilyl chloride |
| **TBDPSCl** | tert-butyl(chloro)diphenylsilane |
| **THF** | tetrahydrofuran |
| **TLC** | thin layer chromatography |

As understood by a person skilled in the art, Compounds synthesized using the protocols as indicated may contain residual solvent or minor impurities.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

**Preparation of intermediates**

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

**Intermediate 1**

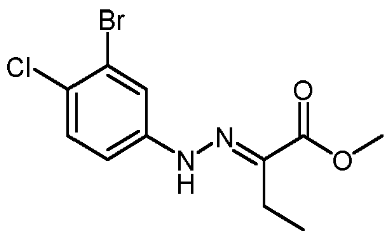

A solution of (3-bromo-4-chlorophenyl)hydrazine (4.655 g, 18.047 mmol) and methyl 2-oxobutanoate (1.02 eq) in HCl (93 mL, 1.25 M in MeOH) was refluxed for 90 min. The reaction mixture was cooled to room temperature and volatiles were removed under reduced pressure to give 5.768 g of Intermediate 1 as a brown oily residue that solidified within minutes, used without further purification in the next step.

## Intermediate 2

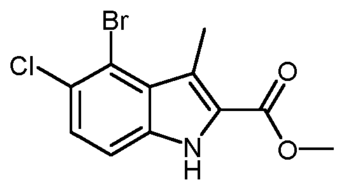

A suspension of Intermediate 1 (5.77 g, 18 mmol) in acetic acid (37 mL) was heated to 70 °C. Sulfuric acid (4.81 mL, 5 eq.) was added dropwise over 10 min (exotherm developed and a precipitate formed). After 15 additional min, the reaction mixture was cooled to room temperature and then to 0 °C by adding ice. The solid precipitate was filtered and washed with water until the filtrate was of neutral pH. The solid was triturated with cold heptane/diisopropylether (8/2, 50 mL) to give an off-white solid. This solid was purified by preparative SFC (Stationary phase: Chiralpak Daicel IG 20 x 250 mm, Mobile phase: $CO_2$, EtOH + 0.4 % $iPrNH_2$) to give Intermediate 2 (1.745 g, 32 %).

## Intermediate 3

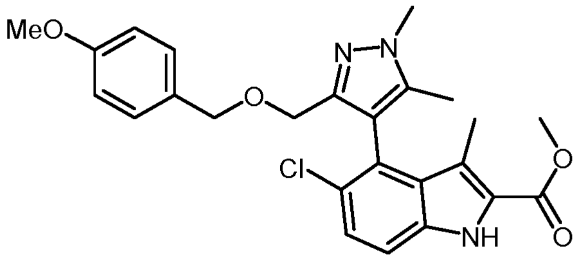

Intermediate 2 (500 mg, 1.65 mmol), 3-(((4-methoxybenzyl)oxy)methyl)-1,5-dimethyl-4-(4,4,5,5-tetramethyl-1,3,2-dioxaborolan-2-yl)-1H-pyrazole (CAS [2143010-90-4]) (800 mg, 1.3 eq.), $Pd_2(dba)_3$ (76 mg, 0.05 eq.), and S-Phos (68 mg, 0.1 eq.) were weighed in a pressure tube under $N_2$. Dioxane (10.5 mL) and a saturated aqueous $NaHCO_3$ solution (4.5 mL) were added and the mixture was heated at 100 °C for 2 h. The reaction mixture was cooled to room temperature, diluted with EtOAc (40 mL) and water (40 mL). The organic layer was separated and the aqueous one was extracted with EtOAc (40 mL). The combined organic layer was dried over $MgSO_4$, filtered and evaporated. The crude mixture was purified by flash chromatography on silica gel (40 g, gradient: from heptane 100 % up to heptane/EtOAc 4/6). Intermediate 3 (790 mg, 89 %) was obtained as a yellowish oil that solidified on standing. Intermediate 3 was used without further purification in the next reaction step.

## Intermediate 4

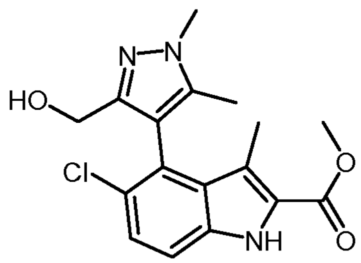

Trifluoromethanesulfonic acid (0.888 mL, 5 eq.) was added to a solution of Intermediate 3 (1080 mg, 2 mmol) in DCM (25 mL). The reaction mixture was stirred at room temperature for 1 h. The reaction mixture was diluted with DCM (100 mL) and treated with saturated aqueous $NaHCO_3$ (30 mL). The organic layer was separated and the aqueous one was extracted with DCM (50 mL x 3). The combined organic layer was dried over $MgSO_4$, filtered, and evaporated. Intermediate 4 (625 mg, 89 %) was obtained as a yellowish solid, used without further purification in the next step.

## Intermediate 5, Intermediate 5a and Intermediate 5b

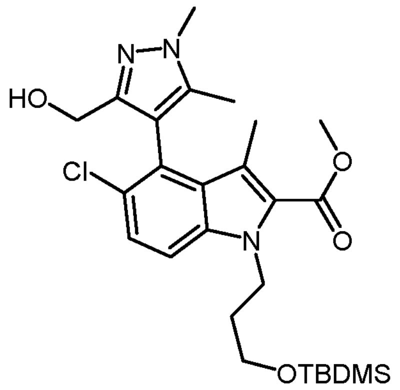

Intermediate 5 - mixture of atropisomers

Intermediate 5a - $S_a$ or $R_a$; one atropisomer but absolute stereochemistry undetermined

Intermediate 5b - $R_a$ or $S_a$; one atropisomer but absolute stereochemistry undetermined

Cesium carbonate (732 mg, 1.25 eq.) was added to a solution of Intermediate 4 (625 mg, 1.79 mmol) in DMF (10 mL) under nitrogen atmosphere. (3-Bromopropoxy)(tert-butyl)dimethylsilane (0.458 mL, 1.1 eq.) was added dropwise and the reaction mixture was stirred at room temperature overnight. The reaction mixture was diluted with EtOAc (100 mL) and water (50 mL). The organic layer was separated and washed with brine (2 x 30 mL). The combined aqueous layer was extracted with EtOAc (50 mL). The combined organic layer was then dried over $MgSO_4$, filtered and evaporated. The crude mixture was purified by flash chromatography on silica gel (40 g, gradient: from heptane 100 % up to EtOAc 100 %) to afford Intermediate 5 (360 mg, 38 %) as a white solid.

A sample of Intermediate 5 (25 g, 48.4 mmol) was separated into its atropoisomers by preparative SFC, (Chiralpak Diacel column, AD 20 x 250 mm, mobile phase: $CO_2$, EtOH + 0.4 % $iPrNH_2$) to afford the two atropoisomers:

Intermediate 5a (11 g, 43.7 %)

OR: -29.3 ° (589 nm, 20 °C, c 0.29 w/v %, DMF).

Intermediate 5b (10.7 g, 42.5 %)

OR: + 19.4 ° (589 nm, 20 °C, c 0.32 w/v %, DMF).

## Intermediate 6

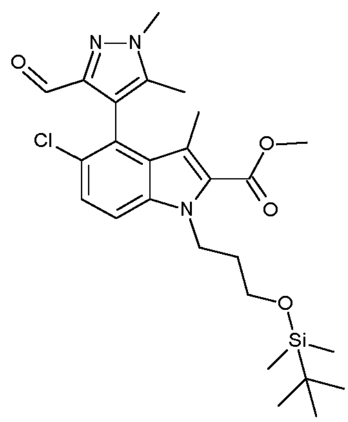

$S_a$ or $R_a$; one atropisomer but absolute stereochemistry undetermined

Dess-Martin periodinane (CAS [87413-09-0], 10.2 g, 24.03 mmol) was added at 0 °C to a stirred solution of Intermediate 5a (10 g, 19.23 mmol) in DCM (25 mL). The ice-water bath was removed and the reaction mixture was stirred at room temperature for 1 h, then it was diluted with DCM (200 mL) and treated with 500 mL of a 1:1 mixture of aqueous $NaHCO_3$ saturated solution (250 mL) and $Na_2S_2O_3$ (10 %, 250 mL). The biphasic mixture was stirred for 30 min, until it became clear, and the layers were separated. The organic layer was washed twice with saturated aqueous $NaHCO_3$, then with brine, dried over $MgSO_4$, filtered, and evaporated to give Intermediate 6 (10.4 g, yield: 92 %), used without further purification.

**Intermediate 7a and Intermediate 7b**

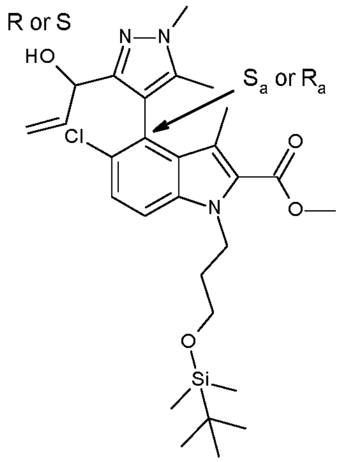

Intermediate 7a

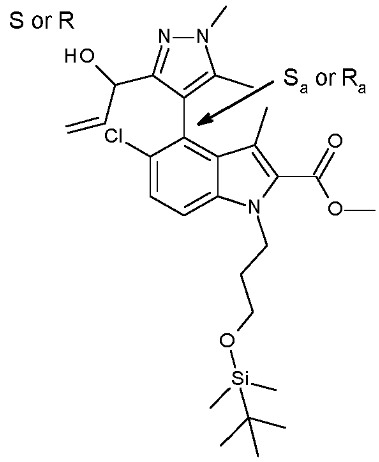

Intermediate 7b

For both compounds, pure stereoisomers but absolute stereochemistry undetermined Vinylmagnesium bromide (CAS [1826-67-1], 17 mL, 1 M in 2-Me-THF, 17 mmol) was added dropwise to a stirred and cold (-78 °C) solution of Intermediate 6 (5.85 g, 11.06 mmol) in anhydrous THF (64.5 mL). The reaction mixture was stirred at this temperature for 1 h, then it was quenched with saturated aqueous $NH_4Cl$ solution and allowed to reach room temperature. The mixture was diluted with EtOAc and water, the layers were separated, and the aqueous layer was extracted twice with EtOAc. The combined organic layer was washed with water followed by brine, dried over $MgSO_4$, filtered, and concentrated in vacuo. The residue was purified by preparative SFC (Chiralpak Daicel column, IG 20 x 250 mm, mobile phase: $CO_2$, iPrOH + 0.4 % $iPrNH_2$), to afford:

Intermediate 7a (2.64 g, 44 % yield); O.R.: + 18.43 ° (589 nm, 20 °C, c 0.23 w/v %, DMF)
Intermediate 7b(1.54 g, 26 % yield); O.R.: ND

**Intermediate 8**

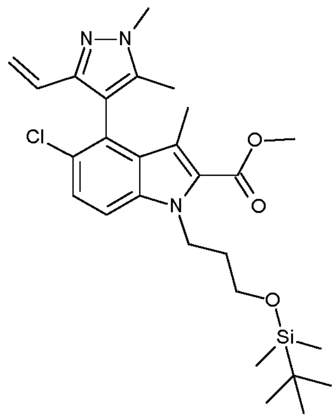

$S_a$ or $R_a$; one atropisomer but absolute stereochemistry undetermined

nBuLi (2.78 mL, 2.5 M, 6.95 mmol) was added at -78 °C to a suspension of methyltriphenylphosphonium bromide (3.2 g, 8.94 mmol) in anhydrous THF (52 mL) under nitrogen atmosphere. The resulting reaction mixture was stirred at room temperature for 1 h. Then a solution of Intermediate 6 (3.1 g, 4.97 mmol) in anhydrous THF (24 mL) was added dropwise to the reaction mixture over 30 min by syringe pump. The reaction mixture was stirred at room temperature for 4 h, then it was diluted with EtOAc and water. The organic layer was separated, then washed with brine, and the aqueous layer was extracted with EtOAc (x 3). The combined organic layer was dried over $MgSO_4$, filtered and concentrated under reduced pressure. The residue was purified by flash column chromatography over silica gel (Heptane/EtOAc, 100/0 to 0/100) to afford Intermediate 8 (2.52 g, yield: 76 %) as a pale yellow oil.

## Intermediate 9

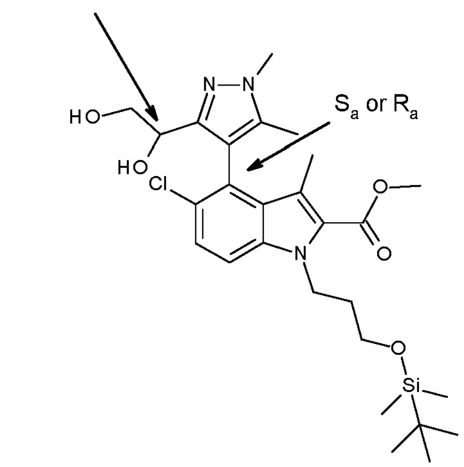


Intermediate 9 - $S_a$ or $R_a$; one atropisomer but absolute stereochemistry undetermined AD-mix-alpha (9 g) and methanesulfonamide (580 mg, 6.1 mmol, 1.25 eq.) were suspended in water (57 mL) and a solution of Intermediate 8 (2.52 g, 4.88 mmol) in tBuOH (56 mL) was added at room temperature. The resulting mixture was stirred vigorously for 15 h. Additional AD-mix-alpha (1 g) was added and the reaction mixture was stirred at room temperature for 4 h, then it was quenched by adding a mixture of 10 % aqueous $Na_2S_2O_3$ and saturated aqueous $NaHCO_3$. The resulting mixture was stirred for 5 min, and then diluted with brine. The layers were separated and the aqueous layer was extracted with EtOAc (x 4). The combined organic layer was dried over $MgSO_4$, filtered, and evaporated. The residue was purified by flash column chromatography over silica gel (DCM/MeOH, 100/0 to 90/10) to afford Intermediate 9 (1.92 g, yield: 72 %) as a pale yellow foam.

## Intermediate 10a and Intermediate 10b

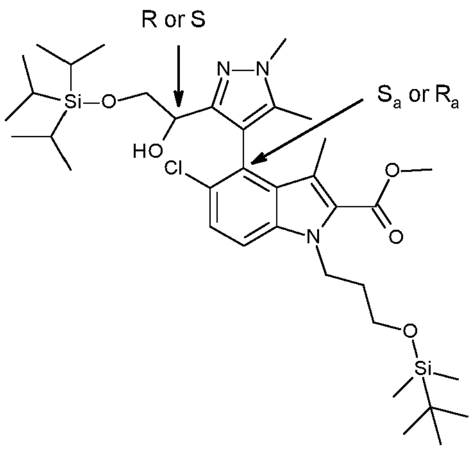


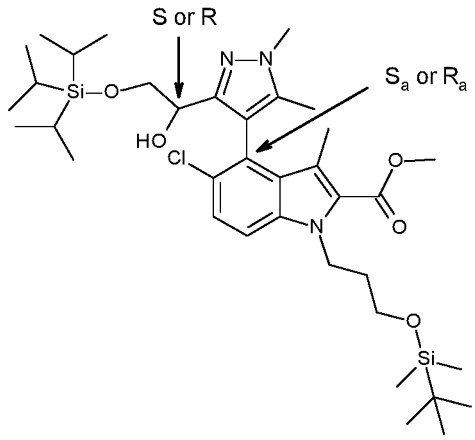


**Intermediate 10a** **Intermediate 10b**

For both compounds, pure stereoisomers but absolute stereochemistry undetermined Triisopropylsilyl chloride

(0.92 mL, 4.19 mmol, 1.2 eq.) was added dropwise at 0 °C to a solution of Intermediate 9 (1.92 g, 3.49 mmol), imidazole (713 mg, 10.5 mmol, 3 eq.) and DMAP (128 mg, 1.05 mmol, 0.3 eq.) in anhydrous DCM (22 mL) under nitrogen atmosphere. The reaction mixture was stirred at 0 °C for 5 h, then it was quenched by adding brine. The layers were separated and the aqueous layer was extracted with DCM (x 3). The combined organic layer was dried over $MgSO_4$, filtered, and evaporated. The residue was purified by flash column chromatography on silica gel (hept/EtOAc, 100/0 to 0/100), followed by preparative HPLC (Stationary phase: RP XBridge Prep C18 OBD -10 μm, 30 x 150 mm, Mobile phase: 0.25 % $NH_4HCO_3$ solution in water, $CH_3CN$) to afford Intermediate 10a (1.03 g, yield: 42 %) and Intermediate 10b (695 mg, yield: 28 %), both as colorless oils.

**Intermediate 11**

mixture of R and S

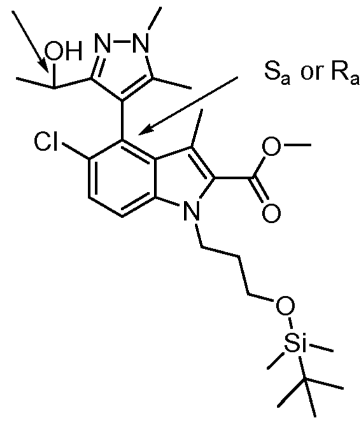

$S_a$ or $R_a$

Methylmagnesium bromide (3.4 M in MeTHF, 6.52 mL, 22.16 mmol, 3 eq.) was added dropwise over 10 min to a solution of Intermediate 6 (4.3 g, 7.39 mmol) in anhydrous THF (66 mL) under nitrogen atmosphere at room temperature. The reaction mixture was stirred at room temperature for 2.5 h. The reaction was quenched by adding saturated aqueous $NH_4Cl$. The reaction mixture was diluted with water and EtOAc. The layers were separated and the organic layer was washed with brine (x 1). The combined aqueous layer was extracted with EtOAc (x 3). The combined organic layer was dried over $MgSO_4$, filtered, and concentrated in vacuo. The residue was purified by flash column chromatography ($SiO_2$, 120 g RediSep, DCM/MeOH, 100/0 to 90/10) to afford Intermediate 11(6.75 g, yield: 90 %) as a pale yellow oil.

**Intermediate 12**

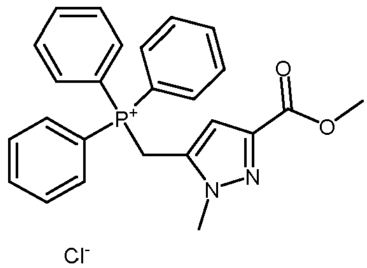

A solution of 5-(chloromethyl)-1-methyl-1H-pyrazole-3-carboxylic acid, methyl ester (CAS [2245938-86-5], 24 g, 0.127 mol) and $PPh_3$ (37 g, 1 eq.) in ACN (250 mL) was stirred under reflux for 16 h. The white suspension was concentrated in vacuo and triturated with EtOAc (100 mL). The resulting solid was collected by filtration and dried to afford Intermediate 12 (54.8 g, yield: 96 %) as a white solid.

## Intermediate 13

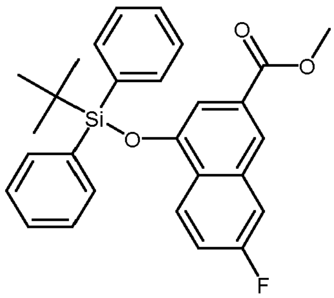

TBDPSCl (14.66 g, 1.5 eq.) was added to a solution of methyl 7-fluoro-4-hydroxy-2-naphthoate (CAS [2092726-85-5], 8 g, 35.555 mmol) and imidazole (7.26, 3 eq.) in DCM (500 mL), cooled to 0 °C under nitrogen atmosphere. Once the addition was complete, the reaction was stirred at room temperature overnight. The reaction was quenched by addition of water (100 mL). The mixture was extracted with EtOAc (3 x 200 mL). The combined organic layer was dried over $Na_2SO_4$, filtered, and concentrated to afford a yellow oil. This oil was purified by flash column chromatography on silica gel (petroleum ether:EtOAc - 1:0 to 1:1) to afford Intermediate 13 (14 g, yield: 86 %) as a yellow oil.

## Intermediate 14

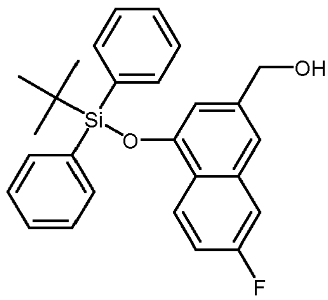

$LiAlH_4$ (1.39 g, 1.2 eq.) was added slowly to a solution of Intermediate 13 (14 g, 30.528 mmol) in THF (200 mL), cooled to 0 °C under nitrogen atmosphere. Once the addition was complete the reaction mixture was stirred at 0 °C for 2 h. The reaction was quenched by slow addition of water (2 mL) followed by a 10 % aqueous NaOH solution (2 mL) at 0 °C. The heterogeneous mixture was filtered, and the filter cake was washed with DCM (200 mL). The filtrate was evaporated and the residue was purified by flash column chromatography on silica gel (petroleum ether:EtOAc - 1:0 to 1:1) to give Intermediate 14 (12 g, yield: 90 %) as a yellow solid.

## Intermediate 15

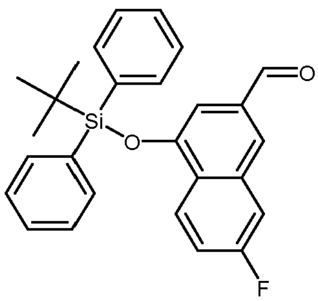

$MnO_2$ (29.074 g, 12 eq.) was added to a solution of Intermediate 14 (12 g, 27.869 mmol) in DCM (200 mL) at room temperature. The resulting solution was stirred at room temperature overnight. The reaction mixture was filtered, and the filtrate was concentrated. The residue was purified by flash column chromatography over silica gel (eluent: petroleum ether/EtOAc, 100/0 to 50/50) to afford Intermediate 15 (12 g, yield: 99 %) as a yellow oil.

## Intermediate 16

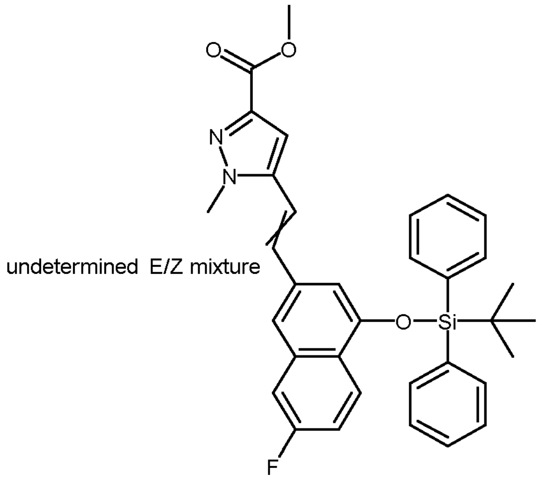


NaH (60 % in mineral oil, 1.448 g, 1.3 eq.) was added to a suspension of Intermediate 12 (13.812 g, 1.1 eq.) in THF (200 mL) at 0 °C. The resulting solution was stirred at this temperature for 1 h before being cooled to -30 °C. Intermediate 15 (12 g, 27.847 mmol) was added slowly to the solution while maintaining the temperature between -20 °C and -30 °C. Once the addition was complete, the reaction was stirred at -30 °C for 2 h. The reaction was quenched by slow addition of water (100 mL). The mixture was extracted with DCM (3 x 300 mL). The combined organic layer was dried over $Na_2SO_4$, filtered, and concentrated under reduced pressure. The crude product was purified by column chromatography on silica gel (petroleum ether:EtOAc - 1:0 to 1:1) to afford Intermediate 16 (13 g, yield: 82 %) as a white solid.

## Intermediate 17

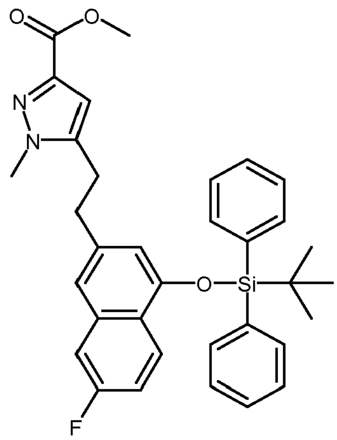

A solution of Intermediate 16 (13 g, 23.02 mmol) in MeOH (75 mL) and THF (75 mL) was hydrogenated at 25 °C (15 psi $H_2$) in the presence of Pd/C (2 g; 10 %). The reaction mixture was stirred for 16 h. After uptake of $H_2$ (1 eq.), the catalyst was filtered off and the filtrate was evaporated to afford Intermediate 17 (13 g, yield: 100 %) as a colorless oil.

## Intermediate 18

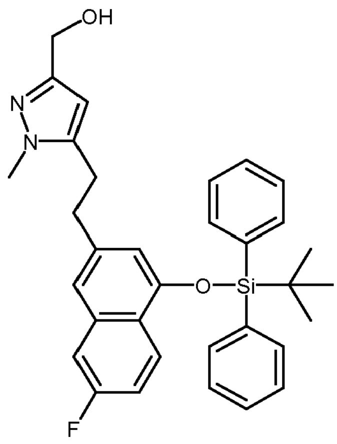

$LiAlH_4$ (1.045 g, 1.2 eq.) was added portionwise to a solution of Intermediate 17 (13 g, 22.94 mmol) in THF (200 mL) at 0 °C, under nitrogen atmosphere. The reaction mixture was stirred at 0 °C for 2 h. Water (1 mL) was then added dropwise, followed by a 10 % aqueous NaOH solution (1 mL), at 0 °C. The reaction mixture was filtered, the filter cake was washed with DCM (200 mL), and the filtrate was evaporated. The crude product was purified by flash column chromatography over silica gel (eluent: petroleum ether/EtOAc, 100/0 to 0/100) to afford Intermediate 18 (10.4 g, yield: 84 %) as a white solid.

## Intermediate 19

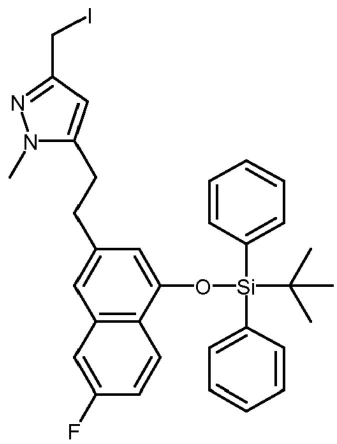

DIPEA (7 mL, 0.75 g/mL, 40.62 mmol), followed by MsCl (2.5 mL, 1.48 g/mL, 32.3 mmol) were added at 0 °C to a solution of Intermediate 18 (10 g, 18.5 mmol) in THF (200 mL) and the reaction mixture was stirred at room temperature for 0.5 h. NaI (10 g, 66.7 mmol) was added to the reaction mixture, which was then stirred at room temperature for 1.5 h. The reaction mixture was diluted with EtOAc and washed with water. The aqueous layer was back-extracted with EtOAc (x 1), the combined organic layer was washed with brine (x 1), dried over $MgSO_4$ and concentrated under reduced pressure. The resulting residue, (11.55 g, yield: 96 %) was purified by flash column chromatography on silica gel, using as eluent a gradient nheptane/EtOAc, 100/0 to 80/20, to provide Intermediate 19 (9.5 g, yield: 79 %).

## Intermediate 20

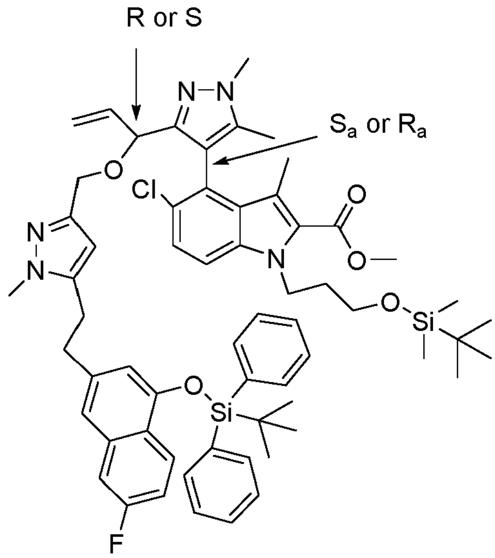

Pure stereoisomer but absolute stereochemistry undetermined

A solution of Intermediate 7a (220 mg, 0.4 mmol) and Intermediate 19 (483 mg, 0.75 mmol, 1.85 eq.) in THF (3 mL) was added at room temperature under nitrogen atmosphere to a previously nitrogen-degassed slurry of NaH (60 % dispersion in mineral oil, 32 mg, 0.81 mmol, 2 eq.) in THF (0.5 mL). The reaction mixture was stirred at room temperature for 1.5 h, then it was cooled to 0 °C, and the reaction was quenched by adding aqueous saturated $NH_4Cl$. The aqueous layer was extracted with EtOAc (x 3). The combined organic layer was washed with brine, dried over $MgSO_4$, filtered, and evaporated. The residue was purified by flash column chromatography on silica gel (DCM/MeOH, 100/0 to 95/5) to provide Intermediate 20 (290 mg, yield: 67 %).

## Intermediate 21

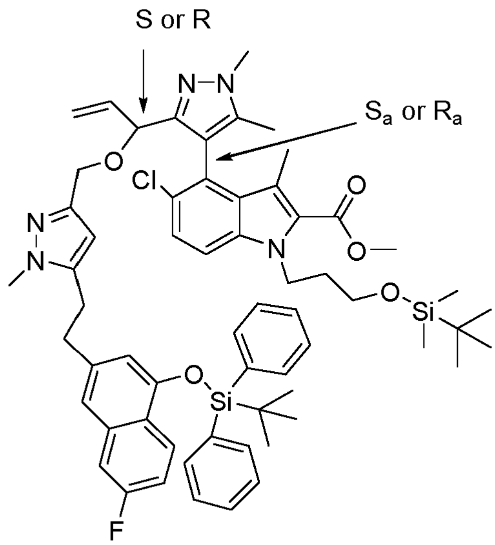

Pure stereoisomer but absolute stereochemistry undetermined

Intermediate 21 was prepared by an analogous reaction protocol as Intermediate 20, starting from Intermediate 7b instead of Intermediate 7a.

## Intermediate 22

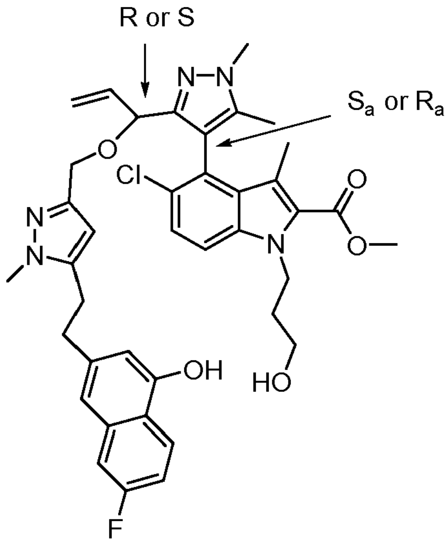

Pure stereoisomer but absolute stereochemistry undetermined

TBAF (0.68 mL, 1 M in THF, 0.68 mmol, 2.5 eq.) was added to a solution of Intermediate 20 (290 mg, 0.27 mmol) in THF (2 mL) at 0 °C. The reaction mixture was then stirred at room temperature for 3 h. The reaction mixture was cooled to 0 °C and was treated with saturated aqueous $NH_4Cl$ (5 mL), stirred for 15 min, then extracted with EtOAc (3 x). The combined organic layer was washed with brine, dried over $MgSO_4$, filtered, and evaporated. The residue was purified by flash column chromatography on silica gel (DCM/MeOH, 100/0 to 95/5) to provide Intermediate 22 (190 mg, yield: 98 %) as a white fluffy solid, containing 3% diastereomer (Intermediate 23), as observed by SFC analysis.

## Intermediate 23

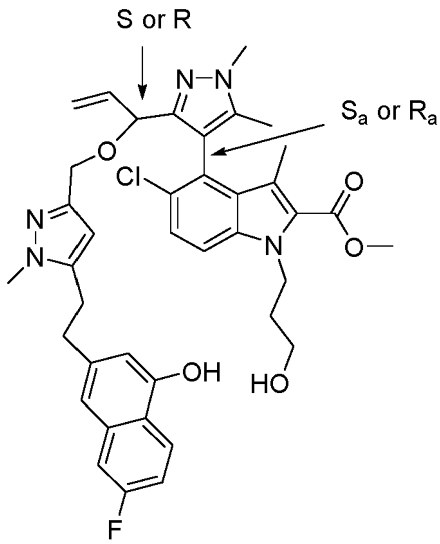

Pure stereoisomer but absolute stereochemistry undetermined

Intermediate 23 was prepared by an analogous reaction protocol as Intermediate 22, starting from Intermediate 21 instead of Intermediate 20.

## Intermediate 24

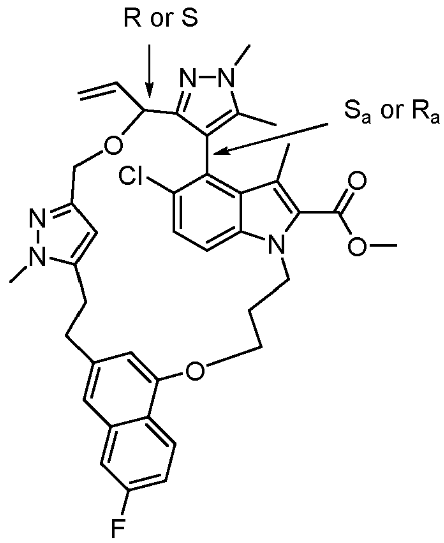


Pure stereoisomer but absolute stereochemistry undetermined

A thoroughly nitrogen-degassed solution of Intermediate 22 (100 mg, 0.14 mmol) and di-tert-butyl azodicarboxylate (129 mg, 0.56 mmol, 4 eq.) in THF (0.8 mL) and toluene (5 mL) was added by syringe pump (0.1 mL/min) to a thoroughly nitrogen-degassed solution of triphenylphosphine (148 mg, 0.56 mmol, 4 eq.) in toluene (5 mL) stirring at 70 °C under nitrogen atmosphere. Once the addition was complete, the reaction mixture was stirred for 10 minutes at 70 °C, then it was cooled to room temperature and solvents were evaporated. The residue was purified by flash column chromatography on silica gel (heptane/EtOAc, 0/100 to 100/0, then DCM/MeOH, 100/0 to 95/5) to afford Intermediate 24 (64 mg, yield: 66 %) as an off-white solid.

## Intermediate 25

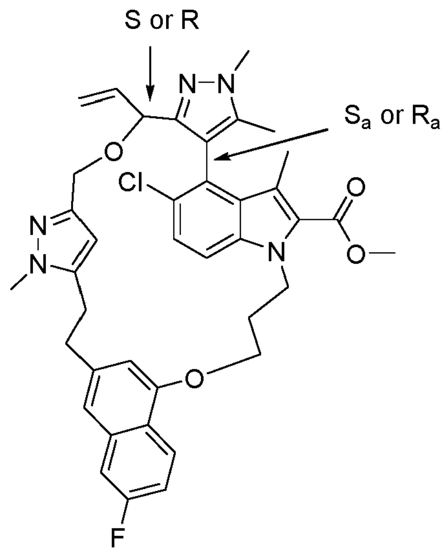


Pure stereoisomer but absolute stereochemistry undetermined

Intermediate 25 was prepared by an analogous reaction protocol as Intermediate 24, starting from Intermediate 23 instead of Intermediate 22.

## Intermediate 26

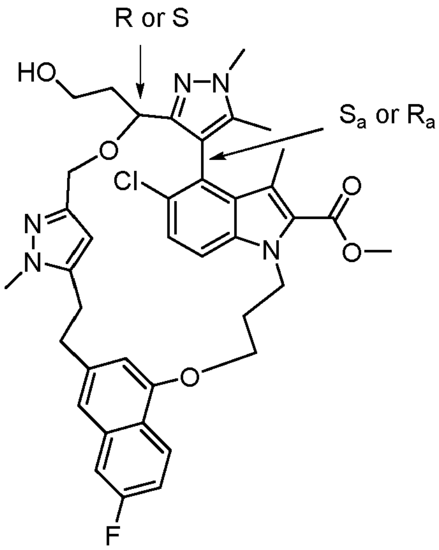

Pure stereoisomer but absolute stereochemistry undetermined

9-borabicyclo[3.3.1]nonane dimer (9-BBN) (0.43 mL, 0.5 M in THF, 0.215 mmol, 5 eq.) was added dropwise at 0 °C to a solution of Intermediate 24 (30 mg, 0.043 mmol) in THF (1.5 mL). The reaction mixture was stirred at 40 °C for 2 h, then it was cooled to 0 °C and was treated with water (0.75 mL) and sodium perborate tetrahydrate (0.034 g, 0.215 mmol, 5 eq.). The resulting mixture was stirred at 35 °C overnight. Another portion of sodium perborate tetrahydrate (0.034 g, 0.215 mmol, 5 eq.) was added and the reaction mixture was stirred for another 5 h at 35 °C. The reaction mixture was diluted with EtOAc and filtered. The biphasic filtrate was separated, and the aqueous layer was extracted with EtOAc. The combined organic layers were washed with brine, dried over $MgSO_4$, filtered, and concentrated in vacuo. The residue was purified by flash column chromatography (DCM/MeOH 100/0 to 95/5) to provide Intermediate 26 (15 mg, yield: 49 %).

## Intermediate 27

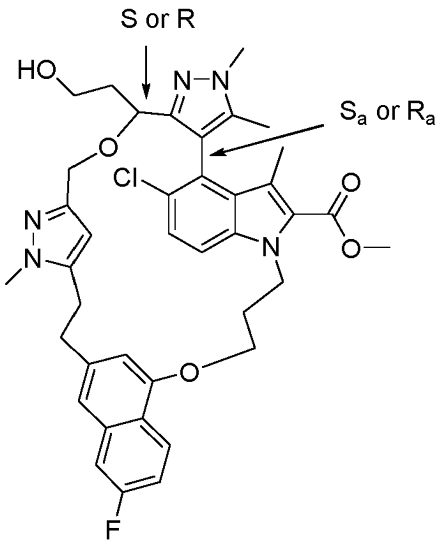

Pure stereoisomer but absolute stereochemistry undetermined

Intermediate 27 was prepared by an analogous reaction protocol as Intermediate 26, starting from Intermediate 25 instead of Intermediate 24.

## Intermediate 28

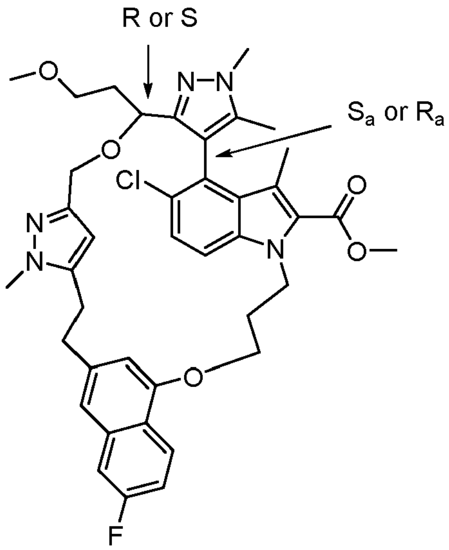


Pure stereoisomer but absolute stereochemistry undetermined

NaH (60 % dispersion in mineral oil, 1 mg, 0.027 mmol, 1.3 eq.) was added to a previously nitrogen-degassed solution of Intermediate 26 (15 mg, 0.021 mmol) and MeI (2 µL, 0.032 mmol, 1.5 eq.) in THF (0.5 mL) at room temperature under nitrogen atmosphere. The reaction mixture was stirred at room temperature for 2.5 h, then it was cooled to 0 °C, quenched with saturated aqueous $NH_4Cl$ (2 mL) and extracted with EtOAc (3 x). The combined organic layers were washed with brine, dried over $MgSO_4$, filtered, and evaporated under reduced pressure, to provide crude Intermediate 28 (17 mg, quantitative yield), used without further purification.

## Intermediate 29

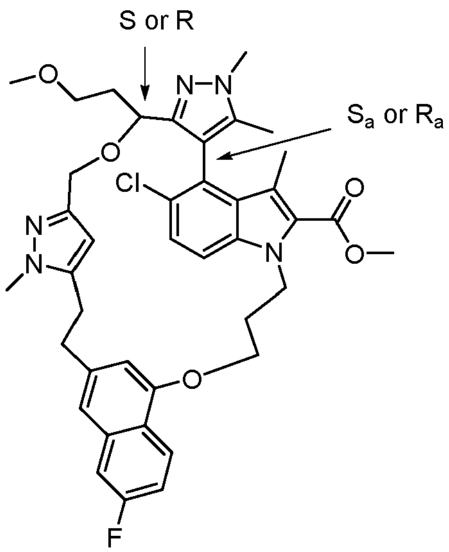


Pure stereoisomer but absolute stereochemistry undetermined

Intermediate 29 was prepared by an analogous reaction protocol as Intermediate 28, starting from Intermediate 27 instead of Intermediate 26. Crude Intermediate 28 was purified by flash column chromatography on silica gel (DCM/MeOH, 100/0 to 97/3), to provide Intermediate 29 as a white foam.

Mixture of Intermediate 30 and Intermediate 27

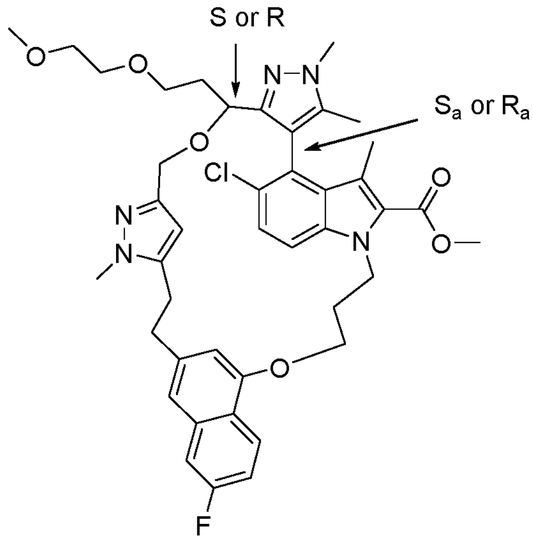

Intermediate 30

Pure stereoisomer but absolute stereochemistry undetermined

NaH (60 % dispersion in mineral oil, 3 mg, 0.08 mmol, 1.3 eq.) was added to a previously degassed solution of Intermediate 27 (44 mg, 0.062 mmol) and 2-bromoethyl methyl ether (9 μL, 0.092 mmol, 1.5 eq.) in THF (0.5 mL) at room temperature under nitrogen atmosphere. The reaction mixture was stirred at room temperature for 1 h, then more 2-bromoethyl methyl ether (150 μL, 1.6 mmol, 26 eq.) was added to the reaction mixture, which was then stirred at 50 °C for 2 days. The reaction mixture was cooled to 0 °C and the reaction was quenched by adding saturated aqueous $NH_4Cl$ (2 mL). The mixture was extracted with EtOAc (3 x). The combined organic layers were washed with brine, dried over $MgSO_4$, filtered, and evaporated to provide a mixture of Intermediate 30 and Intermediate 27 (30 mg of the mixture), used as such in the next step.

## Intermediate 31

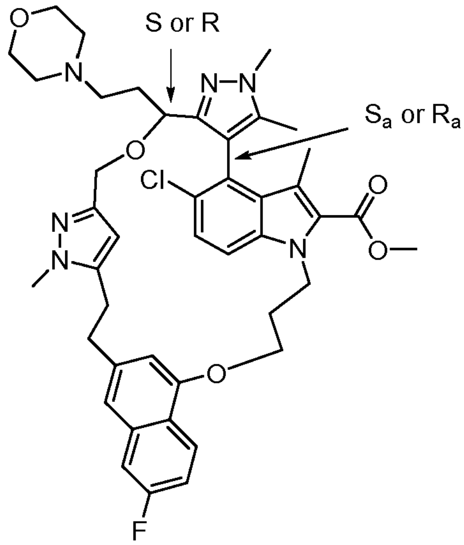

Pure stereoisomer but absolute stereochemistry undetermined

Methanesulfonyl chloride (0.051 mL, 0.66 mmol, 20 eq.) was added dropwise to a solution of Intermediate 27 (25 mg, 0.033 mmol) and triethylamine (114 μL, 0.82 mmol, 25 eq.) in THF (1 mL) at 0 °C. The reaction mixture was stirred for 1 h at room temperature. Morpholine (86 μL, 0.99 mmol, 30 eq.) was then to the reaction mixture at 0 °C and the reaction mixture was then stirred at 45 °C for 12 h. The reaction mixture was cooled to room temperature, treated with saturated aqueous $NaHCO_3$, and extracted with EtOAc (3 x). The combined organic layer was washed with brine, dried over $MgSO_4$, filtered, and evaporated under reduced pressure. The residue was purified by flash column chromatography on silica gel (DCM/MeOH, 100/0 to 95/5, then DCM/$NH_3$ (7 M in MeOH) 97/3 to 95/5) to provide Intermediate 31 (15 mg, yield: 58 %).

## Intermediate 32

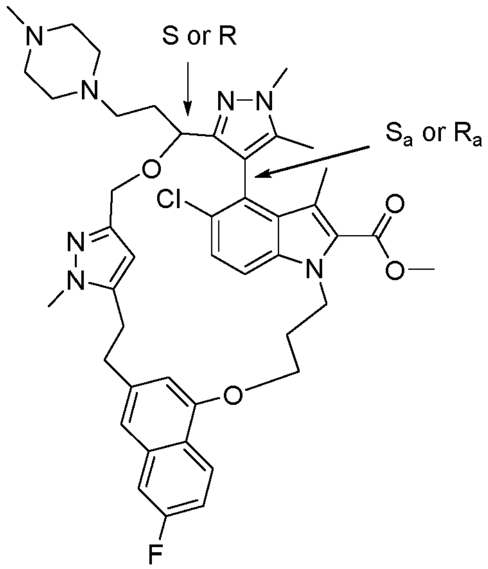

Pure stereoisomer but absolute stereochemistry undetermined

Intermediate 32 was prepared by an analogous reaction protocol as Intermediate 31, starting from N-methylpiperazine instead of morpholine.

## Intermediate 33

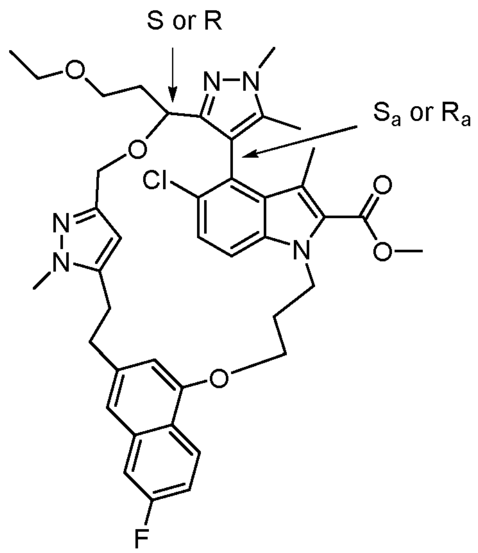

Pure stereoisomer but absolute stereochemistry undetermined

Intermediate 33 was prepared by an analogous reaction protocol as Intermediate 28, starting from Intermediate 27 instead of Intermediate 26 and iodoethane instead of iodomethane.

**Intermediate 34, Intermediate 34a, Intermediate 34b**

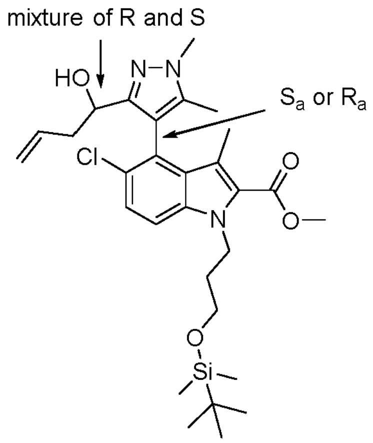


**Intermediate 34**

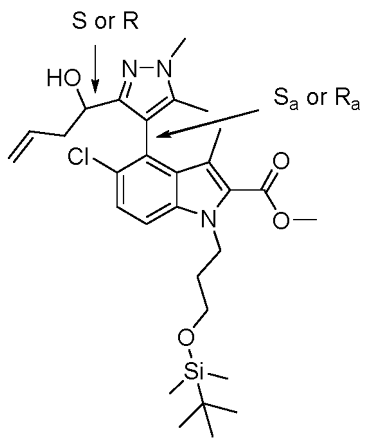


**Intermediate 34a**

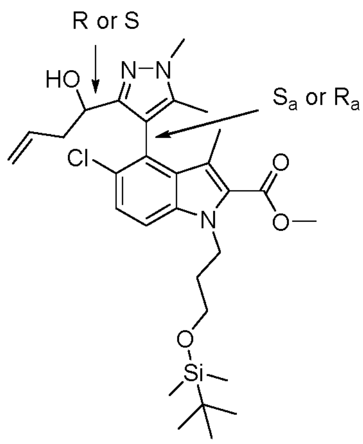


**Intermediate 34b**

Intermediate 34a and Intermediate 34b are pure stereoisomers but absolute stereochemistry is undetermined

Allylmagnesium bromide (1 M in $Et_2O$, 4 mL, 4 mmol, 1.25 eq.) was added dropwise to a solution of Intermediate 6 (1.65 g, 3.2 mmol) in anhydrous THF (25 mL) at -78 °C. The solution was stirred at this temperature for 1 h, then another portion of allylmagnesium bromide (1 M in $Et_2O$, 1 mL, 1 mmol, 0.3 eq.) was added at - 78 °C, and the reaction mixture was stirred at this temperature for 1 h. The reaction mixture was then allowed to reach room temperature and saturated aqueous $NH_4Cl$ (4 mL) was added. The mixture was diluted with EtOAc (15 mL) and water (5 mL). The layers were separated, and the aqueous layer was extracted with EtOAc (2 x). The combined organic layer was washed with water, brine, dried over $MgSO_4$, filtered, and concentrated in vacuo. The residue was purified by flash column chromatography on silica gel (heptane/EtOAc 100/0 to 75/25 to 65/35) to give Intermediate 34b (539 mg, yield: 30 %), Intermediate 34 (579 mg, 32 %), and Intermediate 34a (170 mg, yield: 9 %).

**Intermediate 35**

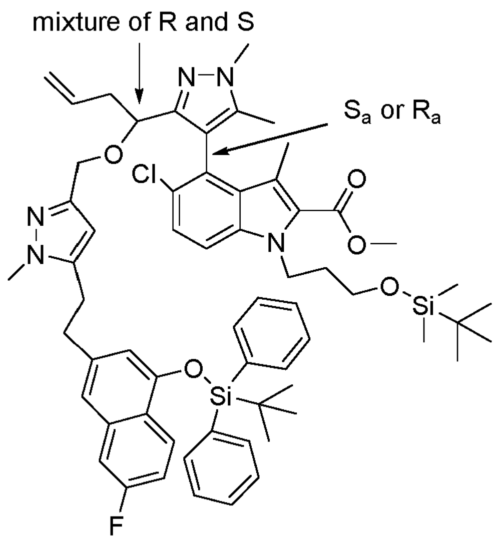

A solution of Intermediate 34 (539 mg, 0.96 mmol) and Intermediate 19 (1.073 g, 1.654 mmol, 1.6 eq.) in THF (10 mL) was added to a previously degassed slurry of NaH (60 % dispersion in mineral oil, 62 mg, 1.55 mmol) in THF (0.5 mL) at room temperature under nitrogen atmosphere. The reaction mixture was stirred at room temperature for 1 h, then it was cooled to 0 °C and the reaction was quenched by adding saturated aqueous $NH_4Cl$. The layers were separated, and the aqueous layer was extracted with EtOAc (x 3). The combined organic layers were washed with brine, dried over $MgSO_4$, filtered, and evaporated. The residue was purified by flash column chromatography on silica gel (DCM/MeOH, 100/0 to 95/5) to provide Intermediate 35 (104 mg, yield: 12 %).

## Intermediate 36

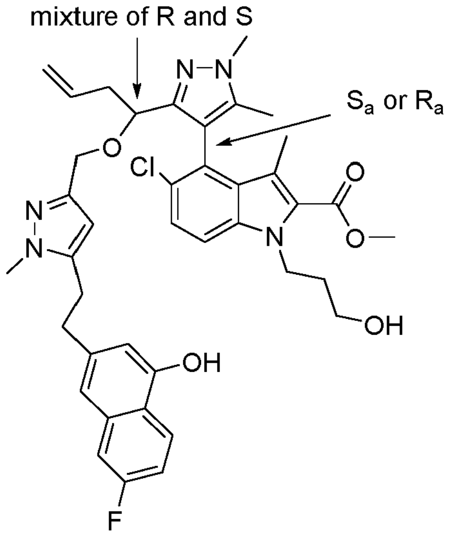


TBAF (1 M in THF, 0.68 mL, 0.68 mmol, 2.5 eq.) was added to a solution of Intermediate 35 (290 mg, 0.27 mmol) in THF (2 mL) at 0 °C. The reaction mixture was stirred at room temperature for 5 h. The reaction mixture was cooled to 0 °C and treated with saturated aqueous $NH_4Cl$ (5 mL), stirred for 15 min, then extracted with EtOAc (3 x). The combined organic layers were washed with brine, dried over $MgSO_4$, filtered, and evaporated. The residue was purified by flash column chromatography on silica gel (DCM/MeOH, 100/0 to 95/5) to provide Intermediate 36 (190 mg, yield: 97 %).

## Intermediate 37

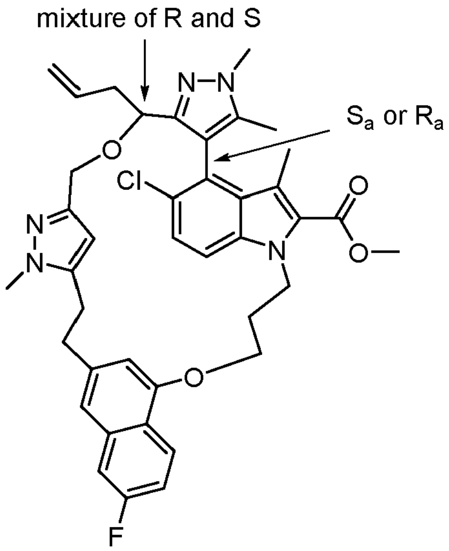


A nitrogen-degassed solution of Intermediate 36 (100 mg, 0.14 mmol) and di-tert-butyl azodicarboxylate (129 mg, 0.56 mmol, 4 eq.) in THF (1 mL) and toluene (5 mL) was added by syringe pump (0.1 mL/min) to a solution of triphenylphosphine (148 mg, 0.56 mmol) in toluene (5 mL), previously degassed with nitrogen, at 70 °C under nitrogen atmosphere. Once the addition was complete, the reaction mixture was stirred for 15 min at 70 °C, then it was cooled to

room temperature and the solvents were evaporated. The residue was purified by flash column chromatography on silica gel (heptane/EtOAc gradient, 0/100 to 100/0 then DCM/MeOH, 100/0 to 95/5) to afford Intermediate 37 (311 mg, yield: 66 %).

**Intermediate 38 and Intermediate 39**

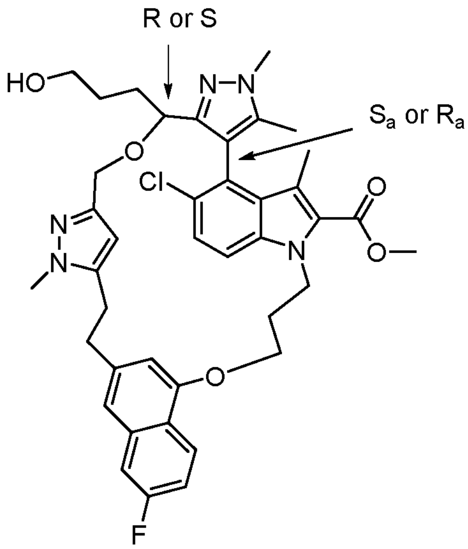


**Intermediate 38**

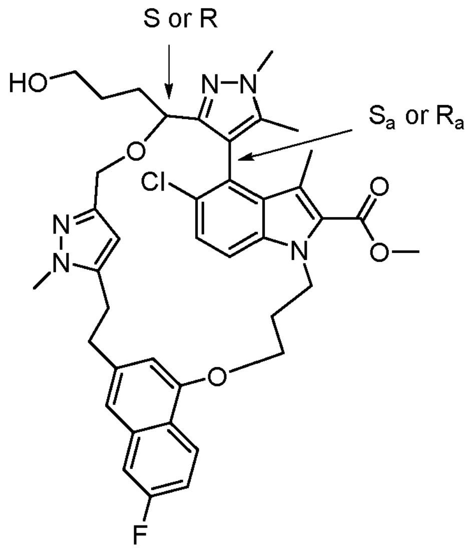


**Intermediate 39**

Both compounds are pure stereoisomers but absolute stereochemistry undetermined

9-borabicyclo[3.3.1]nonane dimer (0.5 M in THF, 4.38 mL, 2.19 mmol, 5 eq.) was added to a solution of Intermediate 37 (311 mg, 0.44 mmol) in THF (4 mL) at 0 °C and the mixture was stirred at 40 °C for 2 h. After cooling to 0 °C, a mixture of sodium perborate tetrahydrate (1.01 g, 9.57 mmol, 15 eq.) in water (9 mL) was added and the mixture was stirred at 27 °C for 2 days. The reaction mixture was diluted with EtOAc and filtered. The layers were separated and the aqueous layer was extracted with EtOAc. The combined organic layers were washed with brine, dried over $MgSO_4$, filtered, and concentrated in vacuo. The residue was purified by flash column chromatography on silica gel (DCM/MeOH, 100/0 to 95/5) followed by preparative SFC (Stationary phase: Chiralpak Daicel ID 20 x 250 mm, Mobile phase: $CO_2$, iPrOH + 0.4 % $iPrNH_2$) to give Intermediate 38 (62 mg, 19 %) and Intermediate 39 (148 mg, 46 %).

Alternatively, Intermediate 38 and Intermediate 39 can be prepared separately following the same sequence, from pure Intermediate 34b and Intermediate 34a, respectively.

## Intermediate 40

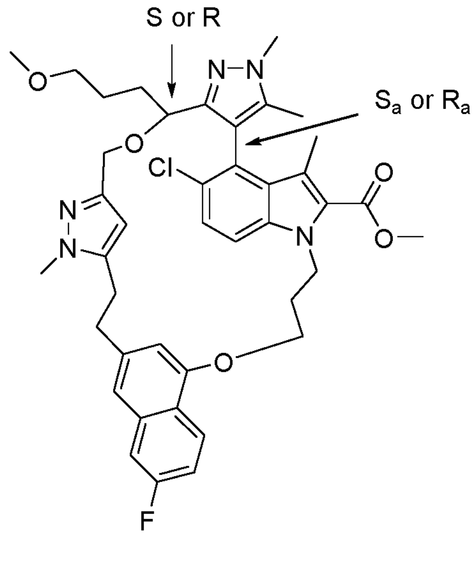


Pure stereoisomer but absolute stereochemistry undetermined

NaH (60 % dispersion in mineral oil, 1.6 mg, 0.041 mmol, 1.2 eq.) was added to a solution of Intermediate 39 (25 mg, 0.034 mmol) and MeI (3 µL, 0.051 mmol, 1.5 eq.) in THF (1 mL), at room temperature. After 1 h, additional MeI (50 µL, 0.8 mmol, 23.5 eq.) was added and the reaction mixture was stirred at 45 °C overnight. The reaction was quenched by adding saturated aqueous $NH_4Cl$ and the mixture was extracted with EtOAc (3 x). The combined organic layers were washed with brine, dried over $MgSO_4$, filtered, and evaporated. The residue was purified by flash column chromatography on silica gel (DCM/MeOH, 100/0 to 95/5) to provide Intermediate 40 (9 mg, yield: 35 %).

## Intermediate 41

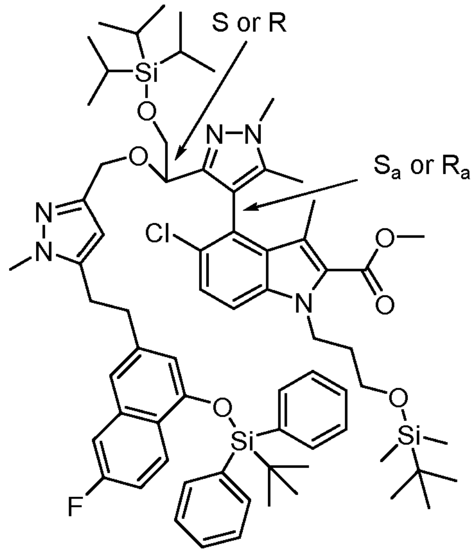


Pure stereoisomer but absolute stereochemistry undetermined

A solution of Intermediate 19 (1002 mg, 1.44 mmol, 1.2 eq.) and Intermediate 10b (863 mg, 1.2 mmol) in anhydrous THF (12 mL) was added dropwise over 5 min to a suspension of NaH (60 % dispersion in mineral oil, 72 mg, 1.8 mmol, 1.5 eq.) in anhydrous THF (12 mL) at 0 °C under nitrogen atmosphere. The reaction mixture was stirred at 0 °C for 5 min then at room temperature for 1 h. The reaction was quenched with a drop of MeOH followed by a drop of saturated aqueous $NH_4Cl$. The mixture was diluted with EtOAc and brine was added. The layers were separated, the organic layer was washed with brine and the combined aqueous layers were extracted with EtOAc (x 3). The combined organic extracts

were dried over $MgSO_4$, filtered, and concentrated under reduced pressure. The residue was purified by flash column chromatography ($SiO_2$, 40 g RediSep, heptane/EtOAc, 100/0 to 0/100) to afford Intermediate 41 (744 mg, yield: 51 %) as a white foam.

## Intermediate 42

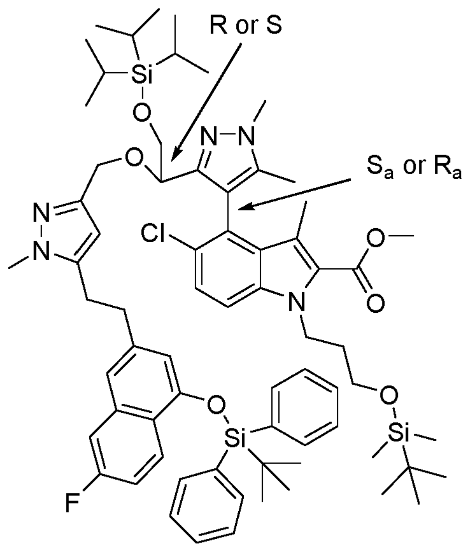

Pure stereoisomer but absolute stereochemistry undetermined

Intermediate 42 was prepared by an analogous reaction protocol as Intermediate 41, starting from Intermediate 10a instead of Intermediate 10b.

## Intermediate 43

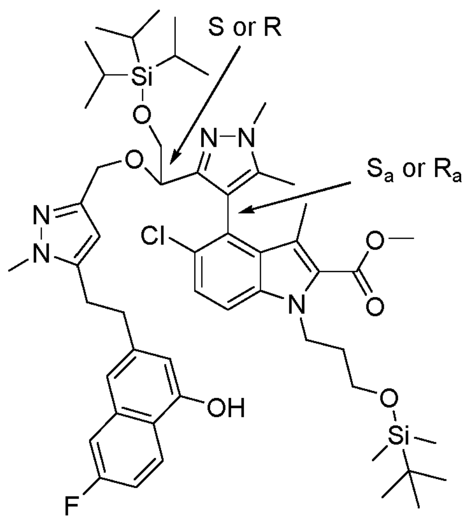

Pure stereoisomer but absolute stereochemistry undetermined

$K_2CO_3$ (335 mg, 2.42 mmol, 4 eq.) was added to a solution of Intermediate 41 (744 mg, 0.61 mmol) in MeOH (12 mL) at room temperature. The reaction mixture was stirred at room temperature for 3 h. The reaction mixture was diluted with EtOAc and saturated aqueous $NH_4Cl$ was added. The layers were separated and the organic layer was washed with brine. The aqueous layer was extracted with EtOAc (x 3), the combined organic extracts were dried over $MgSO_4$, filtered, and concentrated under reduced pressure to afford Intermediate 43 (666 mg, yield: 100 %) as a colorless thick oil.

## Intermediate 44

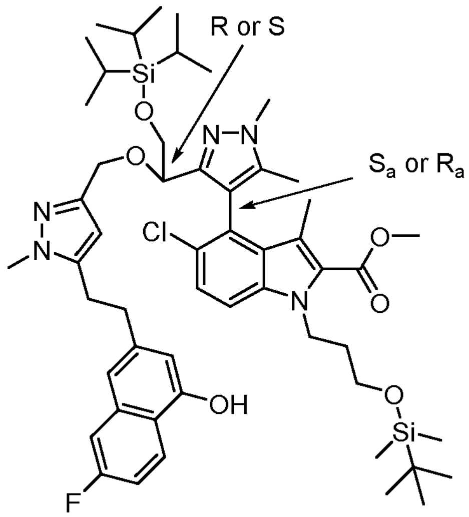


Pure stereoisomer but absolute stereochemistry undetermined

Intermediate 44 was prepared by an analogous reaction protocol as Intermediate 43, starting from Intermediate 42 instead of Intermediate 41.

## Intermediate 45

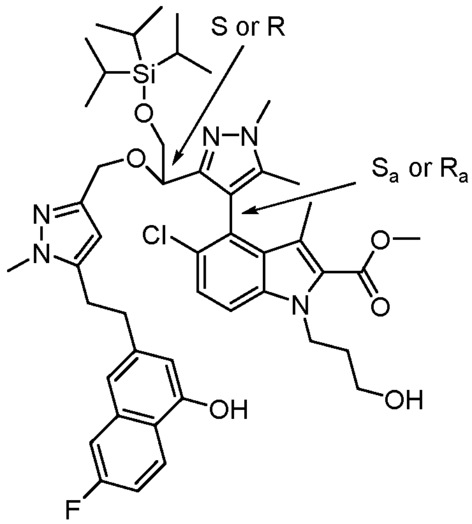


Pure stereoisomer but absolute stereochemistry undetermined

Pyridinium p-toluenesulfonate (305 mg, 1.21 mmol, 2 eq.) was added to a solution of Intermediate 43 (666 mg, 0.61 mmol) in MeOH (13 mL) at room temperature. The reaction mixture was stirred at 50 °C for 5 h. The reaction was quenched by addition of saturated aqueous $NaHCO_3$ the mixture was diluted with DCM. The layers were separated and the aqueous layer was extracted with DCM (x 3). The combined organic layers were dried over $MgSO_4$, filtered, and concentrated under reduced pressure. The residue was purified by flash column chromatography ($SiO_2$, 40 g RediSep, DCM/MeOH, 100/0 to 90/10) to give Intermediate 45 (459 mg, yield: 62 %) as a white foam.

## Intermediate 46

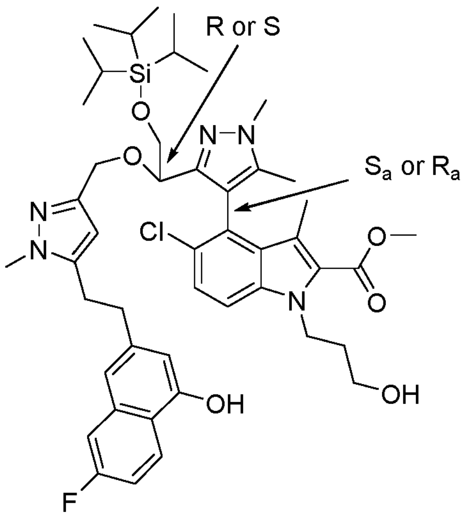

Pure stereoisomer but absolute stereochemistry undetermined

Intermediate 46 was prepared by an analogous reaction protocol as Intermediate 45, starting from Intermediate 44 instead of Intermediate 43.

## Intermediate 47

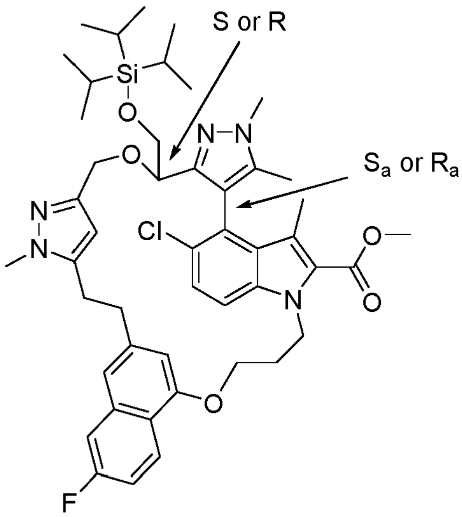

Pure stereoisomer but absolute stereochemistry undetermined

A nitrogen-degassed solution of Intermediate 45 (459 mg, 0.38 mmol) and di-tert-butyl azodicarboxylate (348 mg, 1.51 mmol, 4 eq.) in toluene (12 mL) and THF (2 mL) was added by syringe pump (0.1 mL/min) to a solution of triphenylphosphine (397 mg, 1.51 mmol, 4 eq.) in toluene (12 mL) at 70 °C under nitrogen atmosphere. Once the addition was complete, the reaction mixture was stirred at 70 °C for 15 min, then it was cooled to room temperature and the solvent was removed under reduced pressure. The residue was purified by flash column chromatography ($SiO_2$, 40 g RediSep, heptane/EtOAc, 100/0 to 100/0) to give Intermediate 47 (1.03 g, contaminated with triphenylphosphine oxide) as a pale yellow solid, used without further purification.

## Intermediate 48

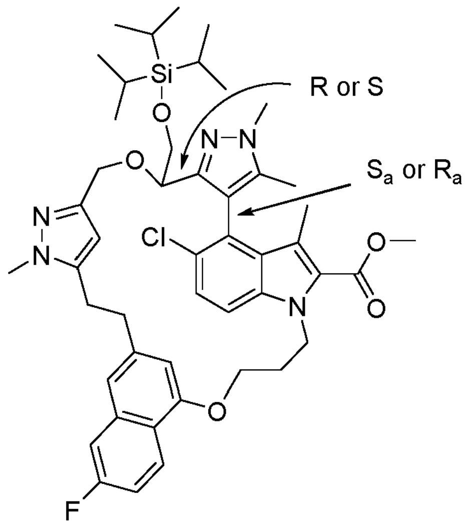


Pure stereoisomer but absolute stereochemistry undetermined

Intermediate 48 was prepared by an analogous reaction protocol as Intermediate 47, starting from Intermediate 46 instead of Intermediate 45.

## Intermediate 49

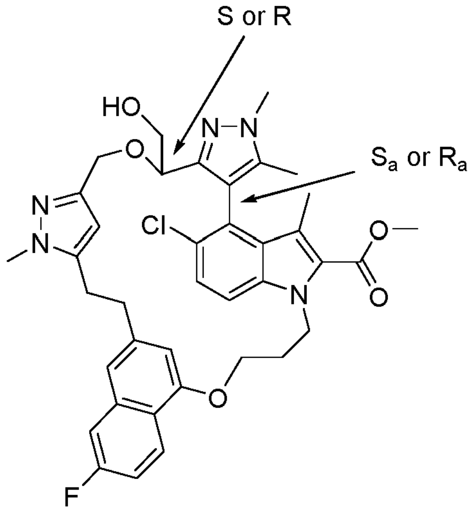


Pure stereoisomer but absolute stereochemistry undetermined

TBAF (1 M solution in THF, 1.14 mL, 1.14 mmol, 2.5 eq.) was added to a solution of Intermediate 47 (1.03 g, 0.46 mmol) in anhydrous THF (9 mL) at 0 °C under nitrogen atmosphere. The reaction mixture was stirred at room temperature for 4 h. The reaction was quenched with saturated aqueous $NH_4Cl$ and the layers were separated. The organic layer was washed with brine and the combined aqueous layers were extracted with EtOAc (x 3). The combined organic layers were dried over $MgSO_4$, filtered, and concentrated under reduced pressure. The residue was purified by flash column chromatography (SiO2, 40 g RediSep, heptane/EtOAc, 100/0 to 0/100 then DCM/MeOH 96/4 to 90/10) to afford Intermediate 49 (299 mg, yield: 77 %) as a pale orange foam.

## Intermediate 50

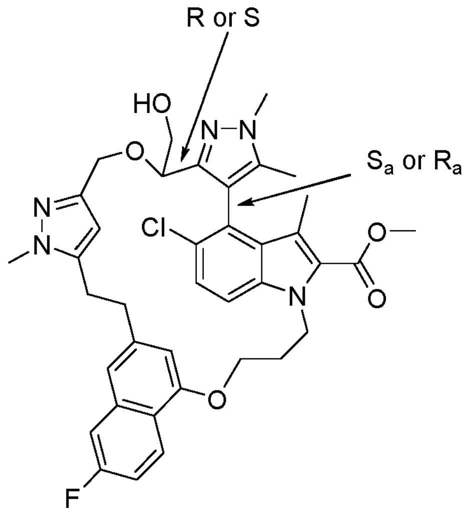

Pure stereoisomer but absolute stereochemistry undetermined

Intemediate 50 was prepared by an analogous reaction protocol as Intermediate 49, starting from Intermediate 48 instead of Intermediate 47.

## Intermediate 51

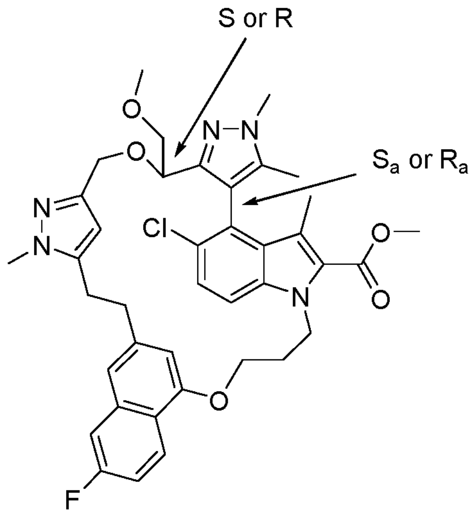

Pure stereoisomer but absolute stereochemistry undetermined

A solution of Intermediate 49 (80 mg, 0.11 mmol) in anhydrous THF (1 mL) was added dropwise to a suspension of NaH (60 % dispersion in mineral oil, 7 mg, 0.17 mmol, 1.5 eq.) in anhydrous THF (1 mL) at 0 °C under nitrogen atmosphere. Once the addition was complete, MeI (12 μL, 0.19 mmol, 1.7 eq.) was added. The reaction mixture was stirred at 0 °C for 5 min then at room temperature for 1 h. The reaction was quenched with a drop of MeOH followed by a drop of saturated aqueous $NH_4Cl$. The mixture was diluted with EtOAc and brine was added. The layers were separated and the organic layer was washed with brine. The combined aqueous layer was extracted with EtOAc (x 5). The combined organic layer was dried over $MgSO_4$, filtered, and concentrated under reduced pressure. The residue was purified by flash column chromatography ($SiO_2$, 40 g RediSep, DCM/MeOH, 100/0 to 90/10) to afford Intermediate 51 (73 mg, yield: 82 %) as a pale yellow solid.

## Intermediate 52

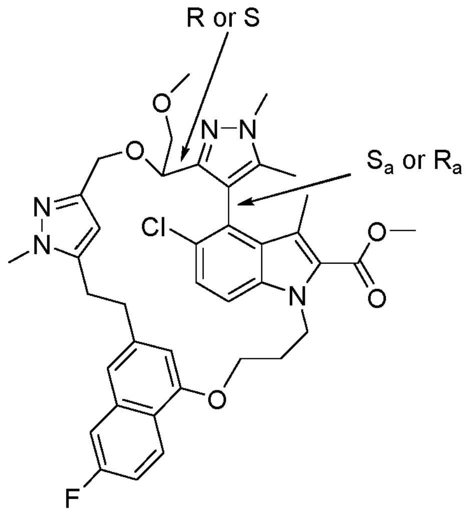

Pure stereoisomer but absolute stereochemistry undetermined

Intermediate 52 was prepared by an analogous reaction protocol as Intermediate 51, starting from Intermediate 50 instead of Intermediate 49.

## Intermediate 53

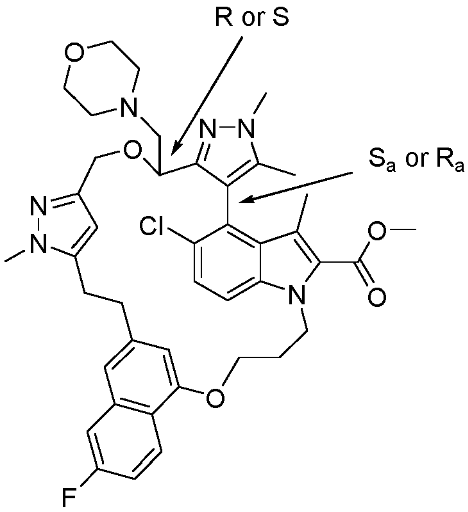

Pure stereoisomer but absolute stereochemistry undetermined

(13 μL, 0.094 mmol, 2 eq.) followed by MsCl (5 μL, 0.07 mmol, 1.4 eq.) were added dropwise to a solution of Intermediate 49 (40 mg, 0.047 mmol) in anhydrous DCM (0.5 mL) at 0 °C under nitrogen atmosphere. The reaction was stirred at 0 °C for 5 min then at room temperature for 1 h. After cooling to 0 °C again, additional (13 μL, 0.094 mmol, 2 eq.) and MsCl (5 μL, 0.07 mmol, 1.4 eq.) were added. The resulting mixture was stirred at room temperature for 30 min. Morpholine (0.12 mL, 1.41 mmol, 30 eq.) was then added under at room temperature under nitrogen atmosphere and the reaction mixture was stirred at 60 °C for 4 h. Additional morpholine (0.12 mL, 1.41 mmol, 30 eq.) was added and the reaction mixture was stirred at 65 °C for 24 h. To push the reaction to completion, additional morpholine (82 μL, 0.94 mmol, 20 eq.) was added and the reaction was stirred at 60 °C for 17 h. The reaction mixture was diluted with DCM and water, and the layers were separated. The organic layer was washed with brine and the combined aqueous layer was extracted with DCM (x 3). The combined organic layer was dried over $MgSO_4$, filtered, and concentrated under reduced pressure. The residue was purified by flash column chromatography ($SiO_2$, 12 g RediSep, DCM/MeOH 100/0 to 90/10) to afford Intermediate 53 (30 mg, yield: 79 %) as a pale yellow solid.

## Intermediate 54

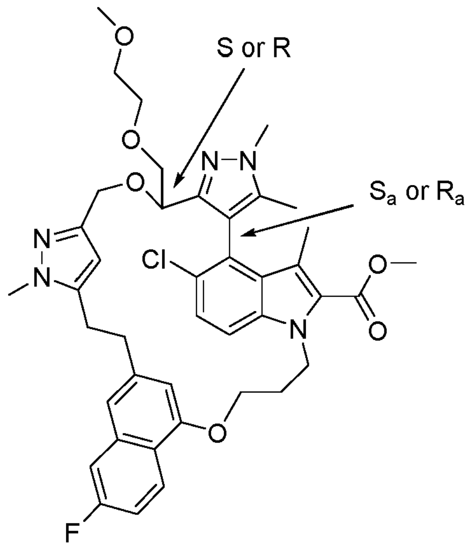

Pure stereoisomer but absolute stereochemistry undetermined

A solution of Intermediate 49 (70 mg, 0.1 mmol) in anhydrous THF (1 mL) was added dropwise to a suspension of NaH (60 % dispersion in mineral oil, 6 mg, 0.15 mmol, 1.5 eq.) in anhydrous THF (1 mL) at 0 °C under nitrogen atmosphere. 2-Bromoethyl methyl ether (3 x 16 μL, 3 x 0.17 mmol, 3 x 1.7 eq.) was added in three portions and the reaction mixture was stirred at room temperature for 5 h. To push the reaction to completion, additional NaH (60 % dispersion in mineral oil, 4 mg, 0.1 mmol, 1 eq.) was added, followed by 2-bromoethyl methyl ether (16 μL, 0.17 mmol, 1.7 eq.) and the reaction mixture was stirred at room temperature for 1 h. The reaction was quenched with a drop of MeOH followed by a drop of saturated aqueous $NH_4Cl$. The mixture was diluted with EtOAc and brine was added. The layers were separated and the organic layer was washed with brine. The combined aqueous layer was extracted with EtOAc (x 5). The combined organic layer was dried over $MgSO_4$, filtered, and concentrated under reduced pressure. The residue was purified by flash column chromatography ($SiO_2$, 12 g RediSep, DCM/MeOH, 100/0 to 90/10) to afford Intermediate 54 (40 mg, yield: 50 %) as a pale yellow solid.

## Intermediate 55

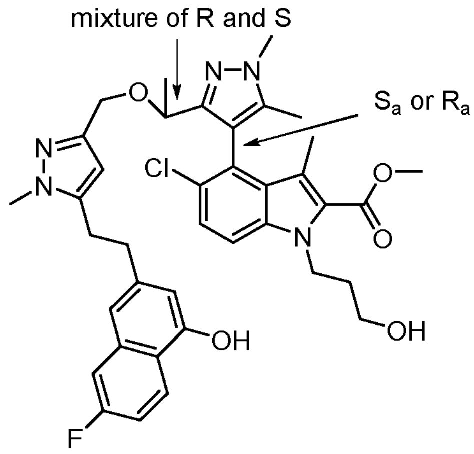

A solution of Intermediate 19 (580 mg, 0.83 mmol, 1.2 eq.) and Intermediate 11 (500 mg, 0.69 mmol) in anhydrous THF (7 mL) was added dropwise over 5 min to a suspension of NaH (60 % dispersion in mineral oil, 42 mg, 1.04 mmol, 1.5 eq.) in anhydrous THF (7 mL) at 0 °C under nitrogen atmosphere. The reaction mixture was stirred at 0 °C for 5 min then at room temperature for 3 h. To push the reaction to completion, additional NaH (60 % dispersion in mineral oil, 14 mg, 0.35 mmol, 0.5 eq.) and Intermediate 19 (338 mg, 0.48 mmol, 0.7 eq.) were added to the mixture under nitrogen atmosphere, and the reaction was stirred at room temperature for 3 h. The reaction was quenched with a drop of MeOH followed by a drop of saturated aqueous $NH_4Cl$. The mixture was diluted with EtOAc and brine was added. The layers were separated

and the organic layer was washed with brine. The combined aqueous layer was extracted with EtOAc (x 3). The combined organic layer was dried over $MgSO_4$, filtered, and concentrated under reduced pressure. The crude residue was dissolved in anhydrous THF (8 mL) and the solution was cooled to 0 °C under nitrogen atmosphere. TBAF (1 M solution in THF, 2.77 mL, 1 M, 2.77 mmol, 4 eq.) was added and the reaction mixture was stirred at room temperature for 3.5 h. The reaction was quenched with saturated aqueous $NH_4Cl$ and the layers were separated. The organic layer was washed twice with brine and the combined aqueous layer was extracted with EtOAc (x 3). The combined organic layer was dried over $MgSO_4$, filtered, and concentrated under reduced pressure. The residue was purified by flash column chromatography ($SiO_2$, 40 g RediSep, DCM/MeOH, 100/0 to 90/10) to afford Intermediate 55 (282 mg, yield: 40 %) as a pale yellow oil.

**Intermediate 56 and Intermediate 57**

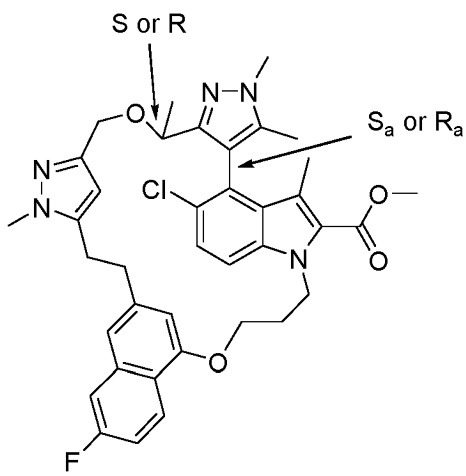

Intermediate 56

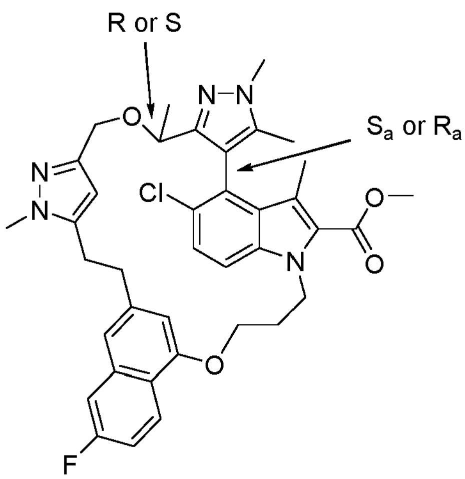

Intermediate 57

Both compounds are pure stereoisomers but absolute stereochemistry undetermined

A nitrogen-degassed solution of Intermediate 55 (282 mg, 0.4 mmol) and di-tert-butyl azodicarboxylate (370 mg, 1.61 mmol, 4 eq.) in toluene (12 mL) and THF (2 mL) was added by syringe pump (0.1 mL/min) to a nitrogen-degassed solution of triphenylphosphine (421 mg, 1.61 mmol, 4 eq.) in toluene (12 mL) stirring at 70 °C under nitrogen atmosphere. Once the addition was complete, the reaction mixture was stirred at 70 °C for 15 min, then it was cooled to room temperature and the solvents were removed under reduced pressure. The residue was purified by flash column chromatography ($SiO_2$, 40 g RediSep, nHept/EtOAc, 100/0 to 100/0 then DCM/MeOH 98/2 to 90/10) followed by preparative HPLC (Stationary phase: RP XBridge Prep C18 OBD- 5 µm, 50 x 250 mm, Mobile phase: 0.25 % $NH_4HCO_3$ solution in water, MeOH) to give Intermediate 56 (51 mg, yield: 17 %) and Intermediate 57 (22 mg, yield: 8 %).

## Intermediate 58

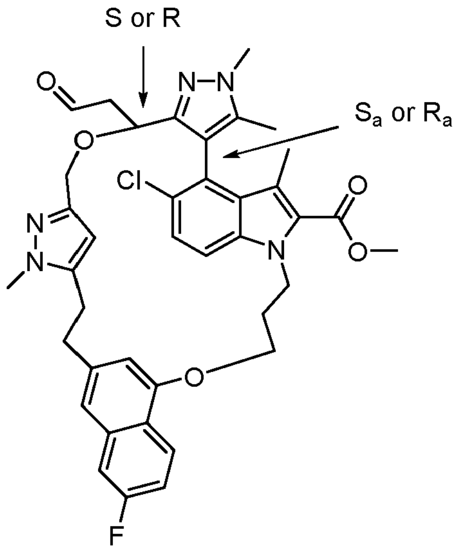

Dess-Martin periodinane (CAS [87413-09-0], 57 mg, 0.13 mmol, 1.2 eq.) was added to a solution of Intermediate 27 (80 mg, 0.11 mmol) in DCM (2 mL) at room temperature. After 2 h, more Dess-Martin periodinane (57.01 mg, 0.13 mmol, 1.2 eq.) was added, and the reaction mixture was stirred at room temperature until completion of the reaction. The reaction mixture was diluted with DCM (10 mL), treated with a 1:1 mixture of saturated aqueous $NaHCO_3$ and 10 % aqueous $Na_2S_2O_3$ (10 mL), and stirred until the biphasic layer became clear. The layers were separated and the aqueous layer was back extracted with DCM (x 2). The combined organic layer was washed with saturated aqueous $NaHCO_3$ (2 x 10 mL), water (10 mL), and brine (10 mL), dried over $MgSO_4$, filtered, and the was solvent was evaporated to provide Intermediate 58 (72 mg, yield: 90 %), used without further purification.

## Intermediate 59 and Intermediate 60

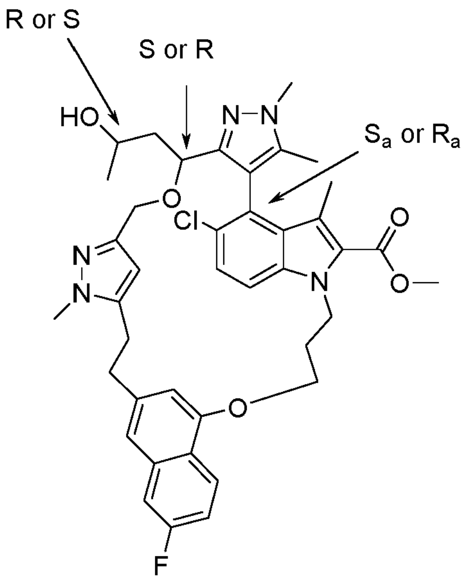

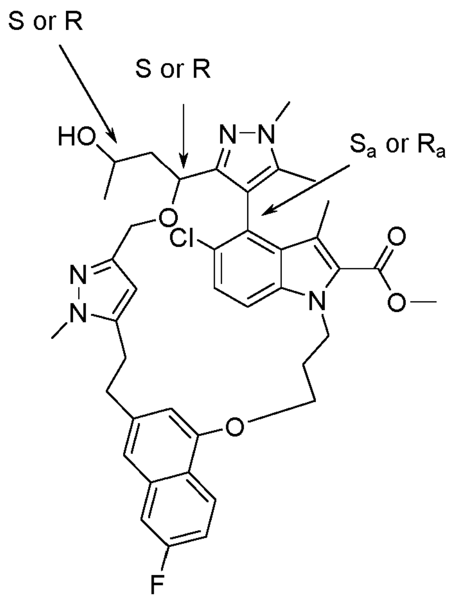

Intermediate 59 Intermediate 60

Both are pure stereoisomers but absolute stereochemistry undetermined

Methylmagnesium bromide (0.14 mL, 3.4 M solution in 2-MeTHF, 0.47 mmol, 4.7 eq.) was added to a solution of Intermediate 58 (72 mg, 0.1 mmol) in THF (1 mL) at -78 °C. After 2 h, methylmagnesium bromide (0.089 mL, 3.4 M in 2-MeTHF, 0.3 mmol, 3 eq.) was added at -78 °C and the resulting mixture was stirred for another 3 h at -78 °C. The reaction

was quenched by adding saturated aqueous $NH_4Cl$. The layers were separated, and the aqueous layer was back extracted with EtOAc (x 2). The combined organic layer was washed with brine, dried over $MgSO_4$, filtered, and evaporated under reduced pressure. The residue was purified by preparative SFC (stationary phase: Chiralpak Daicel ID 20 x 250 mm, mobile phase: $CO_2$, iPrOH + 0.4 % $iPrNH_2$) to provide Intermediate 59 (7 mg, yield: 9 %) and Intermediate 60 (36 mg, yield: 48 %)

## Intermediate 61

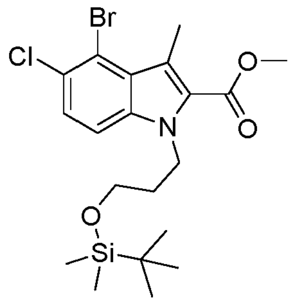

A mixture of Intermediate 2 (37.4 g, 123.6 mmol), (3-bromopropoxy)-tert-butyldimethylsilane (37.567 g, 1.2 eq.), and $K_2CO_3$ (51.25 g, 3 eq.) in ACN (300 mL) was stirred at 80 °C overnight. The reaction mixture was cooled to room temperature and was filtered. The filter cake was washed with EtOAc (100 mL). The filtrate was concentrated and the residue was purified by column chromatography over silica gel (eluent: petroleum ether/EtOAc from 100/0 to 10/90) to afford Intermediate 61 (42 g, yield: 71 %) as a red gum.

## Intermediate 62

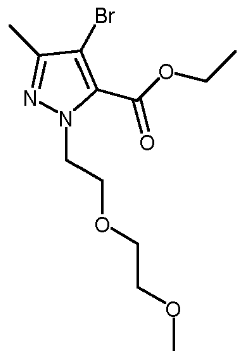

Cyanomethylenetributylphosphorane (CAS [157141-27-0], 45.02 mL, 1 eq.) was added dropwise to a solution of 1H-pyrazole-3-carboxylic acid, 4-bromo-5-methyl-, ethyl ester (CAS [6076-14-8], 20 g, 85.81 mmol) and 2-(2-methoxyethoxy)ethanol (CAS [111-77-3], 14.15 mL, 1.4 eq.) in THF (1.9 L) at room temperature. The reaction mixture was stirred at room temperature overnight. The reaction mixture was poured into water (100 mL) and the mixture was extracted with EtOAc (3 x 100 mL). The combined organic layer was washed with brine, dried over $MgSO_4$, filtered, and concentrated in vacuo. The crude product was purified via flash column chromatography on silica gel (heptane/EtOAc, 100/0 to 50/50) to give Intermediate 62 (24 g, yield: 83 %).

## Intermediate 63

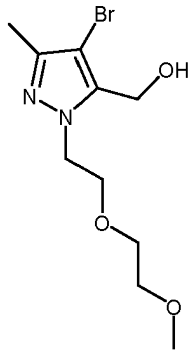

Sodium borohydride (4.26 g, 5 eq.) was added to a solution of Intermediate 62 (7.45 g, 22.23 mmol) in a mixture of THF (130 mL) and MeOH (34 mL) at 0 °C. After 5 min, the resulting mixture was allowed to reach room temperature and was stirred for 3 h. The reaction mixture was diluted by very slow addition of acetone (80 mL) and water (80 mL), followed by EtOAc (100 mL). The layers were separated and the aqueous layer was extracted with EtOAc (2 x 50 mL) followed by a 1:1 mixture of EtOAc/THF (2 x 50 mL). The combined organic layer was dried over $MgSO_4$, filtered, and evaporated to afford Intermediate 63 (7.24 g, quantitative) as a tan oil, used without further purification.

## Intermediate 64

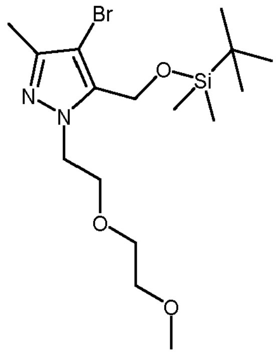

TBDMSCl (617 mg, 1.2 eq.) was added portionwise at 0 °C to a stirred and previously degassed (nitrogen) solution of Intermediate 63 (1 g, 3.41 mmol) and imidazole (325 mg, 1.4 eq.) in dry DCM (10 mL). The reaction mixture was stirred at room temperature under nitrogen for 2 h. To push the reaction to completion, additional TBDMSCl (150 mg, 0.3 eq.) was added and the reaction mixture was stirred at room temperature for another 1.5 h. Saturated aqueous $NH_4Cl$ was added and the layers were separated. The organic layer was dried over $MgSO_4$, filtered, and concentrated in vacuo. The resulting tan oil residue was purified by flash chromatography on silica gel (EtOAc/heptane 0/100 to 30/70) to give Intermediate 64 (1.09 g, yield: 78 %) as a light tan clear oil.

## Intermediate 65

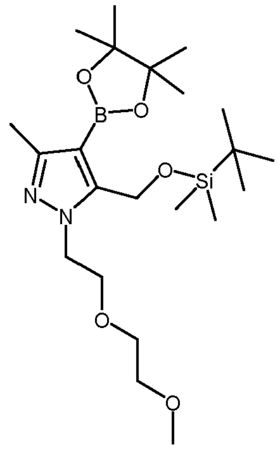

A solution of Intermediate 64 (1.06 g, 2.60 mmol) in dry THF (11 mL) was cooled to - 78 °C under nitrogen atmosphere. *n*-BuLi (2.5 M in hexanes; 1.3 mL, 1.25 eq.) was added dropwise. The reaction mixture was stirred at -78 °C for 1 h. 2-Isopropoxy-4,4,5,5-tetramethyl-1,3,2-dioxaborolane (CAS [61676-62-8], 0.64 mL, 1.2 eq.) was added dropwise. After the addition, the reaction mixture was allowed to warm to room temperature and was stirred for 1 h. The reaction was quenched by slow addition of EtOAc (25 mL), followed by saturated aqueous $NH_4Cl$ (20 mL). The layers were separated and the aqueous layer was extracted with EtOAc (2 x 20 mL). The combined organic layer was washed with brine (20 mL), dried over $MgSO_4$, filtered, and concentrated in vacuo. The residue was purified by flash column chromatography on silica gel (EtOAc/heptane 0/100 to 50/50) to afford Intermediate 65 (934 mg, yield: 79 %) as a yellow clear oil.

## Intermediate 66

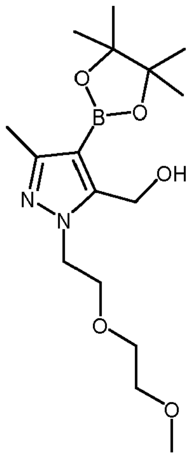

TBAF (1.0 M in THF, 1.2 eq.) was added to a solution of Intermediate 65 (930 mg, 2.05 mmol) in anhydrous Me-THF (12 mL) under nitrogen atmosphere, at 0 °C. The ice bath was removed and the resulting mixture was stirred at room temperature for 16 h. The reaction mixture was diluted with EtOAc and saturated aqueous $NH_4Cl$ was added. The layers were separated and the aqueous layer was extracted twice with EtOAc. The combined organic layer was washed with brine, dried over $MgSO_4$, filtered, and concentrated in vacuo. The residue was purified by flash column chromatography on silica gel (MeOH in DCM 0/100 to 5/95) to afford Intermediate 66 (580 mg, yield: 83 %) as a light yellow clear oil.

**Intermediate 67** and **Intermediate 68**

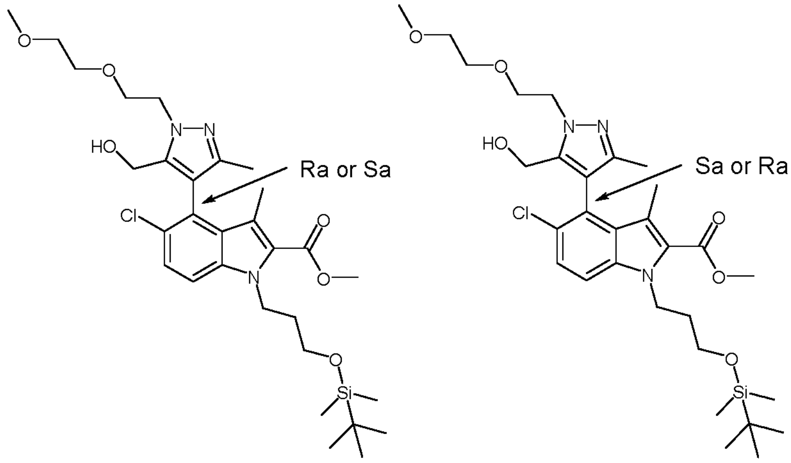


Intermediate 67 Intermediate 68

Both intermediates: one atropisomer but absolute stereochemistry undetermined

Pd(Amphos)$_2$Cl$_2$ (CAS [887919-35-9], 51 mg, 0.05 eq.) was added to a stirred and previously nitrogen-degassed mixture of Intermediate 61 (706 mg, 1.44 mmol), Intermediate 66 (580 mg, 1.2 eq.) and $K_2CO_3$ (400 mg, 2 eq.) in water (2 mL) and 1,4-dioxane (8 mL) in a microwave tube at room temperature and under nitrogen. The reaction mixture was degassed by bubbling nitrogen through. The vial was sealed and the reaction mixture was stirred at 65 °C for 2 h. The reaction mixture was diluted with EtOAc and water and the layers were separated. The aqueous layer was extracted twice with EtOAc. The combined organic layer was dried over $MgSO_4$, filtered, and evaporated. The residue was purified by flash column chromatography (silica; EtOAc in n-heptane 0/100 to 100/0), followed by preparative SFC (stationary phase: Chiralpak Diacel AD 20 x 250 mm, Mobile phase: $CO_2$, EtOH + 0.4 iPrNH$_2$) to give Intermediate 67 (7.32 g, yield: 27 %) and Intermediate 68 (7.74 g, yield: 29 %) as yellow oils.

**Intermediate 69**

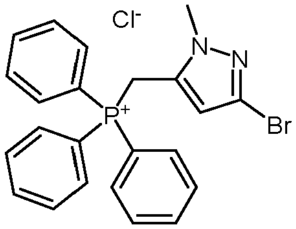

$PPh_3$ (19.53 g, 1.2 eq.) was added to a solution of 3-bromo-5-(chloromethyl)-1-methyl-1H-pyrazole (CAS [2109428-60-4], 13 g, 62 mmol) in ACN (150 mL) and the reaction mixture was stirred at 85 °C for 16 h. The solvent was evaporated. The residue was added to petroleum ether (100 mL) and this mixture was stirred at room temperature for 1 h. The solid was filtered and dried under vacuum to give Intermediate 69 (25 g, yield: 84 %) as a white solid.

**Intermediate 70**

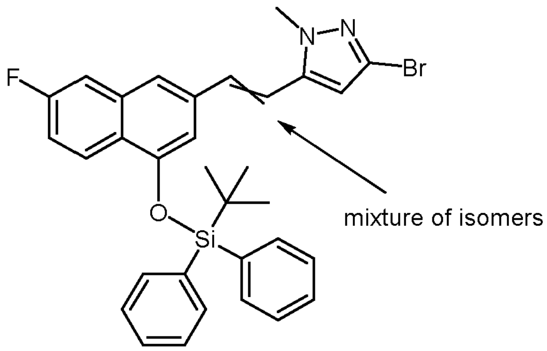


NaH (60 % in mineral oil, 975 mg, 1.3 eq.) was added to a solution of Intermediate 69 (9 g, 18.76 mmol) in THF (100 mL) at 0 °C and the reaction mixture was stirred at 0 °C for 1 h. Intermediate 15 (9.79 g, 1.1 eq.) was then added at -30 °C and the reaction mixture was stirred at -30 °C for 2 h. The reaction was quenched by addition of aqueous $NH_4Cl$ (50 mL). The mixture was extracted with EtOAc (100 mL x 3). The combined organic layer was dried with $Na_2SO_4$ and the solvent was evaporated to give the crude product as a yellow oil. This oil was purified by column chromatography on silica gel (eluent: petroleum ether/EtOAc 100/0 to 85/15) to give Intermediate 70 (9 g, yield: 81 %) as a white solid.

**Intermediate 71**

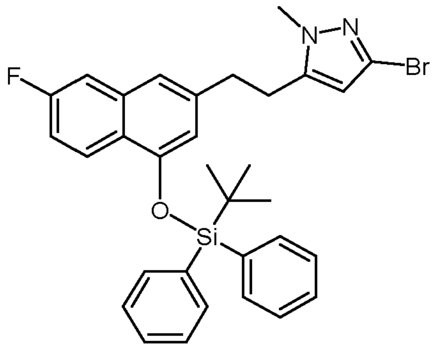

$PtO_2$ (1.04 g, 0.3 eq.) was added to a solution of Intermediate 70 (9 g, 15.25 mmol) in EtOAc (100 mL) under hydrogen atmosphere. The reaction mixture was stirred at room temperature for 6 h. The reaction mixture was filtered and the filtrate was evaporated. The residue was purified by column chromatography on silica gel (eluent: petroleum ether/EtOAc 100/0 to 85/15) to give Intermediate 71 (7.4 g, yield: 83 %) as a white solid.

**Intermediate 72**

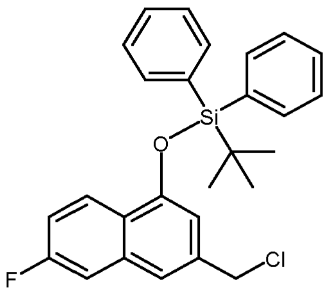

DIPEA (1.115 mL, 6.835 mmol, 3 eq.) was added to a solution of Intermediate 14 (1 g, 2.278 mmol) and methanesulfonic anhydride (0.794 g, 4.556 mmol, 2 eq.) in THF (200 mL) at 0 °C. The reaction mixture was stirred at room temperature overnight. The reaction mixture was cooled to 0 °C and LiCl (0.386 g, 9.113 mmol, 4 eq.) was added. Stirring was continued at room temperature for 2 h. The reaction was quenched by addition of water (20 mL), and the mixture was extracted with EtOAc (20 mL x 3). The combined organic layer was dried with $Na_2SO_4$, filtered, and evaporated. The

residue was purified by column chromatography on silica gel (eluent: petroleum ether/EtOAc = 100/0 to 5/1) to give Intermediate 72 (450 mg, yield: 44 %) as a white solid and its corresponding mesylate (450 mg, yield: 39 %) as a white solid.

## Intermediate 73

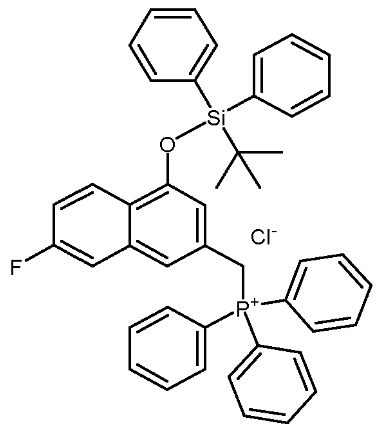

$PPh_3$ (680 mg, 2.592 mmol, 1.2 eq.) was added to a solution of Intermediate 72 (970 mg, 2.16 mmol) in ACN (10 mL). The reaction mixture was stirred at 85 °C for 16 h. The solvent was evaporated and the residue was triturated in petroleum ether (20 mL) at room temperature. The solid was filtered and dried under vacuum to give Intermediate 73 (1.1 g, yield: 70 %) as a white solid.

## Intermediate 74

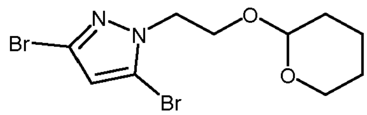

$K_2CO_3$ (22.03 g, 159 mmol, 3 eq.) was added at room temperature to a mixture of 3,5-dibromo-1H-pyrazole (CAS [67460-86-0], 12 g, 53.13 mmol) in DMF (200 mL). The reaction mixture was stirred at 60 °C and 2-(2-bromoethoxy) tetrahydro-2H-pyran (CAS# [17739-45-6], 16.66 g, 1.5 eq.) was added. The reaction mixture was stirred at 100 °C for 4 h. The reaction mixture was filtered and the filtrate was washed with brine (200 mL x 3). The organic layer was dried with $Na_2SO_4$, filtered, and concentrated. The residue was purified by flash column chromatography over silica gel (eluent: petroleum ether/EtOAc from 100/0 to 90/10) to give Intermediate 74 (16 g, yield: 85 %) as a colourless oil.

## Intermediate 75

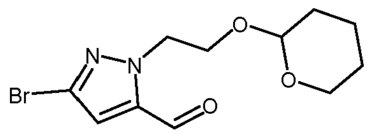

BuLi (2.5 M in THF, 25.4 mL, 63.55 mmol, 1.5 eq.) was added dropwise to a solution of Intermediate 74 (15 g, 42.37 mmol) in anhydrous THF (150 mL) at -78 °C. The reaction mixture was stirred at -78 °C for 1 h before addition of DMF (9.29 g, 127.10 mmol, 3 eq.). The reaction mixture was then stirred for an additional 1 h at -78 °C. The reaction was quenched by addition of aqueous $NH_4Cl$ (100 mL) and the mixture was extracted with EtOAc (200 mL x 3). The organic layer was dried over $Na_2SO_4$, filtered, and evaporated. The residue was purified by column chromatography on silica gel (eluent: petroleum ether/EtOAc = 100/0 to 80/20) to give Intermediate 75 (6 g, yield: 47 %) as a clear oil.

**Intermediate 76**

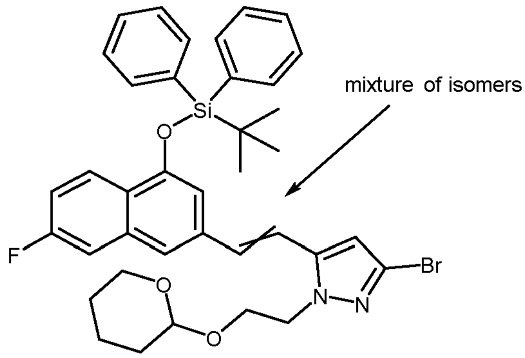


NaH (60 % in mineral oil, 887 mg, 22.17 mmol, 1.2 eq.) was added to a solution of Intermediate 73 (15.04 g, 20.32 mmol, 1.1 eq.) in THF (150 mL) at 0°C. The reaction mixture was stirred at 0 °C for 1 h. Intermediate 75 (5.6 g, 18.47 mmol) was then added at -30 °C and the reaction mixture was stirred at -30 °C for 2 h. The reaction was quenched by addition of aqueous $NH_4Cl$ (50 mL) and the mixture was extracted with EtOAc (100 mL x 3). The combined organic layer was separated, dried with $Na_2SO_4$, filtered, and evaporated. The residue was purified by column chromatography on silica gel (eluent: petroleum ether/EtOAc = 100/0 to 80/20) to give Intermediate 76 (12 g, yield: 93 %) as a white solid.

**Intermediate 77**

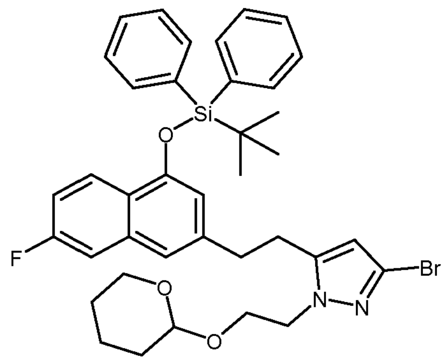

$NaHCO_3$ (2.345 g, 28.58 mmol, 2 eq.) was added to a solution of Intermediate 76 (10 g, 14.29 mmol) and p-toluenesulfonyl hydrazide (9.315 g, 50.01 mmol, 3.5 eq.) in a mixture of THF and water (4/1, 70 mL). The reaction mixture was stirred at 80 °C for 7 h. The solvent was evaporated. The residue was purified by column chromatography on silica gel (eluent: petroleum ether/EtOAc = 100/0 to 80/20) to give Intermediate 77 (6.905 g, yield: 69 %) as a white solid.

**Intermediate 78**

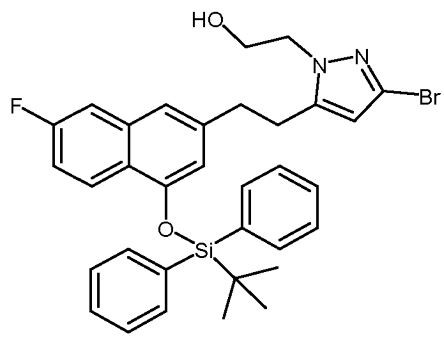

PTSA (68 mg, 0.356 mmol, 0.25 eq.) was added to a suspension of Intermediate 77 (1 g, 1.425 mmol) in MeOH (20 mL). The reaction mixture was stirred at room temperature for 3 h. The solvent was evaporated and the residue was dissolved in DCM (50 mL), and this solution was washed with saturated aqueous $NaHCO_3$ (2 x 10 mL). The organic layer was dried over $MgSO_4$, filtered, and evaporated to give Intermediate 78 (assumed quantitative), used directly in the next step.

## Intermediate 79

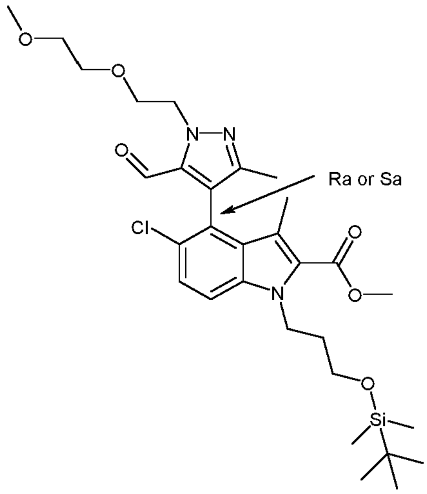


Pure atropisomer but absolute stereochemistry undetermined

Dess-Martin periodinane (1.34 g, 3.15 mmol, 1.05 eq.) was added in one portion to a solution of Intermediate 67 (1.82 g, 3 mmol) in DCM (75 mL). The suspension was stirred at room temperature for 3 h. The mixture was then treated with saturated aqueous $NaHCO_3$ and 10 % aqueous $Na_2S_2O_3$ until two homogeneous layers were obtained. The layers were separated. The aqueous layer was extracted with DCM. The organic layer was dried over $MgSO_4$, filtered, and evaporated to afford Intermediate 79 (1.81 g, yield: 100 %) as an oil, used without further purification.

## Intermediate 80

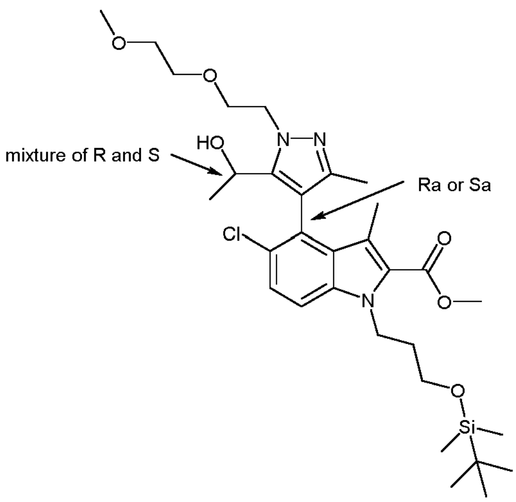


Methylmagnesium bromide (100 µL, 3.4 M in Me-THF, 0.34 mmol, 2 eq.) was added dropwise to a solution of Intermediate 79 (100 mg, 0.16 mmol) in THF (5 mL). The reaction mixture was stirred at room temperature for 1 h. The reaction was quenched by addition of EtOAc and saturated aqueous $NH_4Cl$. The layers were separated and the aqueous layer was extracted with EtOAc. The combined organic layer was washed with brine, dried over $MgSO_4$, filtered, and evaporated to afford Intermediate 80 (1.88 g, yield: 98 %) as an oil.

## Intermediate 81

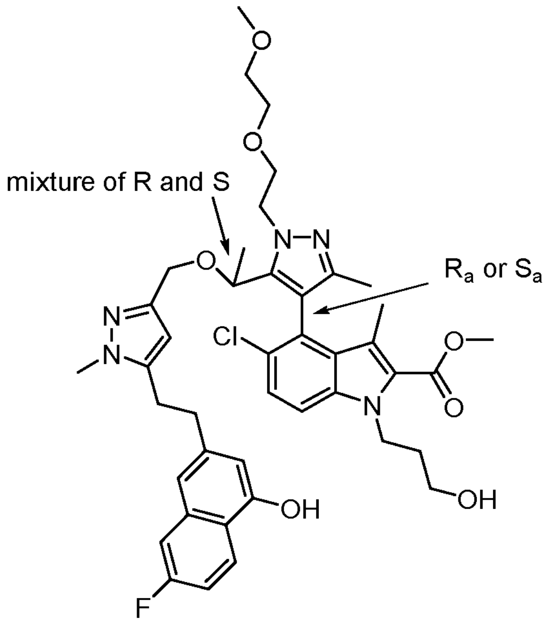


Intermediate 19 (1.06 g, 1.64 mmol, 1.7 eq.) and potassium tert-butoxide (1.38 mL, 1 M in THF, 1.31 mmol, 1.4 eq.) were added to a solution of Intermediate 80 (0.60 g, 0.96 mmol) in THF (5 mL). The reaction mixture was stirred at 50 °C for 3 h. The solution was treated with EtOAc and saturated aqueous $NH_4Cl$. The layers were separated and the aqueous layer was extracted with EtOAc. The combined organic layer was washed with brine, dried over $MgSO_4$, filtered, and concentrated under reduced pressure. The residue was purified by flash column chromatography on silica gel (40 g Redisep flash column, eluting with 0-5 % MeOH in DCM) to afford an oil (1.2 g). This oil was dissolved in THF (10 mL) and treated with tetrabutylammonium fluoride (2.3 mL, 1 M in THF, 2.3 mmol, 2.4 eq.). The reaction mixture was stirred at room temperature for 4 h. The mixture was diluted with EtOAc and saturated aqueous $NH_4Cl$. The layers were separated and the aqueous layer was extracted with EtOAc. The combined organic layer was washed with brine, dried over $MgSO_4$, filtered, and concentrated under reduced pressure. The residue was purified by flash column chromatography on silica gel (40 g Redisep flash column, eluting with 0-5 % MeOH in DCM) to afford Intermediate 81 (0.45 g, yield: 59 %) as a pale orange oil.

## Intermediate 82 and Intermediate 83

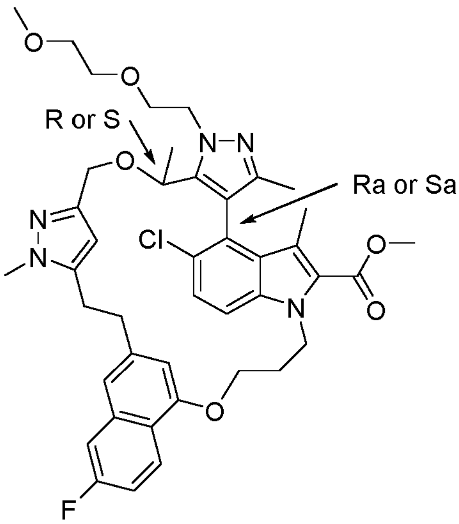


Intermediate 82

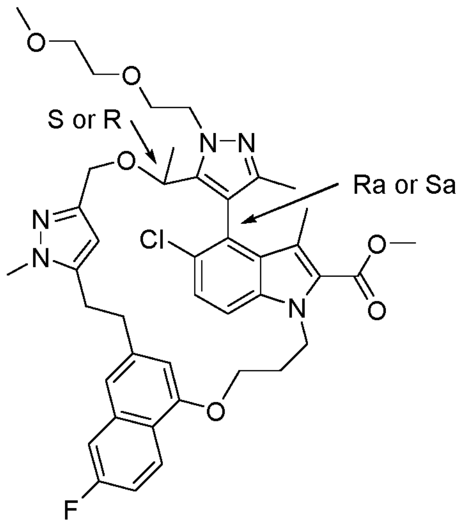


Intermediate 83

Pure stereoisomers but absolute stereochemistry undetermined

A degassed solution of Intermediate 81 (450 mg, 0.57 mmol) and di-tert-butyl azodicarboxylate (525 mg, 2.28 mmol, 4 eq.) in toluene (20 mL) and THF (5 mL) was added dropwise via a syringe pump (rate 0.1 ml/minute) to a degassed solution of triphenylphosphine (600 mg, 2.29 mmol, 4 eq.) in toluene (20 mL) while stirring at 70 °C. Once the addition was complete, the resulting orange solution was cooled to room temperature and stirred overnight. The solution was concentrated under reduced pressure and the residue was purified by flash column chromatography on silica gel (40 g, Redisep flash column, eluting with 0-2 % MeOH in DCM) followed by preparative SFC (stationary phase: Chiralpak Diacel AD 20 x 250 mm, Mobile phase: $CO_2$, EtOH + 0.4 % $iPrNH_2$) to afford Intermediate 82 (impure) and Intermediate 83 (23 mg, yield: 5 %). Impure Intermediate 82 was purified further by flash column chromatography on silica gel (4 g, Redisep flash column, eluting with 0-5 % MeOH in DCM) to afford pure Intermediate 82 (63 mg, yield: 14 %).

## Intermediate 84

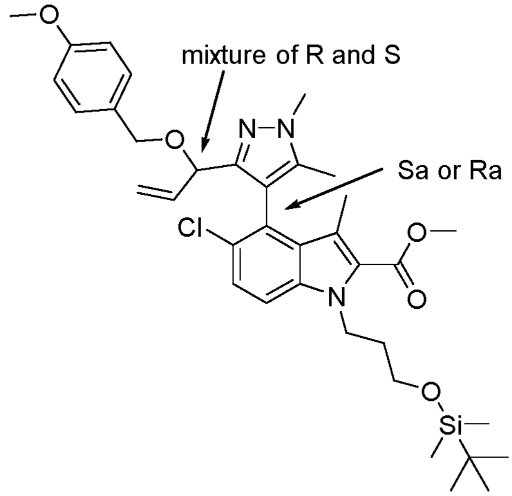


NaH (60 % dispersion in mineral oil, 251 mg, 6.27 mmol, 1.4 eq.), followed by potassium iodide (132 mg, 0.79 mmol, 0.2 eq.) was added to a solution of the racemic mixture of Intermediate 7a and Intermediate 7b (2.46 g, 4.5 mmol) in anhydrous DMF (117 mL) under nitrogen atmosphere at 0 °C. After 10-15 min at 0 °C, 4-methoxybenzyl chloride (0.95 mL, 7.03 mmol, 1.6 eq.) was added dropwise. The resulting mixture was stirred at room temperature for 3.5 h. The reaction was quenched by slow addition of MeOH and saturated aqueous $NH_4Cl$, and then diluted with EtOAc and water. The layers were separated and the aqueous layer was extracted with EtOAc (x 2). The combined organic layer was dried over $MgSO_4$, filtered, and evaporated. The residue was purified by flash column chromatography on silica gel (Redisep CombiFlash, EtOAc in n-heptane 0 % to 50 %) to afford Intermediate 84 (3.3 g) as a yellow oil, used without further purification.

## Intermediate 85

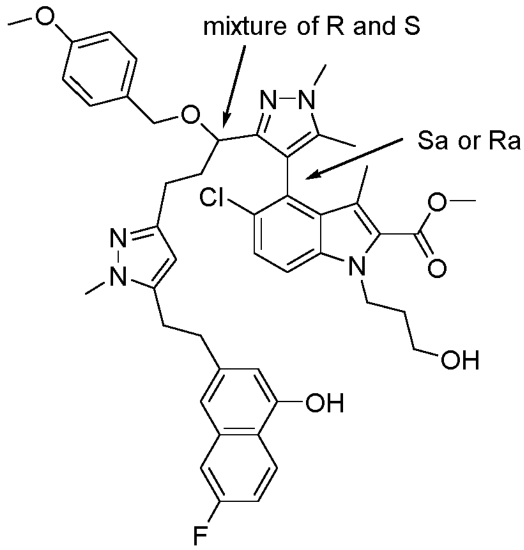


9-BBN (0.5 M in THF, 28 mL, 14 mmol, 5 eq.) was added dropwise to a solution of Intermediate 84 (1.87 g, 2.81 mmol) in anhydrous THF (32 mL) in a pressure tube under nitrogen atmosphere, at room temperature. The tube was sealed, and the mixture was stirred at 75 °C for 45 min. The reaction mixture was cooled to room temperature and Intermediate 71 (2.8 g, 4.77 mmol, 1.7 eq.), potassium phosphate tribasic (1.77 g, 8.36 mmol, 3 eq.) and [1,1'-bis(di-tert-butylphosphino)ferrocene]dichloropalladium(II) (384 mg, 0.59 mmol, 0.2 eq.) were added in one portion under continuous nitrogen flow. Water (3 mL) was then added slowly. The reaction mixture was degassed by bubbling nitrogen through, and the reaction mixture was stirred at 80 °C for 4 h. After cooling, the mixture diluted with EtOAc and water and the layers were separated. The organic layer was washed with brine and the combined aqueous layer was extracted with EtOAc. The combined organic layer was dried over $MgSO_4$, filtered, and evaporated. The residue was dissolved in THF (45 mL) and cooled to 0 °C before TBAF (14 mL, 1.0 M in THF, 14 mmol, 5 eq.) was added dropwise. The reaction mixture was stirred at room temperature for 4 h, then an additional portion of TBAF (7 mL, 1.0 M in THF, 7 mmol, 2.5 eq.) was added at room temperature. The resulting mixture was stirred for 2 h at room temperature. The reaction was quenched by addition of saturated aqueous $NH_4Cl$. The layers were separated and the aqueous layer was extracted twice with EtOAc. The combined organic layer was washed with brine, dried over $MgSO_4$, filtered, and evaporated. The residue was purified by flash column chromatography on silica gel (Redisep CombiFlash 120 g, EtOAc in n-heptane 0 % to 100 %, then MeOH in DCM 0 % to 5 %) to afford a dark brown foam. The foam was suspended in water and a drop of acetic acid was added. The resulting mixture was stirred at room temperature overnight, then the solids were filtered, and these were re-dissolved in DCM. Saturated aqueous $NaHCO_3$ was added and the layers were separated. The aqueous layer was extracted with DCM and the combined organic layer was dried over $MgSO_4$, filtered, and evaporated. The resulting foam was triturated and co-evaporated several times with n-heptane, to afford Intermediate 85 (920 mg, yield: 31 %) as a brown foam.

## Intermediate 86 and Intermediate 87

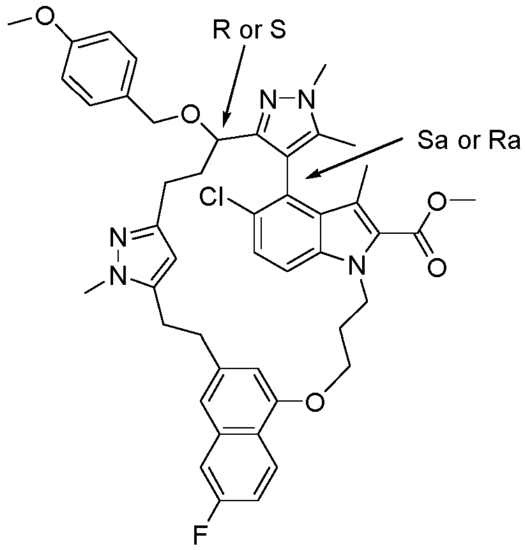

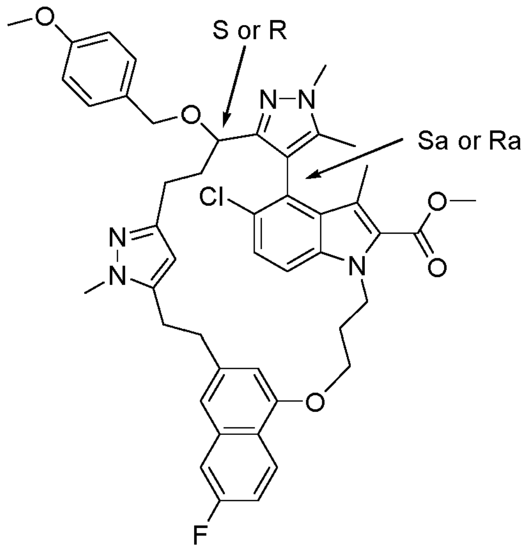

Intermediate 86 Intermediate 87

Pure stereoisomers but absolute stereochemistry undetermined

A solution of triphenylphosphine (1.02 g, 3.88 mmol, 4 eq.) in toluene (30 mL) was degassed and re-filled with nitrogen three times. It was then stirred at 70 °C under nitrogen atmosphere. A second solution of Intermediate 85 (797 mg, 0.97 mmol) and di-tert-butyl azodicarboxylate (893 mg, 3.88 mmol, 4 eq.) in toluene (30 mL) and THF (6 mL) was degassed and re-filled with nitrogen three times. Then it was added to the first solution via syringe pump (0.1 mL/minute) while stirring at 70 °C. Once the addition was complete, the reaction mixture was stirred at 70 °C for 10 minutes, then it was cooled to room temperature and the solvent was removed under reduced pressure. The residue was purified by flash column chromatography ($SiO_2$, 80 g RediSep, n-heptane/EtOAc, 100/0 to 0/100; then DCM/MeOH 100/0 to 95/5), followed by preparative HPLC (stationary phase: RP XBridge Prep C18 OBD- 5 μm, 50 x 250 mm, Mobile phase: 0.25 % $NH_4HCO_3$ solution in water, $CH_3CN$), to yield Intermediate 86 (34 mg, yield: 4 %) and Intermediate 87 (49 mg, yield: 6 %), both as colorless oils.

## Intermediate 88

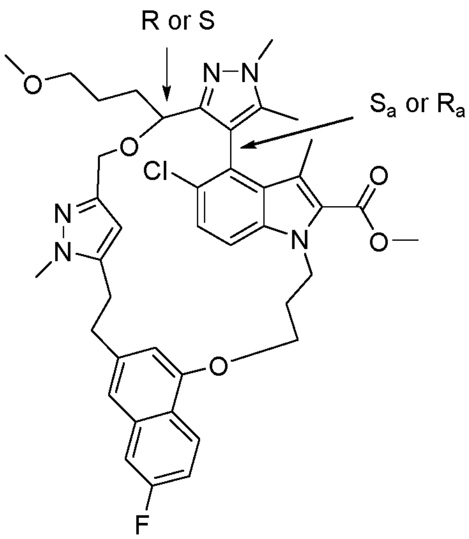

Pure stereoisomer but absolute stereochemistry undetermined

NaH (60 % dispersion in mineral oil, 3 mg, 0.082 mmol, 2 eq.) was added to a solution of Intermediate 38 (30 mg, 0.041 mmol) and MeI (25 µL, 0.041 mmol, 10 eq.) in DMF (1.5 mL), at room temperature and the reaction mixture was stirred at 45 °C overnight. The reaction was quenched by adding saturated aqueous $NH_4Cl$ and the mixture was extracted with EtOAc (3 x). The combined organic layers were washed with brine, dried over $MgSO_4$, filtered, and evaporated to provide Intermediate 88 (15 mg, yield: 49 %), used without further purification.

## Intermediate 89

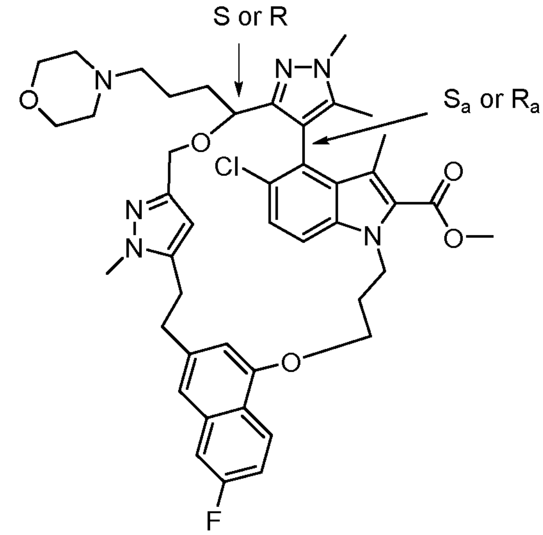

Pure stereoisomer but absolute stereochemistry undetermined

MsCl (22 µL, 0.28 mmol, 6 eq) was added dropwise to a cold (0 °C ) solution of Intermediate 39 (34 mg, 0.047 mmol) and $Et_3N$ (52 µL, 0.73 g/mL, 0.37 mmol, 8 eq) in THF (2 mL). The reaction mixture was then stirred at room temperature for 1 h. After cooling to 0 °C, morpholine (0.12 mL, 1.4 mmol, 30 eq.) was added and the reaction mixture was stirred at 45 °C overnight. After cooling, saturated aqueous $NaHCO_3$ was added. The aqueous layer was extracted with EtOAc (3 x). The combined organic layer was washed with brine, dried over $MgSO_4$, filtered, and evaporated under reduced pressure. The residue was purified by preparative HPLC (Stationary phase: RP XBridge Prep C18 OBD-10 µm, 30 x 150 mm, Mobile phase: 0.25 % $NH_4HCO_3$ solution in water, MeOH) to provide Intermediate 89 (7 mg, yield: 19 %).

## Intermediate 90

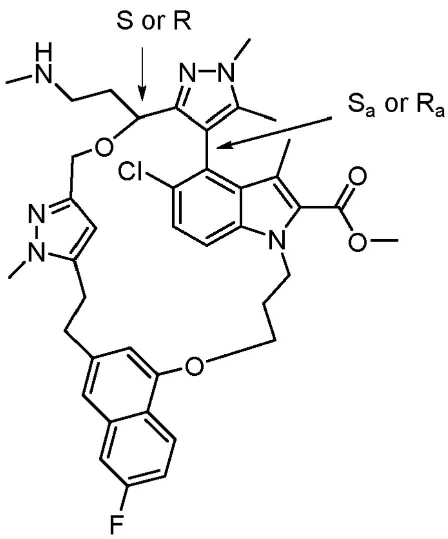

Pure stereoisomer but absolute stereochemistry undetermined

A solution of methylamine in THF (0.26 mL, 2 M, 0.52 mmol, 2.5 eq) was added at room temperature to a solution of Intermediate 58 (150 mg, 0.21 mmol) in MeOH (1.8 mL) and acetic acid (261 μL). Sodium cyanoborohydride (66 mg, 1.05 mmol, 5 eq.) was added after 30 min and the reaction mixture was stirred overnight at room temperature. The solvents were evaporated and the residue was diluted with EtOAc and washed with saturated aqueous $NaHCO_3$. The organic layer was washed with brine, dried over $MgSO_4$, filtered, and evaporated under reduced pressure. The residue was purified by preparative HPLC (Stationary phase: RP XBridge Prep C18 OBD-10 μm, 30 x 150 mm, Mobile phase: 0.25 % $NH_4HCO_3$ solution in water, MeOH), followed by flash column chromatography on silica gel (DCM/$NH_3$(MeOH), 100/0 to 95/5), to provide Intermediate 90 (15 mg, yield: 10 %).

## Intermediate 91

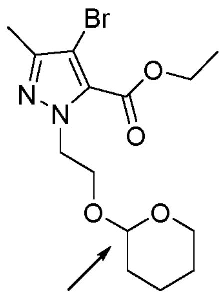

mixture of R and S

2-(Tributylphosphoranylidene)acetonitrile (37.1 mL, 141.592 mmol, 1.5 eq.) was added dropwise via addition funnel to a solution of 1H-pyrazole-3-carboxylic acid, 4-bromo-5-methyl-, ethyl ester (CAS [6076-14-8], 22 g, 94.39 mmol) and 2-(tetrahydro-2H-pyran-2-yloxy)ethanol (15.7 mL, 113.273 mmol, 1.2 eq.) in THF (100 mL) at 0 °C and the reaction mixture was stirred at room temperature overnight. The solvent was evaporated and the residue was taken up with EtOAc and water. The organic layer was separated, dried over $MgSO_4$, filtered, and evaporated under reduced pressure. The residue was purified twice by flash column chromatography on silica gel (n-heptane/EtOAc, 100/0 to 20/80) to give Intermediate 91 (17.21 g, yield: 50 %).

## Intermediate 92

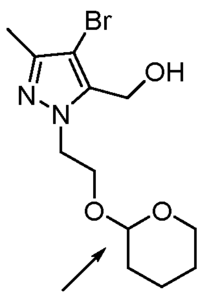

mixture of R and S

Sodium borohydride (21.99 g, 581.34 mmol, 10 eq.) was added to a stirred solution of Intermediate 91 (21 g, 58.13 mmol) in THF (340 mL) and MeOH (75 mL) at 0 °C. The reaction mixture was stirred at room temperature for 24 h. The mixture was cooled to 0 °C, treated with saturated aqueous $NH_4Cl$ and EtOAc, and the resulting mixture was stirred for 15 min at room temperature. The layers were separated and the aqueous layer was extracted with EtOAc. The combined organic layer was dried over $MgSO_4$, filtered, and evaporated. The residue was purified by flash column chromatography on silica gel (MeOH/DCM 0/100 to 10/90) to yield Intermediate 92 (15.4 g yield: 83 %) as a clear oil which solidified to a white solid upon standing.

## Intermediate 93

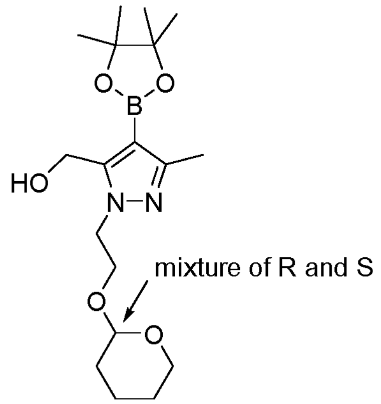


$Et_3N$ (7.7 mL, 55.451 mmol, 3 eq.) followed by pinacolborane (CAS [25015-63-8], 5.9 mL, 39.441 mmol, 2 eq.) were added dropwise to a nitrogen-degassed solution of Intermediate 92 (5.9 g, 18.484 mmol), bis(acetonitrile)dichloropalladium(II) (CAS [14592-56-4], 240 mg, 0.924 mmol, 0.05 eq.) and 2-dicyclohexylphosphino-2',6'-dimethoxybiphenyl (CAS [657408-07-6], 1.518 g, 3.697 mmol, 0.2 eq.) in 1,4-dioxane (65 mL) at room temperature. The resulting mixture was stirred at 80 °C for 1 h. The mixture was cooled to room temperature and was diluted with water (20 mL). The layers were separated and the aqueous layer was extracted with EtOAc (x 3). The combined organic layer was washed with water, then brine, dried over $MgSO_4$, filtered, and evaporated. The residue was purified by flash column chromatography on silica gel (n-heptane/EtOAc, 100/0 to 50/50) to give Intermediate 93 (4.7 g, yield: 69 %) as a pale brown solid.

## Intermediate 94

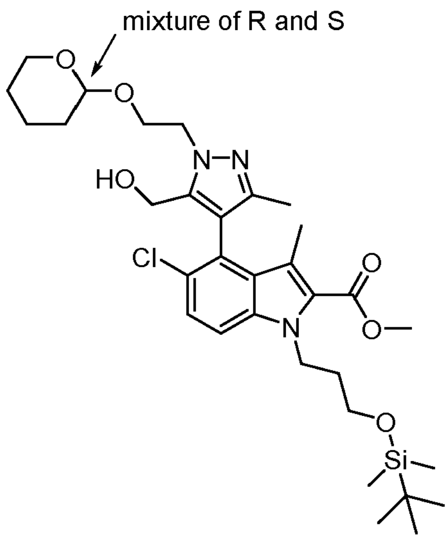


mixture of atropisomers

A reaction pressure tube was charged with Intermediate 61 (5 g, 10.17 mmol), $K_2CO_3$ (2.81 g, 20.33 mmol, 2 eq.), Intermediate 93 (4.43 g, 12.1 mmol, 1.2 eq.) and bis(di-tert-butyl(4-dimethylaminophenyl)phosphine)dichloropalladium(II) (569 mg, 0.8 mmol, 0.08 eq.) and put under nitrogen atmosphere. A mixture of water (16.5 mL) and 1,4-dioxane (82.5 mL), previously degassed by bubbling nitrogen through for 30 min was then added. The resulting mixture was degassed by bubbling nitrogen through for 5 min. The reaction mixture was stirred at 65 °C for 2 h. The reaction mixture was cooled to room temperature and was diluted with EtOAc and water. The layers were separated and the aqueous layer was extracted with EtOAc (x 3). The combined organic layer was dried over $MgSO_4$, filtered, and concentrated under reduced pressure. The residue was purified by flash column chromatography ($SiO_2$, 220 g RediSep, Heptane/EtOAc, 100/0 to 0/100) to give Intermediate 94 (4.61 g, yield: 71 %) as a yellow oil.

## Intermediate 95

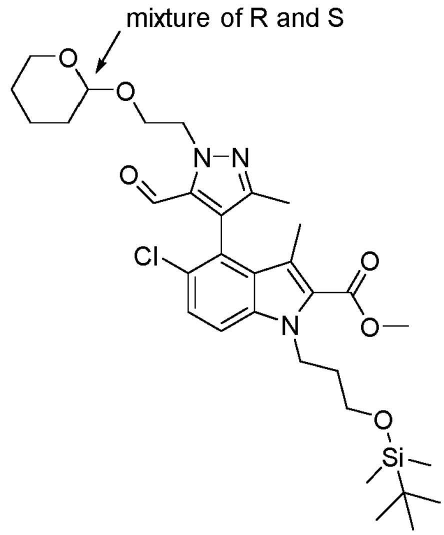

mixture of atropisomers

Solid Dess-Martin periodinane (9.16 g, 21.59 mmol, 3 eq.) was added to a stirred, ice-cooled solution of Intermediate 94 (4.61 g, 7.2 mmol) in anhydrous DCM (92 mL) under nitrogen atmosphere. The suspension was stirred at 0 °C for 5 min then at room temperature for 2 h. Additional Dess-Martin periodinane (1.07 g, 2.52 mmol, 0.35 eq.) was added and the reaction mixture was stirred at room temperature for 1 h. Again, additional Dess-Martin periodinane (1.07 g, 2.52 mmol, 0.35 eq.) was added and the reaction mixture was stirred at room temperature for 1 h. The reaction was then quenched by addition of saturated aqueous $NaHCO_3$ and 10 % aqueous $Na_2S_2O_3$. The mixture was stirred until two homogeneous phases were obtained. The layers were separated and the aqueous layer was extracted with DCM (x 3). The combined organic layer was washed with saturated aqueous $NaHCO_3$ (x 3). The organic layer was dried over $MgSO_4$, filtered, and concentrated under reduced pressure to afford Intermediate 95 (3.5 g, yield: 72 %) as a yellow thick oil, used without further purification.

## Intermediate 96

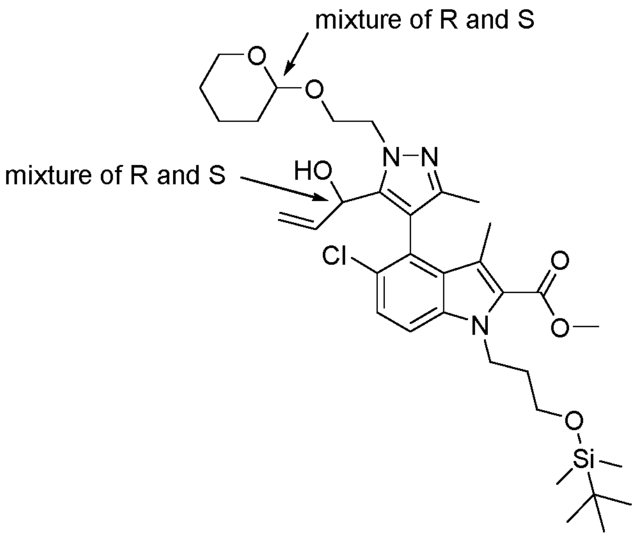

mixture of atropisomers

Vinylmagnesium bromide (1 M in MeTHF, 13.04 mL, 13.04 mmol, 2.2 eq.) was added dropwise over 10 min to a stirred solution of Intermediate 95 (4.03 g, 5.93 mmol) in anhydrous THF (34 mL) under nitrogen atmosphere at -78 °C. The reaction mixture was stirred at -78 °C for 2 h. The reaction was quenched by addition of saturated aqueous $NH_4Cl$. The mixture was diluted with EtOAc and brine was added. The layers were separated and the organic layer was washed with brine (x 1). The combined aqueous layer was extracted with EtOAc (x 3). The combined organic layer was dried over

$MgSO_4$, filtered, and concentrated under reduced pressure. The residue was purified by flash column chromatography ($SiO_2$, 120 g RediSep, heptane/EtOAc, 100/0 to 0/100) to give Intermediate 96 (3.49 g, yield: 89 %) as a thick pale yellow oil.

## Intermediate 97

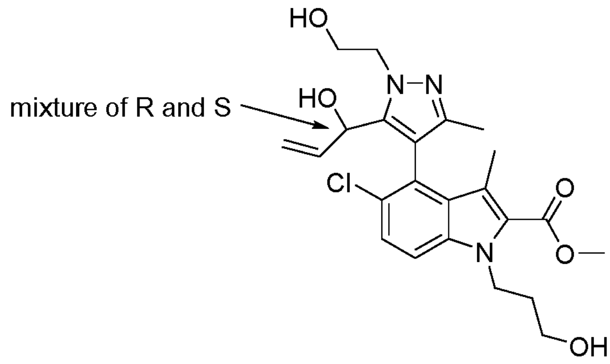


mixture of atropisomers

p-Toluenesulfonic acid, monohydrate (1.51 g, 7.93 mmol, 1.59 eq.) was added to a solution of Intermediate 96 (3.49 g, 5.29 mmol) in MeOH (116 mL). The reaction mixture was stirred at room temperature for 3 h. The solvent was removed under reduced pressure and the residue was diluted with DCM and saturated aqueous $NaHCO_3$ was added. The layers were separated and the organic layer was washed with brine. The combined aqueous layer was extracted with DCM (x 5). The combined organic layer was dried over $MgSO_4$, filtered, and concentrated under reduced pressure to afford Intermediate 97 as a pale yellow foam, used without further purification.

## Intermediate 98

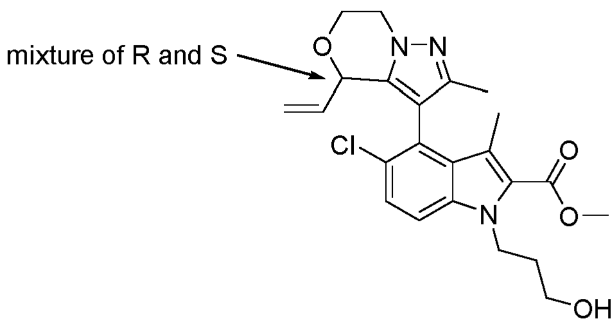


mixture of atropisomers

Pyridinium p-toluenesulfonate (391.7 mg, 1.56 mmol, 1.5 eq.) was added to a solution of Intermediate 97 (500 mg, 1.04 mmol) in toluene (51 mL) in a pressure tube. The tube was capped and the reaction mixture was stirred at 115 °C for 2 h. After cooling, the solvent was removed under reduced pressure. The residue was purified by flash column chromatography ($SiO_2$, 40 g RediSep, DCM/MeOH, 100/0 to 90/10) to give Intermediate 98 (283.3 mg, yield: 61 %) as a thick colorless oil.

## Intermediate 99

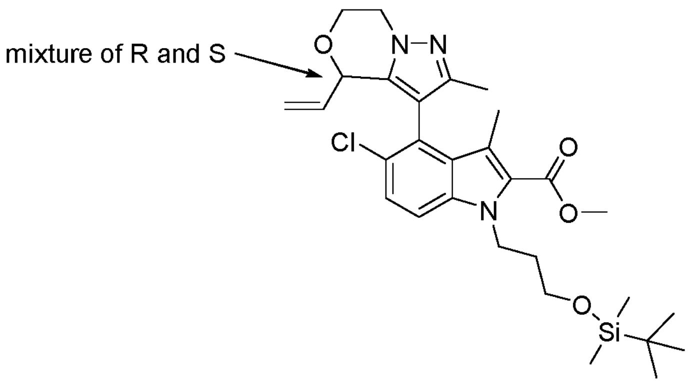


mixture of atropisomers

TBDMSCl (173 mg, 1.15 mmol, 1.8 eq.) was added to a stirred solution of Intermediate 98 (283 mg, 0.64 mmol), imidazole (87 mg, 1.28 mmol, 2 eq.) and DMAP (5 mg, 0.045 mmol, 0.07 eq.) in anhydrous DCM (2.49 mL) at 0 °C under nitrogen atmosphere. After the addition, the reaction mixture was stirred at room temperature under nitrogen for 3 h. The reaction mixture was diluted with DCM and saturated aqueous $NH_4Cl$ was added. The layers were separated and the organic layer was washed with brine. The combined aqueous layer was extracted with DCM (x 3). The combined organic layer was dried over $MgSO_4$, filtered, and evaporated. The residue was purified by flash column chromatography ($SiO_2$, 24 g RediSep, heptane/EtOAc, 100/0 to 0/100) to afford Intermediate 99 (326 mg, yield: 92 %) as a pale yellow oil.

## Intermediate 100

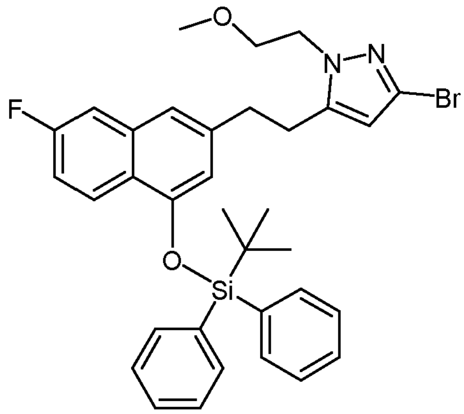

A solution of Intermediate 78 (3 g, 4.86 mmol) in anhydrous THF (24 mL) was added dropwise to a suspension of NaH (60 % dispersion in mineral oil, 583 mg, 14.57 mmol, 3 eq.) in anhydrous THF (12 mL) under nitrogen atmosphere at 0 °C. The reaction mixture was stirred at room temperature for 15 min before dimethyl sulfate (1.98 mL, 20.89 mmol, 4.3 eq.) was added. The reaction mixture was stirred at room temperature for 19 h. The reaction was quenched by addition of saturated aqueous $NH_4Cl$. The mixture was diluted with EtOAc and brine was added. The layers were separated and the organic layer was washed with brine. The combined aqueous layer was extracted with EtOAc (x 3). The combined organic layer was dried over $MgSO_4$, filtered, and concentrated under reduced pressure. The residue was purified by flash column chromatography ($SiO_2$, 80 g RediSep, heptane/EtOAc 100/0 to 0/100) to afford Intermediate 100 (2.38 g, yield: 71 %) as a colorless oil that solidified upon standing.

## Intermediate 101

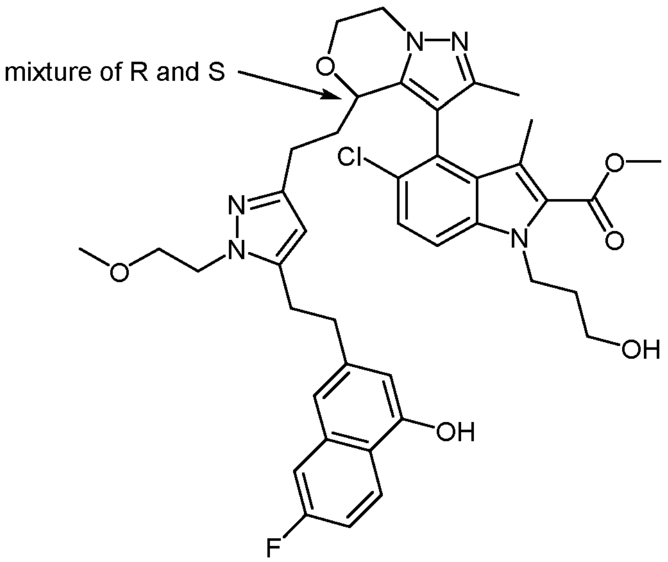


mixture of atropisomers

9-BBN (0.5 M in THF, 8.96 mL, 4.48 mmol, 5 eq.) was added dropwise to a solution of Intermediate 99 (500 mg, 0.9 mmol) in anhydrous THF (10 mL) under nitrogen atmosphere at room temperature. The reaction mixture was stirred at 75 °C for 30 min. After cooling to room temperature, solid Intermediate 100 (1057 mg, 1.52 mmol, 1.7 eq.), potassium phosphate tribasic (570.4 mg, 2.69 mmol, 3 eq.) and [1,1'-bis(di-tert-butylphosphino)ferrocene]dichloropalladium(II) (CAS [95408-45-0], 116.7 mg, 0.18 mmol, 0.2 eq.) were added in one portion to the mixture. The reaction vial was capped, then degassed and re-filled with nitrogen three times. Water (0.97 mL) was then added and the mixture was degassed by bubbling nitrogen for circa 1 min. The resulting reaction mixture was stirred at 80 °C for 6 h. The reaction mixture was diluted with EtOAc and water was added. The layers were separated. The organic layer was washed with brine, and the aqueous layer was extracted with EtOAc (x 3). The combined organic layer was dried over $MgSO_4$, filtered, and concentrated under reduced pressure to give an orange oil. The crude oil was dissolved in THF (14 mL) and was cooled to 0 °C. TBAF (1 M in THF, 5.37 mL, 5.37 mmol, 6 eq.) was added and the reaction mixture was stirred at room temperature for 4 h. Additional TBAF (1 M in THF, 1.79 mL, 1.79 mmol, 2 eq.) was added and the reaction mixture was stirred at room temperature for 3 h. The reaction was quenched by addition of saturated aqueous $NH_4Cl$. The layers were separated and the organic layer was washed with brine. The combined aqueous layer was extracted with EtOAc (x 3) and DCM (x 2) and the combined organic layer was dried over $MgSO_4$, filtered, and concentrated under reduced pressure. The residue was purified by flash column chromatography ($SiO_2$, 120 g RediSep, DCM/MeOH, 100/0 to 90/10) to give Intermediate 101 (854 mg, yield: 73 %) as a brownish foam.

## Intermediate 102, Intermediate 103, Intermediate 104, Intermediate 105

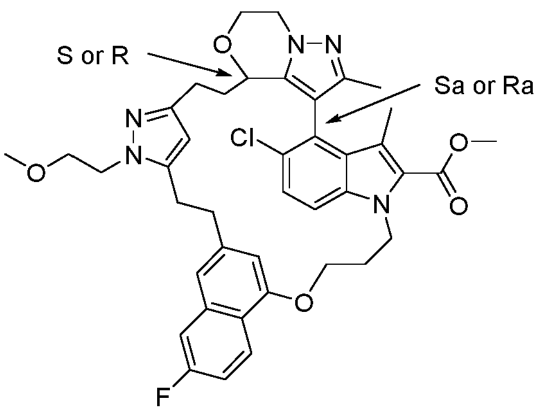


Intermediate 102

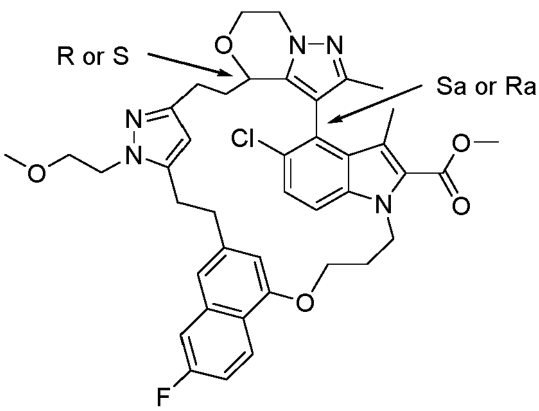


Intermediate 103

Pure stereoisomers but absolute stereochemistry undetermined

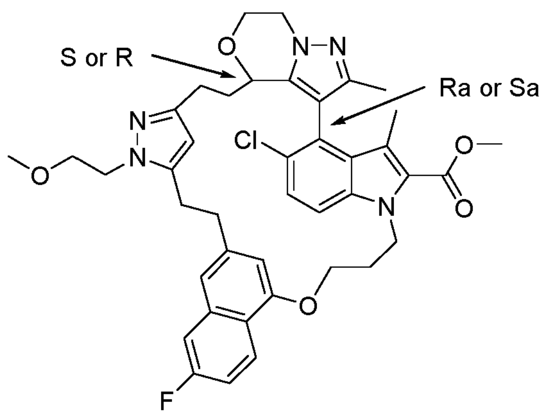

Intermediate 104

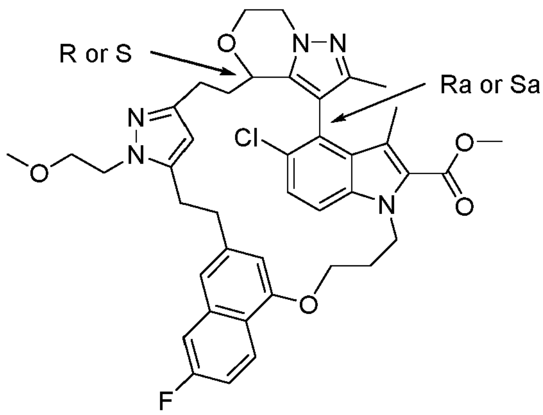

Intermediate 105

Pure stereoisomers but absolute stereochemistry undetermined

A solution of triphenylphosphine (1.3 g, 4.96 mmol, 4 eq.) in toluene (38 mL) was degassed and re-filled with nitrogen three times followed by bubbling nitrogen through for 5 min. This solution was stirred at 70 °C under nitrogen atmosphere. A solution of Intermediate 101 (940 mg, 1.24 mmol) and di-tert-butyl azodicarboxylate (1.14 g, 4.96 mmol, 4 eq.) in toluene (38 mL) and THF (7 mL) was degassed and re-filled with nitrogen three times followed by bubbling nitrogen through for 5 min. This solution was added to the first solution via syringe pump (0.1 mL/minute) while stirring at 70 °C. Once the addition was complete, the reaction mixture was stirred at 70 °C for 15 min, before it was cooled to room temperature. The solvents were evaporated and the residue was purified by flash column chromatography ($SiO_2$, 120 g RediSep, heptane/EtOAc 100/0 to 0/100; then DCM/MeOH 100/0 to 90/10), followed by another flash column chromatography ($SiO_2$, 80 g RediSep, DCM/MeOH 100/0 to 90/10), and finally by preparative HPLC (stationary phase: RP XBridge Prep C18 OBD- 5 μm, 50 x 250 mm, Mobile phase: 0.25 % $NH_4HCO_3$ solution in water, $CH_3CN$) to give two fractions. The first fraction was separated into its stereoisomers by preparative SFC (Stationary phase: Chiralpak Daicel ID 20 x 250 mm, Mobile phase: $CO_2$, iPrOH + 0.4 % $iPrNH_2$) to give Intermediate 102 (98.5 mg, yield: 11 %) and Intermediate 105 (117.5 mg, yield: 12 %), both as pale yellow foams. The second fraction was separated into its stereoisomers by preparative SFC (stationary phase: Chiralpak Daicel IC 20 x 250 mm, Mobile phase: $CO_2$, EtOH + 0.4 % $iPrNH_2$), followed by another preparative SFC (stationary phase: Chiralpak Daicel AD 20 x 250 mm, Mobile phase: $CO_2$, iPrOH + 0.4 % $iPrNH_2$) to give Intermediate 104 (55.4 mg, yield: 6 %) and Intermediate 103 (64.5 mg, yield: 7 %), both as pale yellow foams.

## Intermediate 106

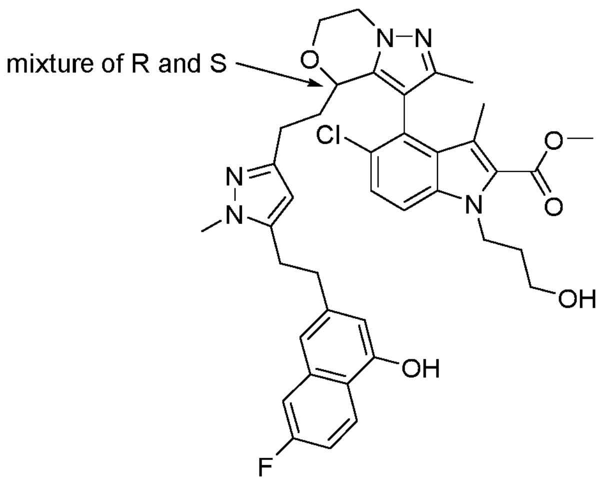

mixture of atropisomers

9-BBN (0.5 M in THF,5.85 mL, 2.92 mmol, 5 eq.) was added dropwise to a solution of Intermediate 99 (326.5 mg, 0.58 mmol) in anhydrous THF (7 mL) under nitrogen atmosphere at room temperature. The reaction mixture was stirred at 75 °C for 30 min. The mixture was cooled to room temperature, and solid Intermediate 71 (584.3 mg, 0.99 mmol, 1.7 eq.), potassium phosphate tribasic (372.5 mg, 1.75 mmol, 3 eq.), and [1,1'-bis(di-tert-butylphosphino)ferrocene]dichloropalladium(II) (76.2 mg, 0.12 mmol, 0.2) were added in one portion to the mixture. The vial was capped, then degassed and re-filled with nitrogen three times. Water (0.6 mL) was added and the mixture was degassed by bubbling nitrogen through for a short time. The resulting mixture was stirred at 80 °C for 7 h. The reaction mixture was diluted with EtOAc and water was added. The layers were separated and the organic layer was washed with brine. The aqueous layer was extracted with EtOAc (x 3). The combined organic layer was dried over $MgSO_4$, filtered, and concentrated under reduced pressure. The residue was dissolved in THF (9.5 mL) and this solution was cooled to 0 °C. Tetrabutylammonium fluoride (1 M in THF, 5.85 mL, 5.85 mmol, 10 eq.) was added and the reaction mixture was allowed to warm to room temperature and was stirred for 3 h. The reaction was quenched by addition of saturated aqueous $NH_4Cl$ and the layers were separated. The organic layer was washed twice with brine. The combined aqueous layer was extracted with EtOAc (x 3) and the combined organic layer was dried over $MgSO_4$, filtered, and concentrated under reduced pressure. The residue was purified by flash column chromatography ($SiO_2$, 120 g RediSep, DCM/MeOH, 100/0 3 to 90/10) to give Intermediate 106 (354 mg, yield: 81 %) as a brown foam.

## Intermediate 107, Intermediate 108, Intermediate 109, and Intermediate 110.

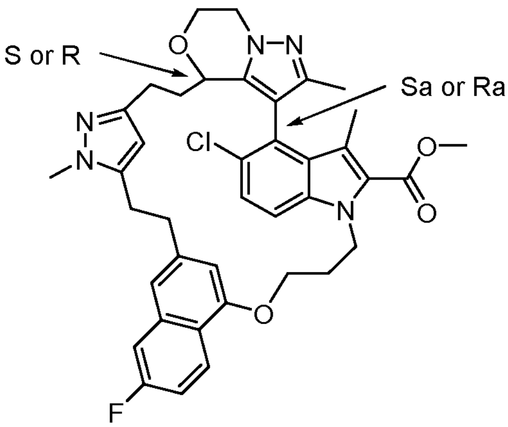

Intermediate 107

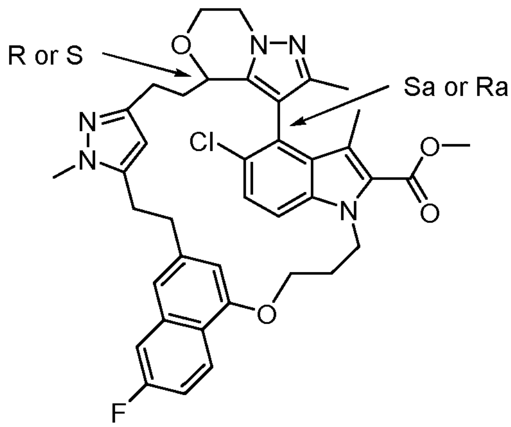

Intermediate 108

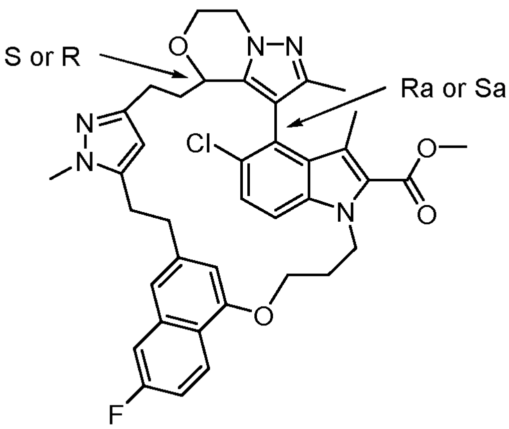

Intermediate 109

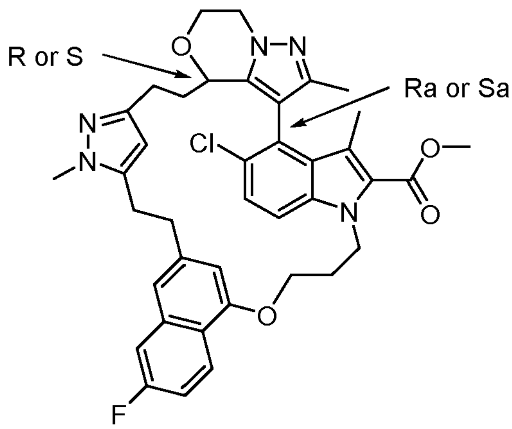

Intermediate 110

A solution of triphenylphosphine (499 mg, 1.9 mmol, 4 eq.) in toluene (15 mL) was degassed and re-filled with nitrogen three times, followed by bubbling nitrogen through for 5 min. This solution was then heated at 70 °C under nitrogen atmosphere. A solution of Intermediate 106 (354 mg, 0.48 mmol) and di-tert-butyl azodicarboxylate (438 mg, 1.9 mmol, 4 eq.) in toluene (15 mL) and THF (3 mL) was degassed and re-filled with nitrogen three times followed by bubbling nitrogen through for 5 min. This solution was then added to the first solution via syringe pump (0.1 mL/minute) while stirring at 70 °C.

Once the addition was complete, the reaction mixture was stirred at 70 °C for 15 min. After cooling, the solvents were removed under reduced pressure. The residue was purified by flash column chromatography ($SiO_2$, 40 g RediSep, heptane/EtOAc, 100/0 to 0/100; then DCM/MeOH 97/3 to 90/10), followed by preparative HPLC (Stationary phase: RP XBridge Prep C18 OBD- 5 μm, 50 x 250 mm, Mobile phase: 0.25 % $NH_4HCO_3$ solution in water, $CH_3CN$). The obtained mixture of stereoisomers was separated by preparative SFC (stationary phase: Chiralpak Diacel AD 20 x 250 mm, Mobile phase: $CO_2$, EtOH-iPrOH (50-50) + 0.4 % $iPrNH_2$) to give Intermediate 107 (31.2 mg, yield: 5 %), Intermediate 108 (44.4 mg, yield: 8 %), Intermediate 109 (46.6 mg, yield: 8 %), and Intermediate 110 (28.1 mg, yield: 5 %).

COMPOUNDS

**Compound 1**

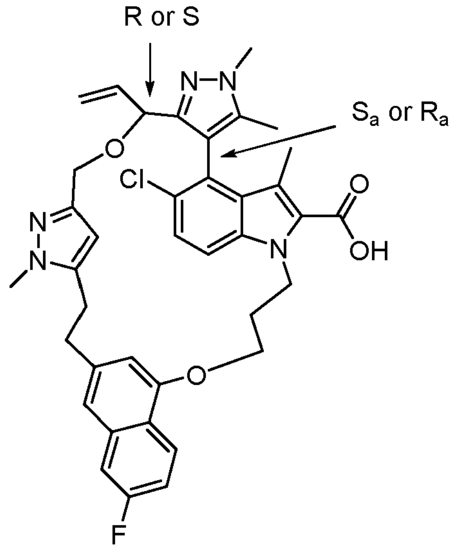

Pure stereoisomer but absolute stereochemistry undetermined

LiOH (15 mg, 0.63 mmol, 17 eq.) was added to a solution of Intermediate 24 (40 mg, 0.057 mmol) in MeOH (1 mL), THF (1 mL), and water (0.5 mL) and the resulting mixture was stirred at 46 °C for 4.5 h. The solvents were evaporated and the residue was dissolved in water (10 mL). The aqueous layer was extracted with DCM. The aqueous layer was treated with HCl (1 M, 0.92 mL, 0.92 mmol, 16 eq.) until pH = 2. The cloudy aqueous layer was then extracted with $CHCl_3$ (3 x). The combined organic layer was dried over $MgSO_4$, filtered, and evaporated to give Compound 1 (25 mg, yield: 64 %).

$^1H$ NMR (400 MHz, CHLOROFORM-*d*) δ ppm 2.12 (s, 3 H) 2.16 (s, 3 H) 2.20 - 2.28 (m, 1 H) 2.31 - 2.41 (m, 1 H) 2.84 - 3.02 (m, 4 H) 3.40 (s, 3 H) 3.50 (td, J=9.09, 3.34 Hz, 1 H) 3.62 - 3.71 (m, 1 H) 3.75 (d, J=11.08 Hz, 1 H) 3.88 (s, 3 H) 4.21 (d, J=11.08 Hz, 1 H) 4.50 - 4.59 (m, 1 H) 4.68 (d, J=7.11 Hz, 1 H) 5.04 - 5.13 (m, 2 H) 5.14 - 5.22 (m, 1 H) 5.52 (s, 1 H) 5.91 (s, 1 H) 5.99 (ddd, J=17.17, 10.22, 7.11 Hz, 1 H) 6.99 (d, J=8.99 Hz, 1 H) 7.18 (d, J=9.09 Hz, 1 H) 7.21 (s, 1 H) 7.22 - 7.25 (m, 1 H) 7.32 - 7.38 (m, 1 H) 8.35 (dd, J=9.15, 5.70 Hz, 1 H).

## Compound 2

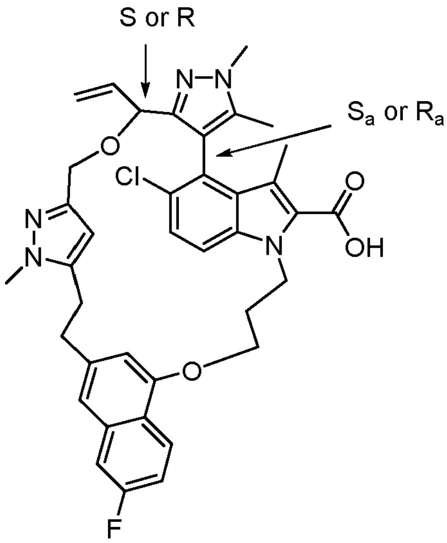

Pure stereoisomer but absolute stereochemistry undetermined

Compound 2 was prepared by an analogous reaction protocol as Compound 1, starting from Intermediate 25 instead of Intermediate 24. The crude Compound 2 was purified by preparative HPLC (Stationary phase: RP XBridge Prep C18 OBD- 5 μm, 50 x 250 mm, Mobile phase: 0.25 % $NH_4HCO_3$ solution in water, $CH_3CN$).

$^1H$ NMR (400 MHz, CHLOROFORM-*d*) δ ppm 2.06 (d, J=9.93 Hz, 6 H) 2.13 - 2.17 (m, 1 H) 2.29 (br s, 2 H) 2.73 - 2.97 (m, 4 H) 3.19 (s, 3 H) 3.35 (br d, J=6.69 Hz, 1 H) 3.46 (br d, J=5.02 Hz, 1 H) 3.61 - 3.78 (m, 1 H) 3.83 (s, 3 H) 3.96 (br d, J=11.39 Hz, 1 H) 4.42 (br d, J=6.17 Hz, 2 H) 4.46 (br d, J=4.70 Hz, 1 H) 5.16 (br d, J=10.97 Hz, 2 H) 5.22 - 5.37 (m, 1 H) 5.53 (s, 1 H) 5.68 (br s, 1 H) 6.08 - 6.20 (m, 1 H) 7.11 (s, 1 H) 7.19 (td, J=8.78, 2.40 Hz, 1 H) 7.24 - 7.26 (m, 1 H) 7.28 (br s, 1 H) 7.30 - 7.37 (m, 1 H) 8.24 (dd, J=9.14, 5.80 Hz, 1 H).

## Compound 3

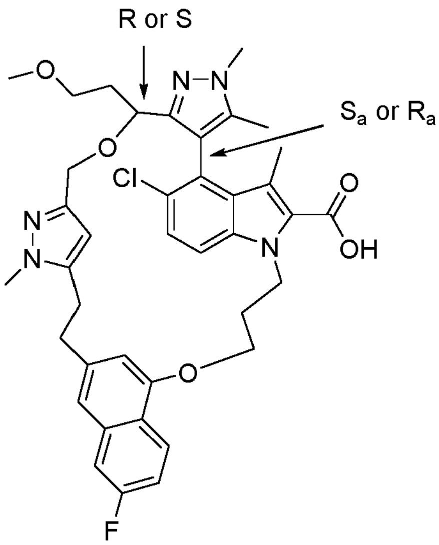

Pure stereoisomer but absolute stereochemistry undetermined

LiOH (15 mg, 0.63 mmol, 27 eq.) was added to a solution of Intermediate 28 (17 mg, 0.023 mmol) in THF (1 mL), MeOH (1 mL), and water (0.5 mL). The resulting mixture was stirred at 45 °C overnight. The solvents were evaporated and the residue was purified by preparative HPLC (Stationary phase: RP XBridge Prep C18 OBD- 5 μm, 50 x 250 mm, Mobile phase: 0.25 % $NH_4HCO_3$ solution in water, $CH_3CN$) to provide Compound 3 (3 mg, 19 % yield).

$^1H$ NMR (400 MHz, CHLOROFORM-*d*) δ ppm 1.82 - 1.90 (m, 1 H) 1.97 (m, J=8.20 Hz, 1 H) 2.08 (br d, J=15.15 Hz, 8 H) 2.19 - 2.32 (m, 2 H) 2.81 - 2.92 (m, 2 H) 2.98 (br d, J=11.29 Hz, 2 H) 3.23 (br s, 7 H) 3.40 (m, J=5.10 Hz, 2 H) 3.45 - 3.52 (m, 2 H) 3.87 (s, 3 H) 4.03 - 4.11 (m, 2 H) 4.37 - 4.45 (m, 1 H) 4.45 - 4.56 (m, 1 H) 5.16 - 5.28 (m, 1 H) 5.59 (br s, 1 H) 5.86 (s, 1 H) 7.04 (br d, J=9.09 Hz, 1 H) 7.18 - 7.25 (m, 3 H) 7.31 - 7.38 (m, 1 H) 8.34 (br dd, J=8.20, 6.64 Hz, 1 H).

## Compound 4

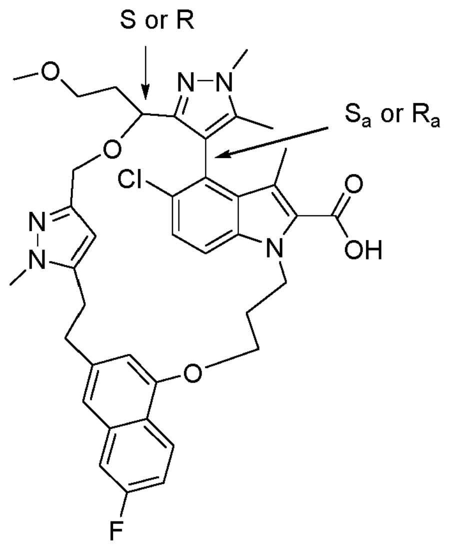


Pure stereoisomer but absolute stereochemistry undetermined

LiOH (16.44 mg, 0.69 mmol, 20 eq.) was added to a solution of Intermediate 29 (25 mg, 0.034 mmol) in THF (1 mL), MeOH (1 mL) and water (0.5 mL). The reaction mixture was stirred at 50 °C for 3 h. The solvents were evaporated to dryness. The residue was dissolved in water (2 mL), cooled to 0 °C, and treated with HCl (1 M, 0.69 mL, 0.69 mmol, 20 eq.). The aqueous layer was extracted with $CHCl_3$ (x 3). The combined organic layer was dried over $MgSO_4$, filtered, and evaporated to give Compound 4 (20 mg, yield: 81 %).

$^1H$ NMR (400 MHz, CHLOROFORM-*d*) δ ppm 1.87 - 1.92 (m, 1 H) 2.09 (s, 3 H) 2.12 (s, 3 H) 2.21 - 2.33 (m, 4 H) 2.67 - 2.82 (m, 2 H) 2.92 (s, 3 H) 2.94 - 3.07 (m, 2 H) 3.00 - 3.07 (m, 1 H) 3.27 (s, 3 H) 3.30 - 3.37 (m, 1 H) 3.40 (dt, J=9.30, 4.55 Hz, 1 H) 3.48 - 3.57 (m, 2 H) 3.78 (d, J=10.35 Hz, 1 H) 3.87 (s, 3 H) 4.05 (t, J=7.32 Hz, 1 H) 4.47 (dt, J=15.10, 3.74 Hz, 1 H) 5.23 (s, 1 H) 5.24 - 5.34 (m, 1 H) 5.83 (s, 1 H) 7.19 (s, 1 H) 7.21 - 7.26 (m, 1 H) 7.31 (dd, J=10.03, 2.51 Hz, 1 H) 7.33 - 7.36 (m, 1 H) 7.36 -7.39 (m, 1 H) 8.30 (dd, J=9.20, 5.85 Hz, 1 H).

**Compound 5 and Compound 6**

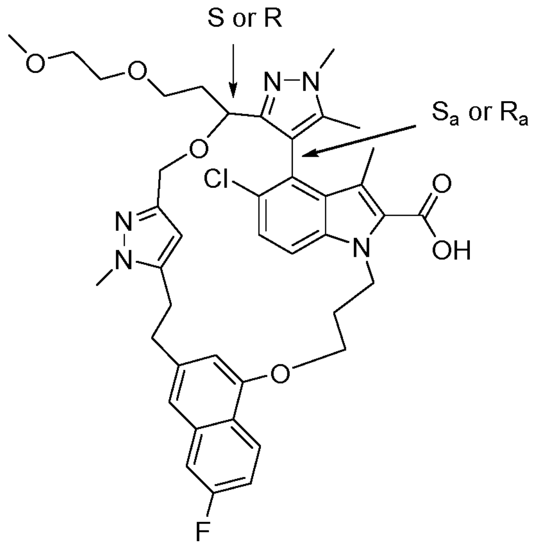

Compound 5

(ammonium salt)

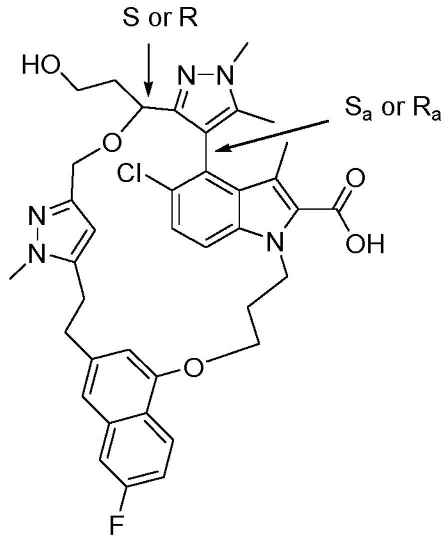

Compound 6

(ammonium salt)

Both compounds are pure stereoisomers but absolute stereochemistry undetermined

LiOH (25 mg, 1.04 mmol, 51 eq.) was added to a solution of the mixture of Intermediate 30 and Intermediate 27 (30 mg, 0.02 mmol) in THF (1 mL), MeOH (1 mL), and water (0.5 mL). The reaction mixture was stirred at 50 °C for 3.5 h. The solvents were evaporated and the residue was purified by preparative HPLC (Stationary phase: RP XBridge Prep C18 OBD- 5 μm, 50 x 250 mm, Mobile phase: 0.25 % $NH_4HCO_3$ solution in water, $CH_3CN$) to afford Compound 5 (4 mg, yield: 25 %) and Compound 6 (9 mg, yield: 62 %), both as ammonium salts.

Compound 5

$^1$H NMR (400 MHz, CHLOROFORM-*d*) δ ppm 1.85 (br s, 2 H); 1.91 - 2.10 (m, 7 H); 2.21 (br s, 2 H); 2.30 (br s, 2 H); 2.42 - 2.53 (m, 1 H); 2.79 (br s, 3 H); 3.15 (br s, 3 H); ; 3.26 - 3.42 (m, 5 H); 3.44 - 3.54 (m, 4 H); 3.69 - 3.91 (m, 4 H); 4.13 (br s, 1 H); 4.31 - 4.49 (m, 1 H); 5.30 (s, 1 H); 5.23 - 6.01 (m, 1 H); 7.09 (br s, 1 H); 7.17 (br s, 2 H); 7.26 - 7.36 (m, 3 H); 7.95 - 8.48 (m, 1 H).

Compound 6

$^1$H NMR (400 MHz, CHLOROFORM-*d*) δ ppm 2.02 (br d, J=6.27 Hz, 4 H) 2.06 (br s, 3 H) 2.29 (br s, 3 H) 2.77 - 2.92 (m, 4 H) 3.17 (br s, 3 H) 3.25 (br s, 1 H) 3.34 (br s, 1 H) 3.67 (br s, 2 H) 3.74 (br d, J=11.39 Hz, 1 H) 3.83 (br s, 4 H) 4.20 - 4.27 (m, 1 H) 4.46 (m, J=15.20 Hz, 1 H) 5.26 (br d, J=14.21 Hz, 1 H) 5.50 (br s, 1 H) 5.55 (br s, 1 H) 7.14 (s, 1 H) 7.17 - 7.24 (m, 2 H) 7.27 - 7.35 (m, 2 H) 8.27 (br s, 1 H).

## Compound 7

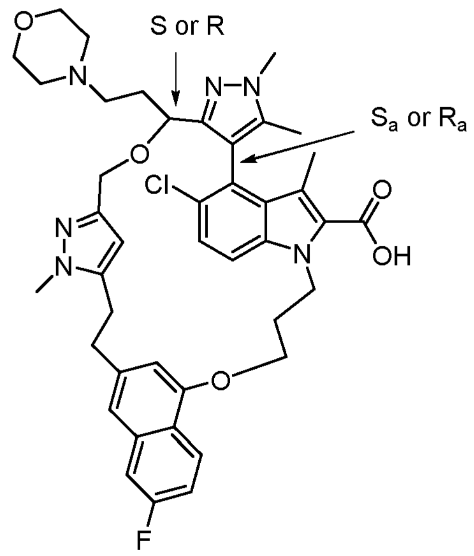

Pure stereoisomer but absolute stereochemistry undetermined

LiOH (10 mg, 0.43 mmol, 25 eq.) was added to a solution of Intermediate 31 (13 mg, 0.017 mmol) in THF (1 mL), MeOH (1 mL), and water (0.5 mL) and the resulting solution was stirred at 50 °C overnight. The volatiles were evaporated to dryness and the residue was dissolved in water (1 mL), and treated with aqueous HCl (1 M, 0.2 mL, 0.2 mmol, 12 eq.) until pH = 6. The layers were separated, and the aqueous layer was extracted with $CHCl_3$ (x 3). The combined organic layer was dried over $MgSO_4$, filtered, and evaporated to provide Compound 7 (8 mg, yield: 60 %).

$^1$H NMR (400 MHz, CHLOROFORM-*d*) δ ppm 2.06 (s, 3 H); 2.07 (s, 3 H); 2.15 - 2.34 (m, 5 H); 2.51 (br s, 6 H); 2.44 - 2.50 (m, 2 H); 2.77 - 2.97 (m, 4 H); 3.13 (s, 3 H); 3.20 (m, J=6.20 Hz, 1 H); 3.32 - 3.40 (m, 1 H); 3.66 - 3.79 (m, 6 H); 3.83 (s, 3 H); 4.06 (br t, J=6.82 Hz, 1 H); 4.42 - 4.52 (m, 2 H); 5.19 - 5.30 (m, 1 H); 5.48 (s, 1 H); 5.56 (s, 1 H); 7.16 (s, 1 H); 7.18 - 7.25 (m, 2 H); 7.27 - 7.36 (m, 2 H); 8.29 (dd, J=9.24, 5.72 Hz, 1 H).

## Compound 8

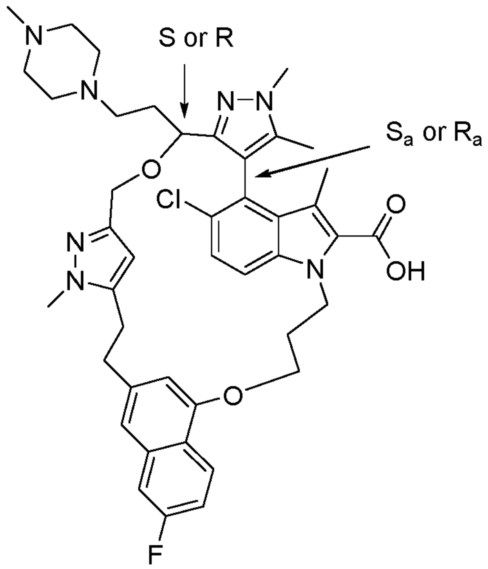

Pure stereoisomer but absolute stereochemistry undetermined

Compound 8 was prepared by an analogous reaction protocol as Compound 7, starting from Intermediate 32 instead of Intermediate 31.

$^1$H NMR (400 MHz, CHLOROFORM-*d*) δ ppm 2.01 (s, 3 H); 2.07 (s, 3 H); 2.13 - 2.16 (m, 2 H); 2.30 - 2.32 (m, 2 H); 2.41 (s, 3 H); 2.60 - 2.77 (m, 8 H); 2.81 - 2.94 (m, 4 H); 3.32 (s, 3 H); 3.34 - 3.44 (m, 2 H); 3.49 (s, 1 H); 3.81 (s, 3 H); 3.93 (br d, J=11.18 Hz, 1 H); 4.15 (br t, J=6.58 Hz, 1 H); 4.41 - 4.50 (m, 1 H); 5.19 - 5.28 (m, 1 H); 5.39 (s, 1 H); 5.73 (s, 1 H); 7.04 (d, J=8.88 Hz, 1 H); 7.14 (s, 1 H); 7.17 - 7.25 (m, 3 H); 7.32 (dd, J=9.98, 2.46 Hz, 1 H); 8.29 (dd, J=9.15, 5.70 Hz, 1 H).

## Compound 9

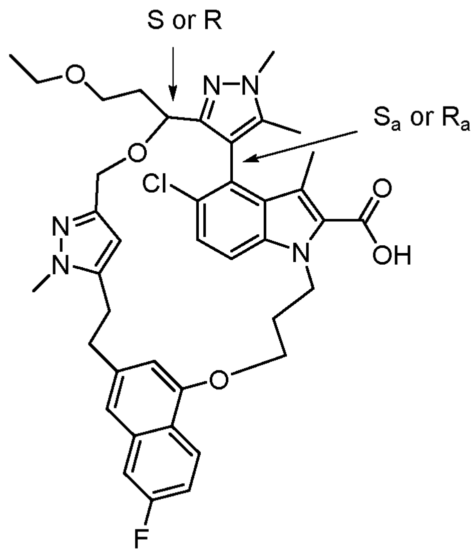

Pure stereoisomer but absolute stereochemistry undetermined

Compound 9 was prepared by an analogous reaction protocol as Compound 3, starting from Intermediate 33 instead of Intermediate 28.

$^1$H NMR (400 MHz, CHLOROFORM-*d*) δ ppm 0.08 (s, 1 H); 1.13 (t, J=7.04 Hz, 3 H); 2.07 (s, 6 H); 2.27 (br s, 4 H); 2.77 (br d, J=10.12 Hz, 2 H); 2.88 - 2.99 (m, 2 H); 3.04 (br s, 3 H); 3.13 (br s, 1 H); 3.31 - 3.44 (m, 4 H); 3.45 - 3.53 (m, 1 H); 3.67 (br d, J=10.12 Hz, 1 H); 3.78 (br d, J=10.78 Hz, 1 H); 3.85 (s, 3 H); 4.11 (br t, J=7.04 Hz, 1 H); 4.45 (br d, J=15.19 Hz, 1 H); 5.22 - 5.30 (m, 1 H); 5.41 (br s, 1 H); 5.65 (br s, 1 H); 7.15 (s, 1 H); 7.23 (br t, J=8.80 Hz, 1 H); 7.28 - 7.36 (m, 3 H); 8.27 (br dd, J=8.91, 5.83 Hz, 1 H).

## Compound 10

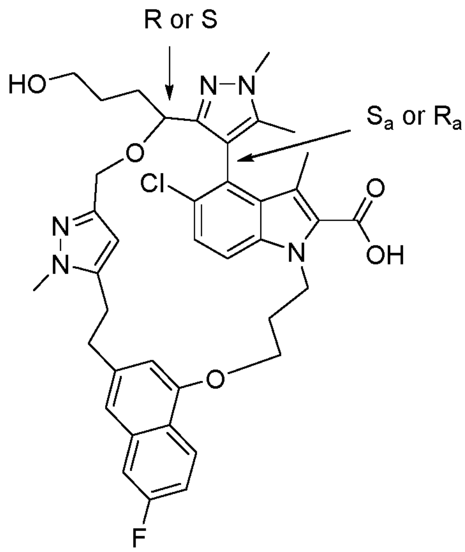

Pure stereoisomer but absolute stereochemistry undetermined

LiOH (15 mg, 0.63 mmol, 14 eq.) was added to a solution of Intermediate 38 (32 mg, 0.044 mmol) in MeOH (1 mL), THF (1 mL), and water (0.5 mL) and the resulting mixture was stirred at 40 °C for 16 h. The solvent was evaporated and the residue was dissolved in water (10 mL). This solution was washed with DCM, and treated with HCl (1 M, 0.92 mL, 0.92 mmol, 14 eq.) until pH = 2. The cloudy aqueous layer was extracted with $CHCl_3$ (3 x). The combined organic layer was dried over $MgSO_4$, filtered, and evaporated to give Compound 10 (30 mg, yield: 96 %).

$^1$H NMR (400 MHz, CHLOROFORM-*d*) δ ppm 1.62 (br s, 3 H), 1.83 - 1.95 (m, 1 H), 2.10 (s, 3 H), 2.12 (s, 3 H), 2.23 (br s, 1 H), 2.27 - 2.35 (m, 1 H), 2.80 - 3.00 (m, 4 H), 3.28 (s, 3 H), 3.37 (dt, J=11.00, 5.50 Hz, 1 H), 3.47 - 3.56 (m, 4 H), 3.87 (s, 3 H), 3.89 - 4.05 (m, 3 H), 4.12 (d, J= 11.00 Hz, 1 H), 4.29 (t, J=5.72 Hz, 1 H), 4.52 (ddd, J=14.47, 7.76, 3.08 Hz, 1 H), 5.22 (ddd, J=14.58, 7.10, 3.19 Hz, 1 H), 5.58 (s, 1 H), 5.90 (s, 1 H), 7.03 (d, J=8.80 Hz, 1 H), 7.18 - 7.25 (m, 2 H), 7.19 - 7.21 (m, 1 H), 7.29 - 7.37 (m, 1 H), 8.35 (dd, J=9.24, 5.72 Hz, 1 H).

## Compound 11

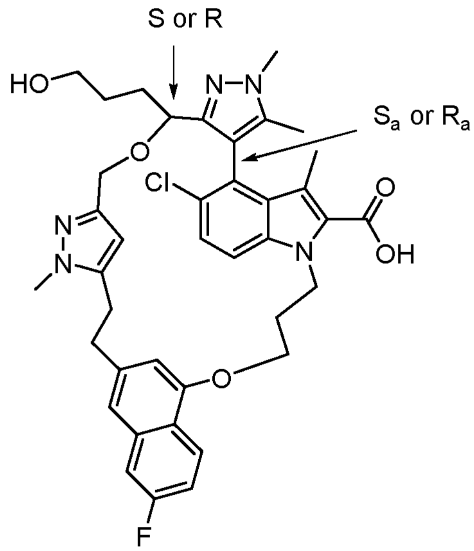


Pure stereoisomer but absolute stereochemistry undetermined

Compound 11 was prepared by an analogous reaction protocol as Compound 10, starting from Intermediate 39 instead of Intermediate 38.

$^1$H NMR (400 MHz, CHLOROFORM-*d*) δ ppm 1.45 - 1.58 (m, 2 H), 1.58 - 1.70 (m, 1 H), 1.91 - 2.05 (m, 2 H), 2.09 (s, 3 H), 2.10 (s, 3 H), 2.12 - 2.23 (m, 1 H), 2.23 - 2.33 (m, 2 H), 2.72 - 2.85 (m, 2 H), 2.92 - 3.03 (m, 3 H), 3.05 (s, 3 H), 3.07 - 3.12 (m, 1 H), 3.34 - 3.43 (m, 1 H), 3.43 - 3.50 (m, 1 H), 3.50 - 3.57 (m, 1 H), 3.64 - 3.72 (m, 1 H), 3.72 - 3.78 (m, 1 H), 3.86 (s, 3 H), 3.90 (t, J=7.04 Hz, 1 H), 4.48 (dt, J=14.91, 4.21 Hz, 1 H), 5.20 - 5.23 (m, 1 H), 5.22 - 5.36 (m, 1 H), 5.40 (s, 1 H), 5.71 (s, 1 H), 5.85 - 5.97 (m, 1 H), 7.16 (s, 1 H), 7.22 (td, J=8.80, 2.64 Hz, 1 H), 7.26 - 7.30 (m, 1 H), 7.32 (s, 1 H), 7.33 - 7.36 (m, 1 H), 8.29 (dd, J=9.24, 5.72 Hz, 1 H).

## Compound 12

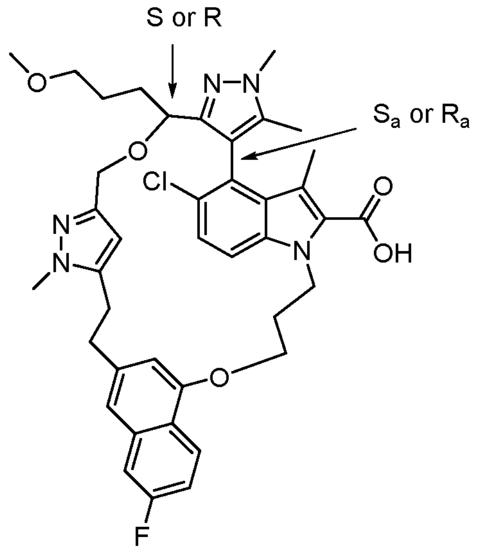

Pure stereoisomer but absolute stereochemistry undetermined

LiOH (6 mg, 0.25 mmol, 25 eq.) was added to a solution of Intermediate 40 (7 mg, 0.01 mmol) in MeOH (1 mL), THF (1 mL), and water (0.5 mL) and the resulting mixture was stirred at 50 °C for 16 h. The solvents were evaporated and the residue was dissolved in water (10 mL). The aqueous layer was washed with DCM, and treated with HCl (1 M, 0.25 mL, 0.25 mmol, 25 eq.) until pH = 2. The cloudy aqueous layer was extracted with $CHCl_3$ (3 x). The combined organic layer was dried over $MgSO_4$, filtered, and evaporated to give Compound 12 (6 mg, yield: 79 %).

$^1$H NMR (400 MHz, CHLOROFORM-*d*) δ ppm 1.49 - 1.68 (m, 5 H), 2.00 - 2.10 (m, 6 H), 2.11 (s, 2 H), 2.26 (br dd, J=8.47, 4.51 Hz, 2 H), 2.69 - 2.81 (m, 2 H) 2.94 (s, 3 H), 2.96 - 3.04 (m, 3 H), 3.28 (s, 3 H), 3.34 (t, J=6.82 Hz, 2 H), 3.39 (dt, J=9.35, 4.79 Hz, 1 H), 3.56 (d, J=10.34 Hz, 1 H), 3.72 (d, J=10.34 Hz, 1 H), 3.81 (t, J=7.26 Hz, 1 H), 3.86 (s, 3 H), 4.46 (dt, J=15.02, 3.82 Hz, 1 H), 5.26 (s, 1 H), 5.27 - 5.34 (m, 1 H), 5.79 (s, 1 H), 7.17 (s, 1 H), 7.21 - 7.25 (m, 1 H), 7.30 (dd, J=10.01, 2.53 Hz, 1 H), 7.32 - 7.38 (m, 2 H), 8.28 (dd, J=9.24, 5.72 Hz, 1 H).

## Compound 13

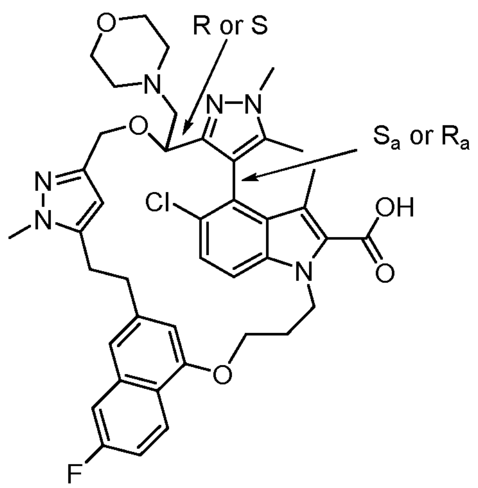

Pure stereoisomer but absolute stereochemistry undetermined

LiOH (13 mg, 0.55 mmol, 15 eq.) was added to a solution of Intermediate 53 (30 mg, 0.037 mmol) in a mixture of MeOH (1 mL), THF (1 mL), and water (0.5 mL). The reaction mixture was stirred for 4 h at 50 °C. The solvents were evaporated and the residue was purified by preparative HPLC (stationary phase: RP XBridge Prep C18 OBD- 5 μm, 50 x 250 mm, Mobile phase: 0.25 % $NH_4HCO_3$ solution in water, $CH_3CN$) to give compound 13 (11 mg, yield: 41 %) as a pale

yellow solid.

$^1$H NMR: (400 MHz, CHLOROFORM-*d*) δ ppm 2.05 (s, 3 H), 2.08 (s, 3 H), 2.29 (br d, J=5.3 Hz, 2 H), 2.41 (br s, 2 H), 2.54 (br s, 2 H), 2.71 - 2.95 (m, 5 H), 3.17 (br s, 3 H), 3.31 (br d, J=12.8 Hz, 3 H), 3.51 - 3.67 (m, 4 H), 3.84 (s, 3 H), 3.92 (br s, 2 H), 4.37 (br d, J=8.8 Hz, 1 H), 4.41 - 4.54 (m, 1 H), 5.19 - 5.29 (m, 1 H), 5.55 (br d, J=18.5 Hz, 2 H), 7.13 (br s, 1 H), 7.17 - 7.25 (m, 2 H), 7.28 - 7.35 (m, 2 H), 8.23 - 8.33 (m, 1 H).

## Compound 14

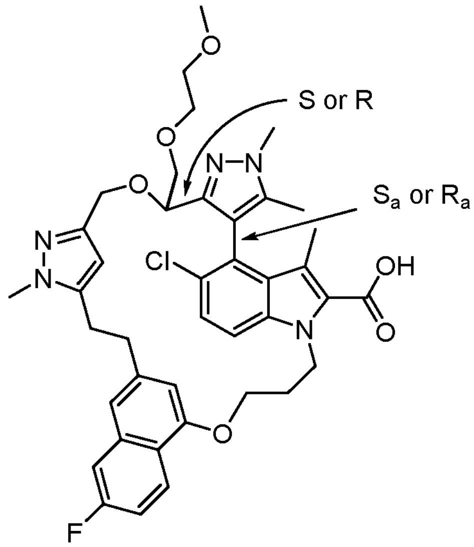


Pure stereoisomer but absolute stereochemistry undetermined

LiOH (18 mg, 0.75 mmol, 15 eq.) was added to a solution of Intermediate 54 (40 mg, 0.05 mmol) in a mixture of MeOH (1.2 mL), THF (1.2 mL), and water (0.6 mL). The reaction mixture was stirred for 4 h at 50 °C. The solvents were evaporated and the residue was dissolved in water and DCM. The mixture was acidified with 1 M aqueous HCl to pH 4-5. The cloudy aqueous layer was extracted with DCM (x 4). The combined organic layer was dried over $MgSO_4$, filtered, and evaporated. The residue was purified by preparative HPLC (Stationary phase: RP XBridge Prep C18 OBD- 5 μm, 50 x 250 mm, Mobile phase: 0.25 % $NH_4HCO_3$ solution in water, $CH_3CN$) to give compound 14 (17 mg, yield: 45 %) as a pale yellow solid.

$^1$H NMR (400 MHz, CHLOROFORM-*d*) δ ppm 1.98 - 2.05 (m, 3 H), 2.06 (br s, 3 H), 2.27 (br s, 2 H), 2.68 - 2.98 (m, 5 H), 3.06 (br s, 3 H), 3.30 (s, 3 H), 3.43 (br s, 4 H), 3.56 (br s, 3 H), 3.81 (s, 3 H), 3.98 (br s, 1 H), 4.04 - 4.12 (m, 1 H), 4.20 (br s, 1 H), 4.41 (br d, J=14.1 Hz, 1 H), 5.20 - 5.34 (m, 1 H), 5.56 (br d, J=66.2 Hz, 2 H), 7.11 (br s, 1 H), 7.14 - 7.25 (m, 2 H), 7.27 - 7.34 (m, 2 H), 8.23 (br s, 1 H).

## Compound 15

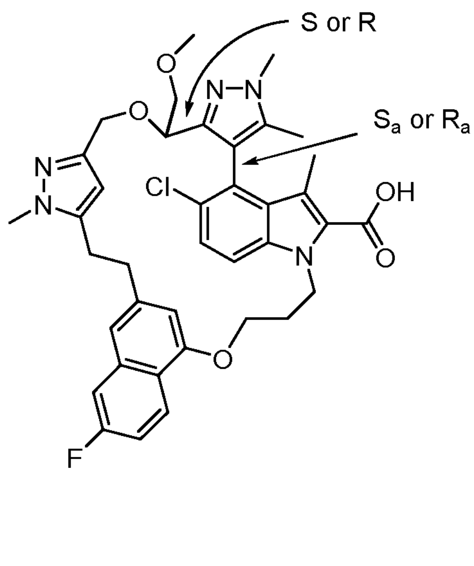

Pure stereoisomer but absolute stereochemistry undetermined

Compound 15 was prepared by an analogous reaction protocol as Compound 14, starting from Intermediate 51 instead of Intermediate 54.

$^1H$ NMR (400 MHz, CHLOROFORM-*d*) δ ppm 2.09 (s, 3 H), 2.11 (s, 3 H), 2.21 - 2.35 (m, 2 H), 2.76 (br d, J=9.6 Hz, 2 H), 2.99 (br d, J=8.3 Hz, 2 H), 3.04 (br s, 3 H), 3.13 (br s, 1 H), 3.36 (s, 3 H), 3.37 - 3.43 (m, 1 H), 3.71 (br d, J=9.6 Hz, 1 H), 3.79 (br d, J=9.6 Hz, 1 H), 3.87 (s, 3 H), 3.98 (br d, J=11.1 Hz, 1 H), 3.99 - 4.06 (m, 1 H), 4.18 (br s, 1 H), 4.43 - 4.52 (m, 1 H), 5.22 - 5.30 (m, 1 H), 5.36 (br s, 1 H), 5.85 (br s, 1 H), 7.13 - 7.19 (m, 1 H), 7.20 - 7.26 (m, 1 H), 7.28 - 7.33 (m, 2 H), 7.33 - 7.37 (m, 1 H), 8.28 (dd, J=9.1, 5.8 Hz, 1 H).

**Compound 16**

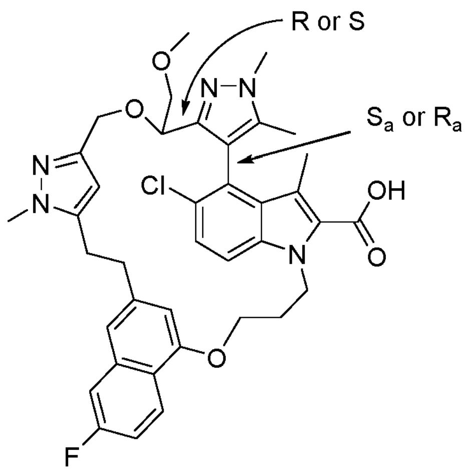

Pure stereoisomer but absolute stereochemistry undetermined

Compound 16 was prepared by an analogous reaction protocol as Compound 14, starting from Intermediate 52 instead of Intermediate 54.

$^1H$ NMR (400 MHz, CHLOROFORM-*d*) δ ppm 2.07 (s, 3 H), 2.13 (s, 3 H), 2.31 (br d, J=16.3 Hz, 2 H), 2.84 - 2.99 (m, 4 H), 3.27 (s, 3 H), 3.40 (br s, 3 H), 3.43 - 3.50 (m, 1 H), 3.64 (br d, J=5.3 Hz, 2 H), 3.70 - 3.77 (m, 1 H), 3.86 (s, 3 H), 4.01 (br d, J=11.4 Hz, 1 H), 4.21 (br d, J=10.7 Hz, 1 H), 4.47 - 4.58 (m, 2 H), 5.20 (br d, J=15.0 Hz, 1 H), 5.45 - 5.55 (m, 1 H), 5.93 (br s, 1 H), 6.94 (br d, J=7.8 Hz, 1 H), 7.19 (s, 1 H), 7.19 - 7.25 (m, 2 H), 7.30 - 7.37 (m, 1 H), 8.34 (dd, J=9.2, 5.7 Hz, 1 H).

**Compound 17**

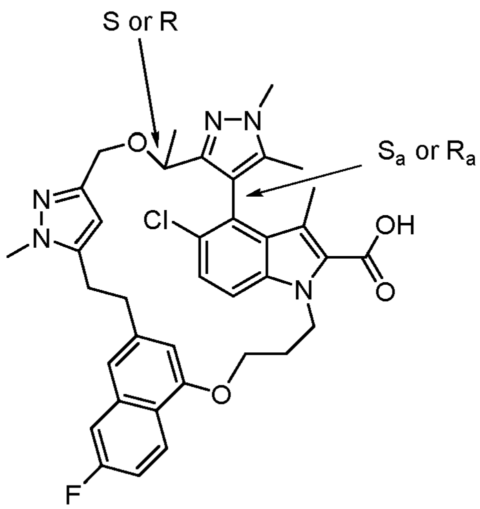

Pure stereoisomer but absolute stereochemistry undetermined

LiOH (27 mg, 1.13 mmol, 15 eq.) was added to a solution of Intermediate 56 (51 mg, 0.075 mmol) in a mixture of MeOH (2 mL), THF (2 mL), and water (1 mL). The reaction mixture was stirred for 6 h at 50 °C. The solvents were evaporated and the residue was dissolved in water and DCM and acidified with 1 M aqueous HCl to pH 4-5. The cloudy aqueous layer was extracted with DCM (x 4). The combined organic layer was dried over $MgSO_4$, filtered, and evaporated. The residue was purified by preparative SFC (stationary phase: Torus Diol 130A 30 x 150 mm, Mobile phase: $CO_2$, MeOH + 0.25 % $NH_3$) to give Compound 17 (22 mg, yield: 44 %) as a pale yellow solid.

$^1$H NMR (400 MHz, DMSO-$d_6$) δ ppm 1.10 (d, J=6.4 Hz, 3 H), 1.92 (s, 3 H), 1.94 (s, 3 H), 2.27 - 2.39 (m, 2 H), 2.86 - 2.94 (m, 2 H), 2.94 - 3.04 (m, 1 H), 3.04 - 3.13 (m, 1 H), 3.61 (s, 3 H), 3.64 - 3.73 (m, 2 H), 3.78 (s, 3 H), 3.79 - 3.85 (m, 1 H), 3.95 - 4.07 (m, 2 H), 4.58 - 4.70 (m, 1 H), 4.89 (s, 1 H), 4.97 - 5.07 (m, 1 H), 6.48 (s, 1 H), 6.94 (d, J=9.0 Hz, 1 H), 7.14 (s, 1 H), 7.30 (td, J=8.9, 2.6 Hz, 1 H), 7.45 - 7.52 (m, 2 H), 8.24 (dd, J=9.2, 5.9 Hz, 1 H), 13.03 - 13.52 (m, 1 H).

## Compound 18

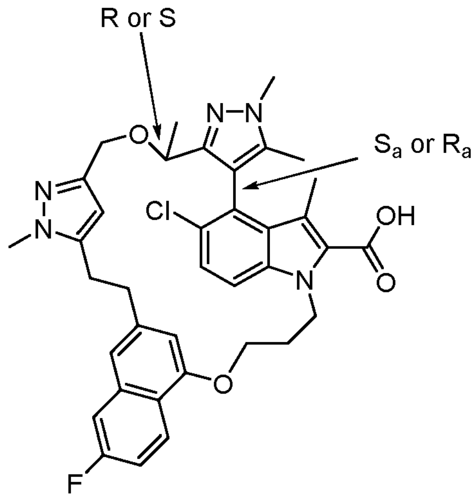


Pure stereoisomer but absolute stereochemistry undetermined

LiOH (12 mg, 0.49 mmol, 15 eq.) was added to a solution of Intermediate 57 (22 mg, 0.033 mmol) in a mixture of MeOH (0.8 mL), THF (0.8 mL), and water (0.4 mL). The reaction mixture was stirred for 6 h at 50 °C. The solvents were evaporated and the residue was dissolved in water and DCM and acidified with 1 M aqueous HCl to pH 4-5. The cloudy aqueous layer was extracted with DCM (x 4). The combined organic layer was dried over $MgSO_4$, filtered, and evaporated. The residue was purified by preparative SFC (stationary phase: Chiralcel Diacel IH 20 x 250 mm, Mobile phase: $CO_2$, EtOH + 0.4 % $iPrNH_2$) to give compound 18 (8 mg, yield: 39 %) as a pale yellow solid.

$^1$H NMR (400 MHz, CHLOROFORM-*d*) δ ppm 1.55 (d, J=6.6 Hz, 3 H), 2.10 (s, 6 H), 2.28 (br s, 2 H), 2.80 (d, J=10.3 Hz, 2 H), 2.98 (br d, J=13.6 Hz, 2 H), 3.08 (s, 3 H), 3.12 - 3.19 (m, 1 H), 3.38 - 3.47 (m, 1 H), 3.64 (br d, J=10.8 Hz, 1 H), 3.79 (d, J=10.8 Hz, 1 H), 3.87 (s, 3 H), 4.07 (q, J=6.4 Hz, 1 H), 4.42 - 4.51 (m, 1 H), 5.31 - 5.37 (m, 1 H), 5.44 (s, 1 H), 5.71 (s, 1 H), 7.16 - 7.19 (m, 1 H), 7.20 - 7.26 (m, 1 H), 7.30 (br d, J=2.4 Hz, 1 H), 7.33 (d, J=2.0 Hz, 2 H), 8.29 (dd, J=9.1, 5.8 Hz, 1 H).

## Compound 19

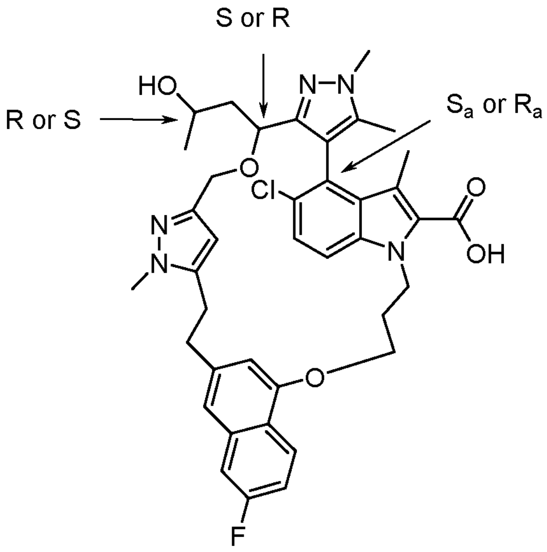

Pure stereoisomer but absolute stereochemistry undetermined

Compound 19 was prepared by an analogous reaction protocol as Compound 1, starting from Intermediate 59 instead of Intermediate 24.

$^1$H NMR (400 MHz, CHLOROFORM-*d*) δ ppm 1.14 - 1.18 (m, 3 H); 1.89 (ddd, J=14.36, 8.97, 5.28 Hz, 1 H); 2.09 (s, 3 H); 2.10 (s, 3 H); 2.22 - 2.35 (m, 3 H); 2.73 - 2.85 (m, 2 H); 2.96 - 3.03 (m, 1 H); 3.00 (s, 3 H); 3.02 - 3.09 (m, 1 H); 3.36 - 3.41 (m, 1 H); 3.73 - 3.76 (m, 1 H); 3.80 - 3.85 (m, 1 H); 3.87 (s, 3 H); 3.94 - 4.01 (m, 1 H); 4.22 (dd, J=9.02, 5.28 Hz, 1 H); 4.48 (dt, J=14.85, 4.24 Hz, 1 H); 5.26 - 5.34 (m, 1 H); 5.37 (s, 1 H); 5.73 (s, 1 H); 7.18 (s, 1 H); 7.20 - 7.25 (m, 1 H); 7.29 - 7.32 (m, 1 H); 7.33 (m, 1 H); 7.33 - 7.36 (m, 1 H); (dd, J=9.24, 5.72 Hz, 1 H).

MS (ESI) *m/z* 714 [M+H]$^+$ RT 1.66 min, Method: B12007B12007

## Compound 20

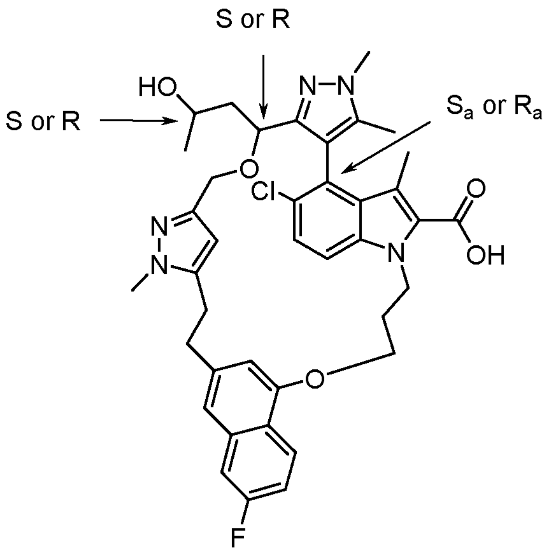

Pure stereoisomer but absolute stereochemistry undetermined

Compound 20 was prepared by an analogous reaction protocol as Compound 1, starting from Intermediate 60 instead of Intermediate 24.

$^1$H NMR (400 MHz, CHLOROFORM-*d*) δ ppm 1.15 (d, J=6.16 Hz, 3 H); 1.96 (ddd, J=14.58, 4.79, 1.76 Hz, 1 H); 2.10 (s, 3 H); 2.11 (s, 3 H); 2.22 (dt, J=14.74, 8.91 Hz, 1 H); 2.27 - 2.35 (m, 2 H); 2.81-2.83 (m, 2 H); 2.84 - 2.91 (m, 1 H); 2.91 -

3.01 (m, 2 H); 3.10 - 3.20 (m, 4 H); 3.29 - 3.39 (m, 1 H); 3.74 (d, J=11.22 Hz, 1 H); 3.89 (s, 3 H); 3.89 - 3.96 (m, 2 H); 4.28 (dd, J=8.36, 4.84 Hz, 1 H); 4.52 (dt, J=14.85, 4.79 Hz, 1 H); 5.21 - 5.31 (m, 1 H); 5.47 (s, 1 H); 5.64 (s, 1 H); 7.18 (s, 1 H); 7.20 - 7.26 (m, 2 H); 7.31 (dd, J=10.01, 2.53 Hz, 1 H); 7.35 (d, J=8.80 Hz, 1 H); 8.31 (dd, J=9.24, 5.72 Hz, 1 H).

**Compound 21**

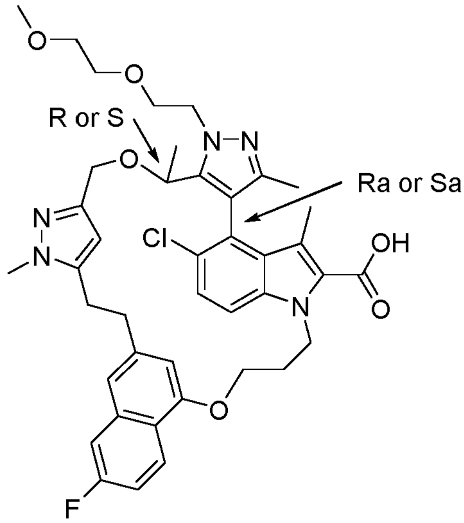


Pure stereoisomer but absolute stereochemistry undetermined

An aqueous solution of LiOH (1 M, 1.25 mL, 1.25 mmol, 15 eq.) was added to a solution of Intermediate 82 (63 mg, 0.082 mmol) in THF (1 mL) and MeOH (1 mL). The reaction mixture was stirred at 50 °C for 16 h. The mixture was concentrated under reduced pressure and the residue was diluted with DCM and water. Aqueous HCl (1 M) was added until pH circa 1. The layers were separated and the aqueous layer was extracted with DCM. The combined organic layer was dried over $Na_2SO_4$, filtered, and the solvent concentrated under reduced pressure. The residue was purified by flash column chromatography on silica gel (4 g, Redisep flash column, MeOH/DCM 0/100 to 5/95) to afford Compound 21 (41 mg, yield: 66 %) as a white solid.

**Compound 22**

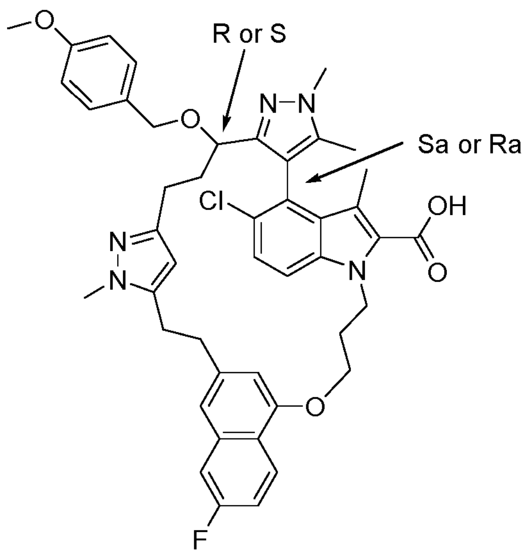


Pure stereoisomer but absolute stereochemistry undetermined

LiOH (15 mg, 0.64 mmol, 15 eq.) was added to a stirred solution of Intermediate 86 (34 mg, 0.042 mmol) in water (0.5 mL), THF (1 mL), and MeOH (1 mL) at room temperature. The reaction mixture was stirred at 50 °C for 16 h. The

solvents were evaporated and the residue was taken up with water and acidified with aqueous 1 M HCl until acidic pH was reached. The aqueous layer was extracted with EtOAc (x 2). The combined organic layer was dried over MgSO4, filtered, and the solvent removed under reduced pressure to yield Compound 22 (32 mg, yield: 96 %) as an off-white solid.

$^1$H NMR (CHLOROFORM-*d*) δ ppm: 8.28 (dd, J=9.2, 5.9 Hz, 1H), 7.30 (d, J=1.8 Hz, 2H), 7.11-7.26 (m, 5H), 6.81 (d, J=8.8 Hz, 2H), 5.72 (s, 1H), 5.63 (s, 1H), 5.32 (ddd, J=14.6, 10.9, 3.1 Hz, 1H), 4.60 (d, J=11.7 Hz, 1H), 4.37-4.51 (m, 2H), 4.24 (d, J=11.7 Hz, 1H), 3.91 (s, 3H), 3.76 (s, 3H), 3.52 (dt, J=9.3, 4.7 Hz, 1H), 3.26 (td, J=9.2, 3.6 Hz, 1H), 2.91-3.05 (m, 5H), 2.71-2.79 (m, 2H), 2.45-2.58 (m, 1H), 2.30-2.44 (m, 2H), 2.18-2.29 (m, 1H), 2.10 (d, J=4.4 Hz, 6H), 1.66-1.82 (m, 3H).

## Compound 23

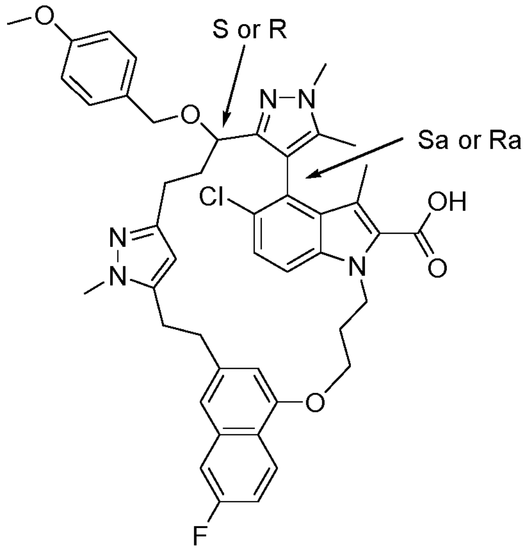

Pure stereoisomer but absolute stereochemistry undetermined

Compound 23 was prepared by an analogous reaction protocol as Compound 22, starting from Intermediate 87 instead of Intermediate 86.

$^1$H NMR (CHLOROFORM-*d*) δ ppm: 8.29 (dd, J=9.1, 5.8 Hz, 1H), 7.27-7.35 (m, 4H; overlapped with CDCl3 signal), 7.14-7.26 (m, 3H; overlapped with CDCl3 signal), 6.82 (d, J=8.8 Hz, 2H), 5.57 (s, 1H), 5.55 (s, 1H), 5.24 (ddd, J=14.9, 10.0, 3.3 Hz, 1H), 4.65 (d, J=11.7 Hz, 1H), 4.47 (dt, J=15.1, 4.3 Hz, 1H), 4.34 (d, J=11.7 Hz, 1H), 4.13 (d, J=6.8 Hz, 1H), 3.93 (s, 3H), 3.78 (s, 3H), 3.47 (dt, J=9.4, 4.8 Hz, 1H), 3.20 (td, J=9.0, 4.2 Hz, 1H), 2.92-3.08 (m, 5H), 2.71-2.85 (m, 2H), 2.18-2.41 (m, 3H), 2.11 (s, 3H), 1.98-2.08 (m, 5H), 1.76-1.94 (m, 3H).

## Compound 24

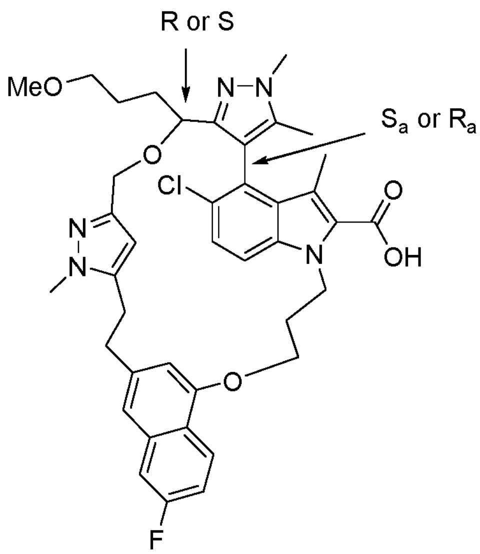

Pure stereoisomer but absolute stereochemistry undetermined

LiOH (19 mg, 0.79 mmol, 39 eq.) was added to a solution of Intermediate 88 (15 mg, 0.02 mmol) in THF (1 mL), MeOH (1 mL), and water (0.5 mL). The reaction mixture was stirred at 50 °C for 3.5 h. The solvents were evaporated and the residue was dissolved in water (1 mL). This solution was treated with aqueous HCl (1 M, 0.81 mL, 0.81 mmol, 40 eq.) until pH = 2. The cloudy aqueous layer was extracted with $CHCl_3$ (3 x). The combined organic layer was dried over $MgSO_4$, filtered, and evaporated under reduced pressure. The residue was purified by preparative HPLC (Stationary phase: RP XBridge Prep C18 OBD - 5 μm, 50 × 250 mm, Mobile phase: 0.25 % $NH_4HCO_3$ solution in water, $CH_3CN$) to afford Compound 24 (1 mg, yield: 7 %).

$^1$H NMR (400 MHz, CHLOROFORM-*d*) δ ppm 1.23 - 1.33 (m, 2 H); 1.50 - 1.56 (m, 1 H); 1.82 - 1.84 (m, 3 H); 2.00 - 2.03 (m, 3 H); 2.33 (br s, 2 H); 2.88 - 2.95 (m, 1 H); 2.99 - 3.11 (m, 3 H); 3.17 (br s, 1 H); 3.43 (m, 1 H); 3.58 (m, 1 H); 3.83 - 3.88 (m, 3 H); 4.00 (m, 2 H); 4.10 (s, 3 H); 4.18 (br d, J=14.28 Hz, 1 H); 4.23 (s, 3 H); 4.31 (m, 1 H); 4.39 (m, 1 H); 5.10 (m, 1 H); 5.68 (s, 1 H); 6.43 (s, 1 H); 6.66 (d, J=8.98 Hz, 1 H); 7.07 (s, 1 H); 7.11 (d, J=8.98 Hz, 1 H); 7.19 (td, J=8.77, 2.45 Hz, 1 H); 7.27 - 7.32 (m, 2 H); 8.26 (dd, J=9.38, 5.71 Hz, 1 H).

## Compound 25

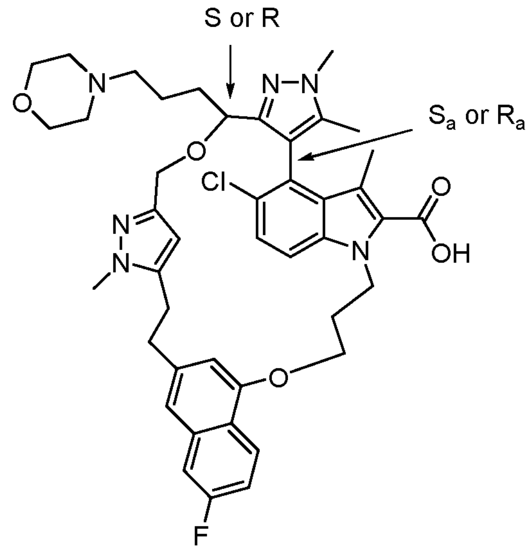

Pure stereoisomer but absolute stereochemistry undetermined

Compound 25 was prepared by an analogous reaction protocol as Compound 24, starting from Intermediate 89 instead of Intermediate 88.

$^1$H NMR (400 MHz, CHLOROFORM-*d*) δ ppm 1.40 (m, J=22.90 Hz, 1 H); 1.84 (br s, 4 H); 1.93 (br s, 3 H); 2.14 - 2.35 (m, 10 H); 2.76 (m, 4 H); 3.15 (br s, 1 H); 3.29 (br s, 3 H); 3.42 - 3.52 (m, 1 H); 3.57 - 3.74 (m, 8 H); 3.96 - 4.14 (m, 2 H); 4.24 - 4.39 (m, 1 H); 5.15 (m, 2 H); 5.70 (br s, 1 H); 6.93 - 7.03 (m, 1 H); 7.06 - 7.17 (m, 3 H); 7.28 - 7.34 (m, 1 H); 8.09 - 8.25 (m, 1 H).

**Compound 26**

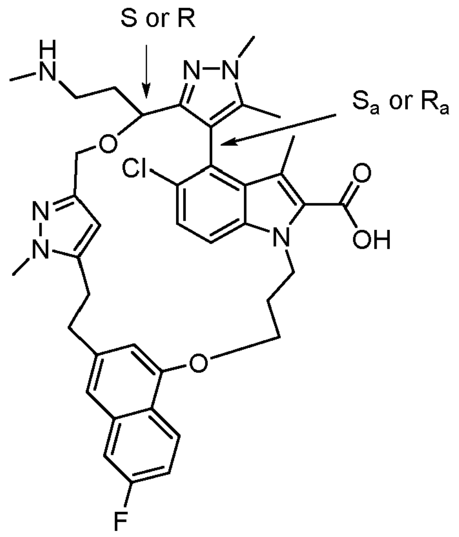


Compound 26 was prepared by an analogous reaction protocol as Compound 7, starting from Intermediate 90 instead of Intermediate 31.

$^1H$ NMR (400 MHz, CHLOROFORM-*d*, 47 °C) δ ppm 1.91 (s, 3 H); 2.00 (s, 3 H); 2.24 (m, 6 H); 2.80 - 3.06 (m, 5 H); 3.25 (br s, 2 H); 3.36 (s, 3 H); 3.82 (s, 4 H); 3.91 (br t, J=5.50 Hz, 3 H); 4.47 (s, 2 H); 4.51 - 4.62 (m, 2 H); 5.94 (s, 1 H); 6.20 (s, 1 H); 7.07 (s, 1 H); 7.08 - 7.12 (m, 1 H); 7.12 - 7.19 (m, 2 H); 7.25 - 7.30 (m, 5 H); 8.18 (dd, J=9.02, 5.72 Hz, 1 H).

**Compound 27**

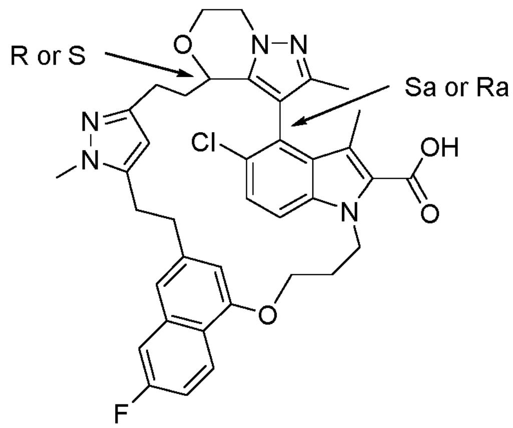


Pure stereoisomer but absolute stereochemistry undetermined

LiOH (22 mg, 0.94 mmol, 15 eq.) was added to a solution of Intermediate 108 (44 mg, 0.062 mmol) in a mixture of MeOH (1.6 mL), THF (1.6 mL), and water (0.8 mL). The resulting reaction mixture was stirred for 4 h at 50 °C. The solvents were evaporated and the residue was purified by reverse phase preparative HPLC (stationary phase: RP XBridge Prep C18 OBD - 5 µm, 50 x 250 mm, Mobile phase: 0.25 % $NH_4HCO_3$ solution in water, $CH_3CN$) to yield Compound 27 (31 mg, yield: 72 %) as a pale yellow solid.

$^1H$ NMR (400 MHz, DMSO-$d_6$) δ ppm 1.27 - 1.37 (m, 1 H), 1.55 - 1.64 (m, 1 H), 1.78 (s, 3 H), 2.03 (s, 3 H), 2.14 (dt, J=15.2, 5.7 Hz, 1 H), 2.24 - 2.37 (m, 3 H), 2.93 (br t, J=8.8 Hz, 2 H), 3.03 - 3.07 (m, 2 H), 3.48 - 3.49 (m, 3 H), 3.68 - 3.75 (m, 1 H), 3.77 - 3.88 (m, 2 H), 4.04 - 4.13 (m, 2 H), 4.13 - 4.20 (m, 1 H), 4.33 - 4.57 (m, 1 H), 4.59 (br d, J=10.3 Hz, 1 H), 4.63 (s, 1 H), 5.01 - 5.08 (m, 1 H), 6.43 (s, 1 H), 7.12 (d, J=8.8 Hz, 1 H), 7.17 (s, 1 H), 7.31 (td, J=8.9, 2.6 Hz, 1 H), 7.49 (dd, J=10.5, 2.5 Hz, 1 H), 7.64 (d, J=9.0 Hz, 1 H), 8.23 (dd, J=9.2, 5.9 Hz, 1 H).

## Compound 28

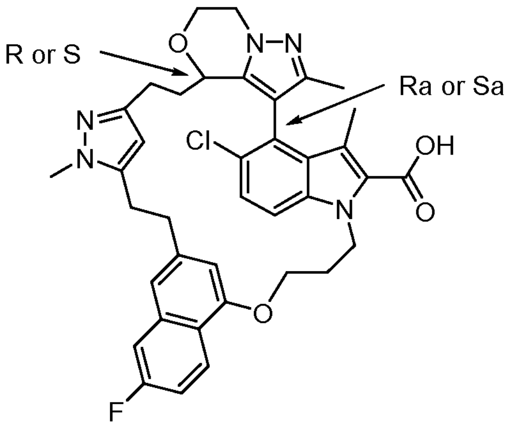

Pure stereoisomer but absolute stereochemistry undetermined

Compound 28 was prepared by an analogous reaction protocol as Compound 27, starting from Intermediate 110 instead of Intermediate 108.

$^1$H NMR (400 MHz, DMSO-$d_6$) δ ppm 1.24 (br s, 2 H), 1.90 (s, 3 H), 1.97 (s, 3 H), 1.99 - 2.06 (m, 1 H), 2.33 (br d, J=1.8 Hz, 3 H), 2.96 - 3.06 (m, 4 H), 3.51 (s, 3 H), 3.83 (br s, 3 H), 4.08 (br s, 2 H), 4.21 - 4.30 (m, 1 H), 4.48 - 4.62 (m, 2 H), 4.98 - 5.03 (m, 1 H), 5.04 (s, 1 H), 6.47 - 6.59 (m, 1 H), 6.85 - 6.97 (m, 1 H), 7.31 (s, 1 H), 7.34 (br d, J=2.4 Hz, 1 H), 7.52 (d, J=9.6 Hz, 2 H), 8.30 (s, 1 H).

## Compound 29

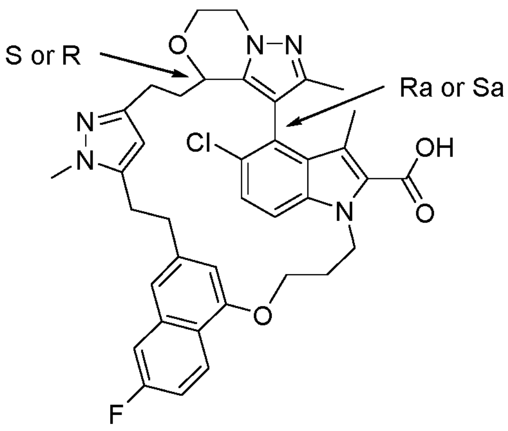

Pure stereoisomer but absolute stereochemistry undetermined

Compound 29 was prepared by an analogous reaction protocol as Compound 27, starting from Intermediate 109 instead of Intermediate 108.

$^1$H NMR (400 MHz, DMSO-$d_6$) δ ppm 1.34 (br s, 1 H), 1.60 (br s, 1 H), 1.78 (s, 3 H), 2.02 (s, 3 H), 2.09 - 2.19 (m, 1 H), 2.25 - 2.40 (m, 3 H), 2.90 - 2.99 (m, 2 H), 3.02 - 3.11 (m, 2 H), 3.50 (s, 3 H), 3.71 (br d, J=3.8 Hz, 1 H), 3.80 - 3.86 (m, 2 H), 4.04 - 4.12 (m, 2 H), 4.14 - 4.21 (m, 1 H), 4.39 (br s, 1 H), 4.57 (br s, 1 H), 4.60 (s, 1 H), 5.04 (br d, J=15.0 Hz, 1 H), 6.45 (s, 1 H), 7.12 (d, J=9.0 Hz, 1 H), 7.17 (s, 1 H), 7.31 (td, J=8.9, 2.6 Hz, 1 H), 7.49 (dd, J=10.5, 2.6 Hz, 1 H), 7.66 (d, J=9.0 Hz, 1 H), 8.23 (dd, J=9.1, 5.9 Hz, 1 H).

**Compound 30**

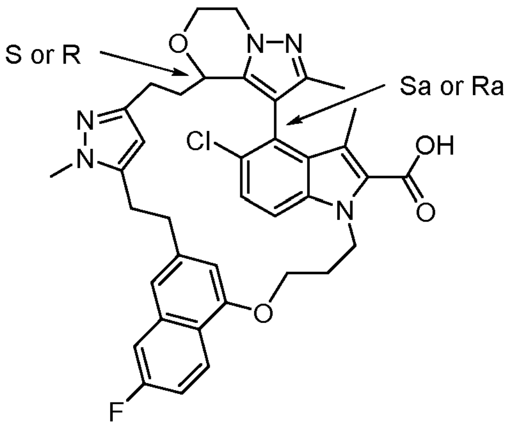

Pure stereoisomer but absolute stereochemistry undetermined

Compound 30 was prepared by an analogous reaction protocol as Compound 27, starting from Intermediate 107 instead of Intermediate 108.

$^1$H NMR (400 MHz, DMSO-$d_6$) δ ppm 1.21 (br dd, J=11.9, 5.3 Hz, 2 H), 1.90 (s, 3 H), 1.99 (s, 3 H), 2.00 - 2.06 (m, 1 H), 2.24 - 2.40 (m, 3 H), 2.95 - 3.05 (m, 4 H), 3.49 - 3.50 (m, 3 H), 3.78 - 3.91 (m, 3 H), 4.05 - 4.12 (m, 2 H), 4.21 - 4.28 (m, 1 H), 4.49 - 4.54 (m, 1 H), 4.59 (br t, J=11.7 Hz, 1 H), 5.00 (br s, 1 H), 5.04 (s, 1 H), 6.51 (s, 1 H), 6.94 (d, J=9.0 Hz, 1 H), 7.31 (s, 1 H), 7.32 - 7.36 (m, 1 H), 7.49 - 7.56 (m, 2 H), 8.30 (dd, J=9.1, 5.8 Hz, 1 H).

**Compound 31**

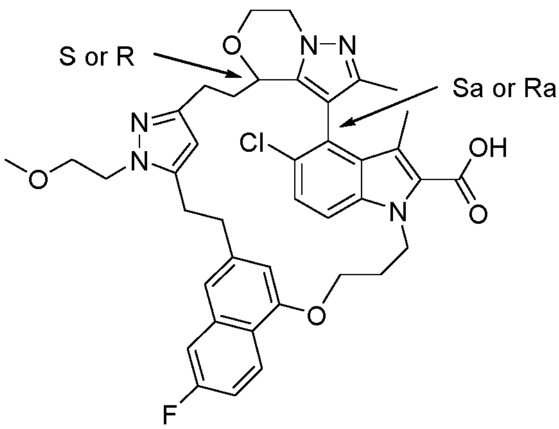

Pure stereoisomer but absolute stereochemistry undetermined

LiOH (47 mg, 1.98 mmol, 15 eq.) was added to a solution of Intermediate 102 (98 mg, 0.13 mmol) in MeOH (3.3 mL), THF (3.3 mL), and water (1.6 mL). The reaction mixture was stirred for 4 h at 50 °C. The solvents were evaporated and the residue was taken up in water and DCM and acidified with 1 M aqueous HCl to pH 4-5. The aqueous layer was extracted with DCM (x 5). The combined organic layer was dried over $MgSO_4$, filtered, and concentrated under reduced pressure to give Compound 31 (59.9 mg, yield: 63 %) as a pale yellow solid.

$^1$H NMR (400 MHz, CHLOROFORM-*d*) δ ppm 1.49 - 1.59 (m, 1 H), 1.64 - 1.76 (m, 1 H), 1.97 (s, 3 H), 2.14 - 2.20 (m, 3 H), 2.32 - 2.46 (m, 2 H), 2.46 - 2.59 (m, 2 H), 2.88 - 2.99 (m, 2 H), 2.99 - 3.09 (m, 2 H), 3.18 (s, 3 H), 3.36 - 3.46 (m, 1 H), 3.49 - 3.65 (m, 3 H), 3.75 - 3.91 (m, 2 H), 3.91 - 4.01 (m, 1 H), 4.16 - 4.34 (m, 3 H), 4.51 - 4.60 (m, 1 H), 4.80 (dd, J=9.7, 2.5 Hz, 1 H), 5.11 - 5.20 (m, 1 H), 5.25 (s, 1 H), 6.03 (s, 1 H), 6.98 (d, J=8.5 Hz, 1 H), 7.15 (s, 1 H), 7.22 (td, J=8.8, 2.5 Hz, 1 H), 7.27 - 7.33 (m, 2 H), 8.34 (dd, J=9.2, 5.7 Hz, 1 H).

## Compound 32

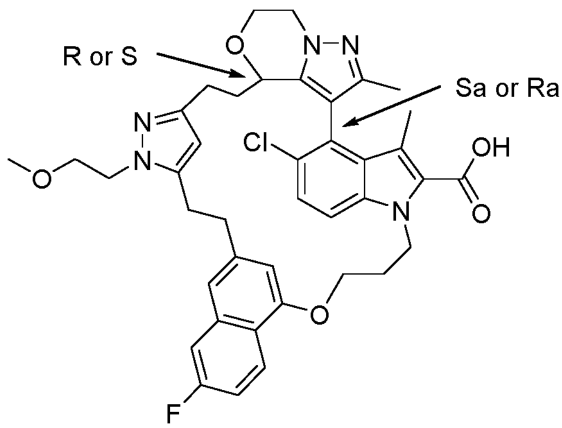

Pure stereoisomer but absolute stereochemistry undetermined

Compound 32 was prepared by an analogous reaction protocol as Compound 31, starting from Intermediate 103 instead of Intermediate 102.

$^1$H NMR (400 MHz, DMSO-$d_6$) δ ppm 1.17 - 1.22 (m, 1 H), 1.91 (s, 3 H), 2.00 (s, 3 H), 2.04 (br d, J=5.7 Hz, 1 H), 2.26 - 2.41 (m, 3 H), 2.92 - 3.08 (m, 4 H), 3.10 (s, 3 H), 3.17 (d, J=5.2 Hz, 1 H), 3.41 - 3.51 (m, 2 H), 3.72 - 3.90 (m, 3 H), 3.93 (t, J=5.6 Hz, 2 H), 4.10 (br d, J=5.3 Hz, 2 H), 4.25 (br d, J=11.1 Hz, 1 H), 4.49 - 4.55 (m, 1 H), 4.62 (br s, 1 H), 5.01 (br d, J=15.0 Hz, 1 H), 5.07 (s, 1 H), 6.48 (s, 1 H), 6.94 (d, J=9.0 Hz, 1 H), 7.31 (s, 1 H), 7.32 - 7.37 (m, 1 H), 7.54 (td, J=6.6, 2.8 Hz, 2 H), 8.31 (dd, J=9.4, 5.9 Hz, 1 H), 13.36 (br s, 1 H).

## Compound 33

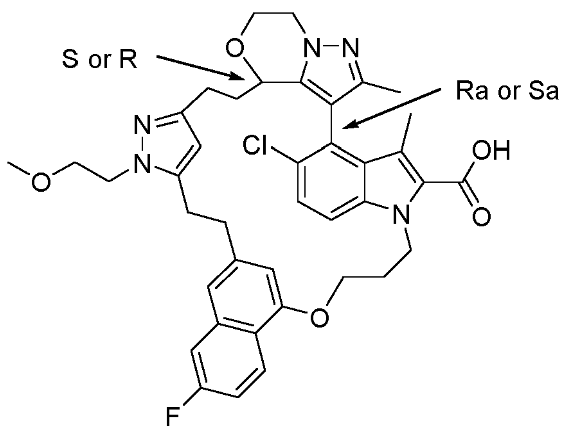

Pure stereoisomer but absolute stereochemistry undetermined

Compound 33 was prepared by an analogous reaction protocol as Compound 31, starting from Intermediate 104 instead of Intermediate 102.

$^1$H NMR (400 MHz, DMSO-$d_6$) δ ppm 1.15 - 1.21 (m, 1 H), 1.91 (s, 3 H), 2.00 (s, 3 H), 2.02 - 2.09 (m, 1 H), 2.26 - 2.41 (m, 3 H), 2.92 - 3.08 (m, 4 H), 3.10 (s, 3 H), 3.17 (d, J=5.2 Hz, 1 H), 3.40 - 3.51 (m, 2 H), 3.72 - 3.90 (m, 3 H), 3.90 - 3.95 (m, 2 H), 4.10 (br d, J=5.2 Hz, 2 H), 4.25 (br d, J=11.7 Hz, 1 H), 4.52 (br d, J=3.9 Hz, 1 H), 4.62 (br s, 1 H), 5.01 (br d, J=14.6 Hz, 1 H), 5.07 (s, 1 H), 6.48 (s, 1 H), 6.94 (d, J=9.0 Hz, 1 H), 7.31 (s, 1 H), 7.32 - 7.37 (m, 1 H), 7.51 - 7.57 (m, 2 H), 8.31 (dd, J=9.3, 6.1 Hz, 1 H), 13.36 (br s, 1 H).

## Compound 34

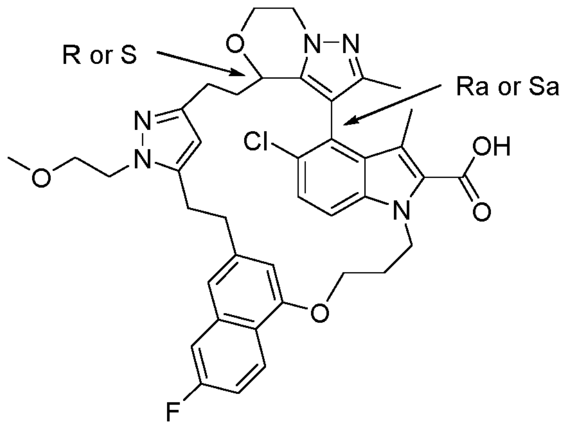

Pure stereoisomer but absolute stereochemistry undetermined

Compound 34 was prepared by an analogous reaction protocol as Compound 31, starting from Intermediate 105 instead of Intermediate 102.

$^1$H NMR (400 MHz, CHLOROFORM-*d*) δ ppm 1.51 - 1.58 (m, 1 H), 1.66 - 1.73 (m, 1 H), 1.97 (s, 3 H), 2.16 (s, 3 H), 2.32 - 2.45 (m, 2 H), 2.45 - 2.59 (m, 2 H), 2.87 - 2.99 (m, 2 H), 2.99 - 3.09 (m, 2 H), 3.18 (s, 3 H), 3.39 - 3.46 (m, 1 H), 3.49 - 3.64 (m, 3 H), 3.71 - 3.88 (m, 2 H), 3.92 - 4.01 (m, 1 H), 4.16 - 4.34 (m, 3 H), 4.50 - 4.61 (m, 1 H), 4.80 (dd, J=9.8, 2.7 Hz, 1 H), 5.10 - 5.22 (m, 1 H), 5.25 (s, 1 H), 6.02 (s, 1 H), 6.98 (d, J=9.0 Hz, 1 H), 7.16 (s, 1 H), 7.22 (td, J=8.8, 2.6 Hz, 1 H), 7.27 - 7.34 (m, 2 H), 8.33 (dd, J=9.1, 5.8 Hz, 1 H).

LCMS results (RT means retention time, in min.)

| Compound number | LCMS results |
|---|---|
| 1 | confirms the MW (RT: 0.95, [M+H]+ 682, LCMS Method 1) |
| 2 | confirms the MW (RT: 1.82, [M+H]+ 682, LCMS Method 2) |
| 3 | confirms the MW (RT: 1.69, [M+H]+ 714, LCMS Method: 2) |
| 4 | confirms the MW (RT: 1.79, [M+H]+ 714, LCMS Method: 4) |
| 5 | confirms the MW (RT: 1.78, [M+H]+ 758, LCMS Method: 5) |
| 6 | confirms the MW (RT: 1.64, [M+H]+ 700, LCMS Method: 5) |
| 7 | confirms the MW (RT: 1.67, [M+H]+ 769, LCMS Method: 3) |
| 8 | confirms the MW (RT: 1.63, [M+H]+ 782, LCMS Method: 3) |
| 9 | confirms the MW (RT: 1.85, [M+H]+ 728, LCMS Method: 2) |
| 10 | confirms the MW (RT: 1.61, [M+H]+ 714, LCMS Method: 3) |
| 11 | confirms the MW (RT: 1.63, [M+H]+ 714, LCMS Method: 3) |
| 12 | confirms the MW (RT: 1.89, [M+H]+ 728, LCMS Method: 3) |
| 13 | confirms the MW (RT: 0.89, [M+H]+ 755, LCMS Method: 6) |
| 14 | confirms the MW (RT: 0.92, [M+H]+ 744, LCMS Method: 6) |
| 15 | confirms the MW (RT: 1.75, [M+H]+ 700, LCMS Method: 3) |
| 16 | confirms the MW (RT: 1.76, [M+H]+ 700, LCMS Method: 3) |
| 17 | confirms the MW (RT: 0.89, [M+H]+ 670, LCMS Method: 6) |
| 18 | confirms the MW (RT: 0.96, [M+H]+ 670, LCMS Method: 6) |

(continued)

| Compound number | LCMS results |
|---|---|
| 19 | confirms the MW (RT: 1.66, [M+H]+ 714, LCMS Method: 3) |
| 20 | confirms the MW (RT: 1.70, [M+H]+ 714, LCMS Method: 4) |
| 21 | confirms the MW (RT: 2.35, [M+H]+ 758, LCMS method 7) |
| 22 | confirms the MW (RT: 1.01, [M+H]+ 790, LCMS method 6) |
| 23 | confirms the MW (RT: 1.06, [M+H]+ 790, LCMS method 6) |
| 24 | confirms the MW (RT: 1.56, [M+H]+ 728, LCMS method 2) |
| 25 | confirms the MW (RT: 1.70, [M+H]+ 783, LCMS method 2) |
| 26 | confirms the MW (RT: 1.63, [M+H]+ 713, LCMS method 4) |
| 27 | confirms the MW (RT: 0.92, [M+H]+ 682, LCMS method 3) |
| 28 | confirms the MW (RT: 0.90, [M+H]+ 682, LCMS method 6) |
| 29 | confirms the MW (RT: 0.92, [M+H]+ 682, LCMS method 6) |
| 30 | confirms the MW (RT: 0.90, [M+H]+ 682, LCMS method 6) |
| 31 | confirms the MW (RT: 0.96, [M+H]+ 726, LCMS method 6) |
| 32 | confirms the MW (RT: 0.94, [M+H]+ 726, LCMS method 6) |
| 33 | confirms the MW (RT: 0.94, [M+H]+ 726, LCMS method 6) |
| 34 | confirms the MW (RT: 0.96, [M+H]+ 726, LCMS method 6) |

Table: Analytical SFC data - $R_t$ means retention time (in minutes), $[M+H]^+$ means the protonated mass of the compound, method refers to the method used for (SFC)MS analysis of enantiomerically pure compounds. No. means number.

| **Compound No.** | **SFC Method** | **Rt** | **$[M+H]^+$** |
|---|---|---|---|
| 1 | 1 | 5.37 | 682 |
| 2 | 2 | 5.58 | 682 |
| 4 | 2 | 4.77 | 714 |
| 13 | 2 | 4.95 | 755 |
| 14 | 2 | 4.79 | 744 |
| 15 | 2 | 5.56 | 700 |
| 16 | 3 | 5.06 | 700 |
| 17 | 2 | 4.47 | 670 |
| 18 | 2 | 5.13 | 670 |
| 21 | 2 | 4.15 | 758 |
| 27 | 2 | 4.74 | 682 |
| 28 | 4 | 5.95 | 682 |
| 29 | 4 | 5.40 | 682 |
| 30 | 2 | 4.49 | 682 |
| 31 | 5 | 4.93 | 726 |
| 32 | 5 | 5.63 | 726 |
| 33 | 5 | 6.85 | 726 |
| 34 | 5 | 5.12 | 726 |

## Analytical Analysis

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rt) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method Codes (Flow expressed in mL/min; column temperature (T) in °C; Run time in minutes)

LC-MS methods:

| Method Code | Instrument | column | mobile phase | gradient | Flow<br>----------<br>Col T | Run time |
|---|---|---|---|---|---|---|
| 1 | Waters: Acquity® UPLC® - DAD and SQD | Waters :BEH (1.8 μm, 2.1 * 50 mm) | A: 0.1 % $NH_4HCO_3$ in 95 % $H_2O$ + 5 % $CH_3CN$<br>B: $CH_3CN$ | From 100 % A to 5 % A in 1.3 min, hold 0.7 min | 0.8<br>-------<br>55 | 2.0 |
| 2 | Waters: Acquity® UPLC® - DAD and SQD | Waters : BEH (1.8 μm, 2.1 * 100 mm) | A: 10 mM $CH_3COONH_4$ in 95 % $H_2O$ + 5 % $CH_3CN$<br>B: $CH_3CN$ | From 100 % A to 5% A in 2.10 min, to 0 % A in 0.90 min, to 5 % A in 0.5 min | 0.6<br>-------<br>55 | 3.5 |
| 3 | Waters: Acquity® UPLC®-DAD and SQD | Waters :BEH (1.8 μm, 2.1 * 100 mm) | A: 10 mM $CH_3COONH_4$ in 95 % $H_2O$ + 5 % $CH_3CN$<br>B: $CH_3CN$ | From 100 % A to 5 % A in 2.10 min, to 0 % A in 0.90 min, to 5 % A in 0.5 min | 0.7<br>-------<br>55 | 3.5 |
| 4 | Waters: Acquity® UPLC® - DAD and SQD | Waters :BEH (1.8 μm, 2.1 * 100 mm) | A: 10mM $CH_3COONH_4$ in 95 % $H_2O$ + 5 % $CH_3CN$<br>B: $CH_3CN$ | From 100 % A to 5 % A in 2.10 min, to 0% A in 0.9 min, to 5 % A in 0.4 min | 0.6<br>-------<br>55 | 3.5 |
| 5 | Waters: Acquity® UPLC® - DAD and SQD | Waters :BEH (1.8 μm, 2.1 * 100 mm) | A: 10mM $CH_3COONH_4$ in 95 % $H_2O$ + 5 % $CH_3CN$<br>B: $CH_3CN$ | From 100 % A to 5 % A in 2.10 min, to 0 % A in 0.9 min, to 5 % A in 0.5 min | 0.6<br>-------<br>55 | 3.5 |

(continued)

| Method Code | Instrument | column | mobile phase | gradient | Flow<br>----------<br>Col T | Run time |
|---|---|---|---|---|---|---|
| 6 | Waters: Acquity® UPLC®-DAD and SQD | Waters : BEH C18 (1.7 μm, 2.1 * 50 mm) | A: 10 mM $CH_3COONH_4$ in 95 % $H_2O$ + 5 % $CH_3CN$<br>B: $CH_3CN$ | From 95 % A to 5 % A in 1.3 min, held for 0.7 min. | 0.8<br>-------<br>55 | 2 |
| 7 | Waters: Acquity® UPLC®-DAD and SQD | Waters :BEH (1.8 μm, 2.1 * 100 mm) | A: 10 mM $NH_4HCO_3$ in 95 % $H_2O$ + 5 % $CH_3CN$<br>B: MeOH | From 100 % A to 5 % A in 2.10 min, to 0 % A in 0.9 min, to 5 % A in 0.5 min | 0.6<br>-------<br>55 | 3.5 |

SFC-MS methods:

The SFC measurement was performed using an Analytical Supercritical fluid chromatography (SFC) system composed by a binary pump for delivering carbon dioxide (CO2) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Analytical SFC-MS Methods (Flow expressed in mL/min; column temperature (Col T) in °C; Run time in minutes, Backpressure (BPR) in bars. "$iPrNH_2$" means isopropylamine, "iPrOH" means 2-propanol, "EtOH" means ethanol, "min" mean minutes.

SFC methods:

| SFC Method | Column | mobile phase | gradient | Flow<br>-------<br>Col T | Run time<br>-------<br>BPR |
|---|---|---|---|---|---|
| 1 | Daicel Chiralpak® IH3 column (3.0 μm, 150 × 4.6 mm) | A:$CO_2$ B: EtOH-iPrOH (50-50) + 0.2 % $iPrNH_2$ | 10 % - 50 % B in 6 min, hold 3.5 min | 2.5<br>-------<br>40 | 9.5<br>-------<br>130 |
| 2 | Daicel Chiralpak® IH3 column (3.0 μm, 150 × 4.6 mm) | A:$CO_2$ B: EtOH + 0.2 % $iPrNH_2$ | 10 % - 50 % B in 6 min, hold 3.5 min | 2.5<br>-------<br>40 | 9.5<br>-------<br>130 |

(continued)

| SFC Method | Column | mobile phase | gradient | Flow ------- Col T | Run time ------- BPR |
|---|---|---|---|---|---|
| 3 | Daicel Chiralpak® AD3 column (3.0 μm, 150 × 4.6 mm) | A:$CO_2$ B: EtOH+0.2 % $iPrNH_2$ | 10 % - 50 % B in 6 min, hold 3.5 min | 2.5 ------- 40 | 9.5 ------- 130 |
| 4 | Daicel Chiralpak® AD-H column (3.0 μm, 150 × 4.6 mm) | A:$CO_2$ B: EtOH-iPrOH+0.2 % $iPrNH_2$ | 10 % - 50 % B in 6 min, hold 3.5 min | 2.5 ------- 40 | 9.5 ------- 110 |
| 5 | Daicel Chiralpak® IG3 column (3.0 μm, 150 × 4.6 mm) | A:$CO_2$ B: EtOH+0.2% $iPrNH_2$ | 10 % - 50 % B in 6 min, hold 3.5 min | 2.5 ------- 40 | 9.5 ------- 130 |

NMR

$^1H$ NMR spectra were recorded on Bruker Avance III 400MHz and Avance NEO 400MHz spectrometers. $CDCl_3$ was used as solvent, unless otherwise mentioned. The chemical shifts are expressed in ppm relative to tetramethylsilane.

**Pharmacological Analysis**

Biological Example 1

Terbium labeled Myeloid Cell Leukemia 1(Mcl-1) homogeneous time-resolved fluorescence (HTRF) binding assay utilizing the BIM BH3 peptide (H2N-(C/Cy5Mal) WIAQELRRIGDEFN-OH) as the binding partner for Mcl-1.

Apoptosis, or programmed cell death, ensures normal tissue homeostasis, and its dysregulation can lead to several human pathologies, including cancer. Whilst the extrinsic apoptosis pathway is initiated through the activation of cell-surface receptors, the intrinsic apoptosis pathway occurs at the mitochondrial outer membrane and is governed by the binding interactions between pro- and anti-apoptotic Bcl-2 family proteins, including Mcl-1. In many cancers, the anti-apoptotic Bcl-2 protein(s), such as the Mcl-1, are upregulated, and in this way the cancer cells can evade apoptosis. Thus, inhibition of the Bcl-2 protein(s), such as Mcl-1, may lead to apoptosis in cancer cells, providing a method for the treatment of said cancers.

This assay evaluated inhibition of the BH3 domain : Mcl-1 interaction by measuring the displacement of Cy5-labeled BIM BH3 peptide ($H_2N$-(C/Cy5Mal) WIAQELRRIGDEFN-OH) in the HTRF assay format.

Assay Procedure

The following assay and stock buffers were prepared for use in the assay: (a) Stock buffer: 10mM Tris-HCl, pH=7.5 + 150mM NaCl, filtered, sterilized, and stored at 4°C; and (b) 1X assay buffer, where the following ingredients were added fresh to stock buffer: 2 mM dithiothreitol (DTT), 0.0025% Tween-20, 0.1 mg/mL bovine serum albumin (BSA). The 1X Tb-Mcl-1 + Cy5 Bim peptide solution was prepared by diluting the protein stock solution using the 1X assay buffer (b) to 25 pM Tb-Mcl-1 and 8 nM Cy5 Bim peptide.

Using the Acoustic ECHO, 100 nL of 100x test compound(s) were dispensed into individual wells of a white 384-well Perkin Elmer Proxiplate, for a final compound concentration of 1x and final DMSO concentration of 1%. Inhibitor

control and neutral control (NC, 100 nL of 100% DMSO) were stamped into columns 23 and 24 of assay plate, respectively. Into each well of the plate was then dispensed 10µL of the 1X Tb-Mcl-1 + Cy5 Bim peptide solution. The plate was centrifuged with a cover plate at 1000 rpm for 1 minute, then incubated for 60 minutes at room temperature with plates covered. The TR-FRET signal was read on an BMG PHERAStar FSX MicroPlate Reader at room temperature using the HTRF optic module (HTRF: excitation: 337nm, light source: laser, emission A: 665nm, emission B: 620nm, integration start: 60 µs, integration time: 400 µs).

Data Analysis

The BMG PHERAStar FSX MicroPlate Reader was used to measure fluorescence intensity at two emission wavelengths - 665nm and 620nm - and report relative fluorescence units (RFU) for both emissions, as well as a ratio of the emissions (665nm/620nm)*10,000. The RFU values were normalized to percent inhibition as follows:

$$\% \; inhibition = (((NC - IC) - (compound - IC)) / (NC - IC)) * 100$$

where IC (inhibitor control, low signal) = mean signal of 1X Tb-MCl-1 + Cy5 Bim peptide+ inhibitor control or 100% inhibition of Mcl-1; NC (neutral control, high signal) = mean signal 1X Tb-MCl-1 + Cy5 Bim peptide with DMSO only or 0% inhibition An 11-point dose response curve was generated to determine $IC_{50}$ values (using GenData) based on the following equation:

$$Y=Bottom + (Top-Bottom)/(1+10^{\wedge}((logIC_{50}-X)*HillSlope))$$

where Y = % inhibition in the presence of X inhibitor concentration; Top = 100% inhibition derived from the IC (mean signal of Mcl-1 + inhibitor control); Bottom = 0% inhibition derived from the NC (mean signal of Mcl-1 + DMSO); Hillslope = Hill coefficient; and $IC_{50}$ = concentration of compound with 50% inhibition in relation to top/neutral control (NC).

$$Ki = IC_{50} / (1 + [L]/Kd)$$

In this assay [L] = 8 nM and Kd = 10 nM

Representative compounds of the present invention were tested according to the procedure as described above, with results as listed in the Table below (n.d. means not determined).

| Compound | TB-MCL1 $K_i$ (nM) |
|---|---|
| 1 | 0.059 |
| 2 | 0.024 |
| 3 | 0.039 |
| 4 | 0.009 |
| 5 | 0.011 |
| 6 | 0.009 |
| 7 | 0.011 |
| 8 | 0.013 |
| 9 | 0.009 |
| 10 | 0.028 |
| 11 | 0.010 |
| 12 | 0.015 |
| 13 | 0.058 |
| 14 | 0.018 |
| 15 | 0.048 |
| 16 | 0.104 |

(continued)

| Compound | TB-MCL1 $K_i$ (nM) |
|---|---|
| 17 | 0.042 |
| 18 | 0.010 |
| 19 | 0.057 |
| 20 | 0.023 |
| 21 | 0.057 |
| 22 | 0.357 |
| 23 | 0.073 |
| 24 | 8.42 |
| 25 | NT |
| 26 | 3.61 |
| 27 | 0.013 |
| 28 | 44.20 |
| 29 | 0.302 |
| 30 | 0.034 |
| 31 | 0.028 |
| 32 | NT |
| 33 | NT |
| 34 | 0.408 |

Biological Example 2

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

The Caspase-Glo® 3/7 Assay is a luminescent assay that measures caspase-3 and -7 activities in purified enzyme preparations or cultures of adherent or suspension cells. The assay provides a proluminescent caspase-3/7 substrate, which contains the tetrapeptide sequence DEVD. This substrate is cleaved to release aminoluciferin, a substrate of luciferase used in the production of light. Addition of the single Caspase-Glo® 3/7 Reagent in an "add-mix-measure" format results in cell lysis, followed by caspase cleavage of the substrate and generation of a "glow-type" luminescent signal.

This assay uses the MOLP-8 human multiple myeloma cell line, which is sensitive to MCL-1 inhibition.

Materials:

- Perkin Elmer Envision
- Multidrop 384 and small volume dispensing cassettes
- Centrifuge
- Countess automated cell counter
- Countess counting chamber slides
- Assay plate: ProxiPlate-384 Plus, White 384-shallow well Microplate
- Sealing tape: Topseal A plus
- T175 culture flask

| Product | Units | Storage |
|---|---|---|
| RPMI1640 (no L-Glutamine, no phenol red) | 500 mL | 4 °C |
| Foetal Bovine Serum (FBS) (Heat inactivated) | 500 mL | 4 °C |
| L-Glutamine (200 mM) | 100 ml | -20 °C |
| Gentamicin (50 mg/mL) | 100 mL | 4 °C |
| Caspase 3/7 Detection kit | 100 mL 10 × 10 mL | -20 °C |

Cell culture media:

| **MOLP8** | |
|---|---|
| | |
| RPMI-1640 medium | 500 mL |
| 20 % FBS (heat inactivated) | 120 mL |
| 2 mM L-Glutamine | 6.2 mL |
| 50 μg/mL Gentamicin | 620 μL |
| | |
| **Assay media** | |
| | |
| RPMI-1640 medium | 500 mL |
| 10 % FBS (Heat inactivated) | 57 mL |
| 2 mM L-Glutamine | 5.7 mL |
| 50 μg/mL Gentamicin | 570 μL |

Cell culture:

Cell cultures were maintained between 0.2 and 2.0 $\times 10^6$ cells/mL. The cells were harvested by collection in 50 mL conical tubes. The cells were then pelleted at 500 g for 5 mins before removing supernatant and resuspension in fresh pre-warmed culture medium. The cells were counted and diluted as needed.

Caspase-Glo reagent:

The assay reagent was prepared by transferring the buffer solution to the substrate vial and mixing. The solution may be stored for up to 1 week at 4 °C with negligible loss of signal.

Assay procedure:

Compounds were delivered in assay-ready plates (Proxiplate) and stored at -20°C. Assays always include 1 reference compound plate containing reference compounds. The plates were spotted with 40 nL of compounds (0.5 % DMSO final in cells; serial dilution; 30 μM highest conc. 1/3 dilution, 10 doses, duplicates). The compounds were used at room temperature and 4 μL of pre-warmed media was added to all wells except column 2 and 23. The negative control was prepared by adding 1 % DMSO in media. The positive control was prepared by adding the appropriate positive control compound in final concentration of 60 μM in media. The plate was prepared by adding 4 μL negative control to column 23, 4 μL positive control to column 2 and 4 μL cell suspension to all wells in the plate. The plate with cells was then incubated at 37 °C for 2 hours. The assay signal reagent is the Caspase-Glo solution described above, and 8 μL was added to all wells. The plates were then sealed and measured after 30 minutes.
The activity of a test compound was calculated as percent change in apoptosis induction as follows:

LC

= median of the Low Control values

= Central Reference in Screener

= DMSO

= 0 %

HC

= Median of the High Control values
= Scale Reference in Screener
= 30 μM of positive control
= 100 % apoptosis induction

$$\%Effect\ (AC_{50}) = 100 - ((sample\text{-}LC) / (HC\text{-}LC)) * 100$$

$$\%Control = (sample / HC) * 100$$

$$\%Control\ min = ((sample\text{-}LC) / (HC\text{-}LC)) * 100$$

Table: Measured $AC_{50}$ for Representative Compounds of Formula (I). Averaged values are reported over all runs on all batches of a particular compound.

| Compound | MOLP8 Caspase-Glo $AC_{50}$ (nM) |
|---|---|
| 1 | 100.7 |
| 2 | 19.5 |
| 3 | 154.0 |
| 4 | 28.4 |
| 5 | 22.6 |
| 6 | 52.7 |
| 7 | 149.4 |
| 8 | 1163.9 |
| 9 | 43.6 |
| 10 | 1060.2 |
| 11 | 303.4 |
| 12 | 57.8 |
| 13 | 1368.4 |
| 14 | 205.0 |
| 15 | 66.1 |
| 16 | 230.0 |
| 17 | 129.3 |
| 18 | 35.4 |
| 19 | 1212.0 |
| 20 | 683.0 |

(continued)

| Compound | MOLP8 Caspase-Glo $AC_{50}$ (nM) |
|---|---|
| 21 | 318.3 |
| 22 | 1003.2 |
| 23 | 168.8 |
| 24 | >25 |
| 25 | NT |
| 26 | >30 |
| 27 | 8.5 |
| 28 | >30 |
| 29 | 146.5 |
| 30 | 52.8 |
| 31 | 48.6 |
| 32 | NT |
| 33 | NT |
| 34 | 889.4 |

**Claims**

**1.** A compound of Formula (I)

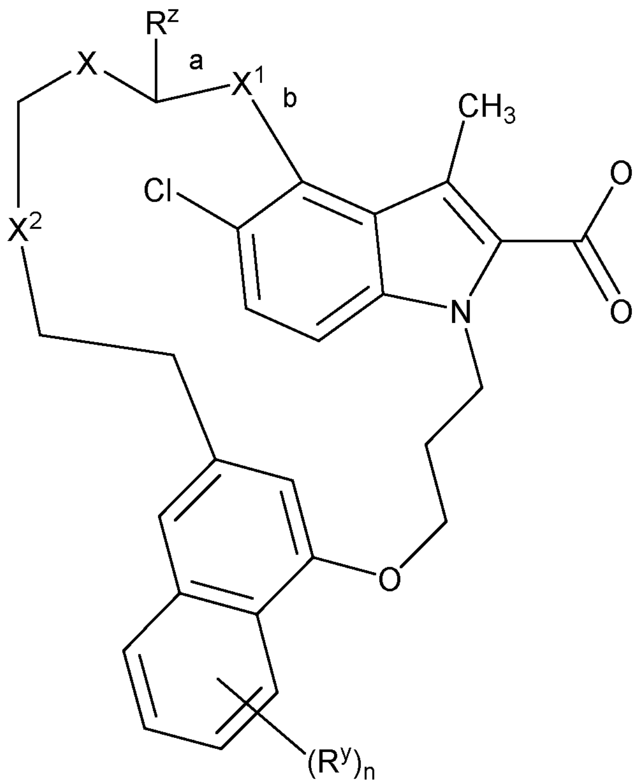

(I)

,

or a tautomer or a stereoisomeric form thereof, wherein
$X^1$ represents (a-1) or (a-2):

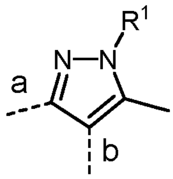 or 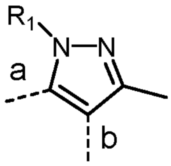

(a-1) (a-2)

,

wherein 'a' and 'b' indicate how variable $X^1$ is attached to the remainder of the molecule;
$R^1$ represents hydrogen; methyl; or $C_{2-6}$alkyl optionally substituted with one substituent selected from the group consisting of $Het^1$, $-OR^3$, and $-NR^{4a}R^{4b}$;
X represents -O- or $-CH_2-$;
in case X represents $-CH_2-$:
$R^z$ represents $-O-C_{1-4}$alkyl optionally substituted with one substituent selected from the group consisting of $-OR^5$, $-NR^{6a}R^{6b}$, $Ar^1$, and $Het^2$; or
$R^z$ is taken together with $R^1$ of (a-2), so that $R^z$ together with the atoms to which it is attached and (a-2) forms a bicyclic ring of formula (b-1) or (b-2):

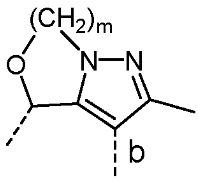 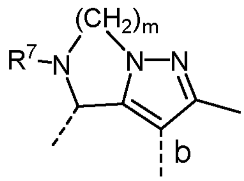

(b-1) (b-2)

;

in case X represents -O- :

$R^z$ represents $C_{2-4}$alkenyl or $C_{1-4}$alkyl, wherein said $C_{2-4}$alkenyl or $C_{1-4}$alkyl is optionally substituted with one substituent selected from the group consisting of $-OR^5$, $-NR^{6a}R^{6b}$, $Ar^1$, and $Het^2$;
$Het^1$ represents morpholinyl, N-methylpiperazinyl, or tetrahydropyranyl;
$R^3$ represents hydrogen, $C_{1-4}$alkyl, or $-C_{2-4}alkyl-O-C_{1-4}$alkyl;
$R^{4a}$ and $R^{4b}$ are each independently selected from the group consisting of hydrogen and $C_{1-4}$alkyl;
$X^2$ represents

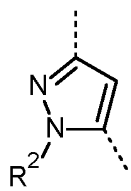

which can be attached to the remainder of the molecule in both directions;
$R^2$ represents hydrogen; methyl; or $C_{2-6}$alkyl optionally substituted with one substituent selected from the group consisting of $Het^1$, $-OR^3$, and $-NR^{4a}R^{4b}$;
$Het^2$ represents morpholinyl or N-methylpiperazinyl;
$Ar^1$ represents phenyl optionally substituted with one $-O-C_{1-4}$alkyl;
$R^y$ represents hydrogen or halo;
$R^5$ represents hydrogen, $C_{1-4}$alkyl, or $-C_{2-4}alkyl-O-C_{1-4}$alkyl;
$R^{6a}$ and $R^{6b}$ are each independently selected from the group consisting of hydrogen and $C_{1-4}$alkyl;
$R^7$ represents hydrogen, $C_{1-4}$alkyl, or $-C_{2-4}alkyl-O-C_{1-4}$alkyl;

n is 1 or 2;
m is 2;
or a pharmaceutically acceptable salt, or a solvate thereof.

**2.** The compound according to claim 1, wherein

$R^1$ represents hydrogen; methyl; or $C_{2-6}$alkyl optionally substituted with one $-OR^3$;
X represents -O- or $-CH_2-$;
in case X represents $-CH_2-$:

$R^z$ represents $-O-C_{1-4}$alkyl optionally substituted with one $Ar^1$ substituent; or
$R^z$ is taken together with $R^1$ of (a-2), so that $R^z$ together with the atoms to which it is attached and (a-2) forms a bicyclic ring of formula (b-1):

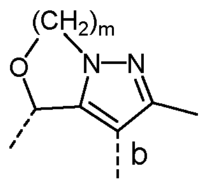

(b-1)

;

in case X represents -O- :

$R^z$ represents $C_{2-4}$alkenyl or $C_{1-4}$alkyl, wherein said $C_{1-4}$alkyl is optionally substituted with one substituent selected from the group consisting of $-OR^5$, $-NR^{6a}R^{6b}$, and $Het^2$;
$R^3$ represents $C_{1-4}$alkyl, or $-C_{2-4}$alkyl-$O-C_{1-4}$alkyl;
$R^2$ represents $C_{2-6}$alkyl optionally substituted with one $-OR^3$;
$Het^2$ represents morpholinyl or N-methylpiperazinyl;
$R^y$ represents halo;
n is 1.

**3.** The compound according to claim 1 or 2, wherein X represents -O-.

**4.** The compound according to claim 1 or 2, wherein X represents $-CH_2-$.

**5.** The compound according to claim 4, wherein
$R^z$ is taken together with $R^1$ of (a-2), so that $R^z$ together with the atoms to which it is attached and (a-2) forms a bicyclic ring of formula (b-1) or (b-2):

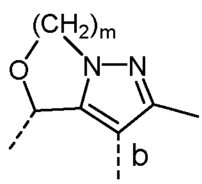

(b-1)

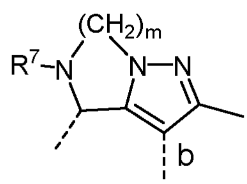

(b-2)

.

**6.** The compound according to claim 4, wherein
$R^z$ represents $-O-C_{1-4}$alkyl optionally substituted with one substituent selected from the group consisting of $-OR^5$, $-NR^{6a}R^{6b}$, $Ar^1$, and $Het^2$.

7. The compound according to any one of the preceding claims, wherein $R^y$ represents fluoro.

8. A pharmaceutical composition comprising a compound as claimed in any one of claims 1 to 7 and a pharmaceutically acceptable carrier or diluent.

9. A process for preparing a pharmaceutical composition as defined in claim 8 comprising mixing a pharmaceutically acceptable carrier with a therapeutically effective amount of a compound according to any one of claims 1 to 7.

10. A compound as claimed in any one of claims 1 to 7 or a pharmaceutical composition as claimed in claim 8 for use as a medicament.

11. A compound as claimed in any one of claims 1 to 7 or a pharmaceutical composition as claimed in claim 8 for use in the prevention or treatment of cancer.

12. The compound or a pharmaceutical composition for use according to claim 11, wherein cancer is selected from prostate, lung, pancreatic, breast, ovarian, cervical, melanoma, B-cell chronic lymphocytic leukemia (CLL), acute myeloid leukemia (AML), and acute lymphoblastic leukemia (ALL).

## Patentansprüche

1. Verbindung der Formel (I)

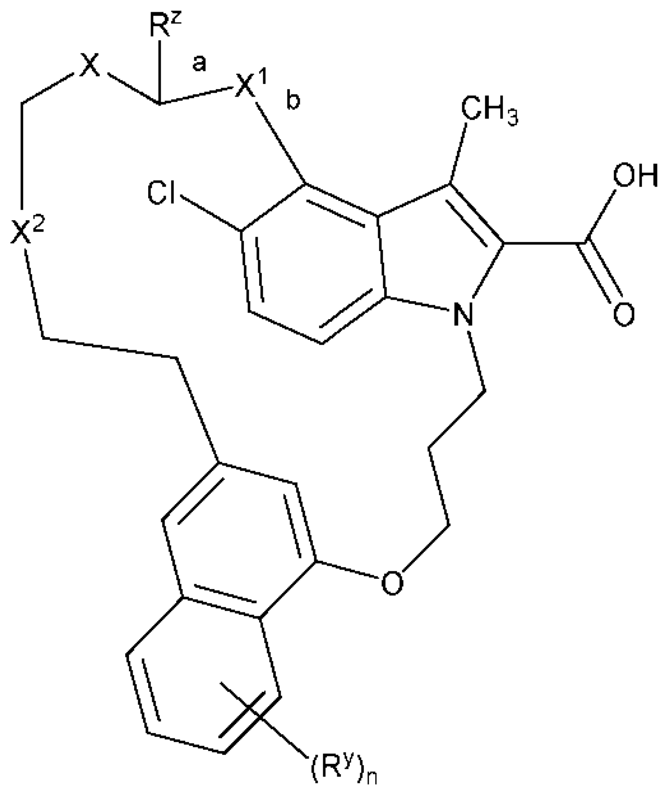

oder ein Tautomer oder eine stereoisomere Form davon, wobei $X^1$ für (a-1) oder (a-2) steht:

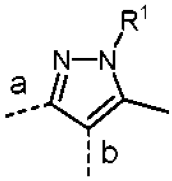

or

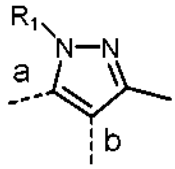

(a-1) (a-2),

wobei "a" und "b" angeben, wie variabel $X^1$ an den Rest des Moleküls angelagert ist;
$R^1$ für Wasserstoff; Methyl; oder $C_{2-6}$-Alkyl steht, optional mit einem Substituenten substituiert, der aus der Gruppe ausgewählt ist, bestehend aus $Het^1$, $-OR^3$ und $-NR^{4a}R^{4b}$;
X für -O- oder $-CH_2$- steht;
falls X für $-CH_2$- steht:

$R^z$ für $-O-C_{1-4}$-Alkyl, optional mit einem Substituenten substituiert, der aus der Gruppe ausgewählt ist, bestehend aus $-OR^5$, $-NR^{6a}R^{6b}$, $Ar^1$ und $Het^2$; oder
$R^z$ zusammen mit $R^1$ aus (a-2) genommen wird, sodass $R^z$ zusammen mit den Atomen, an die es angelagert ist und (a-2) einen bicyclischen Ring der Formel (b-1) oder (b-2) ausbildet:

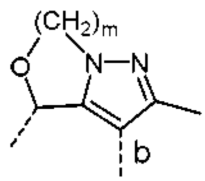

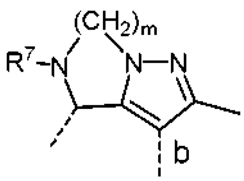

(b-1) (b-2)

falls X für -O- steht:

$R^z$ für $C_{2-4}$-Alkenyl oder $C_{1-4}$-Alkyl steht, wobei das $C_{2-4}$-Alkenyl oder $C_{1-4}$-Alkyl optional mit einem Substituenten substituiert ist, der aus der Gruppe ausgewählt ist, bestehend aus $-OR^6$, $-NR^{6a}R^{6b}$, $Ar^1$ und $Het^2$;
$Het^1$ für Morpholinyl, N-Methylpiperazinyl oder Tetrahydropyranyl steht;
$R^3$ für Wasserstoff, $C_{1-4}$-Alkyl oder $-C_{2-4}$-Alkyl-O-$C_{1-4}$-Alkyl steht;
$R^{4a}$ und $R^{4b}$ jeweils unabhängig aus der Gruppe ausgewählt sind, bestehend aus Wasserstoff und $C_{1-4}$-Alkyl;
$X^2$ für

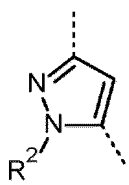

steht, das an den Rest des Moleküls in beiden Richtungen angelagert werden kann;
$R^2$ für Wasserstoff; Methyl; oder $C_{2-6}$-Alkyl steht, optional mit einem Substituenten substituiert, der aus der Gruppe ausgewählt ist, bestehend aus $Het^1$, $-OR^3$ und $-NR^{4a}R^{4b}$;
$Het^2$ für Morpholinyl oder N-Methylpiperazinyl steht;
$Ar^1$ für Phenyl steht, optional substituiert mit einem $-OC_{1-4}$-Alkyl;
$R^y$ für Wasserstoff oder Halogen steht;
$R^5$ für Wasserstoff, $C_{1-4}$-Alkyl oder $-C_{2-4}$-Alkyl-O-$C_{1-4}$-Alkyl steht;
$R^{6a}$ und $R^{6b}$ jeweils unabhängig aus der Gruppe ausgewählt sind, bestehend aus Wasserstoff und $C_{1-4}$-Alkyl;
$R^7$ für Wasserstoff, $C_{1-4}$-Alkyl oder $-C_{2-4}$-Alkyl-O-$C_{1-4}$-Alkyl steht;

n 1 oder 2 ist;
m 2 ist;
oder ein pharmazeutisch unbedenkliches Salz oder ein Solvat davon.

**2.** Verbindung nach Anspruch 1, wobei

$R^1$ für Wasserstoff; Methyl; oder $C_{2-6}$-Alkyl steht, optional substituiert mit einem $-OR^3$;
X für -O- oder $-CH_2-$ steht;
falls X für $-CH_2-$ steht:

$R^z$ für $C_{1-4}$-Alkyl steht, optional substituiert mit einem $Ar^1$-Substituenten; oder
$R^z$ zusammen mit $R^1$ aus (a-2) genommen wird, sodass $R^z$ zusammen mit den Atomen, an die es angelagert ist und (a-2) einen bicyclischen Ring der Formel (b-1) ausbildet:

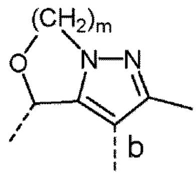

(b-1)

falls X für -O- steht:

$R^z$ für $C_{2-4}$-Alkenyl oder $C_{1-4}$-Alkyl steht, wobei das $C_{1-4}$-Alkyl optional mit einem Substituenten substituiert ist, ausgewählt aus der Gruppe bestehend aus $-OR^6$, $-NR^{6a}R^{6b}$ und $Het^2$;
$R^3$ für Wasserstoff, $C_{1-4}$-Alkyl oder $-C_{2-4}$-Alkyl-O-$C_{1-4}$-Alkyl steht;
$R^2$ für $C_{2-6}$-Alkyl steht, optional substituiert mit einem $-OR^3$;
$Het^2$ für Morpholinyl oder N-Methylpiperazinyl steht;
$R^y$ für Halogen steht;
n 1 ist.

**3.** Verbindung nach Anspruch 1 oder 2, wobei X für -O- steht.

**4.** Verbindung nach Anspruch 1 oder 2, wobei X für $-CH_2-$ steht.

**5.** Verbindung nach Anspruch 4, wobei
$R^z$ zusammen mit $R^1$ aus (a-2) genommen wird, sodass $R^z$ zusammen mit den Atomen, an die es angelagert ist und (a-2) einen bicyclischen Ring der Formel (b-1) oder (b-2) ausbildet:

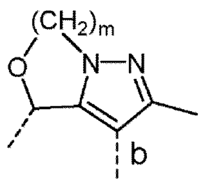

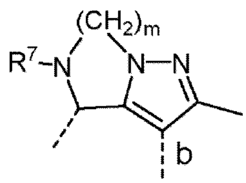

(b-1) (b-2)

**6.** Verbindung nach Anspruch 4, wobei
$R^z$ für $-OC_{1-4}$-Alkyl steht, optional mit einem Substituenten substituiert, ausgewählt aus der Gruppe bestehend aus

$-OR^5$, $-NR^{6a}R^{6b}$, $Ar^1$ und $Het^2$.

**7.** Verbindung nach einem der vorstehenden Ansprüche, wobei $R^y$ für Fluor steht.

**8.** Pharmazeutische Zusammensetzung, umfassend eine Verbindung nach einem der Ansprüche 1 bis 7 und eine pharmazeutisch verträgliche Trägersubstanz oder Verdünnungsmittel.

**9.** Verfahren zum Herstellen einer pharmazeutischen Zusammensetzung wie in Anspruch 8 definiert, umfassend ein Mischen eines pharmazeutisch verträglichen Trägers mit einer therapeutisch wirksamen Menge einer Verbindung nach einem der Ansprüche 1 bis 7.

**10.** Verbindung nach einem der Ansprüche 1 bis 7 oder eine pharmazeutische Zusammensetzung nach Anspruch 8 zur Verwendung als ein Medikament.

**11.** Verbindung nach einem der Ansprüche 1 bis 7 oder eine pharmazeutische Zusammensetzung nach Anspruch 8 zur Verwendung bei der Vorbeugung oder Behandlung von Krebs.

**12.** Verbindung oder pharmazeutische Zusammensetzung zur Verwendung nach Anspruch 11, wobei der Krebs aus Prostata, Lunge, Bauchspeicheldrüse, Brust, Eierstock, Gebärmutterhals, Melanom, B-Zell-chronischer lymphatische Leukämie (CLL), akuter myeloischer Leukämie (AML) und akuter lymphoblastischer Leukämie (ALL) ausgewählt ist.

## Revendications

**1.** Composé de Formule (I)

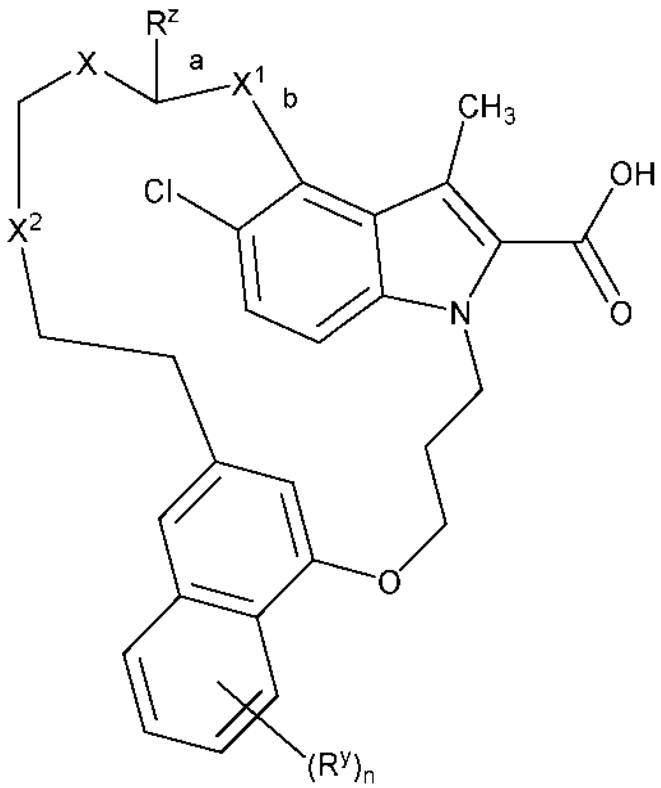

ou tautomère ou forme stéréoisomère de celui-ci, dans lequel
$X^1$ représente (a-1) ou (a-2) :

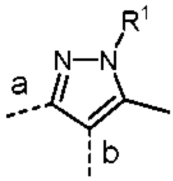

or

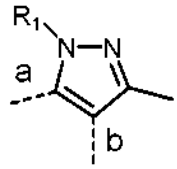

(a-1) (a-2) ,

dans lequel 'a' et 'b' indiquent comment la variable $X^1$ est liée au reste de la molécule ;

$R^1$ représente l'hydrogène ; le méthyle ; ou le $C_{2-6}$alkyle éventuellement substitué par un substituant choisi dans le groupe constitué de $Het^1$, $-OR^3$, et - $NR^{4a}R^{4b}$ ;

X représente -O- ou $-CH_2-$ ;

dans le cas où X représente $-CH_2-$ :

$R^z$ représente -O-$C_{1-4}$alkyle éventuellement substitué par un substituant choisi dans le groupe constitué par $-OR^6$, $-NR^{6a}R^{6b}$, $Ar^1$, et $Het^2$ ; ou

$R^z$ est associé à $R^1$ de (a-2), de sorte que $R^z$ et les atomes auxquels il est fixé et (a-2) forment un anneau bicyclique de formule (b-1) ou (b-2) :

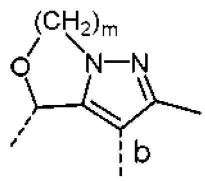

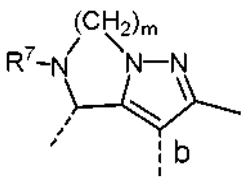

(b-1) (b-2)

dans le cas où X représente -O- :

$R^z$ représente un $C_{2-4}$alcényle ou un $C_{1-4}$alkyle, dans lequel le $C_{2-4}$alcényle ou le $C_{1-4}$alkyle est éventuellement substitué par un substituant choisi dans le groupe constitué par $-OR^5$, $-NR^{6a}R^{6b}$, $Ar^1$, et $Het^2$ ;

$Het^1$ représente le morpholinyle, le N-méthylpipérazinyle ou le tétrahydropyranyle ;

$R^3$ représente hydrogène, $C_{1-4}$alkyle, ou -$C_{2-4}$alkyl-O-$C_{1-4}$alkyle ;

$R^{4a}$ et $R^{4b}$ sont chacun choisis indépendamment dans le groupe constitué par l'hydrogène et le $C_{1-4}$alkyle ;

$X^2$ représente

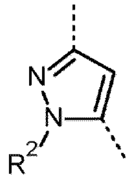

qui peut être fixé au reste de la molécule dans les deux sens ;

$R^2$ représente l'hydrogène ; le méthyle ; ou le $C_{2-6}$alkyle éventuellement substitué par un substituant choisi dans le groupe constitué par $Het^1$, $-OR^3$, et - $NR^{4a}R^{4b}$ ;

$Het^2$ représente le morpholinyle ou le N-méthylpipérazinyle ;

$Ar^1$ représente un phényle éventuellement substitué par un -O-$C_{1-4}$alkyle ;

$R^y$ représente l'hydrogène ou l'halo ;

$R^5$ représente hydrogène, le $C_{1-4}$alkyle, ou -$C_{2-4}$alkyl-O-$C_{1-4}$alkyle ;

$R^{6a}$ et $R^{6b}$ sont chacun choisis indépendamment dans le groupe constitué d'hydrogène et le $C_{1-4}$alkyle ;

$R^7$ représente l'hydrogène, le $C_{1-4}$alkyle, ou -$C_{2-4}$alkyl-O-$C_{1-4}$alkyle ;

n représente 1 ou 2 ;

m vaut 2 ;
ou un sel pharmaceutiquement acceptable, ou un solvate de celui-ci.

2. Composé selon la revendication 1, dans lequel

$R^1$ représente l'hydrogène ; le méthyle ; ou le $C_{2-6}$alkyle éventuellement substitué par un $-OR^3$ ;
X représente -O- ou $-CH_2-$ ;
dans le cas où X représente -CH2- :

$R^z$ représente l'-O-$C_{1-4}$alkyle éventuellement substitué par un substituant $Ar^1$ ; ou
$R^z$ est associé à $R^1$ de (a-2), de sorte que $R^z$ et les atomes auxquels il est fixé et (a-2) forment un anneau bicyclique de formule (b-1) :

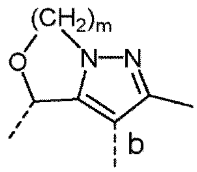

(b-1)

dans le cas où X représente -O- :

$R^z$ représente le $C_{2-4}$alcényle ou un le $C_{1-4}$alkyle, dans lequel ledit $C_{1-4}$alkyle est éventuellement substitué par un substituant choisi dans le groupe constitué par $-OR^6$, $-NR^{6a}R^{6b}$, et $Het^2$ ;
$R^3$ représente l'hydrogène, le $C_{1-4}$alkyle, ou $-C_{2-4}$alkyl-O-$C_{1-4}$alkyle ;
$R^2$ représente le $C_{2-6}$alkyle éventuellement substitué par un $-OR^3$ ;
$Het^2$ représente le morpholinyle ou le N-méthylpipérazinyle ;
$R^y$ représente l'halo ;
n vaut 1.

3. Composé selon la revendication 1 ou 2, dans lequel X représente -O-.

4. Composé selon la revendication 1 ou 2, dans lequel X représente $-CH_2-$.

5. Composé selon la revendication 4, dans lequel
$R^z$ est associé à $R^1$ de (a-2), de sorte que $R^z$ et les atomes auxquels il est fixé et (a-2) forment un anneau bicyclique de formule (b-1) ou (b-2) :

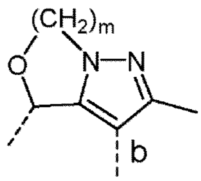
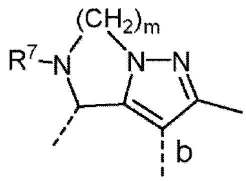

(b-1) (b-2)

6. Composé selon la revendication 4, dans lequel
$R^z$ représente l'-O-$C_{1-4}$alkyle éventuellement substitué par un substituant choisi dans le groupe constitué par $-OR^5$, $-NR^{6a}R^{6b}$, $Ar^1$, et $Het^2$.

**7.** Composé selon l'une quelconque des revendications précédentes, dans lequel $R^y$ représente un composé fluoro.

**8.** Composition pharmaceutique comprenant un composé selon l'une quelconque des revendications 1 à 7 et un support ou diluant pharmaceutiquement acceptable.

**9.** Procédé permettant de préparer une composition pharmaceutique telle que définie dans la revendication 8 comprenant le mélange d'un support pharmaceutiquement acceptable avec une quantité thérapeutiquement efficace d'un composé selon l'une quelconque des revendications 1 à 7.

**10.** Composé selon l'une quelconque des revendications 1 à 7 ou composition pharmaceutique selon la revendication 8 destiné à être utilisé comme médicament.

**11.** Composé selon l'une quelconque des revendications 1 à 7 ou composition pharmaceutique selon la revendication 8 destiné à être utilisé dans la prévention ou le traitement d'un cancer.

**12.** Composé ou composition pharmaceutique destiné à être utilisé selon la revendication 11, dans lequel le cancer est choisi parmi les cancers de la prostate, du poumon, du pancréas, du sein, de l'ovaire, du col de l'utérus, le mélanome, la leucémie lymphoïde chronique à lymphocytes B (LLC), la leucémie aiguë myéloïde (LMA), et la leucémie lymphoblastique aiguë (LLA).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018178226 A
- WO 2017182625 A
- WO 2018178227 A
- WO 2020063792 A
- WO 2020221272 A
- WO 2020103864 A


### Non-patent literature cited in the description


- **CHENG et al.** *eLife*, 2016, vol. 5, e17755
- **JULIAN et al.** *Cell Death and Differentiation*, 2017, vol. 24, 1380-1389
- **HANAHAN** ; **WEINBERG**. *Cell*, 2011, 1-44
- **BEROUKHIM et al.** *Nature*, 2010, vol. 463 (7283), 899-905
- **YECIES et al.** *Blood*, 2010, vol. 115 (16), 3304-3313
- **ROBERTS et al.** *NEJM*, 2016, vol. 374, 311-322
- **KOTSCHY et al.** *Nature*, 2016, vol. 538, 477-486
- **MERINO et al.** *Sci. Transl. Med;*, 2017, vol. 9
- **CHEN et al.** *JCI*, 2018, vol. 128 (1), 500-516
- **WANG et al.** *Genes and Dev*, 2013, vol. 27, 1351-1364
- **STEIMER et al.** *Blood*, 2009 (113), 2805-2815
- **ELIEL, E.L.** ; **WILEN, S. H.** Stereochemistry of Organic Compounds. John Wiley and Sons, Inc., 1994
- **CHARRON, CARLIE L. et al.** *Tetrahedron Lett.*, 2016, vol. 57 (37), 4119-4127
- **AUSTIN R. et al.** *Cancer Letters*, 2016
- *CHEMICAL ABSTRACTS*, 2109428-60-4
- **T. W. GREENE** ; **P. G. M. WUTS**. Protective Groups in Organic Synthesis. Wiley, 2007
- Pharmaceutical preparations and their Manufacture. **GENNARO et al.** Remington's Pharmaceutical Sciences. Mack Publishing Company, 1990
- *CHEMICAL ABSTRACTS*, 2143010-90-4
- *CHEMICAL ABSTRACTS*, 87413-09-0
- *CHEMICAL ABSTRACTS*, 1826-67-1
- *CHEMICAL ABSTRACTS*, 2245938-86-5
- *CHEMICAL ABSTRACTS*, 2092726-85-5
- *CHEMICAL ABSTRACTS*, 157141-27-0
- *CHEMICAL ABSTRACTS*, 6076-14-8
- *CHEMICAL ABSTRACTS*, 61676-62-8
- *CHEMICAL ABSTRACTS*, 887919-35-9
- *CHEMICAL ABSTRACTS*, 67460-86-0
- *CHEMICAL ABSTRACTS*, 17739-45-6
- *CHEMICAL ABSTRACTS*, 25015-63-8
- *CHEMICAL ABSTRACTS*, 14592-56-4
- *CHEMICAL ABSTRACTS*, 657408-07-6
- *CHEMICAL ABSTRACTS*, 95408-45-0